# EUROPEAN PATENT APPLICATION

(11) **EP 1 437 409 A1**
(43) Date of publication of application: **14.07.2004**
(21) Application number: 02799470.6
(22) Date of filing: 13.09.2002
(51) Int. Cl.: C12N 15/29, C12Q 1/68

(54) **METHOD OF IMPARTING OR CONTROLLING FERTILITY WITH THE USE OF FERTILITY RESTORING GENE FOR RICE BT-MALE STERILITY CYTOPLASM AND METHOD OF JUDGING THE EXISTENCE OF FERTILITY RESTORING GENE**

(30) Priority: 19.09.2001 JP 2001285247; 04.10.2001 JP 2001309135; 26.06.2002 JP 2002185709
(71) Applicant: Japan Tobacco Inc., Tokyo 105-8422 (JP); Syngenta Limited, Guildford Surrey GU2 7YH (GB)
(72) Inventor: KOMORI, Toshiyuki, Japan Tobacco Inc., Iwata-gun, Shizuoka 438-0802 (JP); OTA, Shozo, Japan Tobacco Inc., Yokohama-shi, Kanagawa 227-8512 (JP); MURAI, Nobuhiko, Japan Tobacco Inc., Tokyo 105-8422 (JP); HIEI, Yuko, Japan Tobacco Inc., Iwata-gun, Shizuoka 438-0802 (JP)
(74) Representative: Behnisch, Werner, Dr.
(86) International application number: PCT/JP2002/009429
(87) International publication number: WO 2003/027290

(57) **Abstract**

The purpose of the present invention is to provide a method for providing and inhibiting the rice fertility to the rice BT type cytoplasmic male sterility, and discerning the presence of the rice restorer gene. The present invention employs a nucleic acid having the base sequence of SEQ ID NO.27, or a nucleic acid having a base sequence which is identical to at least 70% of the base sequence of SEQ ID NO.27, and which functions to restore fertility. Alternatively, a nucleic acid having the base sequence of bases 38538-54123 of SEQ ID NO.27, or having a base sequence which is identical to at least 70% of the base sequence of bases 38538-54123 of SEQ ID NO.27, and which functions to restore fertility, is used.

SEQUENCE LISTING
<110>  JAPAN TOBACCO INC.
       Syngenta Limited
<120>  A method for providing and inhibiting the rice fertility, and
discerning the presence of the rice restorer gene by using the rice
restorer gene to the rice BT type cytoplasmic male sterility.
<130>  YCT739

<150>  JP 2001-285247
<151>  2001-09-19
<150>  JP 2001-309135
<151>  2001-10-04
<150>  JP 2002-185709
<151>  2002-06-26

<160>  68

<210>  1
<211>  24
<212>  DNA
<213>  artificial sequence
<220>
<223>  Oligonucleotide primer for amplification of R1877 EcoRI marker
sequence.
<400>  1
cattcctgct tccatggaaa cgtc  24

<210>  2
<211>  33

<212>  DNA
<213>  artificial sequence
<220>
<223>  Oligonucleotide primer for amplification of R1877 EcoRI marker
sequence.
<400>  2
ctctttctgt atacttgagc tttgacatct gac  33

<210>  3
<211>  20
<212>  DNA
<213>  artificial sequence
<220>
<223>  Oligonucleotide primer for amplification of G4003 HindIII marker
sequence.
<400>  3
gatcgacgag tacctgaacg  20

<210>  4
<211>  24
<212>  DNA
<213>  artificial sequence
<220>
<223>  Oligonucleotide primer for amplification of G4003 HindIII marker
sequence.
<400>  4
aatagttgga ttgtcctcaa aggg  24

<210>  5

<211> 27
<212> DNA
<213> artificial sequence
<220>
<223> Oligonucleotide primer for amplification of C1361 MwoI marker
sequence.
<400> 5
aaagcaaccg acttcagtgg catcacc 27

<210> 6
<211> 24
<212> DNA
<213> artificial sequence
<220>
<223> Oligonucleotide primer for amplification of C1361 MwoI marker
sequence.
<400> 6
ctggacttca tttccctgca gagc 24

<210> 7
<211> 27
<212> DNA
<213> artificial sequence
<220>
<223> Oligonucleotide primer for amplification of G2155 MwoI marker
sequence.
<400> 7
gaccaccaat taactgatta agctggc 27

<210> 8

<211> 27

<212> DNA

<213> artificial sequence

<220>

<223> Oligonucleotide primer for amplification of G2155 MwoI marker
sequence.

<400> 8

tttctggctc caataatcag ctgtagc 27

<210> 9

<211> 27

<212> DNA

<213> artificial sequence

<220>

<223> Oligonucleotide primer for amplification of G291 MspI marker
sequence.<400> 9

ctgctgcagc aagctgcacc gaaccgg 27

<210> 10

<211> 24

<212> DNA

<213> artificial sequence

<220>

<223> Oligonucleotide primer for amplification of G291 MspI marker
sequence.<400> 10

acattttttc ttccgaaact tccg 24

<210> 11

<211>  24
<212>  DNA
<213>  artificial sequence
<220>
<223>  Oligonucleotide primer for amplification of R2303 BslI marker
sequence.
<400>  11
atggaaagat acactagaat gagc  24

<210>  12
<211>  24
<212>  DNA
<213>  artificial sequence
<220>
<223>  Oligonucleotide primer for amplification of R2303 BslI marker
sequence.
<400>  12
atcttatata gtggcaggaa agcc  24

<210>  13
<211>  24
<212>  DNA
<213>  artificial sequence
<220>
<223>  Oligonucleotide primer for amplification of S10019 BstUI marker
sequence.
<400>  13
aacaatctta tcctgcacag actg  24

<210> 14
<211> 24
<212> DNA
<213> artificial sequence
<220>
<223> Oligonucleotide primer for amplification of S10019 BstUI marker
sequence.
<400> 14
gtcacataga agcagatggg ttcc 24

<210> 15
<211> 24
<212> DNA
<213> artificial sequence
<220>
<223> Oligonucleotide primer for amplification of S10602 KpnI marker
sequence.
<400> 15
agctgttgag agttctatgc cacc 24

<210> 16
<211> 24
<212> DNA
<213> artificial sequence
<220>
<223> Oligonucleotide primer for amplification of S10602 KpnI marker
sequence.
<400> 16
tagccatgca acaagatgtc atac 24

<210> 17
<211> 26
<212> DNA
<213> artificial sequence
<220>
<223> Oligonucleotide primer for amplification of S12564 Tsp509I marker
sequence.
<400> 17
ctagttagac cgaataactg aggttc 26

<210> 18
<211> 27
<212> DNA
<213> artificial sequence
<220>
<223> Oligonucleotide primer for amplification of S12564 Tsp509I marker
sequence.
<400> 18
tttgtgggtt tgtggcattg agaaaat 27

<210> 19
<211> 2240
<212> DNA
<213> Oryza sativa L.
<223> PCR marker G4003 HindIII
<400> 19
   gcggccgctc cgggaagtcg agcgagtaga cgcccctgac gccgtacgcg tcggcgagcc 60
   gcagcggcgt ctctggcggt gtgaaggaca gcccgttcag cgtcgcgcgg cgccgcccgt 120
   tgatcgtcac cggcgccgtg ctccgcagca ggtacgcctg cgtcacgttg atcgacgagt 180
   acctgaacga tccctgtggg ttcggcctcg ccgctccggc actcaggttc cacctgccca 240
   atgcaaaaaa ccaaaaccca aaagcttaat gcgaataata catcattcca cgtatttaaa 300
   aaaataattt ataggtaaaa tttttataat gtattttagc gacgtaaatg tcaatgctga 360
   gaaataaacg ataatacttt aaatgaagtt ctaaaattta aattttggca tcggttgatg 420
   ttggataaag aaaacgatgg aggctagtaa tttttcttct tttttaagta tctagattgt 480
   catatattga atttttcagt ttttcatccc tttgaggaca atccaactat tattttcctt 540
   ttcttatgta aaaggttgaa caacatattc aaacataaaa aaataaaatt aaatgaaata 600
   aatttacaat tcataaaatt tacagaattt atgttaagaa aatattcaaa cttagataat 660
   aataaagcaa caaaatcgta ctaaaaagaa gtataattgt acattgtata ctactactcc 720
   tacaatttta gacttagaat ttttaatttc ctgaaatcta gtaatgccat ttttttcttt 780
   ctagttgaac cagacagtaa gtttaactcg aaacttataa gctaatgagc gaagtcgggc 840
   aattcactcg tacctgacgg agcgagcttg gttcatggag aaggacttgt cgaactggtc 900
   ctggggaggg tcggggagcg ggccggaggc ccgcccccgg gagttggagt agcggaggac 960
   ggcgacgccg gcgacgcggc gccacacggt gtcgttcacc atgcgcgcgc tggcgacgac 1020
   gtagtagtcg gagctcgcgt tctggtcggt ggtgacgagg aaggagtagg actggccgac 1080
   gtggacgtcc aggttggtgt agttctgctg cgtcgtgtag gagccctccg tctccaccag 1140
   caccatgttg tgcccctgga tcctgaagtt gaggctcgtc gacgtcccca cgttgtgcac 1200
   tcggatcctg tacgtcttgc ctgtgtcccc acaccgacgt cgccgacaca cgcgcaaaag 1260
   ataatagact cattgtaagt aggtagtaac cttctccgtt tcatattata aatcgtttga 1320
   ttatattttt gttagttaaa cttctttaag ttttttttct ataaacttaa ttaaatctaa 1380
   agaattttaa taaaaaaaat caaacgactt ataatataaa atggatggag tagttgcatc 1440
   aatttgtgga tgaagcaaac aagattatat ccttttcatg agggtgaaag tattcagtga 1500
   acaattcgtc agtttcaagt ttcatgaaat cggacagggt ctctgaaagt ctgtattttt 1560
   ggtactgttg gattgactac tctggcttct gttgtcacat cttttgtatc ctagtttcgg 1620
   taaaaaaaat tttggcattt ttactcctat cgttgatctg tttaactgaa accattgcat 1680
   gatatactac tagcagacaa aactggtgaa aattcacgag aatgaacttt ttgtcagtta 1740
   agcattagcg gacagcttca gtaagcagag caggctgcct taaggcttaa agcactatct 1800
   tccacaacac tttgtcctac aatcaaattc caaatttact atcacaaaaa gcgaaggaac 1860
   taactaaacc ttactcctac tagtactact gctatgacta tgaaacaaga ttccaatcca 1920
   aagaaaacac agtgctcgat cagcatgata aaagcaacga aacctgctca tccagctgcc 1980
   aaaatgccac cccactgact ctacgtacgt actacgtatt gacgctgtaa aaaactagcc 2040
   gtagtacaga gaagaggacc caaagtttcg tcaaaaattt tattttaccc ggatccacat 2100
   tgatggtctc gtactcgatg ccggccggga caaggctgtc gttgtacctg tacgggccct 2160
   tgccgttaat cagcacgccg tccggcatcc cgaggtcctt gccactgtcc agcatcttcc 2220
   tcagatcctg caacgaattc 2240

<210> 20
<211> 2601
<212> DNA
<213> Oryza sativa L.
<223> PCR marker C1361 MwoI
<400> 20
tcttgctgag atccaagttg cggtaacttt gcccttttct ttttttcttc tcttctgaat 60
tttttcatgg tttttgggag agattttcgt aacttgatta cagttctagg aaaaggccac 120
cttgttcaaa cagggctttc ttgaaaggga tcaatttgct aggagtacat gattctaaaa 180
gcgatttcga aataaaacac agttctcgat ctcatacctg aaaacaaaag gcccatactg 240
tgtaaactgt gattatgctt ctgttaaatg ggatatttgt acaaaattga cgccaaccac 300
ctataaacag attgtgagct tttatcttag taaaataaaa tgtgacattc tactcagtgt 360
tcagtgatcc gatgtcgtct cttctgcgta caacttctaa cagccgtttt cggtagtaca 420
aactagcgaa acaccaaaaa cgcagcattt gagttctgga atacgctgaa attgttagaa 480
tcaaccacga aaccaaaatc attgttcaga aacgttgcaa cgagataaaa cacaagaact 540
tgttttaaca aagcatacgg acagtacata tacggttaca acacccagtc tttatacagt 600
tctgctggag ttccatctac tggctgtcat tgtatctcag gacagacagg ttaacatagg 660
tacaacacaa ttacaggcta aaccgaagcg aactacactg tcagcatctc taacagtatc 720
gtcaagcaag cttatttaca gctgctctag taaatttaca acgtccctgg cagaatccct 780
ctcgtttctg gcagcgacga ggcacggtcc atggccttag caggacatct cacccgtcag 840
ctgcatagaa agcaaccgac ttcagtggaa tcacctcctg ctcctgcaaa aaagttggtt 900

cgatcaatca cgcgtttaat ccaaaacaaa atgggtatta attatgctag cctatgaagc 960
tacctcagag ttctctattt gctctgcagg gaaatgaagt ccagtggaac agttctcaag 1020
cacctcaggg ctcttcatcc atgctttgtg tgcttcaatg gctttcagct tatagcgaaa 1080
catctgcgat acggatctaa aattaaggat gtcgacaatt acttaacaca acaaataatt 1140
gaagcaggtc cagttaaaga aaagtagcag cgaagaatag cactctgaag tctgaacctc 1200
agataaagaa atggttggtt tttccagttc atctccctca acatggattc cagtaccctg 1260
gcattctggg caaaggatgg atgttatttt cttaggtgca ttttttgcct ttcttcctcg 1320
attgcttttt cccttgcttg caattttgtc tgctagcatc tcatattggc ataaaatagt 1380
ccagtgcaca aggcaagaag tgtgaaacaa atgaaatgcc tgcaaaatta gccgtacaaa 1440
gtcattggag gttgcagcag aatactacaa atttttaaag aagaaactat acactgtcta 1500
tgttttgctt gaaatgaatt caaccacttt gcattatacg gtttggaatc cctggtttgt 1560
gagaactgta attccattac aacagtgaag aagttaccat aactaatgaa tggaaattag 1620
tcaaatgcct aattttttag gtttgcttta atttatttat ctgtgagaaa tgctaagcat 1680
gtcatgcgtt gctatcttca agaaatacta agaaactgca aaggcaaaga atgtttgaaa 1740
taacttaccc cgcttgagtt tctactgctg caggctagat ttcctgtctt gcagttgagc 1800
aaggtagcta catccttttc aagaagcatt ggtcgcccac aaatatcaca agctttctca 1860
gcagcaaggc gcttctgctt acgcaactcc ctcctcatag atttggtgga taagaggcca 1920
acttgaagat tgtgtgaagt acctgtcggg gaacctgtta tgatagcttg gctattgtca 1980
tgggcggagc tgctttgctc attcgactcc tctgaagatg cttcttgatc tgaaaatgac 2040
ttctttcttc tctttccacg gtgtccagca tcatcaatca cgaagaaaga tccagcagag 2100
ataggaaggt cctgatcatc agaagaccac ttcctgccca actcaattgt ataagagaag 2160
ttgacaatgg caaagtcaga ttgctcatag gtgtcacact catccaagcc atgggagcca 2220
tcctgtccta cccaagcaca ccagatcttg ctaatctttt tacttccttt gctagcttcc 2280
cataacctgt atgcaatatt tccatatccc aaaagatgca caggcaaatc cgaaacaaca 2340
tcctttagca atacactagg aataacgaga ggaccgtcag ttccactttg gtttgacagc 2400
acatgatctt cagatacaga agcagttcta ccattaccat gcgcatttgc accacggcgt 2460
gtgccttttg cgccattgcg agagctagaa tcatctctca acctcgaagt cacttcagtg 2520
tcgttcgctg gaaccagagc cagctctctg gtgttctgcg agctcgagtc cagcaagagc 2580
gggtccttct cgcgcgagtt g 2601

<210> 21
<211> 1333
<212> DNA
<213> Oryza sativa L.
<223> PCR marker G2155 MwoI
<400> 21
ccctctgctt gatccagtgt acatccatgg gttaggacag attagttact cagttaatta 60
agtgtgagac tggaaaaaaa tatctgacgg cagttttata agttgagtga ttgaactagt 120
gaaagttcag ttaactgtca acggctgtag atttgggatg gcagactgtt ctgagtcaaa 180
atgaagcttt tactgtgcgt ggttaccagg tgcagtaaaa taatttcaga tctaatcgca 240
gtaaaaaaat gtagtactat atgttaagac gagattggtc ggtcaaaatc tatctggccc 300
tttacatctc ccaaatgtta cctcagttgc aggtggtaaa aaaaaatcac tcgtttcacg 360
tgatgtcggc agatcatgga ccatgtctca aatgctgaaa ctctgaacaa tcaacaaaaa 420
aatccaacca gatgagctgt gcaactgata attgatcatc acactatttg caactcatct 480
ttcatgtaga tggaacttca atcccgaaga aataatgaca gcaaaatgct gcgatcctga 540
agaaaggatg gcggcaaaat ggcagcgata aaaaaaaaat ggttggttac tgaagaatta 600
tttgtgcagc agttgagaca gtagcaagat aagagctagc taagctagct aggtagagtt 660
ggatggaaga gtagtagtat gagatagagc atggagcgcg acaactcaag tggatgctaa 720
agtaaaaggc attctcttct cttgtttgga atcagaaaag aaaagaaaag acttgagctg 780
cttggctgga atgtttggtt ggatcatgcg cgctctcctt agcttagctc gccaagaaat 840
cctcgcttca tctctctcaa taattcaaag ccacgagctc tctgctcata tccagtgcga 900
cgattcccgt taatgcaaat gcattatatc cagttcgaaa tgttacaatt cttgcgtttg 960
cagcaagcca gcaagtggtg tgaattgttt aatccctcgt gcatttcaac gaaattctct 1020
cacaaattcg cattgacttc tttcttagca caattagtaa gcagtgacaa ataaagaatt 1080
tttgaacagg atgtctttcc aaggaaggtg agatttttta tgtggatagc aaggatcgcc 1140
tttccttagc atgaagagaa tgtgatcaac tttacacctt gcttacgatt atggccttaa 1200
tttttgatac cctaaacagg agcacatcac atgcatgtcg acctgagacc accaattaac 1260
tgattaagtt ggcatttcag atgcatccgt cagttacatg atcaggtgat cgatggatca 1320

actgtaggtt tca 1333

<210> 22
<211> 863
<212> DNA
<213> Oryza sativa L.
<223> PCR marker G291 MspI
<400> 22
   cgaacaggat caaaagtaga cgacgagggc atttagaagg agaggaattg tatttgttcc 60
   cggtatttaa tttttaaatt tgtggtcgga agtttcggaa gaaaaaatgt gctcatgagt 120
   gattattggc tctgaacacc aacctctctt ttcgttgatt ccttctgagg tgttgggtgt 180
   tgggacacga tgctgccgcc gacacgacac cgggttccac aatacactaa tctactcgcg 240
   acaccttcat tgaactgcat ataattattt agaaagtcca ttaacacatc ttataaaacc 300
   ttgttgaatc atataatcat tctataaagt ctatttgaac atcttatgaa aaaataagat 360
   ctgacctagt cgttacactc tcttacattt tccattagcc taactaattc cgtgcaggaa 420
   acgcccaaaa ataatagtac caatagtcca ctaatcccgt gccagaggcc gccaatgatt 480
   agtgattaac ccaaaaaaca taatcatcat cacacgccgc taatgaccag ctctcgctta 540
   gctcatccca caggcggccc ccacacgcca ctcctgccat gtgggcccac ctttcacacc 600
   ccccaccaac cagaaaaaaa actcccccaa aaaaaaaact tttaatgctt atctcgcggc 660
   agtataaaag gcgaccccac cacccacaca caatcacagt cagcgaccca acccaacccg 720
   agccgaggag tcgagtcgtg tgaaaattac gaaattgccc ttcgactcca ccaccaccac 780
   ccaccggcga ggcgaggaga ggagaaaaat tgggaggaaa aaaaaaggga aaaagaaaaa 840
   gggtggagga gatttttgcg aag 863

<210> 23
<211> 1510
<212> DNA
<213> Oryza sativa L.
<223> PCR marker R2303 BslI

<400> 23
tgccatgaag acctatggaa agaatatctt cttctcactc tgtgaatggt gagtttactc 60
tctgtaacat ttagggctag gtcgaaggaa catgaagcat tgctgattca ctccactgtg 120
tttttttttt ctgtataggg ggaaagaaaa tcctgctaca tgggcaggcc gcatgggtaa 180
cagctggaga acaactggcg acatcgccga caactggggc aggttctact catcctctct 240
ttaaccctgt ttacatagtt cttgagtttt tcagtactga tcgtaattgc cctgttattt 300
cagtatgaca tctcgtgcag acgaaaatga ccaatgggct gcctatgctg gacctggtgg 360
atggaatggt aagaacttga gatgtatctg ttcctaggtt gcttaaccat ttgagagctt 420
caaaatgatc aacatatgtt tctgctgtgc aatatcagat cctgacatgc ttgaagtggg 480
aaatggtggg atgtctgaag ctgagtaccg gtcacacttc agtatctggg cactagcaaa 540
ggtaccatag catgttctat gtactaataa ttttgctgca atgttgaact tctttgcatt 600
tcctcactgc aagttttgct tgaattgttc aggctcctct tttgatcgga tgcgatgtgc 660
gctcaatgag ccagcagacg aagaacatac tcagcaactc ggaggtgatc gctgtcaacc 720
aaggcaagcc ttctcagttt cacatgctta gatttagcca tacctcttgg atatttcacc 780
atactcataa tgtaactctc tgaacagata gtctaggtgt ccaaggaaag aaagtacaat 840
ctgacaacgg attggaggta tcccttcaat ggcttccaaa tttgcagttt ctcattgtcc 900
cataagcctt ggcatgatca tgactaactc tgaagctgac aatactttgt gtaaatttgt 960
cggtaggttt gggccgggcc actcagcaac aacaggaagg ctgtggtgct ctggaacagg 1020
cagtcatacc aggcaaccat cactgcacat tggtcgaaca tcgggctcgc tggatcggtc 1080
gcggtcactg ctcgtgatct atgggcggta aagcctttgc tttcttcaga gctcaaagta 1140
gaacatcttc tcttcagaat tcagagttca taacaaattt ctgtcaattg tgcagcactc 1200
ttcgttcgcg gctcagggac agatatcagc atcggtggcg cctcatgact gcaagatgta 1260
tgtcttgaca ccaaactagt cagcaaagaa aagcagcaca ggttagtacg tgtccggcga 1320
atacagctaa attgatcagg attcaggaag aaggtttgca atttgcaagg attggtagag 1380
ctggaaatgg gatgccattt ggttatgtat gtagaaataa gctgtaagcc tgtaagcgta 1440
tatgtaatca gccgtcaaat gctggcgagt gtatttctga agtttgcaac gaaagttgca 1500
gcaataaaaa 1510

<210> 24

<211> 1016
<212> DNA
<213> Oryza sativa L.
<223> PCR marker BstUI
<400> 24
tggggattct tttctttaag caatttaaca ttattgtcct aacaatatac acaatattgg 60
tttttctttc agtatcaaat aattctttta cttttgaaaa cacatttgca atgtgttgga 120
aacacaatta tatcttgcac ttccttttgg aaatttaatc atttgaaaac tgattcgcgt 180
ttcatggctg taatcttctc ttgcgaacat cgctctttct ttgatggttc tctgttgaga 240
agaagagcaa ccaagtaaat tttcgaaatg tttttttgtt ctttctattc accattgcag 300
gttgtcaaag ccatcgagaa ggccataccg attccgagag cgcaacccat tgccttggat 360
ggcccagcaa gggaagagct gaaggccatg gaggcgcaga aggtcgagat cgaccgcacc 420
gcggcgctcc aggtgcgccg tgagctttgg ctggggctgg catacctcgt cgtccagact 480
gccggcttca tgaggctcac attctgggag ctctcatggg atgtcatgga acccatctgc 540
ttctatgtga cctccatgta cttcatggcc ggctacacct tcttcctccg gaccaagaag 600
gagccctcct tcgagggctt cttcgagagc cggttcgcgg cgaagcagaa gcggttgatg 660
cacgcccggg atttcgatct ccgccggtat gacgagctcc ggcgagcctg tggcctgccg 720
gtggttcgga ctccgacgag cccctgcaga ccgtcgtcgt cgtcgtcgtc gtcttcgacg 780
caggagagcc attgccattc ttactgccat tgccaatgat ctttgtgctg ttctgttctg 840
ttgtcagaat tttttcatgc ccagtttatg ggggttaagc tagcttctcc attgtaccgt 900
tctgatgtgc ggatgatgcg atgcaaagca tagtttgttg aagagatgac aaggcagatt 960
ttagcttgaa aacctggagg tgagaaaaaa aaatcctgat gtgtttgtgt gtgtga 1016

<210> 25
<211> 676
<212> DNA
<213> Oryza sativa L.
<223> PCR marker S10602 KpnI
<400> 25

accaccttca tatgaagaaa ttaacggtgt tttcatgagg aatccaacag tcgctgaatt 60
ggtggaaact gtggaattct tcttggctga ggtaaccaat catcacttca ccacaatgca 120
caagtttgta gcttactact acagtacttc taataagttt tgtctgttga gattttattg 180
ctgatttcta tgcatggtca tctttttgac aggccatcca gtcttatcgt gctgagagtg 240
aaactgagct caacctggca gctggtgact atatagttgt ccggaaggta cggccctatc 300
ttcccattgg acatgtttct aaccataaac atatctttgc tggacttttg tgggcaaagt 360
tggctacact aaacttgtgt tcattaacct gctcaatcag gtgtcaaaca atggatgggc 420
agaaggtgaa tgcagaggga aagctggctg gttcccttac gactacatcg agaaaaggga 480
ccgtgtgctt gcaagtaaag tcgcccaggt cttctaggcg ttcaatgagc catacataca 540
taaccctggt gttgtacact gtattatgat cgttcgtgat cttcaaagac cctctgatca 600
gagaaatcac aaatattctt ttgttctatt attgtcatta tcactacccc ttttgtcaaa 660
accagtgcag cctttt 676

<210> 26
<211> 1059
<212> DNA
<213> Oryza sativa L.
<223> PCR marker Tsp509I
<400> 26
   gcgagatcat gaacttgatt ttctggttgc catattgggc ttgcttgtta accttgtaga 60
   gaaggatagc cttaataggt aagtccctca catgcttcct tccatttgct caattcatat 120
   cagtgttact gttctggcag ttccttgggg tcaggactca gaaacatcca attaatgttc 180
   atgttctctt aacgactcag aaatacttta taacctctcc acaggtacgg gctttcatct 240
   gcccgtgttc ctgttgatct atctcagaat ccacagagtg aagagacaca gagagatgtc 300
   atagcactcc tctgttctgt attcttagca agtcaaggtg ctagtgaagc ttctggaact 360
   atatcaccgg taattcaaaa ttcttcaagt tccttttgta tgtagattat atctttgtaa 420
   aactcggcat ttattacctg ctctttgttt caaaaagcag tattttattt tgctccttag 480
   cataggtcag cagaacagtt gatcttattc agaaaacaat attttgcatg taacatactg 540
   ttatctatga gatgaaaatt aatgcatgtg taataatgtc aatgataaat atttgctatc 600
   tgaatccagt ctaccaactc tagttagacc gaattactga ggttctattt caaagaataa 660
   tttagtgcac catttgttca actactatga agtaaaatgg tattcccttc tattgacatc 720
   gggttagaag tgaaaggcca tcttaatgcg atgttctcaa tgccacaaac ccacaaattt 780
   cattaacaca tacagattat tattaacata gctataaatt ggatttccag aagcttgagt 840
   tgaatttatt ttgttacaat tgaaagcact gggaacatta gcattttttt ttagttcttg 900
   gttattgcaa tttataatgt tatacagaac tgtgtacctc acaatgcatt cattatgaca 960
   ttctatgaac catttgattg actgttgctt gtaaacaaca ggatgatgag gagtctttga 1020
   tgcaaggagc acgggaagct gaaatgatga tcgtagagg 1059

<210> 27
<211> 76363
<212> DNA
<213> Orza sativa IR24
<400> 27
gatcaactaa caacctcttt gcagcaaaaa agcatacaca caagtgtttg tcttggcctg 60
gggctctgca gatggactga tactctgacc tgcagtgggc ttgggagcta acaatggttt 120
cattcttttt ttttttatgt tttcccctgt tgtttttgct catgttttgt gtaatttttt 180
cttctcatct agcgatgtta tttttcttag catgatggga gtagccctcc tttttttttc 240
tctaattaag tgtaaagtag caacagcata gggatgaatg ttcagtgtag tgtgtggtgt 300
ttcagttatt cagagacgtc catacagttt gtaccttgtg accacacgtc ttaatctgat 360
gaagcttaga ataaatcaca tgttagcaat gcaatatcat ctgcgtcttc tctcactttg 420
gtggccatca aattctgtgt agaagtgtat ggttggtgtg ctgttgcaaa tgccgtattc 480
cgctctgttt tgtggaagtt aagaagtccc tagttgaaat accgattttt catgatctcg 540
gagattgatg caactctgat tgcagcattt cttttattta gaatgtacac tccatgctat 600
catgatgttt attgtttagt actacaagat ttggttaacc attattttaa tatcataata 660
attttataaa atcttggagt aacaagttca taatacatga tagcataact ttttgaggct 720
agtctatgta tattgtctcc tttgttttta aactaagcac tcaataaatt attgatggct 780
gtaattttct gaaggtttca ccggtttcgg cccgtgcttt ataaatagct tcggcacaaa 840
agacaaaacg gtccctccaa cacataaatg gttgagttta cgttttcatt atctttggta 900

aaatcaagtc caccacgtag acactcataa caaaagtttg aatatcctca gaaattttga 960
cttgagtcta tcttaccttt gatatcggac atccaaccct ccctccctcc ctgaacttta 1020
tattattcat attacacctg aactttatat tattcatatt acaccctgaa gtggttttca 1080
tttaattgca tacatgctga aatagtttga caacgtgaga tgcactaaaa atctacacgt 1140
tcgtcttaag ttgcaattca ttttatccct tttctttttc tctcttacat aggaatatca 1200
atagtactaa ttcacattac aatatagtat aaattggtaa tcgattattg gcaatatact 1260
atattaaata ttcaaaacta gtcatttaag ctgccaaata agtaaaccac tatcgaaaac 1320
cacaatataa atggcattac aaaacttagg gggttgaata tccaatttta aagttcatga 1380
tgctagagga atttctatca aaagtttatg ggtacatatg gactttttcc tttttaaaag 1440
aagctattct tgtcgtaaac gttaaatatt ttttgtactt tattttttat gattgaaaaa 1500
aaaacttagt tttcaaaatg attggtctgt atacaagcat caattagact taataaattc 1560
atctaacagt ttcctggcag aaactgtaat ttgtttttgt tattagacta cgtttattat 1620
ttcaaatatg tgtacgtata tctgatgtga caaccaaacc caaaaatttt ccctaactcc 1680
atgaggcctt acagatatat ttgatgggtg taaagttttt taagttcttt gggtgcaaag 1740
tttttaaagt atacggacac acatttgaag tattaaatat agacaaataa caaaacatat 1800
tacatattct gcctgtaaac aacgagacaa atttattaag cctaattaat ctgtcattag 1860
caaacgttta ctgcagcatc acattgtcaa atcatagcgt aattaggctc aaaaatattc 1920
gtctcgtaat ttacatgcaa actgtgtaat tggttttttt ttcgtcaaca tttaatactc 1980
catgcatgtc caaatatttg atgcgatctt tttggccaaa ttttgttgga atctaaacaa 2040
ggatcaaatt tgctgaattt ttccagacgt cacggcttgt tcatccatcg ttcgcatcgc 2100
gattcgccac cgacgccttg gtttccaacg aattttatca tccgcttaaa tacatccaaa 2160
gctctccatc gccatcggcg gccaacggcg accgctccgc tctacccaat ccacccatcc 2220
actcgccgcc gccccctgat ccaaagcctc cgccgcgccg ccgtcgagag gaggaggagg 2280
aggaggagga ggaggcgtga gcccctatgg ggaccctcct ccggccgcgt ccgcttgccc 2340
acgccgccgg cgccggcgac gccacgccgt cgaccgcgca cggtagccac gcgcctctcg 2400
agaggccccc cccccccgcc gctcgctgat ctctcttctc atcctgtttg ggtttgggtt 2460
tgtgatttgg gtgttttttt tttttccgca gcggtggtgg tgagcggtgg ccgcggccgt 2520
ggcgtggagt gccagccgca tcgggtgcgc cgccgcccgg gtccgcaggt tgcggtggcg 2580
acggcgagct ggaggaggcg gagggagacc gtggtgagat cggatttcgc cgctggtggt 2640

gccgctacca tgggggattc gccgcaggcg ctctcaggtt tgcagcctcc tccactctct 2700
tctcgcaaaa tgtgttgcta tgttcctctc gctgggctgg cctcatagcc attaatgtag 2760
tttgctggaa cattacattc ggaacgttgt tggcaattgc ttgacaaaat gtggaattgt 2820
ggaggggaga aaaatcgttt gaacctgcag tgacaaaatt gccatctata attttaaaac 2880
tgaaggtgtg gaaatcaaac ataatcattg ccagcacatc attcttgtta accaccttga 2940
catattgttg gcttataaca gttagctcca caccaacttg gaaggtgtca atggaatgta 3000
agtataaatt gaggataact ggcagttgtt aagactttct acagaacttg tagcagctaa 3060
aactagctat tgtgcattta tgtttcatgg aatttgagcg gcaatggata tttcttacta 3120
agacgtataa tgcaaaaaaa aaaaaaaaac tatgtctatg cagtttacat gtaatgtgcg 3180
gatgcaaata aaatcatgtt catggacaaa ctaatgggat tcataccaaa ttccagaatt 3240
gcatttctta tgtggttact tttgtttgtt gatttggtta ccagacatcg atgtggtttc 3300
aagggtcaga ggggtttgct tctacgcggt gactgcagtt gcagcaatct ttttgtttgt 3360
cgccatggtt gtggttcatc cacttgtgct cctatttgac cgataccgga ggagagctca 3420
gcactacatt gcaaagattt gggcaactct gacaatttcc atgttctaca agcttgacgt 3480
cgagggaatg gagaacctgc caccgaatag tagccctgct gtctatgttg cgaaccatca 3540
gagtttcttg gatatctata cccttctaac tctaggaagg tgtttcaagt ttataagcaa 3600
gacaagtata tttatgttcc caattattgg atgggcaatg tatctcttag gagtaattcc 3660
tttgcggcgt atggacagca ggagccagct ggtatggctg tagtctcatc cctgctttct 3720
taagtagaca tatatacatt tacagtattt ggtaaataaa caagatttta tgaatcatat 3780
atgattttgg ggaaaacaca aaactctctt tgttggctgc cttgaacata gttctgttca 3840
cacagttata gcaccttctt taaaatgaag aactttgttg catacacata aggccaaacc 3900
acataatgaa ttttgtttat ttctatcttt gaatgttagc atcgtttttg tttaatgcat 3960
gatcgccttc ctatatattt gtagtatgtc aacattgtat tccatgctga gcataacaaa 4020
tggtttgtta aaattcagga ctgtcttaaa cggtgtgtgg atttggtgaa aaaaggagca 4080
tctgtatttt tctttccaga ggggactaga agcaaagatg gaaagctagg tgcatttaag 4140
gttcagtaac caaacttagg ttacattaca tctaatgaga tttttatatt cagtatataa 4200
tgttaacctt ctcatggtgt actgacgtgg ttataaatgt ccccagagag gtgcattcag 4260
tgtggctaca aagaccggtg ctcctgtgat acctattact cttctcggga cagggaaact 4320
gatgccttct ggaatggaag gcatccttaa ttcaggttca gtaaagctca ttattcacca 4380

tccaattgaa gggaatgatg ctgagaaatt atgttctgaa gcaaggaagg tgatagctga 4440
cactcttatt ctaaacggtt atggagtgca ctaaagaaag atggtgtttt tttttattat 4500
atggaaccta ttcaaaggca cagacaggct ttcaaggcta agcttgttac aggtactgat 4560
actagttact aattactttc gtaatcagta taaataagct tgtgtagtgt aatggcattg 4620
tacatttctg cacttggtaa atttacagaa gaggcaagta atattttaga ggattgagtt 4680
tattcaccca gtcatatagt tgaagaggca agtaacctgt aagagaggac tgaacattaa 4740
cacctcttgt tcgattaaaa atgaccaaag agcatcaaac atgtattcga ggctgttact 4800
ttagatatgg cccattaatt tgtttagttg tctatgtaca tcctagttgg tgtaaatgcc 4860
agttaccatt tctatgatct aaaacaatca actcttttag tatattttca aaaacgaaat 4920
tcagtacaca tgtatgaatc ttaatattct tctctagctc gttacaaaag caacaaaggc 4980
accgtgtcag ctggttcaca ttagctagtt tgtacttagc attatccact agcaccttat 5040
tttcatgcat atcatgctaa tttgcttgcc cacgttgagt gggaattttt ttcatgtttt 5100
ataatttata tatgttttag acttctagtc cacaatttat gtacttcatg ttcctgagcc 5160
tctagtatgg ctgatagcag actaggtgct gagtgctgtc cttttttgca gactgaagag 5220
agaagaaata caagactgtc cattgttagt cagatttgta aaaatagact ctgatgtagt 5280
ttacttttgc ccctatttta tttttaacaa tacaaatata taacagatcc taagaactta 5340
tcttaattta ggagaagttg ctcgtttcat taaattaaat tgtgaagtaa aaatgtgtgc 5400
tcgagtctgt caatgcaatc ctgtgttctt gtttgaagat atggtgtagg gcaggccagg 5460
attgaacact gaatggtaag actgcttctg ccttcagacg ttattgctaa atttttagct 5520
acttgcagtt agtgctgcca cgccgattaa gcagtagaac aaagtagttt tgtcgtgcac 5580
aaatgagtta tatttcattg gaaatcgaag cgaaaacgaa tcaaaagtta gaagaaaagg 5640
ggaaacttgg taattactcc ataaagagag tgcattttat tggtaagatg gtatccggaa 5700
gctgtgagct ccgggctgta tgtattctgg caaatttgat atgagatgct cgattattgg 5760
cttaagttag cgatatcaaa tttggggaag caccaaagga attattgtga aggagttatg 5820
ggtgcgtgac gttatctgct aggttcaaat ccttgtggct atgaatattt atctgctagg 5880
ttcaaatcct agtgactatg aatattaatg ggtaaggtaa gggatttatt gttaatttta 5940
gtttctttaa gattgtgcca tcggacgcca ttcggtaact gtaataatgc tttgtattgg 6000
attcacttgt gttacatgca cgcactaaac atgtgcttta ccttttcatc tgtttttgcg 6060
ttctgggcta gaaactcaaa cgttgaattt tccatggtct gctcaacttg acaattactg 6120

cgtgtcaagc gatcttatac gcatactatg cgcacaagtg attgtatacg gatatgatga 6180
cagtataacg tgtgatattg atttttttaa taaaaaaatg atgttcattt ccttgatgaa 6240
ggaacaaaga ctttttttaa aagaagggta ttactaaaaa caaaaatgac aaaaacaaaa 6300
tatcagtgca catggcaagt gtgctcggca attttttctc tgtactttaa acaaaaatac 6360
ttctatatgt tcttttttat aagggtggca caaatctttt aaatgagcca aatatctaca 6420
ttggatttat taaaaactgt ataaattata atttatactc tgaaaggttg tgtgcatctc 6480
tcttggagaa aatgtataag ttgcaaacaa acattaatcc acgttatgta actttttttc 6540
gccggaaagg ccgaaggagg cctgacggag cgtggggctc ctcaccggga gaccgcgcag 6600
gccccccttt gccggttcgg ccggggactc agggtgaaat tctaagctct ctgtatgtgg 6660
aaggttcgcg accgtcgaaa gagcataaga cacgggcgat gtatacaggt tcgggccgct 6720
gagaagcgta ataccctact cctgtgtttt gggggatctg tgtatgaagg agctacaaag 6780
tatgagccag cctctccctt gttctgggtt ccgaatctgg aaaagtccag tccagtcccc 6840
ccctctaagt gggcaaggtc ctccttttat atcttaaggg gataccacat gcaccatctc 6900
cctcctttct gtggagactt accctacctt ttcataaatg gacggagatt tgtatagttg 6960
ccgtccgaat gaccttctga taggacggcc catacctacc tccacttccg ccgaaagcag 7020
gtgcgacgtg ggattatggc tgtctgctga cgacatgacc agtgtcagac tggtcacaaa 7080
ttgctcattc ctgtccacca cgcgtcagtt tagcaatcta catgttggcc cttcttcaca 7140
caacatcttg cctgtaatgg ttaggatgaa gcctggcata tatctaacca ggactaacgt 7200
gccatctcta ggaggtaaca cgctagctcc agctggggac gagcgcctag aagccctcgt 7260
cctgacggga tggggcgagg cgtgcgtcag atcgcctgtc gccacctaac ctgcgatctg 7320
accggtctgt gactggtcac agaccggata aacgagtgca ctgcacttcg ttacatgcag 7380
cgtgacacgc tcagccaaac cgcaataaat gtggttaggt gagccccgct gtgctcacct 7440
aacccataca cgcggagcaa aaacccacga ggggtcgggg cgcctcggcc ctcggggccg 7500
aggcgggtgc ggtccgaccc cctcgggggg actaagagga gggcgaacac atcaccctcg 7560
ggcccgacgt cccccgaggg tgccaggcca cgtgggcgat tgtgtctgcc tcaaacctct 7620
agtcatgata ctcctgatcc catgtcaccg acagtagccc ccggcgttat gccagggcga 7680
tcgccctctt taagggaagc ggtcgggcgt gacgccactc ctaaggcctg gtgacaggtg 7740
ggaccggtct ccacaattgg gcagaaaccc aacggtcaca aatcacgcac atcggcaatg 7800
gtaactctac tatcaataat gagcggtctc ttcaagactg ccacattact cgagtagcac 7860

acgaatctgg acatggcgat tcgtttcgtc tggagatatg gtaacgtcgc tttggtcggc 7920
gagcgtaatt aacgcgcgca cgatatgatc tatctcgact gccacaaccg catatccacc 7980
tcatgcgccg caagcgggcg aatggattta gtggaagcgt gggcgcgaga aacgaggggg 8040
cgaaatagtg ggcgcgagaa gcgaggagcc gggcacagcg ttggcaagag tataaaggca 8100
ctgaggaaag gatctgtttc cttcctttcg ccatcatttc ccttgtcttc gccgcttgcg 8160
ccctaactcc ttctttcctg tgctctactt tcgccacacg cgctcgctct caatcttctc 8220
ttcctccggc gccatggcac ggggctccgc tctgctcgat ggtagcgtgc tgccgccttc 8280
ccgcatcgtg agcgagaggc aggctgggct gccgcgccgc ttcatgccgg aatctgccac 8340
cggccgggag atagtcacgc tgggcgaggg acgcccggcg ccagactacc cggggcggtc 8400
cgtcttcttt ctccccttttg caatggcagg gctggttccg ccatttcttt ctttcttcat 8460
ggatgttctg aagttctacg atctccagat ggcgcacctc acccccaacg cggtgatgac 8520
attggccatc ttcgcgcatc tgtgcgagat gttcattggg gtgcgcccat ctcttcggct 8580
gttccggtgg ttcttcaccg tgcagtcggt gtcgccgcca tcggtagttg gtggctgcta 8640
cttccagcca cgggggccgg tgctgaatcg ctacatcccc tgcgccctcc gcaagaagtg 8700
ggacgactgg aagagcgact ggttctacac ccccctcgcc gacgaagcgc gcctccgact 8760
tccgagccag cccccggcgc aggcctccag ctggcgggcg ccggtagatc tgggggatgg 8820
ctatgacgcc gtcctcgacc gcctggcggg cctacgatcc caggggctca caggaccatt 8880
ggtgtacggc gactacctcc gtcgtcggat tgcgccgctc cagcggcgcg ctcggggcgc 8940
ctgggagtac accgggtccg aagactacat gaggacccac cagggagtca gatgggactg 9000
ggctcctgag gatttcaaga tagtggtcca acgggtgctg aatctcaact ccatggaggc 9060
gtccctcatt ccccaaggaa tcctccctct ctgcagcgat ccagaccgcg cctccatcct 9120
gaccattatg acggcggtcg gggcctcaga ggagtgagct ccaaagggcc acgacggcgc 9180
aggcgggagc cgtagggggg atcaatctac ccggggaggg ggtcgtgctt ctgggtctcg 9240
cgacggaggc ccgaggagca gccgccctgc cgacgcccgg gggaagagga agcagggagg 9300
aacacctccc ccatctcctc cccgaggggg cggggcggtg cgtgccagca gcaggcgccc 9360
ggagggggcc gcgccgacat cgcagcccga gggggagcgc aagaagaagc ggctccgcaa 9420
gatgggggag acagaaccat ctcaggaaaa ccttatttcc cctctaaagt ggtcgtttaa 9480
ccgaccccct cgcaggttcg tctctcaccc atcgtggctg tattcattct ctcaacgcga 9540
gttttcactc acccatcttg ttcgtcttct ggtcttttct tctgtttcag cgagatcccg 9600

tcgcgtccct cccgccattc caagtccggc cagtctgagg ccgaggatcc ggcggccgca 9660
gaggcccgga ggcgggaatc tgaccggcga gaggccgcgg atcgcctacg ggaagccgag 9720
gaggccgccc aggaggccgc ccgggctcgc caggtcgagg aaaccgctcg ggaggaggcc 9780
gcccgggccc gccaggccga ggaagccgct cgggaggagg ccgcccgagc ccaccaggcc 9840
gaggaagccg ctcgggagaa agccggattt cgccaggacg aggcaatggc gacttccgag 9900
gcagctcgcg atgaggtcgc gggcgcgtcg cttgagccca cttcctcggg cgacgctcag 9960
gcgacaactt ccggggcagc tggcgacgag gctgcgggcg cgtcgcttgg gcccactccc 10020
tcaggcgacg cccaggacca accaggtccg agggacatcc ctgagtccgg cacttccatc 10080
ggcggcccga gccgcgtggc atcctctcca aggcggctct tccccacgcc ttctatcgcc 10140
ccactgagcg cagagcccct tctgcaggcc ttggccgccg caaacaccgc ggtgttggac 10200
gggcttagtg cccaggtgga ggccctgcaa gcagagtggg cggagctcga cgccgcgtgg 10260
gcgcatgtcg aggaggggcg gcgctcagtg gaggccatgg tggaggtggg ccgcaaggca 10320
caccgccggc atgtctcgga gcttgaagcc cgtaagaagg tgttggcgga aatcgccaag 10380
gaagtggagg aggagcgggg ggctgccctc attgccacca gcgtgatgaa cgaggcgcag 10440
gacaccctcc gccttcaata cgggagctgg gaggcggagc tagggaaaaa gctcgacacc 10500
gcccaggggg tgcttgacgc tgccgctgcc cgagaacagc gggcggggga gaccgaagcg 10560
gcgtcccgac ggcgcgaaga gacccttgag gcgcgcgcca tggcgctgga agagcgcgcc 10620
tgcgtcgtgg agagggatct ggcggaccgc gaggccgccg tcactatccg ggaggcaaca 10680
ctggcggcgc acgagtccgc ctgtgccgaa gaggagtccg cactccgcct ccacgaggac 10740
gcgctcaccg agcgggagcg agctctcgag gaggccgagg ccgcggcgca acggctggcg 10800
gacagcctgt ccctccgcga ggcagcgcag gaggagcagg cgcgccgcac tctggaatgt 10860
gtccgcgccg agaggaccgc actgaaccag caggccgctg acctcgaggc gcgggagaag 10920
gagctggacg cgagggcgcg cagcgacggg gcggctgcgg gcgaaaacga cttagccgcc 10980
cgcctcgctg ctgccgaaca taccatcgcc gatctgcagg gcgcgctaaa ctcgtccgcc 11040
ggggaggtcg aggccctccg cttggcaggc gaggtagggc ccggcatgct ttgggacgcc 11100
gtctcccgcc tagatcgcgc cggtcggcag gtgggcctct ggagagggcg gaccgtaaag 11160
tacgccgcca accatggagg cctcgcccag cgcctctcga agatggccag ggctctccaa 11220
cggctccccg aggagctcga gaagacaatt aagtcatcct cgagggacct cgcccaagga 11280
gcggtggagc tcgtactggc gagttaccag gccagggacc ccaatttctc tccatggatg 11340

gcgctggatg agttccctcc tgggaccgag gacagcgcgc gcgcaggtcc gggatgccgc 11400
cgaccatatc gtccacagct tcgagggctc agcccctcgg ctcgcgttcg cccccaactc 11460
cgacgaggag gacaatgccg gtggtgcaga cgacagtgac gatgaggccg gcgacccggg 11520
cgtatcggat tgatccccca agcccccgcc attctttagt tttttcttct tttccttctt 11580
ctaaggcctt cgggcctctt ttttgtatag atcaacttaa tctgtaatca aaaatgaaga 11640
aatttttgtg tcaatttcat cttgctgtgt gtatgagatg aggatgatct gtgacgtggt 11700
ccttttgcgt cttagcttga ttaagggctc gtgcccaggt cccagtcctc aaaaggcgtg 11760
ggtcggggct agtgcctggg gagatccaca tgtcgagact ggccaggccg ggaacgtggt 11820
gaccgagggt tatgggtgac ccgattgtgg gtttttgccg attccccccc ggagttcacc 11880
acgccccggg gcacggctcg gttctgggcc ccgtttggcg attttagccg acccgagccc 11940
ccgagggcag gattgagcac gagtgaccta tttcaagtca agattcttca aaaggaaaaa 12000
aaaacacaga tacagccttt aggaaattga aactgctttt attgaaatac tgaaataaga 12060
gaaataagaa tgtgcatgtg tggcagcccc cggccaacgc tgcacgcccg agggggtgcg 12120
gggttggccc gagcccgaaa cctgacaccc gacccccccc tcaggggtag aagcgacgaa 12180
ggtgttcgat gttccacggg ttaggcagct caatgccgtc gcccgtggcc agccgtatgg 12240
agcccggccg ggggacgccg accactcgat acggaccctc ccacattggt gagagcttgc 12300
tcaatccagc acgcgtttgg acgcggcgta ggacgaggtc gtcgacgcag agtgatcggg 12360
cccggacgtg acgctgatgg tagcgccgca ggctctgctg gtagcgcgcg gctctgaggg 12420
ccgcgcgccg ccttcgctct tccaagtagt cgaggtcatc tctgcgaagt tgatcttgat 12480
cagcctcgca gtacatggtg gcccgaggag acctcagggt gagctcggat gggagaaccg 12540
cttccgcgcc gtagacgagg aagaaaggcg tttccccggt tgctcggctt ggtgtagttc 12600
ggtttgccca gagcaccgct agcaactcct cgatccatga atcgtcgtgc ttcttgagta 12660
tgttgaaggt cttggtttta aggcctttga ggatttctga attggcgcgc tccacttggc 12720
cattgcttct ggggtgggca ggtgaggcga agcagagctt gatgcccatg tcttcgcagt 12780
agtcgccgaa gagttcacta gtgaattggg tgccattatc cgtaataata cggttaggca 12840
ctccaaaccg ggccgtgatg cccttaatga atttaagtgc ggagtgctta tcgatcttga 12900
cgaccggata agcctcgggc cacttagtga acttgtcgat cgcgacatac agatactcaa 12960
acccgcccgg ggcccgccta aacggtccca ggatatcgag cccctagaca gcaaatggcc 13020
acgaaagtgg tatggtctgc agggcctggg ccggctgatg gatttgcttg gcgtggaatt 13080

gacacgctct acatcgccgg accaggtcga ccgcatcatt gagagctgtc ggccaataga 13140
aaccctggcg aaaagcttta ccaaccaagg tgcgcgaggc ggagtgggct ccgcattcgc 13200
cttcatggat atcggcaaga agcacaacgc cttgttcccg aggaatgcac ttcaggagga 13260
ttccattagc cgcgcgccga tagagggtcc cttctaccag cacgtagcgt ttggagatgc 13320
gatggacgcg ttcactccct tcgcggtcct cgggtaaagt cttatctgtg aggtatgctt 13380
ggatctcggc aatccaagca atcaatctaa gggagctggg agcgctcccc tcgggtcccg 13440
aggcctggac ttcgacgggc ctcgggggcc ggtcaggcgc gtccgtctcc cctaaggggt 13500
cgggtcgcgc cgacggctgg gcaagccttt cttcaaaggc gcccggtggg gtctgggctc 13560
gcgtggacgc gagccgtgag agttcgtcgg caatcatgtt atcccgtctg ggcacatgcc 13620
gaagctcaat cccgtcaaaa tggcgctcca tacgccgtac ttggcgcacg taggcgtcca 13680
tctgcgggtc agagcaccgg tactccttac agacttggtt aacgaccagc tgggagtcgc 13740
ctaacaccag gaggcggcgg atccccagtc cagctgccac tctgagtccg gcaaggagtc 13800
cctcgtactc tgccatattg ttagtcgctc gaaagtcgag gcggaccaag tatctgagga 13860
cgtctccgct cggagaggtc aacgtgaccc ccgcaccggc gccctgaaga gacagggagc 13920
cgtcgaactg cattacccag tgggcggtgt gaggcagctg cgaggggtcc gtgctggcct 13980
cggggattga gacgggctcg ggagccgggg tccactctgc cacaaaatcg gcgagagcct 14040
ggctcttgat agcgtgacgt ggttcaaagt gcaaatcgaa ctcagaaagt tcgattgccc 14100
atttcaccac ccgtcctgta ccctctcgat tatgcaagat ttgaccgagg gggtaagacg 14160
taaccacagt gacccgatgc gcctggaaat aatggcgcag tttcctcgag gccatcagaa 14220
tagcgtaaag catcttctgg gcctgagggt atcgggtttt ggcgtcccgg agggcctcac 14280
taacaaagta gacgggccgc tgcacctttc ggtggggccg atcctcttcg ctaggggccg 14340
catccctggg gcactcttcg tccaagcagc ctcgcggggc gcacttgtct tctgtgctga 14400
tgacctcggg gtcggaggat aacaggggcg gccttcccac agtggctttg gggccgtcct 14460
gggggtcagg ggctcctggc gtcgtcggac aagcgggcaa agggccaact ccggtcgtca 14520
ggggccttag gcctccgttc ggctcggggg cctcttctcc ctgctctttc ccgggtcgag 14580
tcagcacagg gttagcctcg gggtcaaagg gcgataggtg cggccttccc acagtggcct 14640
cagggccttc ctgggggtcg ggggctccta gcaccgtctg acaagcgggc agagggccaa 14700
ctccggtcgt cgggggcctc aggccaccgt tcggctcggg ggcctctcct ccctgctctc 14760
tcccgggcca agtcggcaca gggtggggaa gcgcgaaatg agaattatcc tcatcgcgct 14820

ccacaaccaa tgccgcacta actacttgcg gggtcgccgc taagtagagt agcaagggct 14880
cgtctggctc cggggcgacc ataactgggg gagagcttag atacgccttc aactgggtga 14940
gggcattttc agcttccttc gtccaggtaa acggtccgga gcgtttgaga agcttaaata 15000
agggtaacgc cttctctccc agcctcgata tgaaccgact tagggcggcc atgcaaccgg 15060
tgacgtattg cacatcccta agtttgctgg ggggcgcatc cgctctatag cccgtatctt 15120
ctcggggttg gcctcaatgc cccgggcaga gaccaagaac ccgagaagct tgcccgcagg 15180
tacaccgaac acacacttat cggggtttaa ttttatgcgg gcggagcgga gactctcaaa 15240
agtttccgct agatctatga gtaacgtttc ctggttgcgc gtctttacaa ccaagtcatc 15300
gacataagcc tcaatattac gtcctaattg gctaccgaaa gaaattcgag tagtacgttg 15360
aaaagtagga cctgcattct ttaacccgaa gggcattgtc gtataacaat aggttcctat 15420
gggggtaatg aacgcagttt tttcctcatc ctccctagcc atgcgaatct gatggtaacc 15480
agagtatgca tctagaaaac acaaaaggtc gcaccccgca gtggagtcga caatctgatc 15540
tatgcgaggc agggggtaag gatccttagg acatgccttg ttaaggtcgg tgtagtcgat 15600
gcacatccga agcttgccgt tcgccttggg aacgaccacc gggttcgcca gccactcggc 15660
ggggttgacg ctgccatcat atttttcggc gatggtgggc cggaaccttg ggggccaacg 15720
gacattccga agactcgcca caaaggctct acagccgaca ccaccaaccg ggggcacgga 15780
gggctgattc ccgcgtccgt gttgaggtga cactctggac gaggaagcgc cctccgttgc 15840
gtgggcagca cttcggtcat tacgccggcg ctcgatgctg gtgcgggcgt ccggcccccc 15900
acgcagatct ttctgggtcg aaggagtcga cgaaggagtg gcggccgaat ggcgaacagc 15960
ggctgccgct cgtcgtgccc tccgtcttga cgacgcggag ccggtggtag cagcaccaga 16020
ggccttggtg gcggaggacc gcccaccagc atctaggcgc tgccgtgccg tcatgactaa 16080
tttggccacg tcgtccagcc atcgttgggc tggagactcc gggtcaggga cgacaggcgg 16140
gtgacgtaag agcgcgcccg cagcttggag cgcgccctgg ggcgtgctgc cgtcgccgta 16200
gacgaggagg cgacgctccc catctcgccg ttcttctcca tcgcccgcga tcggtgaagt 16260
cgcggatctt tcgaccctct cgagcgcctc cccccgctta ggactttggc atggagggag 16320
cggtggagta cgagctcgac ggcgtgggtt cggctccccg tcgtcgccac tcacactcgg 16380
agagaggtcg tgcgcctttg cttgctcggc catcaggctg aacaggaaaa gcttggcgca 16440
cacggaagag tacgagagct cagaaaaaca cacactgagt cccctacctg gcgcgccaga 16500
tgacggagcg tggggctcct caccgggaga ccgcgcaggc ccccctttgc cggttcggcc 16560

ggggactcaa ggtgaaattc taagctctct gtatgtggaa ggtttgcgac cgtcgaaaga 16620
gcataagaca cgggcgatgt atacaggttc gggccgctga gaagcgtaat accctactcc 16680
tgtgttttgg gggatctgtg tatgaaggag ctacaaagta tgagccagcc tctcccttgt 16740
tctgggttcc gaatctggaa aagtccagtc cagtccagtc cccccctcta agtgggcaag 16800
gtcctccttt tatatcttaa ggggatacca catgcaccat ctccctcctt tctgtggaga 16860
cttaccctat cttttcataa atggacggag atttgtatag ttgccgtccg aatgaccttc 16920
tgataggacg gcccatacct acctccactt ccgccgaaag caggtgcgac gtgggattat 16980
ggctgtctgc tgacgacatg accagtgtca gactggtcac aaattgctca ttcctgtcca 17040
ccacgcgtca gtttagcaat ctacatgttg gcccttcttc acacaacatc ttgcctgtaa 17100
tggttaggat gaagcctggc atatatctaa ccaggactaa cgtgccatct ctaggaggta 17160
acacgctagc tccagctggg gacgagcgcc tagaaaccct cgtcctgacg ggatggggcg 17220
aggcgtgcgt cagatcgcct gtcgccacct aacccgcgat ctgaccggtc tgtgactggt 17280
cacagaccgg ataaacgagt gcactgcact tcgttacatg cggcgtgaca cgctcagcca 17340
aaccacaata aatgtggtta ggtgagcccc gctgtgctca cctaacccat acacgcggag 17400
caaaaaccca cgaggggtcg ggcgcctcgg gccctcgggg ccgaggcggg tgcggtccga 17460
ccccctcggg gggactaaga ggagggcgaa cacatcaccc tcgggcccga cgtcccccga 17520
gggtgccagg ccacgtgggc gattgtgtct gcctcaaacc tctagtcatg atactcctga 17580
tcccatgtca ccgacaaggc catccgaatg tattaaggag taaaagttac aagaaaaaac 17640
accataatgc accaatgtgc atgaccacac accatacact acccccaagc acaaaccact 17700
gagggtgaag cctagcacca aacgaccgcc actaagtgtg accaaacgcc gctaggccta 17760
cggcagcaac acatagatga gacttcgaaa acgatgccac caaggtggtc acgacatcta 17820
ggatgctgcc atcgtccatc taaaaagatg tggttttcac ccagagaaac tcatcaagaa 17880
ggggagaggg taacccttga cagcgcccca aggaggttac gacgcccgaa ggcgtagccg 17940
ctgccggtcc ggtgaaccac cggactaggc ttccgcctag gaccctatag ccttgatcgc 18000
agatcaccgt ccaccactca gaaccaccac acagacaaaa ggtagcacgt agcttccacc 18060
acaccgcacc gacgcccctt cgtcggccga ctccatcgaa ccaccatccc tgagagctgg 18120
cccaggaccc ctccgttcca ccacccgccg gccgccttgc cagttttggc caaaggagaa 18180
cccgggactg ggtgacattg cttcggcagc ctgagcttcc cccgctggcg agctgctgtc 18240
tcaatccaac ctagaaactc cccgcaaaag aaggggatga gctctaggaa gggcgagggt 18300

gccgaccggc aacgaggaag acaacccatc gactccagct ccctttgcac taccatctgg 18360
ccctgcgcca atgccggata cgctgtcgct ccggctccgg cgccacccac ctgcaccccc 18420
tttgcctggt ctccgcgccc ctcctggctg cgtcgcgccg cccagctggc cgctaagggc 18480
accgcgacgg ccgcccggct accgaggcct ggccgcgcca tgggacagct cgcgctggca 18540
ccagcgagcc acggccgtcg cgctgttgcc ggcgccagcg agcacaaccg ccagctccaa 18600
gggccgagca tgccactgag ccgccgccgc tgccgcccgg gccggctgca cgtcaccggc 18660
gcacacgacc gcacgccgcc acgctccgcc tccgcgcccg aggcagcccc atgccattgc 18720
cgcgcacctc gcccgcccgc tgccgagccg ccaccgcgca ccttgctgag ccgccaccgc 18780
cgtccctagc cgcctcgtgc cgccgccacg ccagatccag gcgcgggatg gccggatccg 18840
gccttggggg cgccggatcc accgcctccc cacaccgcca cggcgtcacc acctccgacc 18900
gcagtgaggg cttcgtcgtt tgccccatcc tcatcgcgtc gaggaggaag acgccaagaa 18960
aaaagggcct cgccgctgcc ttccttgctc gctgccggct tcgccgccgg cgagctccgg 19020
cggcggcgag gtgggggaga agaagtgggg agtgggcagc tagggttttt tcgcccccca 19080
agccgcccgt gcgagagcga cggtgggggg gggggggact ttccaacctc ttccagtgtt 19140
ctagttctcc acgttatgta actcaatttg tttaaccata gaaagtaaga aacctaccag 19200
cgtgttaagc tctctttcat tccctttctt cttcctggtt ttgcttccat cacatgtcaa 19260
gtgaagggtt cttaactacc attactccta cacatctaat ttttttctca gatctttcgc 19320
aggtatatat tgatgctaca ttttatgatc ttaagataat ctccttcaca ttaccctctg 19380
ctgaaacttt agcttgaacc gtcatcttca ccacaatttg agcccaattt gcacagagca 19440
caacgagcaa tagcttgccc ttacgttcat tatttagcat gaactactac taactaccca 19500
agaatcaata caccggttta ataacgccat tttatcacgt taatatatgt ttcattcaac 19560
acaccggttt tggcacagtt gcaaacttgc aataaattct ttcctacttc tccatcccat 19620
aatataacaa attggtatgt ctcgtctggt actaagttac tatattatga gatggaggga 19680
gcacttcttt tcttccaaaa tataagaata tagtattgga ttagatatta tctagattca 19740
cgaattcgat taggttgtct agatttatag ttgtatgtaa tgtataattc ggtaataggt 19800
tattacctct caggatggag ggagtagttt tgactttttt tttcttataa atcgctttga 19860
tttttatatt agtcaaattt tatcgagttt aactaagttt atagaaaaaa attagcaaca 19920
tttaagcacc acactagttt cattaaattt agcatggaat atattttgat aatatatttg 19980
ttctgtgtta aaaatgctgc tatatttttc tataaacgta gtcaaattta aataagttag 20040

actaaaaaaa atcaaaacga cttataatat gaaatggagg aagtagtaga ctataacaaa 20100
tttaaaccgt gctttgattt tagagcatca ctaatatgtt agcaataatc tatccctaaa 20160
atttattttt tttcctaaac tgaaaatagg aagtggaaat actcctccat ctaagagaga 20220
gcctaaattc aataaaaaac taaaaaacta aaggtggatc cctctattaa actaccgcaa 20280
aaaatttatg ttttttttct cttccacgcg cgcagaacag atatctcgat caagttagca 20340
tgtaaaattt ttaaagagat accttatacg actccttccg tatttccaaa agcaaacgga 20400
tttaaaatct gactcaaata aagatctata tatccaattt acatgacaca tgtttcgccg 20460
aatttttata ttaataataa ttaatatttt taaaattaaa ttattagcaa tttgtttgga 20520
ggatttatca aaacaggatg gacgttgttt ataacagcgt ctagacctag acgcgcttgc 20580
aaactgcggc caccctttta tcacacaaat tttgacaatt ttgacacttt ccaaaaatta 20640
attttataaa ttaaccgtga ccaaaactta tttaaaaatg atctttttgt tgagcgcaaa 20700
atcgtatact tcagcgccaa atagcacggc gccgacctcc cccttcccct cccctctatc 20760
ctccactgct gccgcccacc tctccgtatc agctgcgtcg cgttggtttc cgccggcgct 20820
gctgctgctg caccagtccg ctagggcggg cgggcatggc gcgccgcgcc gcttcccgcg 20880
tccgcgccgg cgctgttggc gcccttcgct cggagggctc gacccaaggg cgagggggcc 20940
gcacgggggg cagtggcgcc gaggacgcac gccacgtgtt cgacgaattg ctccggcgtg 21000
gcaggggcgc ctcgatctac ggcttgaact gcgccctcgc cgacgtcgcg cgtcacagcc 21060
ccgcggccgc cgtgtcccgc tacaaccgca tggcccgagc cggcgccgac gaggtaactc 21120
ccaacttgtg cacctacggc attctcatcg gttcctgctg ctgcgcgggc cgcttggacc 21180
tcggtttcgc ggccttgggc aatgtcatta agaaggattt tagagtggat gccatcgcct 21240
tcactcctct gctcaagggc ctctgtgctg acaagaggac gagcgacgca atggacatag 21300
tgctccgcag aatgacccag cttggctgca taccaaatgt cttctcctac aatattcttc 21360
tcaaggggct gtgtgatgag aacagaagcc aagaagctct cgagctgctc caaatgatgc 21420
ctgatgatgg aggtgactgc ccacctgatg tggtgtcgta taccactgtc atcaatggct 21480
tcttcaagga gggggatctg gacaaagctt acggtacata ccatgaaatg ctggaccggg 21540
ggattttacc aaatgttgtt acctacaact ctattattgc tgcgttatgc aaggctcaag 21600
ctatggacaa agccatggag gtacttacca gcatggttaa gaatggtgtc atgcctaatt 21660
gcaggacgta taatagtatc gtgcatgggt attgctcttc agggcagccg aaagaggcta 21720
ttggatttct caaaaagatg cacagtgatg gtgtcgaacc agatgttgtt acttataact 21780

cgctcatgga ttatctttgc aagaacggaa gatgcacgga agctagaaag atgttcgatt 21840
ctatgaccaa gaggggccta aagcctgaaa ttactaccta tggtaccctg cttcaggggt 21900
atgctaccaa aggagccctt gttgagatgc atggtctctt ggatttgatg gtacgaaacg 21960
gtatccaccc taatcattat gttttcagca ttctaatatg tgcatacgct aaacaaggga 22020
aagtagatca ggcaatgctt gtgttcagca aaatgaggca gcaaggattg aatccggata 22080
cagtgaccta tggaacagtt ataggcatac tttgcaagtc aggcagagta gaagatgcta 22140
tgcgttattt tgagcagatg atcgatgaaa gactaagccc tggcaacatt gtttataact 22200
ccctaattca tagtctctgt atctttgaca aatggacaag gctaaagag ttaattcttg 22260
aaatgttgga tcgaggcatc tgtctggaca ctattttctt taattcaata attgacagtc 22320
attgcaaaga agggagggtt atagaatctg aaaaactctt tgacctgatg gtacgtattg 22380
gtgtgaagcc caatatcatt acgtacagta ctctcatcga tggatattgc ttggcaggta 22440
agatggatga agcaacgaag ttacttgcca gcatggtctc agttggaatg aaacctgatt 22500
gtgttacata taatactttg attaatggct actgtaaaat tagcaggatg gaagatgcgt 22560
tagttctttt tagggagatg gagagcagtg gtgttagtcc tgatattatt acgtataata 22620
taattctgca aggtttattt caaaccagaa gaactgctgc tgcaaaagaa ctctatgtcg 22680
ggattaccga aagtggaacg cagcttgaac ttagcacata caacataatc cttcatgggc 22740
tttgcaaaaa caatctcact gacgaggcac ttcgaatgtt tcagaaccta tgtttgacgg 22800
atttacagct ggagactagg acttttaaca ttatgattgg tgcattgctt aaagttggca 22860
gaaatgatga agccaaggat ttgtttgcag ctctctcggc taacggttta gtgccagatg 22920
ttaggaccta cagtttaatg gcagaaaatc ttatagagca ggggttgcta gaagaattgg 22980
atgatctatt tctttcaatg gaggagaatg gctgtactgc caactcccgc atgctaaatt 23040
ccattgttag gaaactgtta cagaggggtg atataaccag ggctggcact tacctgttca 23100
tgattgatga gaagcacttc tccctcgaag catccactgc ttccttgttt ttagatcttt 23160
tgtctggggg aaaatatcaa gaatatcata ggtttctccc tgaaaaatat aagtccttta 23220
tagaatcttt gagctgctga agccttttgc agctttgaaa ttctgtgttg gagttctttt 23280
ctcctacagt cgtattagag gagggatctt ctctttatgt gtaaatagcg aggtatgtat 23340
gtcacctctc cgaattattt ttactctggt tcctagacgg taaacaagca attatgttct 23400
gcctttgatg ccagaaaaaa cacaaaagtt tgtcgttatc tctactaacg gatcataaag 23460
gaatttgtaa ctggagtttc aaacttaatt tgtctaggca gtagttttgg cattagatcc 23520

aacattgtgt aggattcatt tgtgtgtatc aatctatagg gtttcattaa atttcgttta 23580
tgtgtactgt ttaggtgttg aatagtttga cttgtttttt aactgaacaa aagatactga 23640
aatcgttcca ttcaacaaac acatgttccg ttaatgaaat tattgtacgt taccttttgt 23700
tttcttactc acaagtgtcc tcttttctta tatcctatag attggtacaa caaattattg 23760
attcaatttt ggttttgaac attgatgatc ctccctgcac tattggtgca gctgctcttc 23820
tattcatttt gtgaagtgat gtgagtacct ctcaatccca tccttatgct tctgtgcatg 23880
cttcattcca attttttacg catatcgatt gttttctttt atataacagt ccataaagat 23940
aatcacatca tgacaaagtt atttatttct acagtatagt tatataagta ttcaccagtt 24000
ttccatgaat attttggcat gtgattacaa agaagattat ttgagaaaat ccatgctttt 24060
atttcatcat tttgtttgaa gttgaacttt aatttatggt gtaaatttca gttattattg 24120
ctagcagctc gtactcttta atggtataac ttcacttgtg cttattctcc aatatctccc 24180
ttcttgttgt tcaggttcaa gaaaatcatt tgttggattc agaatctggt gtccattttc 24240
ttcttaaatt attaaatcct ccagtgaatc ttgttgattc caaagcacca tcgataggtt 24300
ccaaacttct tggaatcagt aaagttcaaa tgcttaatgg atcaaataag gattctgact 24360
gcatttcaga ggaaatcctt tcaaaagttg aagagattct cttaagctgt caagtgatca 24420
agtcgctcga caaagatgac aagaaaacaa caaggccaga actgtgtcca aagtggcttg 24480
ctttgttgac aatggaaaat gcatgcttgt ctgctgtttc agtagagggt aagttttaat 24540
caaatttctt ggtcatgatt tccctttatg accattatat ttatttatat gagccaaata 24600
agcagttgtc aacttgtcat aagttacata gcacctattt gcaatattca tgggtggttt 24660
gcttagccct tttcttcacc tgcttttgat tgatgacttc catctgtgtt gcagaattga 24720
attggagtag tggactgcac tagaagcacc tatggccatt gtcatactag gaaggttttc 24780
ccttatcaaa tatttgattg ttacagagac ttctgacaca gtgtccagag ttggaggaaa 24840
ttttaaagag acattaaggg agatgggagg tcttgatagt atttttgacg ttatggtgga 24900
ttttcattca acattggaga tgagatctcg ctaacatcgc atattttaca tttcctttgt 24960
tcaactctaa tagattgtgc aggcttgttc cttttcgcca ttttagcttt aatgcgcttg 25020
aagccacatg aaagtaatgc ttgtccagat acatagccaa aggttgttat attttggggc 25080
atggaaaatg cttgaggtag taactatttt catcaggaca tggaaaattg gctgcaacac 25140
aaattatgtt gttttatgtt gcaaaaatag ttttttaata cttttttatt ctgcatgtgg 25200
tgttagtatc ttacagttcc tctgatgatt atatccccca cgataataac acttgaaacg 25260

ataataacac ttgacatatc tacaccaagt gaacattatt catttggatg ttacttttcc 25320
agctatactt gctgttcttg catgtgtaag caagtttgga gtaaattgcg cattaattta 25380
aatgcttggt gttcctatct gtgtactttt tattccccaa ctaataatgc aatcatatta 25440
cgctgataaa ctgaataaat aaattaacaa tatacttctg gtggcaaacc ttgtgtatca 25500
gaatctcata aaggatacat ccacttcagc tttggaccga aatgaaggaa catctttgca 25560
aagtgctgct ctcctcttga aatgtttgaa aatattggaa aatgccatat ttctaagcga 25620
tgataacaag gtaatgctcc ttatatgttc tgtttcagtt tagtacccat ttccttcttc 25680
tgtactatct tctctcctga tttgttctgt gcaaaatgtg caaacagtgc gactttgtat 25740
gtctgcttaa caatttcttt ttcttcctga aaaagcaata tgaactctta cattcatttt 25800
gcttcttgca gacccatttg cttaatatga gtagaaaatt gaacccgaaa cgctccttgc 25860
tttcttttgt tggtgtcatt atcaatacta ttgagttatt atcaggtatt tttcttaata 25920
atacaatgtg ttcgctaaca caataaaatg ttttaaacat ccagtatgtt aaagttgcag 25980
tctgacgcct atttgttttt gctgcagctc tttcaatact tcagaattct tctgttgttt 26040
ccagctctac atatccgaaa tcgtctaaag tctctcaaca gagttactct ggtaataaca 26100
aacaccaatt ttgtttgatc agttgatctc gttggctttt ctatgcactg tctcaatata 26160
gtttggtcgc cattcaagtc tcactacaga tgttgaactt ggcctgacac caaatattta 26220
taaaatgcta cctgatattt ttaatatttc atgtttcctg acccagatta tcttgttggt 26280
tcctcgtata agtttaatta gtgacattct tgaagctttg ttatgcagca gatgtcatgg 26340
ggggaacttc atttaatgat ggaaagagca agaactcgaa aaaaaaaaac ttttgtcgaa 26400
ccagacacgt cattgttgct tatcttcaaa atcagaagtt tctcatatta ctatatcttc 26460
tggtagtgat gctggtctgt cacagaaggc attcaattgt tctccattta tatcaagcaa 26520
tggggcatca agtggttcat taggcgagag gcacagcaat ggtagtggtt tgaagttgaa 26580
tataaaaaag gatcgtggca atgcaaatcc aattagaggc tcaactggat ggatttcaat 26640
aagagcgcac agttctgatg ggaactccag agaaatggca aaaagactcc gtctatctta 26700
aaatgtaatc accgacagtg gtggtggtga tgaccctttt gcatttgacc gccgcgtcgg 26760
cgtcgccacc acgtaatcgc ccacgtcgct gcccccgctg ccacgtcgtc gaccgcgcac 26820
ggtaatcaca cgcatctcga ggccgccgct agctgatatc ttctcatccg gttgatttgt 26880
gattttggcg tttttgcagt ggtgatggcg gggggcgacc gtggccgagg cgtggagtgc 26940
catccgcatc agggtgtatc ggccgcgctg ctccgccctg gtccgcaggc tttggcggcg 27000

agctggcggc ggagggagac tgtggtgaga tcggatttcg ccgctggtgg tgtcgctacc 27060
atgggggatt cgccgcaggc gctctcaggt ttgcagcctc ctccactctc ttcccttttt 27120
tatttttttt tctcgcaaaa tgtgttgtga tgttcgtctc gctgggctgg cctcatagcc 27180
attaatgtag tttgctggaa catttacatt tggaacgttg ttggcaattg ctttacaaaa 27240
tgtggaattg tggaggggag aaaaatcatt tgaacctgca gtgacaaaat tgccatctct 27300
aattttaaaa ctgaaggtgt ggaaatcaaa cataatcatt gccagcgcat cattcttgtt 27360
aaccaccatg atatattgtt ggttataaca gttagctcca caccaacctt gaaggtgtca 27420
atagaatgtt tagtataaat tgaggagaac aggcagttgt taagactttc taaagaactt 27480
gtagcagcta atactagcta ttgtgcattt gtgtttcatg gaatttgagc agcaatggat 27540
atttcttact aagatgtatg atgcaaaaca aaaaactatg tctatacagt ttacatgtaa 27600
tgtgcggatg caaataaaat catgtacatg gacaaactca tgggattcat accgaattcc 27660
agaattgcat ttcttatgtg gttacttttg ttgttgattt ggttaccaga catcgatgtg 27720
atttcaaggg tcagaggggt ttgcttctac gcggtggctg cagttgcagc aatctttttg 27780
tttgtcgcca tggttgtggt tcatccactt gtgctcctat ttgaccgata ccggaggaga 27840
gttcaggaaa aaaatttgaa aatacccatt ttttgaaaaa gatttacgtt tatatacact 27900
agtatgaaga atttgcgaaa atataactaa tccgcagatc ggttatgcgg gagcgcaaca 27960
aaagtatggc gtggcggcgc ggagtggacg gccgaggcgt tcgcgcggaa tggggctgcg 28020
ggaccgagcc agtctcgctt gccggtaacg cggaaccggt acgctcccgc agcgccagtg 28080
tgcggaaccg cggcgccaac atttttttac tgcatggcac tgtgtttaat actgtttgac 28140
actgtttctg gtactgtttt acacagttcc cgggtcagtt ccgcacaatg gaggcgcggc 28200
accgaccatg aacaatgtgt gaacagtgct gcacagggtt aaaacagtgt ataaactgcg 28260
ctgcacagtg ctggagtcgc tggccactgc ggttccgcgt tttggaaccg cgggaccgtc 28320
gcgattccgc gttttggagc tgccggacca tgacggttcc gcgcaggatc gtcggtcccg 28380
tattttgaat ctgcggaacc gtcgctgtcc cgcgtttcca tttcgcggga tgcgtatatt 28440
tttataaaac ctctccatgc atgtatataa acataaatta ttgaaaaaat aagtatattt 28500
gcaaattttt ttcgagagct cagcactaca ttgcaaagat ttgggcaact ctgacaattt 28560
ccatgttcta caagcttgac gtcgagggaa tggagaacct gccaccgaat agtagccctg 28620
ctatctatgt tgcgaaccat cagagttttt tggatatcta tacccttcta actctaggaa 28680
ggtgtttcaa gtttataagc aagacaagta tatttatgtt ccgaattatt tgatgggcaa 28740

tgtatctctt aggagtaatt cctttgcggc gtatggacag caggagccag ctggtatggc 28800
tgtagtctca tccctgcttt cttaagtaga catatatgca attacagaat ttggtaaaca 28860
aacaagattt tatgaatcat atatgatttt ggggaaaaca ccaaactctc tttggtggct 28920
gccttgaaca tagttctatt cacacagtta tagcaccttc tttaaaatga agaactttgt 28980
tgcatacaca tatggccaaa ccacataatg aattttgttt atttctatct ttgaatgtta 29040
gcaccttatt ttcatgcata tcatgctaat ttgcttgccc acgttgagtg ggaatttttt 29100
tccatgtttt ataatttata tatgttctag acttctagtc cacaatttat ctacttcatg 29160
ttcctgagcc tctagtatgg ctggtagcag actaggtgct gagtgctgtc catttttgca 29220
gactgaagag aggagaaata caggactgtc cgttgttagt cagatttgta aaaatagact 29280
ctgatgtagt ttattttagc ccctatttta tatttaacaa tacaaatata taacgtatcc 29340
taagaactta tcgtaattta ggagaagttg ctcgtttcat taaattaaac tgtgaagtaa 29400
aaatgtgtgc tcgagtctgt caatgcaatc ctgtgttctt gtttgaagat atggtgtagg 29460
gcaggctagg atcgaacact gaatggtaag actgcttctg ccttcatttg tgcacttggt 29520
gctgccacgc cgattaagca gtagaacaaa gtaattttgt cgtgcacaaa tgagttatat 29580
ttcattgaaa atcgaagtga aaatgaacca aaagatagaa gaaaagggga aacttggtaa 29640
ttatatactc cacaaattta ttggtaagat ttgatattag acgctcgatt acttggctta 29700
agttaaggat atcaaatttg gggaagcacc aaaggaatta ttgtgaagga gttgtgggtg 29760
cataacgtta tctactagtt caaatcctag tgactatgaa tattaatgag taaggtaagg 29820
gatttattgt taattttagt ttctttaaga ttgtgtccga gtacaccatt cggtaagtgt 29880
aataatgttt tgtattggat tcacttgtgt tacgtgcatg tgcttttacc ttttcatttg 29940
tttctgcgtt ctgggtatga atttgacgag attccatggt cagctcaaca tatcagttac 30000
tgcgtgtcaa gcgatcttat atggtatgcg cacaagcgat tgtatacgga tatgacagta 30060
taatgtgtga tattgatacg atgttccttt cctttataaa ggaacaaaga ctttttttaa 30120
aaaaagaagg ggtattacta aaaaccaaaa tgtcaaaaac aaaatatcag tgcacatggc 30180
aagtgtgcac gagcaatagc ttgcccttac gttcattatt tagcatgtac tactactaac 30240
tacgcaaaaa tcaattcacc gattattaaa ctgttaacat cattttagca cgttaacata 30300
tgtttcattc aacacaccgg ttttggcaca tttacaaact tgcaaagttg caatactccc 30360
ttcgttacat agcataagag attttaggtg aatgtgacac atctatccaa attcattata 30420
ctagaatgta tcaccgcctc cacgccggga gggagagcgc cgccggtgga gaaaggggga 30480

gggagtggtc gaggggaacc agtagggtgc cctccccgtc gccgcctccc cgtggccgcg 30540
ccggcgagac aggaggaaga gggggagatg gagcggcgcc gccggtgagg gcgcgcgtgc 30600
gcgggggggg ggggggggga gcggcgacgc cggtgaggaa gggaagggga gtggtggctt 30660
tgagagagat aggggagagg gaaaatgatt ttagagttag ggtttgggct gctgagtttt 30720
tatatagatc gggatcaatc aggaccgtcc atcagatcgg acaactacgg tttctcccgc 30780
gttgggccgg gtgccactcc taggttgccc acactattgg gccacatgta cgctccgcgt 30840
gaaataagtt cactttaggt cctttaagtt gcctctgaat tgttcccagg ccggccgcac 30900
tattgggcca ccccataggc catgtgtacg ctccgcacag aataatttcg ctttagctcc 30960
cttaatttgt cccctcaaac ttctaaaacc agtgcaaatc tttaattttt agttcaccca 31020
ttgcaactca cgggcatatt tgctagtgac atataatatg aaacgaagga tgtagcagac 31080
tatagaattt aaactgtgct ttcattttag agcatcacta actgttattt agatttttat 31140
ttaaataaat gcagaaatga tgtttttatt atgaaattta gcaataaagc tcccaaaatt 31200
tcaaaaaaaa attaaaagag atttattaat catggttaat ttaattaaaa attaaatcta 31260
accatatcat attatttcac ggtccgtgat gaggaaatgg cagctgctat cacttatggt 31320
gggagagaag ggcattgttt tatttttata actatctctt ataactccca tgaaactata 31380
aaataaatat aatcattatc ataacattag tttttttcca ttgcaacgca agggtaattt 31440
ttcagtacaa taaaaaaata aaagtgggcc attctgaacg gaaatttctg gttttttttc 31500
ccaagagcgc cgcacacaac tgcgcaagag atcgatcgcg atcaccctgc tcgtcgccga 31560
tctcctacac catccctgcc atctccttcc cctccactgg ctgctgctgc acctgtcagc 31620
tagggcgggc atggcgcgcc gcgccgcttc ccgcgctgct ggcgcccttc gctcggaggg 31680
ctcgatccaa gggcgagggg gccgcgcggg gggcagtggc ggtggcgcgg aggacgcacg 31740
ccacgtgttc gacgaattgc tccgtcgtgg cataccagat gtcttctcct acaatattct 31800
tctcaacggg ctgtgtgatg agaacagaag ccaagaagct ctcgagctac tgcacataat 31860
ggctgatgat ggaggtgact gcccacctga tgtggtgtcg tacagcaccg tcatcaatgg 31920
cttcttcaag gagggggatc tggacaaaac ttacagtaca tacaatgaaa tgcttgacca 31980
gaggatttcg ccaaatgttg tgacctacaa ctctattatt gctgcgctat gcaaggctca 32040
aactgtggac aaggccatgg aggtacttac caccatggtt aagagtggtg tcatgcctga 32100
ttgcatgaca tataatagta ttgtgcatgg gttttgctct tcagggcagc cgaaagaggc 32160
tattgtattt ctcaaaaaga tgcgcagtga tggtgtcgaa ccagatgttg ttacttataa 32220

ctcgctcatg gattatcttt gcaagaacgg aagatgcacg gaagcaagaa agatttttga 32280
ttctatgacc aagaggggcc taaagcctga aattactacc tatggtaccc tgcttcaggg 32340
gtatgctacc aaaggagccc ttgttgagat gcatggtctc ttggatttga tggtacgaaa 32400
cggtatccac cctaatcatt atgttttcag cattctagta tgtgcatacg ctaaacaaga 32460
gaaagtagaa gaggcaatgc ttgtgttcag caaaatgagg cagcaaggat tgaatccgaa 32520
tgcagtgacg tatggagcag ttataggcat actttgcaag tcaggcagag tagaagatgc 32580
tatgctttat tttgagcaga tgatcgatga aggactaagc cctggcaaca ttgtttataa 32640
ctccctaatt catggtttgt gcacctgtaa caaatgggag agagctgaag agttaattct 32700
tgaaatgttg gatcgaggca tctgtctgaa cactattttc tttaattcaa taattgacag 32760
tcattgcaaa gaagggaggg ttatagaatc tgaaaaactc tttgacctga tggtacgtat 32820
tggtgtgaag cccgatatca ttacgtacag tactctcatc gatggatatt gcttggcagg 32880
taagatggat gaagcaacga agttacttgc cagcatggtc tcagttggaa tgaaacctga 32940
ttgtgttaca tatagtactt tgattaatgg ctactgtaaa attagcagga tgaaagatgc 33000
gttagttctt tttagggaga tggagagcag tggtgttagt cctgatatta ttacgtataa 33060
tataattctg caaggtttat ttcaaaccag aagaactgct gctgcaaaag aactctatgt 33120
cgggattacc aaaagtggaa ggcagcttga acttagcaca tacaacataa tccttcatgg 33180
actttgcaaa aacaaactca ctgatgatgc acttcggatg tttcagaacc tatgtttgat 33240
ggatttgaag cttgaggcta ggactttcaa cattatgatt gatgcattgc ttaaagttgg 33300
cagaaatgat gaagccaagg atttgtttgt tgctttctcg tctaacggtt tagtgccgaa 33360
ttattggacg tacaggttga tggctgaaaa tattatagga caggggttgc tagaagaatt 33420
ggatcaactc tttctttcaa tggaggacaa tggctgtact gttgactctg gcatgctaaa 33480
tttcattgtt agggaactgt tgcagagagg tgagataacc agggctggca cttacctttc 33540
catgattgat gagaagcact ttccctcgaa agcatccact gcttccttgt ttatagatct 33600
tttgtctggg ggaaaatatc aagaatatca tagatttctc cctgaaaaat acaagtcctt 33660
tatagaatct ttgagctgct gaagcatttt gcagctttga aattctgtgt tggaattctt 33720
ttctcctaca gtccgattag aggagggatc ttctctgtat gtgtaaatag cgaggtatgt 33780
atgtcacctc tccgaattat tttgactgtg gttcctggac tgtaaacaag ctattatctt 33840
ctggtgttga tgccagaaaa aacacaaaag tttgtcgtta tctctactaa cggatcataa 33900
aggggtttgt aactggagtt tcaaacttaa ggtatctagg cagtaggtat atattgatcc 33960

tacatcttat gatcttaaga tgatatcctt ctcattatcc tctgctgaaa ctttagcttg 34020
aaccgtcatc tacaccacaa tttgagcccc ttagcacaga gcacaacgag caatagcttg 34080
cccttacgtt cattatttag catgcactac tactaactac ccaataatca atacatcggt 34140
tattaaactg tttgtacagt ttaataatgt catttatcca cgttaacata tgtttcattc 34200
aacaccacac cggttttggc acagttgcaa acttgcaata acatttttac tacttctccg 34260
ccccataata taacaatctc gttccatact atattgctat attacaggat ggatgaagta 34320
cttcttttct tccaaaatat aagaatctag tactagatta gatattattt ggattcacga 34380
atttgattag gctgtctaga tttgtagtcg tatgtaatgt ctaattcggt aataggttat 34440
tacctctttg gatggaggga gtagttttta tttcgtactc cctccgtttc atattataag 34500
ttgttttgac tttttcttta gtcaaatttt attgagtttg attaaattta tagaaaaaaa 34560
ttagcaacat ttaagcacca cattagtttc attaaatgta gcatggaata tatttttata 34620
atatgtttgt tttttattaa aatgctacta tatttttcta taaatgtagt caaatttaaa 34680
gaagtttgat tatgaaaaaa tcaaaatgac atataatatg aaactgagga tgtagcagac 34740
tatagcaaat ttaaactatg cttttatttt agagcatcac caaaagatta gcaataattt 34800
atccctaaaa ttcaagtttt gggtttctta aactgaaaat aggaagtgaa aaatcttttc 34860
cgtccaagag atagcctaaa tcttatctta actaattaaa atattcataa ttttcctttc 34920
gtcacattaa attttcgtcc gtaaatctga ttgaaatcca attggacaat ccaaaaaata 34980
gagaaaaaga acagaaaaaa taataaaaag cacacaaatc ttatctcaat cccgcgggaa 35040
gctgccgacg ccgccgaatc cgctcgagcg ccgccgccgc cgctcacggg gaacgatgtc 35100
gctgctgtcg cacgcggtat gggagggcgc cgctgccact gcttgggaga taggatatgg 35160
agagagaagg aaatgtgagg gttagggtta ggtttttccc cgtccgtatc ttcagcgaca 35220
cggaggcgat ccaagctgtc catcagatcg gacggctcag aatgcctcca tcgtcgggcc 35280
gcgcatgctt gatgggccga gggaaggccg gagggtcgaa caaacgcaat caaaggagga 35340
gttggaggag gtaaattaga atttatttgc gggctgagat agtaaatgga ctgaaaatgg 35400
cccatagaga aattgggaat tttatttaaa taaatgttga aaaggtgttt atattatcaa 35460
aattaaaaat taagctccga aaattctaaa aaatattcaa agagcattat taatcatggt 35520
taatttaata aaaattaaat ccaaccatat catattattt cacggcgcgc ggtaggaaaa 35580
tgcgcagctg ttgtcgttta cggtgggaga gaagggacat tgtttatttc cagaactatc 35640
ttttataact cccatggaac tttaaaataa atataatcat tattatagca ttagtttttt 35700

tctgtctttt ttttccccaa gagcgccgcg cagaagagat cgatcgcgat ctccctgccc 35760
cgacgtcgcc ggccgatctc tcattctctc cacgccctgc tcgtcgccga tctcctacac 35820
catccctgcc atctcctcct tcccctcccc tctatcctcc actggtgccg cccacctctc 35880
cgtataagac aaactgcgtt gcggcgttgg tttccgccgg cgctgctgct gcacctgtca 35940
gctagggcag gcatggcgcg ccgcgccgct tcccgcgctg ttggcgccct tcgctcggac 36000
ggctcgatcc aagggcgagg aggccgcgcg gggggcagtg gcgccgagga cgcacgccac 36060
gtgttcgagg aattgctccg gcgtggcagg ggcgcctcga tctacggctt gaaccgcgcc 36120
ctcgccgacg tcgcgcgtca cagccccgcg gccgccgtgt cccgctacaa ccgcatggcc 36180
cgagccggcg ccggcaaggt aactcccacc gtgcacacct atggcattct catcggttgc 36240
tgctgccgcg cgggccgctt ggacctcggt ttcgcggcct tgggcaatgt cgtcaagaag 36300
ggatttagag tggaagccat caccttcact cctctgctca agggcctctg tgccgacaag 36360
aggacgagcg acgcaatgga catagtgctc cgcagaatga ccgagctcag ctgcatgcca 36420
gatgttttct cctgcaccat tcttctcaag ggtctgtgtg atgagaacag aagccaagaa 36480
gctctcgagc tgctgcacat gatggctgat gatcgaggag gaggtagcgc acctgatgtg 36540
gtgtcgtata ccactgtcat caatggcttc ttcaaagagg gggattcaga caaagcttac 36600
agtacatacc atgaaatgct tgatcggagg atttcaccag atgttgtgac ttacagctct 36660
attattgctg cgttatgcaa gggtcaagct atggacaaag ccatggaggt acttaccacg 36720
atggttaaga atggtgtcat gcctaattgc atgacatata atagtattct gcatggatat 36780
tgctcttcag agcagccgaa agaggctatt ggatttctca aaaagatgcg cagtgatggt 36840
gtcgaaccag atgttgttac ttataactcg ctcatggatt atctttgcaa gaacggaaga 36900
tccaccgaag ctagaaagat ttttgattct atgaccaaga ggggcctaga gcctgatatt 36960
gctacctatt gtaccctgct tcaggggtat gctaccaaag gagcccttgt tgagatgcat 37020
gctctcttgg atttgatggt acgaaacggc atccaccctg atcatcatgt attcaacatt 37080
ctaatatgtg catacgctaa acaagagaaa gtagatgagg caatgcttgt attcagcaaa 37140
atgaggcagc atggattgaa tccgaatgta gtgacgtatg gagcagttat aggcatactt 37200
tgcaagtcag gcagtgtaga cgatgctatg ctttattttg agcagatgat cgatgaagga 37260
ctaaccccta acattattgt gtatacctcc ctaattcata gtctctgtat ctttgacaaa 37320
tgggacaagg ctgaagagtt aattcttgaa atgttggatc gaggcatctg tctgaacact 37380
attttcttta attcaataat tcacagtcat tgcaaagaag ggagggttat agaatctgaa 37440

aaactctttg acctgatggt acgtattggt gtgaagccca atgtcattac gtacagtact 37500
ctcatcgatg gatattgctt ggcaggtaag atggatgaag caacgaagtt actctccagc 37560
atgttctcag ttggaatgaa acctgattgt gttacatata atactttgat taatggctac 37620
tgtagagtta gcaggatgga tgacgcatta gctcttttca aagagatggt gagcagtggt 37680
gttagtccta atattattac gtataacata attctgcaag gtttatttca taccagaaga 37740
actgctgctg caaaagaact ctatgtcggg attaccaaaa gtggaacgca gcttgaactt 37800
agcacataca acataatcct tcatggcctt tgcaaaaaca atctcactga cgaggcactt 37860
cgaatgtttc agaacctatg tttgacggat ttacagctgg agactaggac ttttaacatt 37920
atgattggtg cattgcttaa agttggcaga aatgatgaag ccaaggattt gtttgcagct 37980
ctctcggcta acggtttagt gccagatgtt aggacctaca gtttaatggc agaaaatctt 38040
atagagcagg ggttgctaga agaattggat gatctatttc tttcaatgga ggagaatggc 38100
tgtactgcca actcccgcat gctaaattcc attgttagga aactgttaca gaggggtgat 38160
ataaccaggg ctggcactta cctttccatg attgatgaga agcacttttc cctcgaagca 38220
tccactgctt ccttgttata gatcttttgt ctgggggaaa atatcaagaa tatcatagat 38280
ttctccctga aaaatacaag tcctttatag aatctttgag ctgctgaagc attttgcagc 38340
tttgaaattc tgtgttggaa ttcttttctc ctacagtccg attagaggag ggatcttctc 38400
tgtatgtgta aatagcgagg tatgtatgtc acctctccga attattttga ctgtggttcc 38460
tggactgtaa acaagctatt atcttctggt gttgatgcca gaaaaaacac aaaagtttgt 38520
cgttatctct actaacggat cataaagggg tttgtaactg gagtttcaaa cttaaggtat 38580
ctaggcagta gttttgacat tagatccaac attgtgtagt attcatttgt gtgtatcaat 38640
ctatagggtt tcattaaatt tcatttgtgt actgtttagg tgttgaatat attgttttac 38700
ttgtttttta actgaacaaa agatagctga agctttgttc tttaccaaat gcagtagtga 38760
tcatcacaat atattttttt acggaacagg agattgtata aaatggtttc catcggcggc 38820
caacggcgac cgctctgctc tgacccacca cccaatccat ccatccactc gccgccgccc 38880
ctgatccaag cctccgccgc gcgacagcga cgcaccgccg tcgagaggag gaggcgtgag 38940
ccccatgggg accctcctcc ggccgcgtaa tgccgctgca cggtaaccac gcgcctctcg 39000
aggcctccgc cgctagctga tctcttctca tcctgtttgg gtttgggttt gtgatttggg 39060
tgttttttcc gcagcggtgg tggtggtggt ggttgcggcg ggagggggcg gtggccgcgg 39120
ccgtggcgtg gagtgccagc tgcatcgggt gcaccgccgc cggggtccgc aggttgtggt 39180

ggcgacggcg agctgaggag gcggagggag actggtgagg gacacaggca ggcaggctct 39240
caaggctaag cttgttacag gtactgagac tagttactaa ttactttgat aatcagtata 39300
aataagcttg tgtagtgtaa tggcattgtg catttctgca cttgtaaatt ttacagaaga 39360
tggtcattca atttgaacct gcatctaata ttttagtggt ttgagtttat tctcccagtc 39420
acagagttga agaggcaagt aacctgtaag agaggactga acattaacac ctcttgttcg 39480
attaaaaatg accaaagagc atcaaacatg tattcgaggc tgttacttta atatggccca 39540
ttaatttgtt tagttggcta tgtacatcct agttggtgca gtgttgtgga aaacggaata 39600
cgggtgtcgg atggacgagg tgccgtcaag cgattaatcg taatacggat gattaaacgg 39660
aattatatgg atttttggcg ttcgcactaa gatgtacata attgatgtta atggcaatgg 39720
tggagacaaa atgcatcatc ttaataaaaa atatttgtat aaatctctaa ctatattatg 39780
aaaatgccat ttattagttc aatagatatc aacactgatg gttagtagcg caatagcatt 39840
gggcttgtta gtcaaaatag tgcagctggg ctgcaagttg caagtttatg ttagtttcat 39900
aaacagacat ctgatttgtc gataaataac cgactaatcg tgccatacaa ctgtataatt 39960
actctgaaat agtaatgttg ctccgacttg atgatacggt acggtctggc taccgtttcc 40020
gttttgacag acgattaaac ggctgtgccg gtcgacttcc acaacactga gttggtgtaa 40080
atgccagtta ccatttctat gatctaaaat aatcaactct tttagtatat tttcaaaaac 40140
gaaaattcag tacacatgca tgaatcttaa tcttcatatc tagctcgtta caaaatcaac 40200
aaaggcaccg tgtcagctgg tgcacattag ctagttcgta cttagcatta tccactagca 40260
ccttattttc atgcatatca tgctaatttg cttgcccacg ttgagtggga atttttttcc 40320
atgttttata atttatatat gttctagact tctacttcat gttcctgagc ctctagtatg 40380
gctggtagca gactaggtgc tgaatgctgt ccttttttgc agactgaaga gaggagaaat 40440
acaagactgt ccgttgttag tcagatttgt aaaaatagac actgatgtag tttatttttg 40500
cccctatttt atatttaaca atacaaatat ataacgtatc ctaagaattt atcgtaattt 40560
aggagaagtt gctcgtttca ttaaattaaa ttgggaagta aaaatgtgtg ctcgagtatg 40620
tcaatgcaat cctgtgttct tgtttgaaga tatggtgtag ggcaggccag gattgaacac 40680
tgaatggtaa gactgcttct gctttcagac gttattgcta aatttttagc tagttgcaat 40740
tagtgctgtc acgccgatta agcagtagaa caaagtaatt ttgtcgtgac aaatgagtta 40800
tatttctttg aaaatcgaag cgaaaacgaa ccaaaagata gaagaaaagg gaaacttggt 40860
aattactcca caaagagaac aaatttattg gtaagatttg atatgagatg ctcgattact 40920

tggcttaagt taacaatatc aaatttgggg aagcaccaaa agaattattg tgacttaagt 40980
taaagatatc aaatttgggg aagcaccaaa ggaattattg tgatggagtt gtgggtgcat 41040
aacgttattt gctttgttca aatcctagtg actatgaata tgaatattaa tgcgtaaggt 41100
aaggaattta ttgttaattt taggttcttt acgattgtgt ccggggacgc cattcggtaa 41160
ctgtaataat gttttgtatt ggattcactt gtgttacatg cacgcactaa acatgtgctt 41220
taccttttca tttgtttgtg cgttctgcgt ttgaatttga cgagattcca tggtcagctc 41280
aacatgtcag ttactgcgtg tcaagcagtt actgcgtgtc aagcgatctt atatggtatg 41340
cgcacaagcg attgtatacg gatatgacag tataacgtgt gatattgatt tttttatata 41400
aaaaaatacg atgttacttt ccttcataaa ggaacaaaga cttttttttt aaaaaaaaga 41460
aggggtatta ctaaaaacaa aaatgtcaaa aacaaaatat cagtgcacat ggcaagtgtg 41520
ctcggcaatt ttttgtctgt actttaaaca aaaatatttc tatatggtat tttttacaag 41580
ggtgtcacaa atattttaaa ttagccaaac atctgcattt tattaaaaac tgtataaatt 41640
ataatttata ctctaaaagg ttgtgtacat ctctcttgga gaaaatgtat aagttgcgaa 41700
caaacattaa tccacgttat ataagtcaat ctgttattta accatagaaa gtaagaaacc 41760
tactagcgtg ttaagctaag ctctctttca ttctctttct tcttcctggt tttgcttcaa 41820
tcacttgtca agtgaagggt tcttaactac cattactcct actcaccaaa tttttttctc 41880
agatctttcg taggtatata ttgatcctac atcttatgat cttaagatga tatccttctc 41940
attatcctct gctgaaactt tagcttgaac cgtcatctac accacaattt gagcccctta 42000
gcacagagca caacgagcaa tagcttgccc ttacgttcat tatttagcat gcactactac 42060
taactaccca ataatcaata catcggttat taaactgttt gtacagttta ataatgtcat 42120
tttatcacgt taacatatgt ttcattcaac accacaccgg ttttggcaca gttgcaaact 42180
tgcaataaca tttttactac ttctccaccc cataatataa caatctcgtt ccatactaga 42240
ttgctatatt acggacggga tgaagtactt ctttccttcc aaaatataag aatatagtac 42300
tagattagat attatttgga ttcacgaatt tgattaggct atctagattt gtagtcgtac 42360
gtaatgtcta attcggtaat aggttattac ctctttggat ggagggagta gtttttattt 42420
cgtactccct ccgtttcata ttataagttg ttttgacttt tttcttagtc aaattttatt 42480
gagtttgact aaatttatag aaaaaaatta gcaacattta agcaccacat tagtttcatt 42540
aaatgtagca tggaatatat ttttataata tgtttgtttt tttattaaaa tgctactata 42600
tttttctata aatgtagcca aatttaaaga agtttgatta cgaaaaaaaa tcaaaatgac 42660

atataatatg aaactgagga tgtagcagac tatagcaaat ttaaactatg cttttatttt 42720
agagcatcac caaaagatta gcaataattt atccctaaaa ttcaagtttt gggtttctta 42780
aactgaaaat aggaagtgaa aaatcttttc cgtccaagag atagcctaaa tcttatctta 42840
actaattaaa atattcataa ttttcctttc gtcacattaa attttcgtcc gtaaatccga 42900
ttgaaatcca attggacaat ccaaaaaata gagaaaaaga acagaaaaaa taataaaaag 42960
cacacaaatc ttatctcaat cccgcgggaa gctgccgacg ccgccgaatc cgctcgagcg 43020
ccgccgccgc cgccgccgct cacggggaac gatgtcgctg ctgtcgcacg cggtatggga 43080
gggcgccgcc gccgctgctt gggagatagg atatggagag agaaggaaat gtgagggagg 43140
gttaggtttt tccccatccg tatcttcagc gacacggagg cgatccaagc tgtccatcag 43200
atcggacggc tcagaacgcc tccatcgtca ggccgcgcat gcttgatggg ccgagggaag 43260
gccggagggt cgaacaaacg cagtcagagg aggagttgga ggaggtaaag tagaatttat 43320
ttgcgggctg agatagtaaa tggactgaaa atggcccata gagaaattgg gaattttatt 43380
taaataaatg ttgaaaaggt gtttatatta tcaaaattag aaattaagct ccgaaaattt 43440
taaaaaatat tcaaagagca ttattaatca tgattaattt aataaaaatt aaatccaacc 43500
atatcatatt atttcacggc gcacggtagg aaaatgcgca gctgttgtcg ctgacggtgg 43560
gagagaaggg acattgttta tttccagaac tatcttttat aactcccatg gaactttaaa 43620
ataaatataa tcattattat agcattagtt tttttctgtc ttttttttcc ccaagagcgc 43680
cgcgcagaag agatcgatcg cgatctccct gccccgacgt cgccggccga tctctcattc 43740
tctccacgcc ctgctcgtcg ccgatctcct acaccatccc tgccatctcc tccttcccct 43800
cccctctatc ctccactggt gccgcccacc tctccgtata agacaaactg cgttgcggcg 43860
ttggtttccg ccggcgctgc tgctgcacct gtcagctagg gcgggcatgg cgcgccgcgc 43920
cgcttcccgc gctgttggcg cccttcgctc ggacggctcg atccaagggc gaggaggccg 43980
cgcggggggc agtggcgccg aggacgcacg ccacgtgttc gacgaattgc tccgccgtgg 44040
caggggcgcc tcgatctacg gcttgaaccg cgccctcgcc gacgtcgcgc gtgacagccc 44100
cgcggccgcc gtgtcccgct acaaccgcat ggcccgagcc ggcgccgacg aggtaactcc 44160
cgacttgtgc acctacggca ttctcatcgg ttgctgctgc cgcgcgggcc gcttggacct 44220
cggtttcgcg gccttgggca atgtcattaa gaaggatttt agagtggacg ccatcgcctt 44280
cactcctctg ctcaagggcc tctgtgccga caagaggacg agcgacgcaa tggacatagt 44340
gctccgcaga atgaccgagc tcggctgcat accaaatgtc ttctcctaca atattcttct 44400

caaggggctg tgtgatgaga acagaagcca agaagctctc gagctgctgc acatgatggc 44460
tgatgatcga ggaggaggta gcccacctga tgtggtgtcg tataccactg tcatcaatgg 44520
cttcttcaaa gagggggatt cagacaaagc ttacagtaca taccatgaaa tgctggaccg 44580
gggggattta cctgatgttg tgacctacaa ctctattatt gctgcgttat gcaaggctca 44640
agctatggac aaagccatgg aggtacttaa caccatggtt aagaatggtg tcatgcctga 44700
ttgcatgaca tataatagta ttctgcatgg atattgctct tcagggcagc cgaaagaggc 44760
tattggattt ctcaaaaaga tgcgcagtga tggtgtcgaa ccagatgttg ttacttatag 44820
cttgctcatg gattatcttt gcaagaacgg aagatgcatg gaagctagaa agattttcga 44880
ttctatgacc aagaggggcc taaagcctga aattactacc tatggtaccc tgcttcaggg 44940
gtatgctacc aaaggagccc ttgttgagat gcatggtctc ttggatttga tggtacgaaa 45000
cggtatccac cctgatcatt atgttttcag cattctaata tgtgcatacg ctaaacaagg 45060
gaaagtagat caggcaatgc ttgtgttcag caaaatgagg cagcaaggat tgaatccgaa 45120
tgcagtgacg tatggagcag ttataggcat actttgcaag tcaggcagag tagaagatgc 45180
tatgctttat tttgagcaga tgatcgatga aggactaagc cctggcaaca ttgtttataa 45240
ctccctaatt catggtttgt gcacctgtaa caaatgggag agggctgaag agttaattct 45300
tgaaatgttg gatcgaggca tctgtctgaa cactattttc tttaattcaa taattgacag 45360
tcattgcaaa gaaggagggg ttatagaatc tgaaaaactc tttgagctga tggtacgtat 45420
tggtgtgaag cccaatgtca ttacctacaa tactcttatc aatggatatt gcttggcagg 45480
taagatggat gaagcaatga agttactttc tggcatggtc tcagttgggt tgaaacctaa 45540
tactgttact tatagcactt tgattaatgg ctactgcaaa attagtagga tggaagacgc 45600
gttagttctt tttaaggaga tggagagcag tggtgttagt cctgatatta ttacgtataa 45660
cataattctg caaggtttat ttcaaaccag aagaactgct gctgcaaaag aactctatgt 45720
taggattacc gaaagtggaa cgcagattga acttagcaca tacaacataa tccttcatgg 45780
actttgcaaa aacaaactca ctgatgatgc acttcagatg tttcagaacc tatgtttgat 45840
ggatttgaag cttgaggcta ggactttcaa cattatgatt gatgcattgc ttaaagttgg 45900
cagaaatgat gaagccaagg atttgtttgt tgctttctcg tctaacggtt tagtgccgaa 45960
ttattggacg tacaggttga tggctgaaaa tattatagga caggggttgc tagaagaatt 46020
ggatcaactc tttctttcaa tggaggacaa tggctgtact gttgactctg gcatgctaaa 46080
tttcattgtt agggaactgt tgcagagagg tgagataacc agggctggca cttacctttc 46140

catgattgat gagaagcact tttccctcga agcatccact gcttccttgt ttatagatct 46200
tttgtctggg ggaaaatatc aagaatatta taggtttctc cctgaaaaat acaagtcctt 46260
tatagaatct ttgagctgct gaagcatttt gcagctttga aattctgtgt tggaattctt 46320
ttctcctaca gtcctattag aggagggatc ttctctgtat gtgtaaatag cgaggtatgt 46380
atgccacctc tccgaattat ttttactgtg gttcctagac tgtaaacaag caattatgtt 46440
atgctgttga tgccagaaaa aacataaaag tttgtcgtta tctctactaa cggatcataa 46500
agggatttgt gactggagtt tcaaacttaa tgtgtctagg cagtaatttt gacattagat 46560
ccaaaacaat ttatagggtt tcattaaatt tcatctatgt gtactgttta ggtgttgaat 46620
agtttgactt gttttttaac tgaacaaaag atatgtctga agctttgttc tttaccaaat 46680
gcagtactga tcatcacaat atatttttta tggaacaaga ttggattgta tagaatggtt 46740
tctgatctga ttatcttatc tcaacgtatt attatgcaca tgtactaatc atgaaatatc 46800
tgatggaatg atgtttctat ttacctgtgt gaggcagcaa ggagtgagat ggataacacc 46860
acatactccc tctgtcccag aatataagaa gttttagagt tggacacgat tattaagaaa 46920
gtaggtagaa gtgagtagtg gagggttgtg attgcatgag tagtggaggt aggtgggaaa 46980
agtgaatggt ggagggttgt gattggttgg gaagagaatg ttggtagaga agttgttata 47040
ttttggggag tacattatta ttctagaaca atactgttgt gctcaagaag cgttccaaag 47100
atgtttcaca acctgtgctc gatgggtttt gagcttaatc ctggacattt cagtatcatg 47160
atctgtctca ttcttaaaca tggaataaag gatgacagca tgatttcttt gtctctataa 47220
tcttttggct acccacagat aatagctgta aatctatact actttaaaag gagtagtggt 47280
ggtggtgagt ggtgaatctg ccaccacccc accaccaact ctcaaaattc tgacatgtgg 47340
gatcactgtc aatcccttct ccaagacatg tgggatcact gtcaatccct tctccaaacc 47400
aattgtatga tagaacagtg gaaatcacgg acagaccatg gagctctcaa ccataatcat 47460
ccttgcgagt taataacaaa tggagcgtaa acttggcaag caaaaaactc aaattaattc 47520
taaaattaag ctctaggatt caaaatagat ttcctctctg cattgtgctg ttatgatttt 47580
taattccgta acaacgcaaa tgcattttgc tagtcttata aagaagggtt aatgcaaata 47640
ttctgattaa atgattgtat ctatgaagtt tgaatgctag tggaagctcc tttgaccatg 47700
ttttgttgtg cgagcattta agagagtgaa gagaatgctt ctttggtgct gttctggtat 47760
ggaaggatcc acagataaaa ttcaggttct actgcttctc tgcttgtaat tttcatgaag 47820
ctgcagtgaa taccttgttg accacttgat ctgttgcttt gaaggagaat atagtagtgg 47880

ccaaggttgg tgacggtgat ggtggcatgt gatcccccag atcttcagtg acccagagag 47940
gaggggacgg cgcgtggtga gctacaaggc atactcagtg gagggcaaga tcaaggcctc 48000
ccgtccgtag gggactccgc tgcatcaagg ccaactgctc cgaactgatc aatttctggt 48060
acggatcact tctcctttcc tttttttttt caccttaagc actctcttga ttcttcgctg 48120
ctacctccct taatttcttt caatatattg tggcacttga tcatggcgga gacccacctt 48180
ccagtgtgaa tggattttgt caaagaacta aatttattcc attagcttat tttccgatta 48240
catggaagac attcttttct ggaataaata cagaactaaa tcctgtttcc tgaataaaag 48300
ttgttagtgt gtggcatggt gcatttccgc gcttctaaat tttataaaac ctgttcattc 48360
aatttgaacc tgcatccaat ccaatatttt aggtgcagac aggtgcttgc ggtcaggtta 48420
aagaagttgg caaaaatgct tctgaagaaa ggttaattgt tgtttcatct caggaggtaa 48480
tatgcagatg attattccaa ttggcattgc cttgccattt ttatcacgag tctttacaat 48540
tttatatcct cctacatatt ctttccagat tccagatgat ccagtgtctc caacaattga 48600
ggcgcttatt ttgctccata gtaaagtaag tacacttgct gagaaccacc agttgacaac 48660
acggcttgtt gtaccatcaa acaaagttgg ttgtattctt ggggaaggtg gaaaggtaat 48720
tactgaaatg agaagacgga ctggggctga aatccgagtc tactcaaaag cagataaacc 48780
taagtacctg tcttttgatg aggagcttgt gcaggtaatt tatttggcca tacctacacc 48840
agagatccat atattacttt tataactgca gtttttactt gttaacattt cattgtgctt 48900
ttacatttgt tccaagcttt caggttgctg ggcttccagc tattgaaaga ggagccctga 48960
cagagattgc ttcgaggctt tgaactagga cactcagaga tggaagttct tccaataatc 49020
cgacaccttt tgcccctgtt gatggtcctc ctgttgatat cttgcctaac aaggaattca 49080
tgctatatgg acgatctgct aatagtcccc catatggagg gcctgctaat gatccaccat 49140
atggaagacc tgccattgat ccaccatatg gaagaccaat atccacaata tggaagacct 49200
gccaatgatc caccatatag aagacctgtc aatgatacat catattgagg gttgaacaat 49260
gatgggcctc gtgatcaggc ccggtcctga ggggggtcga atggggcgat cgctccgggc 49320
cccccgattc ccaggccccc cacctatctg tgcaacgagt agtagcgatc ttccagcgcg 49380
caacgtgagg cgatgtttct ccgtgatttc gccggcctgc aactgcgaga tcgcgagtat 49440
aacgatcagc cgatcgatct catctgccga ctgccatgct gatgccacac gcaagcgcag 49500
catatcagcc ttatcttggt tgatcggcat gctggacgag cacatctgtt gtcgcatcaa 49560
ctgctgactg ctatatatgt gctggtgctg aatcgatcga ttgtcgtcac ggaagtgaag 49620

aacaaccacg gcactgctgc ctgctgggct ctagccgcca tcagtaagta cgctatactg 49680
cctatctaga tctagatcga gattacatag tggaattatc tgtttataac aaaattacaa 49740
ggtatcaatt gataatttaa ggttataacc gtacaaactt cagtgatttg ctggtttcac 49800
attggttaga tttgtttcaa ctaatttggt acttctgtag ccttgtaatt tacgaatcta 49860
gtattaatat tttcttaagt attagcctgt tccttgatat tatgctgttg agaaagtatg 49920
caatagataa caaaaacaag taggtgtgtt gaggatgctc aagagtaata caggcacttc 49980
aataattctg atattatcag gacatcatca ataattctgc gcctacaaat cttcaaagaa 50040
aattttaata taatgcgtat gattttttaa atacgaatat tgattgctat ttaaagatat 50100
ttatattata tggtaattat tatttgaagg tttataataa aggcctccgt ttttagtttc 50160
acgctgggcc ttcagaatct caggaccggc cctgctcatg atccttacac cgtgtatcct 50220
gtagagtact tctctaaaag agagtaccct agtggaagta gcaaagttgc accatctgct 50280
tcatacgaaa gatatgcagc aactactcgc ttgcctaata gagaactgcc ctcatctatt 50340
agtcctggtg ccgattatat gtcctgccgt tcttatcttg accaagtacc tactgatagg 50400
tactctaata gggttacact acaattaggc ctcttgagag ccgggaatag taatgtgcaa 50460
caattaggaa tcaccagagc tggaaattcc aatgcttatg attatactga ggtacatttc 50520
caatgcgtta gcttgcctct tctttgcaaa tggccctcgc ctgatatgtt tccattagaa 50580
acatgaaacc atatatttga ctgttgcatt atgtctattt tcttccatga tggttcagac 50640
gtctgaaaaa aggacaaaaa tattctagaa tatgtcatgg tgatccaaat atatccttct 50700
gtcttgtgcc cactctaata tctatcgttg gtaacactat tcaattgtta ccatgttgtt 50760
gcaaacccta gattcagtta ttcagctgtt ctctgctgct gttgcttacc agtttcttta 50820
gttgggtgtt gatcttttct cattttttat ttccttgttt cctggttcac ctgctgcctc 50880
tctgatgcat ctgaatgtat atttttgttc tcttcagtgc ttaatagatt taaatttcat 50940
tcttttcagg ctgcggagct gatccatgga cgtgaggatt accgaagact gtcaggtctc 51000
actgggtatg gcttacgcag actgaatttt tacaggacac aaacatgaat tttgtcctca 51060
taatcattga gtgatgatct ctttgcaggt atccaggtgg ctctgtcgaa ttgtggattc 51120
caaatagtta actggagtct gtcattggtg ttggtggtgt caatctagct gagatccgtc 51180
tggtatagcg taagagaaac atcatgcact atccccagtc ataaccatgc cccaatggcc 51240
accaatagtt ttcctcgtga aaatctcccc ttgatcccag atctctggtg cgagagtgaa 51300
gttgcacgaa gcccatcctg gttcttccga gtccattgtg gagatccagg gcattccgga 51360

tcaagtgaaa gccgcacaga gccttctgca aggcttcatc ggcgcaagca gcaacagcag 51420
gcaggcgccc cagtcctctc gcatggccca ttatttttag taagctggag gacattcgca 51480
acaggggggt cagtggtcac tgcaaagctg agtttgttct tcagttcaac tgcagaaaat 51540
tgcagatcgg ttgccgtagt tgctagaacg gtacatagtt gccacctaac tgtagcgagt 51600
ggcataactt attgtgtgtt actgcccaat gttgtctctc cttgtgttca tggattcaga 51660
cttgtgattg tagtatttct ggatcagact ggagtaaaag aaaaaaaaaa aggaagacat 51720
gggtttaaca gtaagctcaa aacgttgaca gtagtaaaat aaaaggggtt tgttcacttt 51780
atttccaata tcaaccttac caacatttgg cgttgaatca tttataccac atcgcttgtg 51840
cagctgaatt tggggctgtt taaaagatgg tctcttggat tgctaattgc ctcgcggcaa 51900
gcgtggtacc ttgtacaata taaatataat tataactatt taatttcata attaaacatg 51960
ttgttacaaa tctctactat tataaaaatt gaagatgttt tttgccggta ttttggtacg 52020
tcatctgtgt atgaatccgt ttttaagttc gtttgctttt ggaaatacat atctgtattt 52080
gattcagttt ataagatcgt tcactttggg taatacagaa ggaatcatat aagaattctg 52140
tttaaaaaca ctcgtatagt aacttgagac gatcagacgc ctaactacag ctcatgattt 52200
tctaaatata tatatatata tatatatata tactagaaaa aatatatgtg tgttaaaagc 52260
tatcttaatc ttattattgt tatatatttt agttaacaag aaatctattg tgggaacttg 52320
tttggatata tattttttta aaaaaaatca tgagctgcaa ttaggaatcc aatcgtctca 52380
agt·tagcagg agggcgagtt tttttaaaga gatttcttat acgatttctt ctatatttct 52440
aaaagcaaac gaacttaaaa accgactcaa acatggatct gtatttccaa aaacgaataa 52500
acttaaaaac cgactcatgc acagatgatt aatttttata atagtagaga taaacgaact 52560
cccacagtga attttatttt aactgaacca tataacaata ataagattaa aatagacttc 52620
acccgttgca atgcacgggc attttttcta gttaaagaag aaataaaaaa acacaaaaat 52680
ttataaaatg taaaaaagaa aaatattata attttgttag aattattatt ataatataga 52740
aaaatagttg ccaaaatttc tcaacgaatg tcgaataaac tcagcaatgt catatattta 52800
aatatgatgg taatatttgt tcgcaaaact ttaatcttca atccttcaac aacatagata 52860
tacaacgtcg taatcgccaa caagcccgag tgaccataca ggatagccga gcggtggatc 52920
tgtactgttc ttgggtgaaa taaatctagt acattgtata tcttatctta atatctacta 52980
ttataaaaat tgaagatatt tcttcaaaga tttccatacg ttctctactc cgttacaata 53040
tcggttctac tccgttacaa tatcggtttt gtacaccccg cgcacgcgtt gtgtgttctc 53100

ccgttccaat acatgaagct agagtcttgc ttctccctgg tctggcaggc cctttttcca 53160
ccatccccac cagggccagc gggttacatt gaccgatcac ggcccacatt agtggatgca 53220
gccagccacg ctcttcacaa atcatgtgat gaacattagc tgagttaaaa tttatccttt 53280
gatgattgtt agaaatgttt ttttctccac atcttctctt tcaattttgg aaaaatagat 53340
ttcttgattt ttgtgctcgt acatcactaa taaatcagtt gttacccttc cacacattgt 53400
caatttacca tgtctatttc agctcttacc ttgtatagtc ttgactcttg agtcctcgct 53460
attgactaag ttgctacatg cctcctacaa atcaatagac tgccataaca atattttcta 53520
cgacatgatc catattagtc catgcaatgc aagtacacac acactactgc acgaaaaaac 53580
tatgcaccat aacttcaaaa ctaacatgtt agaatgacgt taatttttca ttacaattat 53640
attcatcgac cgttaattta ctaggcatcc tgtttaaaaa aaatattcac cgaccatacc 53700
cacatgttcc gtagttcatt aggtgatgga tcggtagtta cagcagctgg atttttatat 53760
tttggtcatt ttgaaaaatt tatttcgcaa atagactcct gaaaaaactt atcccagaaa 53820
tagtcccttt tggagcgtca gagtggctgg cgccgtggtc caacgggaca gcgccaacct 53880
ctctggcgcc gccccccgcc tctattcttg tttctctata tagagttgca aactttttat 53940
ttttgtttta tttttttgga tgttttttca ctcttagaat cacgatacaa ccaactacaa 54000
aaaaaattaa actcgaacgg aatatatcac ttagctagaa gtctgaaaat atagcatacc 54060
acttatctac tttgcacctt caccaaaatt agaccataac ttctttagta aaatcctttg 54120
atcagcatat taaacataat gcactctatc actaggtgaa attacttaat ctaattcaaa 54180
atataactac atgtagcctt gaaaaattct acatgccaca tatttcgtcc gtttgagttt 54240
attattttta tggttcgttc atgtgagttc ccaagtgtga aaaaaaaata aaataaaaat 54300
aaaaaagttg cacatcctct cctctgcatt agagaggaga ggagaggaaa aattctacag 54360
gtcacatatt tcgtccattt gagttcattt tttctatggt tggttcttgt gtgttcctaa 54420
gcgtgaaaaa aatatcaaaa aaataataat aaataaaaaa attcgggggg ggggggcgcc 54480
agccactctt aggggtgaaa acgatcggat aatatccgat ccaatctgct ccgaatccat 54540
ccgaaataag gatatggtat gggtttttag aaatctggcg gatatggatg cggatgagga 54600
tatggtatct ccgaaatacg acggattatc cgacattttt gtcggattat ccgataggcc 54660
ctttaccgga taatccgaaa ttatgaacac atgtaaccac tctatctatt gcatataaca 54720
taagttggtc catccaatga cctaattcat caattaccct agatttctta ctatgtggtt 54780
ttcaccattt catgtcacac ttgcgtagct gtatttttat aaaatggaca tcatgtattt 54840

atgttgttta gcacttaagc acataattat tacaatgggt cgtttattga cattgtgtta 54900
tttttacttg cattgctaac tcaatgttgt attgattgca tacacacgta acatctgata 54960
aaatttaatc cgtttctgaa ccgattccgc accatttccg acatctgcat ccgtacacta 55020
tccacaccca ctccgaatcc gcttaaaaat atggtttagg atatggtatg accactatcc 55080
gtccgaatcc gctttatttt cacccctagc cactctggcg cgcttcccct gccacctcag 55140
catcgtccca ccacgtcggc agaaggacgg cggctccagc cactctggcg ccacaaaaaa 55200
ggaccatttc tagcataagt tttttagggg gtctatttac gaaataagtt tttaaaagga 55260
ccaaaatgtg aaaaatccag gttacagcag actgtgataa gcaatagcta tattgcctat 55320
atatacacgt atatgcattg ctaatccttc aattttgtcc aattctttta aattgtcttc 55380
acctgttgca acgcatgatt ttttttctag tcttaacctt aactaatctt aataactaac 55440
taaaagattc gtatctttcc gatcgtcacc ttgtccatac gctaattttt cgtccgtccc 55500
ccctccccct caaaaaaaaa gggaaaaatc cattttacac cctcgaactc ttatgcttgt 55560
ctaaaataca cccccgaact ataaaaccgg gtataataca ccctcgagct atcaataccg 55620
gacagttcaa gggtgtatta tacctggttt tgtagtttgg gggtgtattt tagataagca 55680
taagagttca agggcgtaaa tggacttttc cccaaaaaaa atcccagtcg ttactttcca 55740
tcctgagaat cggagacagg gaaaactgaa gcatacacgc aaatagaatc aaagataggg 55800
aaaactaagc atatacacac aaatatatcc aaaaattccc atgcagctag atcgggtgcc 55860
accgttgttg ccaaaccacc acattgcaat gtaaatctaa gactaaagcc taaatcctat 55920
gctaagtcat caaattagac tcggttctac caatttggta atatatcaaa ttagacttga 55980
tttttactga tttgaggttc tcgaggtgtc acactatgaa acggaagttt ttcccgttgc 56040
aacgcacggg cactatgcaa tatcttaact aattaaaaga ttcatatttt tcctttcgtc 56100
acaccgatct ttcgtccgtc tgtaacatca cgtgcacctc ctctccaaat cccacatcat 56160
cataatccga cccaaaaaca aaatctcaat ctcaatccaa tcagaatcat cacaaaatca 56220
tccaaaatat caagagatga ttataggaga tggaggggtg agcaggagca acatcatcat 56280
cgcataaaaa ccccaaaatc aatcacaaca acgacatcat tatcacataa gaaaaacaat 56340
acaaacaaca tacacaatca acaacactgg cggatccagc cgaggggaca acggcgtggc 56400
agcgggcaga tcctctcggt cagatccgcc cacgggtgcc actgacgtcg ccgccgccac 56460
cggatccaag ggagaagctt cggacagagg gagagggggg tagaggaccg ctaaatccgc 56520
ccaccggaaa tgccgccgcc accacctccg tcggatttgc ccgagggagc gccgatgccg 56580

ccaccgccat cgcgggagaa gcttgggcac ggagggtgag gaggaggggg ggtagagaat 56640
cgccggatcc atccgctgga aaagcctccg ccggatccgc ctgccggaaa caccggtgtc 56700
gccgcctccg ccggattcgg tagcgggagc cgccgatgcc accaccgccg ccggatccgg 56760
tcggtgggag ccactgacac catcgccgcc gcctcctctg ctaccgacaa gggagagacg 56820
agaggggcgg gggcgagggc gggggacgag agggttagag ggagggaccg agtgggagag 56880
agagggacga gtgagaggag ggggacgagt gaataaggat gcgtgacctt atccactcgc 56940
gcggtcgcac cccggctctt tctctcgctc agctgttgcg cttgtggaga ggatgcgaga 57000
tttttttttg agtaaaatgc acgggcggtc cttaaacttg tagcggtctg tcatctaggt 57060
tcccaaactc tcaaaatgca tatccaggtc ctagaatttg tcaaagtgta tcatctagat 57120
cccaaaccga cacatcctct cttggatcct acatggcgct aatgtgactt gtcacatgga 57180
cgtgacacgt cttttttttt cttcttttct ttttcttttc cgttttcttc tcattcttct 57240
ttttttccat cttctgctcg ggtcacatag aaaggaaaag aaaggaaaat acaagagaag 57300
aaaaaaagaa aaaagaaaat ttttaaatgg gtctcattcg tcagtcaaaa ttatgccaca 57360
tcatgtccct gcgacatgcc acatcagcac cacgtagcat cctgaagggg ttgtggcgat 57420
ttgggaccta aatgacacac tatgacaagt tctaggactt ggatatgtat tttgagagtt 57480
taaggattta tatgacacac tactataagt ttaaggaccg cccatgccct ttactttttt 57540
tttttacacg gagagaatgc gaatttgttg gttagttgcg gctgagggtt tctcgcacgg 57600
agaaatttgc ggtgggagaa ttttttttcg aggttctttc tattgggaga agacgggatt 57660
atagggatta ttactggtgt ggtggcccct gttttctttc tttttcgagc ttctttccgt 57720
taaattcact tttctctctt caaggagcgt aggacatgac tgaatgcagc tgctgtaaat 57780
tagaaataaa aaagaaacat attctgtttt tcattttttt caataggtaa atataaagat 57840
ttttaagtaa tatttaaaaa tatatagtgc tgatcaacga cattgttaag tgagattttg 57900
ctgttactat cacttttttt tccattgggc tcacgtacgg cattaaaagt tttagttttg 57960
gttctctcct tttgagtttg ggcatatacc aatattgaga taggtatact aaagttcatt 58020
tggattttat tcgattcaac ttttttgggt tttgttcagt tcttttttac atgtttctca 58080
tctgaaatta ggaaattagg tttggtaaag tcttgaatag ataacgctgt tgacgtttga 58140
acatatattt atctatttat ttatttaaaa atatatgaat aatttttatt ttgttatgac 58200
ttttgtcggt gacatgggac cgggagtatc atgactagag gcttgggcag gagcgatcac 58260
ccacgtggcc tgatgtaaca tcctgaaaat tcccaacaat aaaaatcact aaaattttga 58320

actttttaaa acttttgcat catgctggtt gttatgattg ctattgcttg ccaaaccgta 58380
aatgatcaca aagaaagtaa agtaaggatc taaaatttaa gtaatagata aatttacgag 58440
aatataatat ttaattgcta accctacaaa taattacgca caagaaaaca aagccagaca 58500
aacggaaggt taattactaa tttaaattat ggattaatta ttaaatactt gaaccatgtg 58560
ttgcgtgcca tggcatctaa atacacatga aataatggtc atataattaa attaagcttt 58620
ataaaattat gtgaggtttt aattaagcaa ttagcttaat gttgtaccga gtcttaatat 58680
actatttata gaataaataa attcaaccta tccgtgtaaa atatattgct ataagttcat 58740
tcaatgtact attgtaataa taatggccac attaggatat tttaattaat tttggaaccc 58800
tcaaagcctc caaaattatc taggttaatt ttgaaattat acctcattta agtaatgcaa 58860
tagaaaaata tacataaaaa taaaatatgg gtaatattag aaattgagta aattttcatc 58920
taaattaaaa catatattgg gtaaacctcc tttatgtaaa aattaagatt tatagaatga 58980
aatttgtaca agggataaac taaaatcggg ttaaatagaa aatggcactg ttcattgcac 59040
tctaggtgct cgacgtggtc cctggcccta ttttccccct cagccgcgcg cgcctggctg 59100
cctcgcgccc cgcgccacgc cacccgcgtc gcgtcgccgc tgccgcgccg tcgccgtcgg 59160
ccgttccgcg ccgctcgtcc gtcgctccgc cgcctcgcgc cccgcgccgc gtcgtcatcg 59220
cgtcgccgtc gccatcaccg cgcctggccg cccctgaccc cgcgccgcgc cgcgccgtcc 59280
cgtagccgcg tgcgcgttcc atcgccgctg ccgcgccgcg cgccgtcacc gcgcgccgct 59340
cgtccgccgc gcatagcccc gcgccgccgc gccatcgtgt cgccgcgccg tcgcgtcgct 59400
ctcgagcccc gcatccctct cgagccccgc acgtcgcgtc ttgtcgccgt tgctgccgcg 59460
tcgtcgtcgc cgatgctgtc gcgtcgccgc tgccgcccgt cgcgtcgcct tgcgccccgt 59520
gccgccgctg ccgcgttgtc gctgtcacct tcgcgtcccg cctcgtgccg cgcgccaccg 59580
ctgccgcccc gtcatcgccc gctcgtcgcg cgcgccgccg ccgctgccgc gccgtcaccg 59640
tcgtgtcgcc gtcggcctcg cgccttgagc cgccgcgcgc ccgtcccctc gcgcctgcgc 59700
cccgccgcac ggccgtcccc tcgccgtcgc cctgcgccac tgccgcgccg cccgtcccat 59760
cgcgccgagc cccgtgccgc cgcgcgcgtc gcgtcgcccc gcctgtcacg ccgctcgccg 59820
cctcgagcca cacgcgtcgc gccgtcgcgt cgccattagg gccggccacc cctttccccg 59880
cgccctataa aaccccccgg ccacccccct ttcaccccac accatcccca cccattcccc 59940
tcttcctctc ctccttcccc tcttcgtccc ctccaccgcg ccgcgccgcc gccttcgtgc 60000
cgccgcgccg tgcgccgtcg tcgcgccgcc ctcgcgccgc cgcaccgccg ccttcgtgcc 60060

gccgcgccgt gcgccgacgt cgtgccgccg tcgccgtcgc cgtcgtcgtg ccgccgtcgc 60120
cgtcgccgtc gtcggtaagc cgccgtccct tccctcgttc cgacgccgtc gccgcccggg 60180
tgggaaggag ccgagagaga gagggaggaa ggagccggga gtaggaagaa agaaaagaaa 60240
agagagagag agaaaagaaa agagaagaaa agagaaaaga gagaaaagaa aagaaaagag 60300
attagagaag ggagggaaga gtgggcccca cctgtcatta gccccatcca attcccctta 60360
gaaaaataat tctgtagaaa agaaaatcaa gatcttgacc ccacctgtca gtcactatag 60420
cgtgtggata aggttgtatt aaaaataaat gaattaggaa cagtactatt tcgcaactat 60480
tagaattaat tcaaatttga atctttacac tagcataact aattcatttt agctccgatt 60540
tgagtggaac ttgaacctaa attcatctaa attcataagc tttccaatgg tatataattt 60600
actattaaat aaaatatatt tataattatt aagtaattaa tatcatatga ttaggttatg 60660
gtcaacttaa aaatatgcta ataaataaaa ttagtattgt ggatgtaata atatttgtct 60720
ctaacatgtc ttgccactgt aacaaccaca caaactaata ttaagtgatg tctgaaatga 60780
atgaatgaat aggaaaatac tagtacttgt ttaatattcg atagccatat aattaaaccc 60840
atggcttata ggttatttaa atcaaatgta gccttgtgat tatgcaacta aaatataaac 60900
acatatagat gaatctttag cttgattagg aggaataata acagagctag tgtgactagt 60960
tatgatatag cttgttgtcg gttgcctata tttagtaaat ggttcaatgt taatacactg 61020
atgcacacac ataccctttt tgataaccta ctagttgcat atattaaact tggtaataaa 61080
tgaagaacca atatattagc taaatactgg tgctagttat aaatcttgac cacacataat 61140
tttagttcaa accacacctg aggattgttc gttataaagt tataaagtta taaagttata 61200
caaaagataa tatgtaacta taatagtatt aaaccacaaa tctaaaatac agggcgcata 61260
attgtcaacc ttttatgcaa acggataata tccatatata tacatcatgt ggataattcg 61320
aataatagct ccattggtaa aataataatg taggcgaatc atggtgatga gatggtttat 61380
cctaaacctc cccatcgaca tagccatgct atagggacct gaccatttta ccttcataac 61440
agatctcttc cataagccaa tagctagact aaaccacaga ttagcaaatg tgtacatcat 61500
atattgtgct agttagtacc aatagaacca tcaggacaat ataaatacta aggaatctta 61560
gctcttagct tgattagaat ccaatagcaa acacgagtag tatgagcagc cttaggttcg 61620
acctcaataa ttatattttg cttgtgcata attgcttctt gttgaatatt ggtttttctc 61680
gcatattata gaaattgtat atcggttagt cgtgaggcaa cgtatgcagc tttcaggagg 61740
tgaaggttga tcaagattgt atcaagaata atgactattc taagcaggca agtcatcact 61800

attccttgaa catgttgatc ctaattgcga aattattttg tttacaaata aaattgcatg 61860
caatgatgaa catcctactt gtgattatgc catgccttga ttattgttta cccttaaaat 61920
ccttgtaacc atgattacgt atgagtccct agtcaattat gacaattgct tagagatgct 61980
attctagaat catgcatact catatttatc aaatgctata tgcttgggca attacctttg 62040
ggaaggtaat tgagatgcgg catgtggaga catgaacgcc acattgccat gatattaatg 62100
acatgatttg tgaaaggaga aataaaatta aacaactgtt ttcgactggg gcggacggag 62160
gatttgggtg gtatctggaa aaggctagta ccgtccccgg tcaattaagg accgagccat 62220
gaagttaagc atgaaacgac ccccgtacaa ccgcacttct cgtatgggta tagacctagc 62280
ggagtagata gctgagcgga ggcagtatcc atgcatagtg gtttcttgat gtgtgaggca 62340
ggggctctac ggtggggcag ccattggtag gaccgcaagg cgggtatcta cagtggtgtc 62400
gccatcggta ggactgccat gtgagaatct aaaacataat tataacttaa tgcatgtgtg 62460
agtcttccct tcccgggtgc gccagaactc ctctcactgc tagaaaccgt gtacgcctag 62520
agtgcatgag gatgaaaagt tcatggagcg ggtactgcca atgcgaggtt atcgaaaagc 62580
tctgccgtga cgcatctcat gtgttgggac gaggctcatg tgttgggcag tcgcggagtg 62640
cgggtaaagt gtacatccac tgcagtgtga gtaaaccaaa tctattcgaa tagccgtgct 62700
cgcggttatt gagcaccggg acatgtatta cacttggcta gactctaaat tcttaacttg 62760
tggggaatgg gatattgcat gatgaatttt atgctgatgg agccacatcc cgagaggagg 62820
gaaggtggac atcctcagaa aaccatgacg attcaatggc gggaagctat ccttgggatc 62880
acaatggatg gtggacagaa ccgtcgttgt ttaaagtgaa cactggtact aaaatttgat 62940
cgatctatgc taggttttag gcttgtgaaa agaattgtaa aattagcttt atgcaaaagg 63000
acctgaagcc attccttgaa ataccctcta tcatatgcat tgttattatg gtggcttgct 63060
gagtacggtt ggtactcacc cttgctattt atatatcttt taggagagtg ttgaagagaa 63120
gcccttgtcg gtacgcttgc gtatcccaca agatgatcgg agtgcggtct tgttctaggt 63180
ctcgtttccc cagtcgactg cctgtggcat gttaaccggg cccttatatt atttgtcttt 63240
tcgctgttgt tctctgatag ttgttggcct acctggccct aatgtaagta tttaactctt 63300
ttagcctaaa ttcattcgtg atatgttgtg atccaactat gtatgtgtgt accaactact 63360
gatccaggga ttggtacgga taaacacaga agatttccga tttccaaaat cgggggtcta 63420
cacctgaccc cctcaggggg ggggggtcgg gcccgagggt gatgtggccg cccccctctt 63480
tgtctccccg aggggtcgga ccgctcccgt ttctgccccg agggctgagg cgccccgacc 63540

ccttgtgggt tttgcgccgc gtgtatgggt taggtgagca caacggggct cacctaaccg 63600
tatttattgt ggtttggacg agcgcgtcac gccgcatgta gcgcagtgca gcgcgctcgt 63660
ttatccggtc tgtgaccagt cacagaccgg tcagatcgtg ggttaggtgg caacaggcgg 63720
tctgacacac gcctcgcccc atcccgtcag gataagagcc tccaggcact tgtccctagc 63780
ccggagccag catgctaact cctggagatg acacgttggt cccggtcaga tatatgccag 63840
gcttcatccc aaccattaca agcaagatat tgtatgaaga agggcgaaca tgcagattgc 63900
tggactgaca cgtggtggac aagaatgacc gatttgtgac cggtctgaca ctggtcatgt 63960
cgtcggcaga caaccatgtt cccacgttgc acctgctttc ggcggagtgg aggtaggtat 64020
gggccatccc atcagaaggt cgttcggaca gcagccattg caagtctccg cccatttatg 64080
aagagatgac agggtgatcc cctggagaga aaaaaaggag gaccttgccc acttaggagg 64140
tgaggacgac tggaagggga gaggatctgg agagtagatc ccacgagagg aaaaaaggga 64200
gaagagggtt tctagagtaa gagctctctg actctccagc tctttgtagc ttcttcgtac 64260
acagatccac cagaaaatag gagtagggta ttacgcttct cagcggcccg aacctgtata 64320
catcgcccgt gtcttgtgct tttttcattc tcgcgaactt tccacagact aggagcttag 64380
aatctcgccc agggcccccg gccgaaccgg caaagggggg cctgcgcggt ctcccggtga 64440
ggagccccac gctccgtcaa ctttggctta taattaaaaa tactctaagg atattttttt 64500
atattttatt ttcttatgtc tatatgaaat tttaaataag atagatggtt aaacatatat 64560
tggaaaaaca tatatccaaa agtccactat cacaagcgta gcatagatac gattacaata 64620
cgtttccgcg aagactgttt atacctactc tattccctgt tccttgtgcg gttgtgccat 64680
ttggggctgt tttttcatct cggattaact cgcgtggaaa ccgcgagacg aatgttttga 64740
gcctaattaa tccgtcatta gcatatatgg gttattatag cacttatggc taatcatggc 64800
ctaattagac ttaaaagatt cgtctcatga tttacatgca aactatgcaa ttagtttttc 64860
ttttatctta tatttaatgc ttcatatatg tgtccaaaga tttgatgcga tgttctggga 64920
aaatcttttt ttaactaaac atgcccaagg tgtttctcca attaagttga cccaaaatca 64980
ttcggcgtca cctttgtctt tcactttcct tccactacaa ggtgatgaca ctgacaaaag 65040
gtccaaaagc tacaggatct gatttttgtt catccatctg tgatgtgtcg gcaagccatc 65100
catggagttc atccactcaa ctcctctctc tcagagagag agagagagag agagacagac 65160
agacacatgc atgatagatt gtgctagtac ggtagtaaca ttttattgcc tccttttcta 65220
aaattctagg ttgtttggaa aacaaaaatt ctagattgtt caataaatta ataatattag 65280

gtatttattt taagtcactt taggtgttaa tttttgaatt ttaaactgct taaactctct 65340
ttcgacgcat ctgagagcag gtacaatagc agactataag ccagctataa atatatttta 65400
agtagataaa agaggaaaaa taagagtagc gggctataga tttgtagaca gctgcagcgc 65460
gagctccaag atacatatgt gtatgacatg tgagaccaaa cattaattat gtagtatatg 65520
tttatatgta tctattgtat gaattggcta ttaaattgac tatgggtgtg ttcggaggtg 65580
ggtgttggga accatctccc aagcacggaa aacggagcgg tccattatgg cgtgattaat 65640
taagtattag ctatttttta aaaaaataaa tcaatatgat ttttttaaac aacttttgta 65700
tagaaacttt ttgcaaaaac tcaccgttta gtagtttgaa aagcgtgcgc gcggaatatg 65760
agggagaggg gttgggaacc tcctcatccg aacgcagcct atacatgatt tggagccaat 65820
agttggctat aatattaaac ttgctctgag tggctcttga atcatcgaag tgatagaaat 65880
catatgcaga aatgtttata tttgtgatgt aaaatttgaa tctaaattta tttatatttt 65940
gaaatggagg aagtactacc taaaacaagt atgagaaaga gacatgaaaa acacaaaatc 66000
tagacttaaa aataattgga attactagca ggaggtcgaa gtcaatcaag acggcgaaga 66060
aaagcacagg ggacagcaga cacgttaaca cgtaagtaaa caaacaagtg gttaattaat 66120
taggggggccc tcaagtctcc cctaaagcca ctaaacatga caggtttgtg taccatggaa 66180
aaaagggtga agcaaaactt tattctctct ctcattagat taccagttgg aaagcaatcc 66240
tgggacctct agctaatctc attattgtag aacaacgttt tcttagagag agagagagag 66300
agaaataagt caataaaaat tactactaat ccacttgaac cagttctgtc ggtgtcggat 66360
gatttaccac atttgacgaa acggactatt tattcgacgt ttcgaaaaac acactttttt 66420
agaaaaaaaa aactttcctc tattagccac tcgttttagt tatataccta tccgagtatc 66480
tgttaagttt atttatcaaa atatttaatt tatctctata attaaatata caatccgtaa 66540
aaacaatcac gcagtaattc gtttcaaact gagcctcagc tagaaaatca aaatggaaat 66600
gaataacaat agcaacagta gagttagttt ttcggcttat catccgcaac ccaaatgcga 66660
attttaaact tagccttaga gttaattttt aaggcttgtt taccatactt catttcccca 66720
gcattagttt cttttgtcac taaaaattgt ttttttaagt tgtttcgttc attttctcac 66780
ggtttatcag cagtagagcg aagccattct tggagcctgt ttggcacagc tctagctcca 66840
gctctagctc cactctttct ggagctggag ctcagcccaa cagttttagg tgcaccaaaa 66900
ttaggagtgt agttgggtgg aactctctca caaaaaattg tggagctgga tttagacagc 66960
tccacaactt cactccaaac ccaactcctg aagttaaatt gataagttga agctctatct 67020

atcaagccct ttttcttgat catgcttcta cctactccat ttttgtttct tggccctcac 67080
aggaattgga aaggaaaggc gtatatgcat caatgcatgc atgcgcacat caacctcgtc 67140
catcaaccat cataatcatc atcatctcgc cagctgacga aaatgacctg catccatcca 67200
tcacggacaa tccaagcgaa caccgctacc aacatcacag ccaacctgtt tatcactagc 67260
tcttgatacc actcctacat aaacactacg cgcaggttaa ttaattaagc gtgattactg 67320
aagtaacatc taatcacgtc ctggttagcc tttaataaga caacagttag agcaggtaca 67380
atagcagcag gatataagcc agctataaaa aaagagagaa aagagcaacg ggctacagat 67440
ctatagccag ctgtagcatg gacttcaaga cacaacgtgt gtataacagg tgggaccaga 67500
taataatagt gtagtatagt aagtaactat tatatatatt gactatagat gatttggagc 67560
tattagtgtg ctatagtatt aaacttgctc atagagcagg tacaatagta ggatattagc 67620
cagctataaa catattataa tgagataaac attgatagag aagagcagcg ggctacagat 67680
ctgtagccag ctacaacacg gactccaaga cacaacgagt gtatgacaga tgggaccaga 67740
tattagtagt atagtaagca actattatat aaattaacta ttacattggc tatagatgat 67800
ttggagttag tagtgggcta tactattaaa ctttttctct tagcaaaaat caagcgccta 67860
atcacattag aggagtagct ttgagacaaa ccaattagcg gcgaatcaag cgatctgcgt 67920
ggtcgtacag tgatgggccg ggccgggccc acagcccgac agtgacaggg ggcctgacgc 67980
atgtcagcct cagccctgga cgggagctag ccgttgtgtc cccgggggag gggagggggg 68040
cattcccatc atttcgcccc tcctccgggc ccacatctca gtgggggtaa aggtgtaaat 68100
tactgcgacc gcgagtccag cgagcctaga tttggacctt gtgtccgttt gactgaaccg 68160
gagctactcc ccaatacggg gggattgcgt tgtgtgcatg ccatgtgggc ccgagcgccc 68220
tttgttcgtg gctttgggtt ggaaaggtga ccgtgtgagc tgtgcggtgt tgtactacgt 68280
attagtataa atcatttttg ggtactactc cctccgtcca aagcttattt ataatttgtt 68340
gtactccaac cgtccgtctt atttaaaaaa aatataaaaa aaattaaaaa aataagtcac 68400
acataaaata ttaatcatgt tttatcatct aacaataaaa aatactaatt ataaaaaaat 68460
ttcatataaa acggacagtc aaacattgtc acgaaaatct aatgtttgcc ttttttttta 68520
agaccaaggg agtatctacg aacaaagata atacatgtta taatcatgaa gcccatgatg 68580
tgattagccc ggccgtttga ctaacctcac gagctacgtg gctgacaagt ttaacttgtt 68640
aactccatca tttcggatac ttagagcatg tacaatagca gactattagc cagctataaa 68700
catattttaa tgggataaaa gatgagagag aagagcagcg ggctacagat ttatagccag 68760

ctgcagcacg gactccaaga cgcaatatgt gtatgacagg taagaccata tgttaatagt 68820
atagtaagca actattttat aaactggcta ttagatcggc tatagataaa ttggagctag 68880
tagtggacta tactattcaa cttgctctta tatgatataa atattgatat aactatatga 68940
ttttgttaat gacatgtttg tttatggatg gactatgtgg ggtcggtcgc ctccgtagct 69000
gaccaaaata caaacttaaa acccctatct ataaaaatct aacttttgtt tataaatata 69060
gatataaaag ttcataatta gagcctcatc ttttaaacga aaagagtact atgaaaacaa 69120
ctcgtaatac aaagactaat tacgacgaaa agaaaatagt actgacaaga ggaaagcagt 69180
gaacttgcat actccctccg taaaaaaaac caacctagac acggatataa cactatatat 69240
ctagattcgt tcgttgtaat gaagtgtcac ctccgtatct aggttggttt tttcgtacga 69300
aagaagtatg agtaaatcta aagctatgta tacccttcgt caaaaaaaaa aagtaaacct 69360
tgtactggtg cgtgtcacat cctaatataa tattgttttt tatggagggt gtacagttga 69420
aaaaaattga tgtgttttaa ggatgaaaaa tattggtaat gttggctatg taactctaga 69480
aaaaaaaatg cagtaataat aaaatgctaa tttgctggag tactagatta tagacaatcc 69540
agtccaggac acgacaccct ccctactctc tccacttcca ctctcaccgg ccaccgcgcg 69600
ctctctctct ctctctcccc cttctcccgc aagattcttc ccccaaatcc cacccgatcc 69660
accgccgccg cccgctcgcc ggagtcccat cgctgccacc gccgccggag ccgcggcccg 69720
acgcccgccg ggcctgcttg ctgtgtgtgt gaggaggtgg agttgctcgc gctcgttccc 69780
gcggccacct ccgcctgctg ctgcttctgc ttccgctggc attgcgggga ggtcgtgtgc 69840
cgggggacgt gggggctcgt gttggagcgc ggctgccggt gaggtggggg gtgcggcgcg 69900
gcgcggctcg cgctcgtgcg ccggtggcgc gggcgcgggg ggaagcgtac gggggagggg 69960
gagtgtggcg gcggcggcgc gcggggtagg gacgggcgcc gccaccacca ccggctcgtt 70020
cgctggcagg cgctacgcgt ccagatccgt acgccggtat gcttcgtctc gccgcaactc 70080
tctccatttg attagtatcc cctcgccgaa acgaggcctg tgaggcgccc gctttctggc 70140
tggcttccct gtactcgctg cttgctcctg cctgttgggt taacccgttt ccatcgaatt 70200
tgggtaagcg aaacatcgcc tcatatgggc atttggggtt ctggcagcct taggctcgcc 70260
atccgtcgcc gagcttccaa gtgaccggcg cttgttggta tatttgcttg cttgttcctg 70320
tttggtggct gcgctaaatc ttttgtgctg cattgaattt atgccaccca tatacagcaa 70380
attactgagc tgaaataatt cggctaatta ggtccagcaa tatgacatct cgtggattga 70440
atgctaagct gacattgtat cactgatgct ggcttatata taggttgttg agaagtgaag 70500

atgtcgacag gtgaaaccct gcgtgcagag ctatcatcca ggacgccgcc tttcggtttg 70560
aggctatgga ttgtgattgg aatcagtatt tgggtggtga tcttctttat actaggtttc 70620
atgtgcctct ggtccatata ccgaaggaag ccgaagaagt cctttgataa gattccagta 70680
tctcaaatcc cggatgtttc caaggagatt gcagtagatg aagttcgtga gcatgctgtt 70740
gtcgaaaact tccgtgtgca agaaagccac gcgatatcgg tgcaggagaa acattacgag 70800
aaagattcag ggaaaatgct ggcacacttg gttaggagta aatcgagtga tgccgataat 70860
ttgagccaat gcagctcggt gtaccaatgt gatagggctg gtagctcgta ttctggtgat 70920
gaaggcagct cgggcaatgc taggaggcac ttttctcaat atgcaactgt ctcagcatcc 70980
cctctggttg gtctcccaga attctctcat ctgggctggg gtcattggtt tactctgaga 71040
gatttggagc atgcaacaaa tcggttttcc aaggagaatg tcattggaga gggtggatat 71100
ggggtagttt accgtggtcg actcataaat ggaactgacg tcgcaataaa gaagcttctt 71160
aataatatgt aagagatcct gaaatctatt ctgcgtttta cagaacttgt gactccttct 71220
gatgccatca tattaatttt cttttgatat ggtgctgcag gggccaggca gaaaaggagt 71280
tcagggttga agttgaggct attggccacg tcaggcataa gaatcttgtc cgccttctag 71340
gatattgtgt tgagggaatc cacaggtaaa gctatttatc aatcaccttt gctgatggat 71400
ggctagcttt tgtttctact ggcacattat ttacttgcat agggatgtag gattgctctt 71460
ggtctatgtc cacctactca ccagattatc tcaagggata ggttattcct gactgcactc 71520
cttatgctat cgattttttc ccttccaaat ctgatggtgg gattcagcat gcccagtgac 71580
agattatgct cagtccacag aaaccttctt tggaccacca ttcttttacc atgaaaatgt 71640
ggccatagct ccgaaagcta ggattcacta gaagcgcaca actgcttatt ggtttgttag 71700
ttggctataa caaggtctta ctgaaatgta cttccatagt tcattacttt gtgaatgcct 71760
gttcttgttc ttcacgtttc ttctcatgca tgttcaattc taaatttgta ttcatgatat 71820
gtccaagcta ctgtattctc caaagaaaat cagaagtcca ttcacctatg tattttccag 71880
ttttccgcca ttttggatac tgctctagaa acaagttaat aatatagata tttatatggt 71940
ttggccagtg ctgcttaagt gaccatcgag atagaaattg cttaagaaat atactaagat 72000
gttgagtgtc aggtgttttc ggataatctt gttaccaaca aataggtcct atgaatataa 72060
tggtgtctgc ttcacgtaat tcaaaatcca cactcagcca aaataatctg caatagggtg 72120
ttgaaaatat gattatgttt ctcccttgtt ttcatcatga ctacagaaat gaacaatgtt 72180
gctacatctt gtaataattt gtggttttca attgaacaaa acatccatca aatgatatct 72240

acagcaatat attttgcact tctgagcaca caataggttt gagtgtattc gagtcatggt 72300
cattgattta agctttttat ttcactacat aaccattgat ttgagtgtat ctaaggagtt 72360
ctgtttccac aagtacttta tgttaatggt gtctccttat gctttggcca tccaaactca 72420
ttactgttgt ttaatatttt tagtggttag tggtgtccaa atctttcttt gtgtacatca 72480
tactatgttt ttgtagtcta ttaaacttcc atcctatcat ctgacttgtt atattccagg 72540
atgcttgtat acgaatatgt gaataacggg aacttagaac agtggcttca tggtgccatg 72600
cgccaacatg gtgttcttac ctgggaagcc cgaatgaaag ttgttcttgg aattgctaaa 72660
gcgtaagaaa caaaccatcg tccccgtcaa aaagaaaaga attgttcttc actttagctc 72720
ttttatatgt atatgtttag ttgcataacc cattttccat aactgaattg gtatacaggc 72780
ttgcttattt acatgaagca atagagccaa aagttgtaca ccgggatatc aaatcaagca 72840
acatactaat cgatgaagaa ttcaatggca aactttctga ttttggcttg gctaagatgc 72900
tgggtgcagg gaagagccat atcacaactc gagttatggg aacttttggg tatgttgata 72960
tttttttgga gttagtatta atctttccta tgcttagctt ttactgttgg aatgtgcagt 73020
acttcgctta ttcatacagt ataaaatttt acatgctgcg aactttgtcc ttcgtatatt 73080
ataacaggta gctttctcat tgctatcatt gattcatttc aggtatgtgg cccctgagta 73140
tgccaacaca ggtctgttaa acgagaagag tgatgtctac agttttggtg tgctattact 73200
ggaagcagtg actggtagag atccagttga ttatggccgg cctgctaatg aggtgagcat 73260
atatcctaca atctcatgcg tattatgtat gttacaaaag tccgtactat tggaaattat 73320
tttacggcaa aataacgtct atactaggag agacgaattt gcttcaggtg tatggctgtc 73380
tggcagttgt ctactgtcta gttacccttg tctcactttt acagtctatt gttttatttt 73440
tcaggagctg actagctgta taccttgtca tatataacaa cactgtaacg tggatgcctt 73500
gcaggtgcat ctagtggagt ggctcaaaat gatggttggc acaagaagag ctgaagaggt 73560
agttgaccct gacatggagg tcaaaccgac cattcgggct cttaagcgtg ctctcctagt 73620
ggcactgagg tgcgtcgacc cagactctga gaaaagacct actatgggtc atgttgttcg 73680
gatgctcgag gcagaagatg tcccatcccg tgaggtggta acgctttctc ctttcctgca 73740
ataacattca tcatattata tcattgcaat aaatctgaag cttttgctgt aatcctactg 73800
aaggaccgga ggagccggag gggcaacact gccaatgcag ataccgagtc caagacaagc 73860
tcaagcgaat tcgagataag tggcgataga agggactcag ggccatcagc aaggtttcaa 73920
ctctaagaag acggtgatca tagtcaagaa caatggcttc aaaactctat gcagtaacat 73980

ggtggttggc agagaaaaag gggtatttct ggagggcatt gcattttgta ttgtaggtct 74040
gcatggcggt agagactgga gagagcacag tgtctgatga tggatacccg gagacctgta 74100
attcccattc agtattctgt ttgttagtca agcagcttgt acagatcgtt gtctgttcca 74160
ttttttcatt cttctggttt ttttgtttag gaggctcttg gattaccagt acgaaccgct 74220
gtctcttttc tagaatcacc aacatggaac ctatcaatat ttactactag tactacgact 74280
tgctttcttc ttgctgagat ctatcatgta ctgtacataa ctgacgtgtt cagctgcact 74340
tggacaagta gatgctcgtt ctgtatgtcg aatttacttg atgaggtcga gcattaagta 74400
ccatggctgc agccggcttc tgtttagttg tgctgacatg cggcggcgac ctcacgctgt 74460
gtggcccatt cttgatcttg ggccgaaact gtagcaacgg gcgtacggcc catctatatc 74520
gggattgttc ggcccgttgt agatgggccg gatcgggatt gcgacttacg tgcgacccat 74580
ttcggttggg ccggtggtcc gctacttcat ctagcagtgg tcggcggcag ggttcacaat 74640
tccaatagaa tccaaacatt attggattga gttaaaaaca caaaccaatc ggctttttgt 74700
caggttcaga aaattttaaa ctgaatttta attttttgac aaaaatctat ttagatttcg 74760
tctgtttttt taggtttgtc aacggattca gcgaaatccg atgatatcgc tcgtgagtgg 74820
atttttgatc cggtatcgag attgtgaacc cttgtcgcgc attgcctgac aaagacaacc 74880
agtgaagcgc cgtgcgcgcc gcgtgcgcgc cgcgtgacgc gaagatgcgc aggaaggaac 74940
aagctggcaa gcggcgcgcc catgacggcg gcggcgacga cgacccgcgc gcgtgcgtgc 75000
gtcaacgcac gcgaccggcc gagatccgtc agtggccgcg gctatatata atacatcgtc 75060
gcctcacacc ccccacacac cgagtcatcg ctcgccggag ttagagttcg tagcggcgaa 75120
ggatatagcc atatattata gatggcgatt ggtgttggtg gctgctgcgc cgtgctgctc 75180
gcggcggcgc tgctcttctc ctctccggcc accacatgta agcacgccca tcttcttctt 75240
cttcttcttt ttttctttct tttttttttt tttttggaaa tgagccgcag ctgacaaaaa 75300
gatcactcac acatggatac actgtcgtga cactaaccaa tgcctaagcc attttgtttt 75360
cttgttttgg atttttcttt ttatgtgtat cacttttgct tgttgctctt gcagatgctt 75420
atgattccct ggatccaaac ggcaacatca cgataaaatg ggatgtgatg caatggactc 75480
ctgatggcta tgctgtaagt agcggtggca gtacaccaac atctctacct ttattttcgt 75540
ctcaacctgt acatttacac tatcttgttc tactacctct aataaaaaaa tatatttgat 75600
gttttaaaat ctattaagtt ctagagatta ggaaagctac acatggtttt atgttttgat 75660
actattaagt agtatatttt ataagttata ttgaaggctg gggtttcaaa agtttgacta 75720

cactagatct tattcaaagc gtctaatgat tactgaacgg aggaagtatg aacttataga 75780
cttgaagtta aacagcatag ccacatctct tcatgtatac ttcatccgtt tcatattata 75840
agattttcta gcattatcca tattcatata tgtgcgtcta gattcattaa tatctatatg 75900
aattgggcaa tgctataaaa tcttataacc tgagaaacgg agggagtatg tcgcaaacaa 75960
caacaacaat aacaacgagc aaaatctgta tcgaatccgg tttccctctt gtaactgtat 76020
caaagatctg tcctctgaaa cgtcccctgt tcatcaggcc gttgtcacac tgtccaacta 76080
ccagcaattc cggcacatcc agccaccggg gtggcagctg gggtggacat ggcagcagaa 76140
ggaggtgatc tggtccatgt acggcgcgca ggccatcgag cagggcgact gctccatgtc 76200
caaggagggc agcaatgtcc cccacagctg caagaagcat cccaccgtcg tcgacctcct 76260
cccgggcacc ccaatcgacc tgcagatcgc caactgctgc aaggctggat cactgagcgc 76320
attcagccag gacccggcaa attctgccgc gtcgtttcag atc 76363

<210> 28
<211> 53905
<212> DNA
<213> Orza sativa Asominori
<400> 28
gatcagtgag tgagagtgat gtgctattga ttttcgtcta ggattttgct gtgctcttct 60
tcttcttctc ctctctacca agaaagatcg atggaggaga atttgtagga cgcgtttctc 120
acgaattact tagctgttaa tgatcagctt gatgtgtacg atatgatggt gcagagtgaa 180
agttgtgttg ttcactggtg gatcatggga tgggaatatg ggattgttgt aagatgtaac 240
tcaagtgttt tcttttttgg gattactttt ggtaataaga gcttgggtga tcgaaaacta 300
cagatggttt ttcttttaag ttgtatgatc tctgtagagt ttttgagtaa tttgtagttt 360
tgtaccctat caaagatcat ctctagctgc ctctgagctc tccaactcta tatgtccatc 420
tctagtatat atgtcccata tttctgactg aaaattttca agtcggttgg ttccctccgc 480
ctggatattc tttcagctaa ttagattttt tttaaatgat aaatttgcta aaagcttgtt 540
caaattcagc taagatctat tcaaacttca atttctctat cgaaattccc ggaaatttca 600
attcaatcat tccccaatac atgccgattt ccgtaatatt gaaccatgac atgtaaacaa 660
cgaaggaatc aagggcatat ttagtttcat ctcacatcga atatacggac acacatttga 720

agtattaaat gcactctaat aacaaaacaa attacagatt ccgccagaaa actacgagac 780
gaatctatta agcctaatta atacatcatt agcaaatgtt tactatagca ccacattgtc 840
aactcatgac gcaattaggc ttaaaagatt cgtctcgcag tttcctgacg aaccgtgtaa 900
ttattatttt ttctacgttt aatactttat gtatgtgccc aaatattcaa tgtgacaacg 960
tgaaaatttt tatttggaac taaataggcc ctaatattct ttcaagatat tagaatagtt 1020
atccctctcc acctccctgc acaaacagtg aacttctttc tccttgggca caggagtagt 1080
agcagctccc ggaaacagaa agcaatcaag caaagtcctg aacctgaagc atcctgaaac 1140
cagcagacgg cagaaaccag tgggcgcagg cgatagcagt ttttcgtggt ccggcgtaca 1200
gccaaaatac tggccatcgg gtgcctacat agaatgagtc cactggacgc agctaccacc 1260
gtgtgtgcta cactgaccgc cgctgctcgt cgaccagttg tacggggctg acttattctg 1320
aatttctaat ggtttatttg ggggtttaga acactgaggg gtgctttaga tccaaagatg 1380
tgaagtttgg gcgtgtcaca tcgggtatta tatatagtgt cgcacagggt gtttgggcac 1440
taataaaaat actaattatt gatcctatac gataagctat ataatactcg atgtgacacg 1500
ccaaaacttt acatccctga atctaaacac ccttttaaat agagtatttg gtgtgaaata 1560
taattttgat ttgggaagaa ggtgagtgag atttggaaaa aaaaagcatt tcaattaaaa 1620
aatttgccag cagtaaataa agaaactact cggttttgta attaaagtga ggttttggca 1680
cttctttgcc ctaaactggc ctccatttta taaagtgaga accgtgcagc aaaagcctga 1740
aaaggcaaaa agaaagaaat tgtagaggtt tttcaggagg atacaactag gtgggtctct 1800
aactctctat gcagctgtgg tctgtggagc aaaacgatga aatggaagac gggacgttga 1860
cgagggtgaa gaaaacgagc gtttgaccag cgtcaaccat ggcgtgaaca gtagcaccac 1920
taacctgacc gagaggttga agaagatgca atcaacgggg tactatagtt cccacgaatt 1980
tcccagcaac aacgggttgg ttctcactac tcacgaattc cctgtggctc aacaactact 2040
agtacatcct tttgtccatt atgataaaag ttctatctta atttttattt acacgttttt 2100
caaactgttt ttttaatttc tatataaaaa atacttaaaa tatcaaataa aatctatttt 2160
tggagtttta aaaaactcaa ttaatcatat atattattga cttattttat tttacgtgga 2220
ctaaaatatc ttcatcttca tttaggttat gttcttttct catcaagata catgatacat 2280
tagcatgttt ttcaaactgt tttttaattt tgtatataaa cttactctaa aatatcaaat 2340
aaaatttact tttaggtttt ataaaagtaa aactcaatta atcattacta acttgtttca 2400
ttttacgtgg actaaaatat cttcatcttc atctaaggtg gtgtttggat ccaaggacta 2460

aattttaatc cctatcacat cggatatttg acactaatta gaagtattaa acatagatta 2520
atgatgaaac ccattccata accctggact aattcgcgag acgaatatat tgagcataat 2580
taatccatga ttagcctatg tgatgctgta gtaaacatgt actaattacg gattaattaa 2640
gcttaaaaaa tttatcttac gaattagctc tcatttatac aattaatttt attgttagtt 2700
tacgtttaat acttttaatt agtatacatc cgacgtaaca ctgatcgata caaacaccaa 2760
ctaaatcgaa aatcaccgaa tggctcgtca tcctcccaca tgagatgcca agatggaaca 2820
ccaacaatcc aacggctagg aagcgcccca tcccacccac cgcctaaccg ccttcctatg 2880
caagtgggtc ccaccccttc cttccttttt tttttctttt tacaaatccc cttccctttc 2940
ttggctagct agctagcttg gcccaacgcc acgagccgag ccgagcacat ccggagccaa 3000
gccgagctca gcgcctcagc tccccctcct cctcgtccca ttcccggttt cctcctccga 3060
tttcccccaa atccgcacgc ctctcccctc cgcctccatt tttcccgatt cccaattccc 3120
aaatccggat cagccgcagc cgcagcagca aaaaatttcg aaatccaaat ccaaacccat 3180
cccccccacg acgacgtcac ccacatcccc acccccgcga gacgagacga gacgactccc 3240
aaatctctct ctcctctctc ctatgcgcgc cgccgccgcc gccgcagcag cagcagctag 3300
gaggcggagc agcagcagca gcagcagctg agatgatcgt gcgcacctac ggccgcagat 3360
cccgctcctt ctccgacggg ggaggagggg agcgcggcgg cggcggtggg ttctcgtcgt 3420
cgcaagacgc gttcgaattc gacggggagg aggaggacga cctcgtcctg ctggggtcgt 3480
cgtcgcagtc gtcgcacccg cccgcgccgt cgcaggagtc gtcgtcgatg tgggacttcg 3540
acgaggaccc gccgccgccg ccccggcggc ggcgggggag gggtgggggt ggggactacg 3600
cggagcccgc cacggcggcg gcggcggcgg cggcggccac ctcgctcatg gaggcggagg 3660
agtacggcga gatgatggag agcgtggacg aggcgaactt cgcgctcgac gggctgcgcg 3720
ccaccgcgcc gaggcgggtg cgccgggcca gcttcctcgc gctgctcggg atctgcgcct 3780
ccgcgccgcg ccgccgcgtc ctccgggccc aggggtcggt acaccaaaga accctccttt 3840
tttttttctt acttgtctgc gctgtaagta aagaataaca attcgcgttc ttgctcttgc 3900
ttcgcgggca atcttggtga ggaatcttgt tagggttatg aaattgggca gccagttctt 3960
gtttcttctg cgtaatcttg gcggaaacag tgggattttg tacgattatg gctccgtaat 4020
cggcatttct gtgggaaatg aaccaccttt agggcatttg accttcgaac agcatgcttg 4080
gtgttgcaat ccgtagctat tgccttcatc ttaggcacaa gaacttgttc tgaattatga 4140
tttaccaact tgtgtttgtt ttcttgttct gagttttctt gcttggttag ggttagggtt 4200

atcaccgtgg tggtgcagaa ttagatgttc gctacttgtc ttaacctctg ccttgcccaa 4260
tttggtaccg agtgttacag ctgggtttag gaagtgtgat ctttgagcat ttctagcatg 4320
ttggtctctt tattttgcta atctcacatg gttgtagagg aaggaagcat agtgactgat 4380
gatgaatgcc tagatactag aaatacatct ttattaactg aattaggatt gcttgggtat 4440
ctatgtagat atgactgtag aatgttactg ctggaaatgc tatccaatat ccattgatct 4500
ctagcctaat atatctctcg aggccaagag atcagtcaat tttgaacttt caggagagtt 4560
tctatttggt acttaatctc ttttatttgt tacttttggt gcctggctct cttttcatga 4620
ttgctaagta gacaggtaaa gttctaccta aaattattct taaaagttca aaatcgcttt 4680
agattaagga gtgccagcca gagccttagg cagagtctta taaaccaaaa gcacaatgct 4740
acaatgttca caaaactttt gtggaatttc cacttgagct gtataaacat cgcaatctac 4800
tgtgaataaa agaagcactt gatggaagtt catgttagca aatgacatgt tttctgtgag 4860
gaggttgatt gcttgaactg ttatggactc ttgcaacttt ttattttact tcgtacccat 4920
ttatgctaat gtgcacaaat aaaattgctg agagtaaaaa tgtacaactt gttacgcacc 4980
agcacacttc ctatttgtat ccattttcct gttgaatttc aaatgtattc aattgctgaa 5040
attgttccat tcaacaaaca catattccgt taatgaaatt attatacatt gcgttttgtt 5100
ttcttactca caagtgtcct cttttcttat atcctataga ttggtgcaac aaattattga 5160
tgcaattttg gttttgaaca ttgatgatcc tccctgcact attggtgcag ctgctcttct 5220
attcgttttg gcaagtgatg tgagtacctc tcaatcccat ccttgtgctt ctgtgcatgc 5280
ttcattctat tttttacgca tatcgattgt tttcttttat ataacagccc ataaaaataa 5340
tcacatcatg gcaaagttat ttatttctcc agtacagtta tataagtatt caccactttt 5400
ccatgaatat cttggcatgt gattacaaag aagattattt aagaaagtcc atgcttttat 5460
ttcatcattt tgtttgaagt tgaactttaa tttatggtgt aaatttcagt taatattgct 5520
agcagctcgt attctttaat ggcataactt cacttgtgct tattctccaa tatctccctt 5580
cttgttgttc aggttcaaga aaatcatttg ttggattcag aatcttgtgt ccattttctt 5640
cttaaattat taaatcctcc agtgaatctt gttgattcca aagcaccatc gataggttcc 5700
aaacttcttg gaatcagtaa agttcaaatg cttaatggat caaataagga ttctgactgc 5760
atttcagagg aaatcctttc aaaagttgaa gagattctct taagctgtca agagatcaag 5820
tcgctcgaca aagatgacaa gaaaacaaca aggccagaac tgtgtccaaa gtggcttgct 5880
ttgttgacaa tggaaaaggc atgcttgtct gctgtttcag tggagggtaa gttttaatca 5940

aatttcttgg tcatgatttc cctttatgac cattataatt atttttatga gccaaataag 6000
cagttgccat aagttacata gcacctgttt acaatattca tgggtggttt gcttagccct 6060
ttgcttcacc tgcctttgat tgatgacttc catccgtgtt gcacaactga attggagtaa 6120
ttgactgcac tagaagcacc tatggccatt gtcatactag gaaggttttc ccttatcaaa 6180
tatttgattg ttacagagac ttctgacact gtgtccagag tcggaggaaa ttttaaagag 6240
acattaaggg agttgggcgg tcttgatagt atttttgacg ttatgatgga ttgccattca 6300
acattggagg tgagatctcg ctaacatcgc atattttaca cttcctttgt tcaactctaa 6360
aggatggtgc aagtttgttt ccttttgcc atttagcttt taatgtgctt gaagccacat 6420
gaaagcaatg cttgtccaga tacatagcca aaggttgtta tattttggga catggaaaat 6480
gcttgaggta gtaactattt tcatcaggac atggaaaatt ggctgcatca caaattatgt 6540
tgtttcatgt tgcaaaatag tttttaaata ctttttattt ctgcatgtgg tgttagtgtc 6600
ttacagtgat tcctctgatg attatatccc ccacgataat aatacttgac atatctacac 6660
caagtggaca ttattcattt ggatgttact tttccagcta tacttgctgt tcttgcataa 6720
actttggagt aaattgcgta tccctttaag agataaactg cttggtgctc ctatctgtgt 6780
actttttatg cccccaacta ataatgcaat catattacgc tgataaactg aataaataaa 6840
ttaacaatat acttctggtg gaaaccttgt gtatcagaat ctcataaagg atacctcaac 6900
ttcagctttg gacctaaatg aaggaacatc tttgcaaagt gccgctctcc tcttgaaatg 6960
tttgaaaata ttggaaaatg ccacatttct aagcgatgat aacaaggtaa tgttccttat 7020
atattctgtt tcagtttagt acccatttcc ttcttctgta ccatcttctc ccctcatttg 7080
ttctgtgcaa aatgtgcaaa cagtgtgact ttgtatttct gcttaacatt tttctttttt 7140
tcctgaaaag cagtataaac tcttacactc attttgcttc ttgcagaccc atttgcttaa 7200
tatgagtaga aaattgtacc cgaaacgctc ctcgctttct tttgttggtg tcattatcag 7260
tattattgag ttattatcag gtatttttct taataataca atatgtccgc taacacaata 7320
aaatgtttta aacatccagt atgttaaagt tgcagtctga cgcctatttt gttttgctgc 7380
agctctttca atactgcaga attcttctgt tgtttccagc tctacatatc cgaaatcgtc 7440
taaagtctct caacagagtt gctctggtaa taacaaacac caaatttgtt tgatcaactc 7500
gttggctttt ctgtgcactg tttcaatata gtttggtcgc cattcaagtc tcactacaga 7560
tgttgaactt gacctgacac ggtggcacca atatttataa aacgctacct gatattttta 7620
atatttcatg tttcctgacc cagattatct tgttggttcc tcatataagt ttaattagtg 7680

tcgttcttga aactttgtta tgcagcagat gtcatggggg gaacttcatt taatgatgga 7740
aagcgcaaga actcgaagaa aaaaaacctt ttgtcgaacc agacacgcca tagttgctta 7800
tcttcaaaat cagaagtttc tcatattact atatcttctg gtagtgatgc tggtctgtca 7860
cagaaggcat tcaattgttc tccatctata tcaagcaatg gggcatcaag tggttcatta 7920
ggcgagagac atagcaatgg tggtgctttg aagttgaata taaaaaagga tcgtggcaat 7980
gcaaatccaa ttagaggctc aagtgggtgg atttcaataa gagcgcacag ttctgatggg 8040
aactccagag aaatggcaaa aagacgccgt ctatctgaaa atgtaatcac cgacagtggt 8100
ggcggtgatg acccttttgc ttttgatgat gttgatcagg agccttcaaa ttgggaactg 8160
cttggtccaa aaaagaaatc gcctcagaaa catcaagaca aatcaggaaa tggagtgcta 8220
gttgcaagtc atgaaccaga ccaacctgaa gatcttaatc agtcgggtac aacatctctt 8280
tttagtgcta aagatgaatc cagtcttttg gaagactgcc tcttggcatc agttaaggta 8340
attaaatatg tttccttctg atctttcttg tttcttcttc aagagaatat acattcttgg 8400
gtcacagttt ctcggtttgt ctttgtgact ttgttgagtg acatattttg aattcacaaa 8460
atttcctttt caatatggct cctcaatcta tagcatctgt cgtgtatgta ttctgtacaa 8520
aatagtattg taacatctcc tagaagaaat tggcaccatc catatcatac agtagcaatt 8580
tatgagacgt gatcctgatt ggaggtttag gacagagcct cgagctaaat tgctattgta 8640
ttgtatctac tatcttttag tacatgatat gtgctgggca ctctgtgtct gagtgtagtg 8700
agtgcttaag tttacatagt tcagctaaca tgcatatgta agacagttta tgattaaatt 8760
taagtgtaga aagaaggtac tttcaaaaga tttttaagga caatataatt gtttcaccgg 8820
gactcatgct tgttctgact gtgagcctaa tgttaccttt acatgccctt acattgtcta 8880
ttttttatcg ttttatgaga tcttccaaac aacttgatct gtcttaatgt ttttttgcta 8940
gctcctttct tggatatctg gtaaatggtt aggccgaagt atgaactttg ccttattgtt 9000
tcaaagaaaa tgtaacaact cctggaaaag tctaattttg gttgcccttt attttgctga 9060
ccgtattggc acacatctaa ttctgctgtt cctttctggc aggttcttat gaacttagca 9120
aatgacaacc catctggttg tgaattgatt gcgtcatgtg gtggacttaa caccatggcc 9180
tccttgatca tgaagcattt cccctcattt tgttttgtcg tggacaacaa ctataacacg 9240
agagatgtca atcttgatca tgagttatca tcttctcaaa acagcaaggc acaccaggtc 9300
aaaattaagc aattgcgaga tcatgaactt gattttctgg ttgccatatt gggcttgctt 9360
gttaaccttg tagagaagga tagccttaat aggtaagtcc ctcacatgct tccttccatt 9420

tgctcaattc atatcagtgt tactgttctg gcagttcctt ggggtcagga ctcagaaaca 9480
tccaattaat gttcatgttc tcttaacgac tcagaaatac tttataacct ctccacaggg 9540
tacggctttc atctgcccgt gttcctgttg atctatctca gaatccacag agtgaagaga 9600
cacagagaga tgtcatagca ctcctctgtt ctgtattctt agcaagtcaa ggtgctagtg 9660
aagcttctgg aactatatca ccggtaattc aaaattcttc aagttccttt tgtatgtaga 9720
ttatatcttt gtaaaactcg gcatttatta cctgctcttt gtttcaaaaa gcagtatttt 9780
attttgctcc ttagcatagg tcagcagaac agttgatctt attcagaaaa caatattttg 9840
catgtaacat actgttatct atgagatgaa aattaatgca tgtgtaataa tgtcaatgat 9900
aaatatttgc tatctgaatc cagtctacca actctagtta gaccgaaatt actgaggttc 9960
tatttcaaag aataatttag tgcaccattt gttcaactac tatgaagtaa aatggtattc 10020
ccttctattg acatcgggtt agaagtgaaa ggccatctta atgcaatgtt ctcaatgcca 10080
caaacccaca aatttcatta acacatacag attattatta acatagctat aaattggatt 10140
tccagaagct tgagttgaat ttattttgtt acaattgaaa gcactgggaa cattagcatt 10200
tttttttagt tcttggttat tgcaatttat aatgttatac agaactgtgt acctcacaat 10260
gcattcatta tgacattcta tgaaccattt gattgactgt tgcttgtaaa caacaggatg 10320
atgaggagtc tttgatgcaa ggagcacggg aagctgaaat gatgatcgta gaggcctatg 10380
cagcccttct tcttgcgttt ctttcaactg aaaggtttgc aatctgtagt tgatggattg 10440
ttttattaat gtctaactac ttgcataatg tcagcactat ggcatttaac ttatactgtc 10500
tgttaactgc aacagcatga aggttcgtgg agccatttcc agctgccttc caaataacag 10560
cttaaaaatc cttgtgcctg cgctagagaa atttgtggta tgtctccata attcttgaac 10620
tactgtttgt ataaaaaagt atggatgatc tttgaattta ctccattttg gaaatcatta 10680
atttttcatg tctgaggtgt gaggtgtcac cataattgta cttcccatcc aggaagcctg 10740
tttgcaaaat ttcacataaa taaggaaaat ttgaacttgt ttcaagtttg aatagtaaca 10800
ggatgtttta tttctcaact ggagaaaaca ttccggctgg gacttttaac ccttaaaatg 10860
ctagtgtgct cccactgtaa gattgtctgc tgtcacattt gaaactttgt gtaatacctt 10920
tatcactacc cttgagatga gagacacaat ctggtaccga gttaagttat tgataactcc 10980
cagttgaagt acagcaccaa atcaagccaa catgttggct acgtaattaa atgttctctt 11040
acaacagata gaggtaaaaa gggagtttct aagtatctaa cctcttaccc tcttggctta 11100
gcactccagg cacaactctt tcttaacttg cgatttagga cttgactctg agaatattgt 11160

gtgcccacac tggttgagtg catgcctatc taagctgcta gtttttgttc attttgatta 11220
actctgaagc tgcctgagct tattctgctt ccatcattta ttaatccatc atgtttctct 11280
ttcagtcgtt ccatctgcag ctcaatatga tcacagagga aacgcactca gctgtcacag 11340
aagttatcga gaaatgcaaa ctttcataga aagagtgaag aggggcctgt acagatcaac 11400
taacaacctc tttgcagcaa aaaagcatac acacaagtgt ttgtcttggc ctggggctct 11460
gcagatggac tgatactctg acctgcagtg ggcttgggag ctaacaatgg tttcattctt 11520
ttttttttta tgttttcccc tgttgttttt gctcatgttt tgtgtaattt tttcttctca 11580
tctagcgatg ttatttttct tagcatgatg ggagtagccc tccttttttt tttctctaat 11640
taagtgtaaa gtagcaacag catagggatg aatgttcagt gtagtgtgtg gtgtttcagt 11700
tattcagaga cgtccataca gtttgtacct tgtgaccaca cgtcttaatc tgatgaagct 11760
tagaataaat cacatgttag caatgcaata tcatctgcgt cttctctcac tttggtggcc 11820
atcaaattct gtgtagaagt gtatggttgg tgtgctgttg caaatgccgt attccgctct 11880
gttttgtgga agttaagaag tccctagttg aaataccgat ttttcatgat ctcggagatt 11940
gatgcaactc tgattgcagc atttcttttt attagaatgt acactccatg ctatcatgat 12000
gtttattgtt tagtactaca agatttggtt aaccattatt ttaatatcat aataatttta 12060
taaaatcttg gagtaacaag ttcataatac atgatagcat aactttttga ggctagtcta 12120
tgtatattgt ctcctttgtt tttaaactaa gcactcaata aattattgat ggctgtaatt 12180
ttctgaaggt ttcaccggtt tcggcccgtg ctttataaat agcttcggca caaaagacaa 12240
aacggtccct ccaacacata aatggttgag tttacgtttt cattatcttt ggtaaaatca 12300
agtccaccac gtagacactc ataacaaaag tttgaatatc ctcagaaatt ttgacttgag 12360
tctatcttac ctttgatatc ggacatccaa ccctccctcc ctccctgaac tttatattat 12420
tcatattaca cctgaacttt atattattca tattacaccc tgaagtggtt ttcatttaat 12480
tgcatacatg ctgaaatagt ttgacaacgt gagatgcaca aaatctacac gttcgtctta 12540
agttgcaatt catttatcc ctttcttttt tctctcttac ataggaatat caatagtact 12600
aattcacatt acaatatagt ataaattggt gatcgattat tggcaatata ctatattaaa 12660
tattcaaaac tagtcattta agctgccaaa taagtaaacc actatcgaaa accacaatat 12720
aaatggcatt acaaaactta gggggttgaa tatccaattt taaagttcat gatgctagag 12780
gaatttctat caaaagttta tgggtacata tggacttttt cctttttaaa agaagctatt 12840
cttatcgtaa acgttaaata ttttttgtac tttatttttt atgattgaaa aaaaaactta 12900

gttttcaaaa tgattggtct gtatacaagc atcaattaga cttaataaat tcatctaaca 12960
gtttcctggc agaaactgta atttgttttt gttattagac tacgtttatt atttcaaatg 13020
tgtgtacgta tatccgatgt gacaaccaaa cccaaaaatt ttccctaact ccatgaggcc 13080
ttacagatat atttgatggg tgtaaagttt tttaagttct ttgggtgcaa agtttttaaa 13140
gtatacggac acacatttga agtattaaat atagacaaat aacaaaacat attacatatt 13200
ctgcctgtaa acaacgagac aaatttatta agcctaatta atctgtcatt agcaaacgtt 13260
tactgcagca tcacattgtc aaatcatagc gtaattaggc tcaaaaatat tcgtctcgta 13320
atttacatgc aaactgtgta attggttttt tttttcgtca acatttaata ctccatgcat 13380
gtccaaatat ttgatgcgat ctttttggcc aaattttgtt ggaatctaaa caaggatcaa 13440
atttgctgaa tttttccaga cgtcacggct tgttcatcca tcgttcgcat cgcgattcgc 13500
caccgacgcc ttggtttcca acgaatttta tcatccgctt aaatacatcc aaagctctcc 13560
atcgccatcg gcggccaacg gcgaccgctc cgctctaccc aatccaccca tccactcgcc 13620
gccgccccct gatccaaagc ctccgccgcg ccgccgtcga gaggaggagg aggaggagga 13680
ggaggaggag gaggcgtgag cccctatggg gaccctcctc cggccgcgtc cgctcgccca 13740
cgccgccggc gccggcgacg ccacgccgtc gaccgcgcac ggtagccacg cgcctctcga 13800
gaggcccccc ccccgccgct cgctgatctc tcttctcatc ctgtttgggt ttgggtttgt 13860
gatttgggtg tttttttttt tccgcagcgg tggtggtgag cggtggccgc ggccgtggcg 13920
tggagtgcca gccgcatcgg gtgcgccgcc gcccgggtcc gcaggttgcg gtggcgacgg 13980
cgagctggag gaggcggagg gagaccgtgg tgagatcgga tttcgccgct ggtggtgccg 14040
ctaccatggg ggattcgccg caggcgctct caggtttgca gcctcctcca ctctcttctc 14100
gcaaaatgtg ttgctatgtt cctctcgctg ggctggcctc atagccatta atgtagtttg 14160
ctggaacatt acattcggaa cgttgttggc aattgcttga caaaatgtgg aattgtggag 14220
gggagaaaaa tcgtttgaac ctgcagtgac aaaattgcca tctataattt taaaactgaa 14280
ggtgtggaaa tcaaacataa tcattgccag cacatcattc ttgttaacca ccttgacata 14340
ttgttggctt ataacagtta gctccacacc aacttggaag gtgtcaatgg aatgtaagta 14400
taaattgagg ataactggca gttgttaaga ctttctacag aacttgtagc agctaaaact 14460
agctattgtg catttatgtt tcatggaatt tgagcggcaa tggatatttc ttactaagac 14520
gtataatgca aaacaaaaaa aaaaaaaact atgtctatgc agtttacatg taatgtgcgg 14580
atgcaaataa aatcatgttc atggacaaac taatgggatt cataccaaat tccagaattg 14640

catttcttat gtggttactt ttgtttgttg atttggttac cagacatcga tgtggtttca 14700
agggtcagag gggtttgctt ctacgcggtg actgcagttg cagcaatctt tttgtttgtc 14760
gccatggttg tggttcatcc acttgtgctc ctatttgacc gataccggag gagagctcag 14820
cactacattg caaagatttg ggcaactctg acaatttcca tgttctacaa gcttgacgtc 14880
gagggaatgg agaacctgcc accgaatagt agccctgctg tctatgttgc gaaccatcag 14940
agtttcttgg atatctatac ccttctaact ctaggaaggt gtttcaagtt tataagcaag 15000
acaagtatat ttatgttccc aattattgga tgggcaatgt atctcttagg agtaattcct 15060
ttgcggcgta tggacagcag gagccagctg gtatggctgt agtctcatcc ctgctttctt 15120
aagtagacat atatacattt acagtatttg gtaaataaac aagattttat gaatcatata 15180
tgattttggg gaaaacacaa aactctcttt gttggctgcc ttgaacatag ttctgttcac 15240
acagttatag caccttcttt aaaatgaaga actttgttgc atacacataa ggccaaacca 15300
cataatgaat tttgtttatt tctatctttg aatgttagca tcgtttttgt ttaatgcatg 15360
atcgccttcc tatatatttg tagtatgtca acattgtatt ccatgctgag cataacaaat 15420
ggtttgttaa aattcaggac tgtcttaaac ggtgtgtgga tttggtgaaa aaaggagcat 15480
ctgtattttt ctttccagag gggactagaa gcaaagatgg aaagctaggt gcatttaagg 15540
ttcagtaacc aaacttaggt tacattacat ctaatgagat ttttatattc agtatataat 15600
gttaaccttc tcatggtgta ctgacgtggt tataaatgtc cccagagagg tgcattcagt 15660
gtggctacaa agaccggtgc tcctgtgata cctattactc ttctcgggac agggaaactg 15720
atgccttctg gaatggaagg catccttaat tcaggttcag taaagctcat tattcaccat 15780
ccaattgaag ggaatgatgc tgagaaatta tgttctgaag caaggaaggt gatagctgac 15840
actcttattc taaacggtta tggagtgcac taaagaaaga tggtgttttt ttttattata 15900
tggaacctat tcaaaggcac agacaggctt tcaaggctaa gcttgttaca ggtactgata 15960
ctagttacta attactttcg taatcagtat aaataagctt gtgtagtgta atggcattgt 16020
acatttctgc acttggtaaa tttacagaag aggcaagtaa tattttagag gattgagttt 16080
attcacccag tcatatagtt gaagaggcaa gtaacctgta agagaggact gaacattaac 16140
acctcttgtt cgattaaaaa tgaccaaaga gcatcaaaca tgtattcgag gctgttactt 16200
tagatatggc ccattaattt gtttagttgt ctatgtacat cctagttggt gtaaatgcca 16260
gttaccattt ctatgatcta aaacaatcaa ctcttttagt atattttcaa aaacgaaaat 16320
tcagtacaca tgtatgaatc ttaatattct tctctagctc gttacaaaag caacaaaggc 16380

accgtgtcag ctggttcaca ttagctagtt tgtacttagc attatccact agcaccttat 16440
tttcatgcat atcatgctaa tttgcttgcc cacgttgagt gggaattttt ttcatgtttt 16500
ataatttata tatgttttag acttctagtc cacaatttat gtacttcatg ttcctgagcc 16560
tctagtatgg ctgatagcag actaggtgct gagtgctgtc cttttttgca gactgaagag 16620
agaagaaata caagactgtc cattgttagt cagatttgta aaaatagact ctgatgtagt 16680
ttacttttgc ccctatttta tttttaacaa tacaaatata taacagatcc taagaactta 16740
tcttaattta ggagaagttg ctcgtttcat taaattaaat tgtgaagtaa aaatgtgtgc 16800
tcgagtctgt caatgcaatc ctgtgttctt gtttgaagat atggtgtagg gcaggccagg 16860
attgaacact gaatggtaag actgcttctg ccttcagacg ttattgctaa atttttagct 16920
acttgcagtt agtgctgcca cgccgattaa gcagtagaac aaagtagttt tgtcgtgcac 16980
aaatgagtta tatttcattg gaaatcgaag cgaaaacgaa tcaaaagtta gaagaaaagg 17040
ggaaacttgg taattactcc ataaagagag tgcattttat tggtaagatg gtatccggaa 17100
gctgtgagct ccgggctgta tgtattctgg caaatttgat atgagatgct cgattattgg 17160
cttaagttag cgatatcaaa tttggggaag caccaaagga attattgtga aggagttatg 17220
ggtgcgtgac gttatctgct aggttcaaat ccttgtggct atgaatattt atctgctagg 17280
ttcaaatcct agtgactatg aatattaatg ggtaaggtaa gggatttatt gttaatttta 17340
gtttctttaa gattgtgcca tcggacgcca ttcggtaact gtaataatgc tttgtattgg 17400
attcacttgt gttacatgca cgcactaaac atgtgcttta ccttttcatc tgtttttgcg 17460
ttctgggcta gaaactcaaa cgttgaattt tccatggtct gctcaacttg acaattactg 17520
cgtgtcaagc gatcttatac gcatactatg cgcacaagtg attgtatacg gatatgatga 17580
cagtataacg tgtgatattg atttttttaa taaaaaaatg atgttccttt ccttgatgaa 17640
ggaacaaaga ctttttttaa aagaagggta ttactaaaaa caaaaatgac aaaaacaaaa 17700
tatcagtgca catggcaagt gtgctcggca attttttctc tgtactttaa acaaaaatac 17760
ttctatatgt tcttttttat aagggtggca caaatctttt aaatgagcca aatatctaca 17820
ttggatttat taaaaactgt ataaattata atttatactc tgaaaggttg tgtgcatctc 17880
tcttggagaa aatgtataag ttgcaaacaa acattaatcc acgttatgta actttttttc 17940
gccggaaagg ccgaaggagg cctgacggag cgtggggctc ctcaccggga gaccgcgcag 18000
gccccccttt gccggttcgg ccggggactc agggtgaaat tctaagctct ctgtatgtgg 18060
aaggttcgcg accgtcgaaa gagcataaga cacgggcgat gtatacaggt tcgggccgct 18120

gagaagcgta ataccctact cctgtgtttt ggggggatct gtgtatgaag gagctacaaa 18180
gtatgagcca gcctctccct tgttctgggt tccgaatctg gaaaagtcca gtccagtccc 18240
cccctctaag tgggcaaggt cctcctttta tatcttaagg ggataccaca tgcaccatct 18300
ccctcctttc tgtggggact taccctacct tttcataaat ggacggagat ttgtatagtt 18360
gccgtccgaa tgaccttctg ataggacggc ccatacctac ctccacttcc gccgaaagca 18420
ggtgcgacgt gggattatgg ctgtctgctg acgacatgac cagtgtcaga ctggtcacaa 18480
attgctcatt cctgtccacc acgcgtcagt ttagcaatct acatgttggc ccttcttcac 18540
acaacatctt gcctgtaatg gttaggatga agcctggcat atatctaacc aggactaacg 18600
tgccatctct aggaggtaac acgctagctc cagctgggga cgagcgccta gaagccctcg 18660
tcctgacggg atggggcgag gcgtgcgtca gatcgcctgt cgccacctaa cccgcgatct 18720
gaccggtctg tgactggtca cagaccggat aaacgagtgc actgcacttc gttacatgcc 18780
gcgtgacacg ctcagccaaa ccgcaataaa tgtggttagg tgagccccgc tgtgctcacc 18840
taacccatac acgcggagca aaaacccacg aggggtcggg gcgcctcggc cctcggggcc 18900
gaggcgggtg cggtccgacc ccctcggggg gactaagagg agggcgaaca catcaccctc 18960
gggcccgacg tcccccgagg gtgccaggcc acgtgggcga ttgtgtctgc ctcaaacctc 19020
tagtcatgat actcctgatc ccatgtcacc gacagtagcc cccggcgtta tgccagggcg 19080
atcgccctct ttaagggaag cggtcgggcg tgacgccact cctaaggcct ggtgacaggt 19140
gggaccggtc tccacaattg ggcagaaacc caacggtcac aaatcacgca catcggcaat 19200
ggtaactcta ctatcaataa tgagcggtct cttcaagact gccacattac tcgagtagca 19260
cacgaatctg gacatggcga ttcgtttcgt ctggagatat ggtaacgtcg ctttggtcgg 19320
cgagcgtaat taacgcgcgc acgatatgat ctatctcgac tgccacaacc gcatatccac 19380
ctcatgcgcc gcaagcgggc gaatgggatt agtggaagcg tgggcgcgag aaacgagggg 19440
gcgaaatagt gggcgcgaga agcgaggagc cgggcacagc gttggcaaga gtataaaggc 19500
actgaggaaa ggatctgttt ccttcctttc gccatcattt cccttgtctt cgccgcttgc 19560
gccctaactc cttctttcct gtgctctact ttcgccacac gcgctcgctc tcaatcttct 19620
cttcctccgg cgccatggca cggggctccg ctctgctcga tggtagcgtg ctgccgcctt 19680
cccgcatcgt gagcgagagg caggctgggc tgccgcgccg cttcatgccg gaatctgcca 19740
ccggccggga gatagtcacg ctgggtgagg gacgcccggc gccagactac ccggggcggt 19800
ccgtcttctt tctccccttt gcaatggcag ggctggttcc gccattttct tctttcttca 19860

tggatgttct gaagttctac gatctccaga tggcgcacct cacccccaac gcggtgatga 19920
cattggccat cttcgcgcat ctgtgcgaga tgttcattgg ggtgcgccca tctcttcggc 19980
tgttccggtg gttcttcacc gtgcagtcgg tgtcgccgcc atcggtagtt ggtggctgct 20040
acttccagcc atgggggccg gtgctgaatc gctacatccc ctgcgccctc cgcaagaagt 20100
gggacgactg gaagagcgac tggttctaca cccccctcgc cgacgaagcg cgcctctgac 20160
ttccgagcca gcccccggcg caggcctcca gctggcgggc gccggtagat ctgggggatg 20220
gctatgacgc cgtcctcgac cgcctggcgg gcctacgatc ccaggggctc acaggggcca 20280
tggtgtacgg cgactacctc cgtcgtcgga ttgcgccgct ccagcggcgc gctcggggcg 20340
cctgggagta caccgggtcc gaagactaca tgaggaccca ccagggagtc agatgggact 20400
gggctcctga ggatttcaag atagtggtcc aacgggtgct gaatctcaac tccatggagg 20460
cgtccctcat tccccaagga atcctccctc tctgcagcga tccagaccgc gcctccatcc 20520
tgaccattat gacggcggtc ggggcctcag aggagtgagc tccaaagggc cacgacggcg 20580
caggcgggag ccgtaggggg gatcaatcta ccccgggagg gggtcgtgct tctgggtctc 20640
gcgacggagg cccgaggagc agccgccctg ccgacgcccg ggggaagagg aagcagggag 20700
gaacacctcc cccatctcct ccccgagggg gcggggcggt gcgtgccaac agcaggcgcc 20760
cggagggggc cgcgccgaca tcgcagcccg agggggagcg caagaagaag cggctccgca 20820
agatggggga gacagaacca tctcggggaa accttatttc ccctccaaag tggtcgttta 20880
accgaccccc tcgcaggttc gtctctcacc catcgtggct gtattcattc tctcaacgcg 20940
agttttcact cacccatctt gttcgtcttc tggtcttttc ttctgtttca gcgagatccc 21000
gtcgcgtccc tcccgccatt ccaagtccgg ccagtctgag gccgaggatc cggcggccgc 21060
agaggcccgg aggcgggaat ctgaccggcg agaggccgcg gatcgcctac gggaagccga 21120
ggaggccgcc caggaggccg cccgggctcg ccaggcgcag gaaaccgctc gggaggaggc 21180
cgcccgggcc cgccaggccg aggaagccgc tcgggaggag gccgcccgag cccaccaggc 21240
cgaggaagcc gctcgggaga aagccggatt tcgccaggac gaggcaatgg cgacttccga 21300
ggcagctcgc gatgaggtcg cgggcgcgtc gcttgagccc gcttcctcgg gcgacgctca 21360
ggcgacaact tccggggcag ctggcgacga ggctgcgggc gcgtcgcttg ggcccactcc 21420
ctcaggcgac gcccaggacc aaccaggtct gagggacatc cccgagtccg gcacttccat 21480
cggcggcccg agccgcgtgg catcctctcc aaggcggctc ttccccacgc cttctatcgc 21540
cccgctgagc gcagagcccc ttctgcaggc cttggccgcc gcaaacatcg cggtgttgga 21600

cgggcttagt gcccaggtgg aggccctgca agcagagtgg gcggagctcg acgccgcgtg 21660
ggcgcgtgtc gaggaggggc ggcgctcagt ggaggccatg gtggaggtgg gccgcaaggc 21720
acaccgccgg catgtctcgg agcttgaagc ccgtaagaag gtgttggcgg aaatcgccaa 21780
ggaagtggag gaggagcggg gggctgccct cattgccacc agcgtgatga acgaggcgca 21840
ggacaccctc cgccttcaat acgggagctg ggaggcggag ctagggaaaa agctcgacgc 21900
cgcccagggg gtgcttgacg ttgccgctgc ccgagaacag cgggcggggg agaccgaagc 21960
ggcgtcccga cggcgcgaag agacccttga ggcgcgcgcc atggcgctgg aagagcgcgc 22020
ctgcgtcgtg gagagggatc tggcggaccg cgaggccgcc gtcactatcc gggaggcaac 22080
actggcggcg cacgagtccg cctgtgccga agaggagtcc gcactccgcc tccacgagga 22140
cgcgctcacc gagcgggagc gagctctcga ggaggccgag gccgcggcgc aacggctggc 22200
ggacagcctg tccctccgcg aggcagcgca ggaggagcag gcgcgccgca ctctggaatg 22260
tgtccgcgcc gagaggaccg cactaaacca gcgggccgct gacctcgagg cgcgggagaa 22320
ggagctggac gcgagggcgc gcagcggcgg ggcggctgcg ggcgaaaacg acttagccgc 22380
ccgcctcgct gctgccgaac ataccatcgc cgatctgcag ggcacgctaa actcgtccgc 22440
cggggaggtc gaggccctcc gcttggcagg cgaggtaggg cccggcatgc tttgggacgc 22500
cgtctcccgc ctagatcgcg ccggtcggca ggtgggcctc tggagagggc ggaccgtaaa 22560
gtacgccgcc aaccatggag gcctcgccca gcgcctctcg aagatggccg gggctctcca 22620
acggctcccc gaggagctcg agaagacaat taagtcatcc tcgagggacc tcgcccaagg 22680
agcggtggag ctcgtactgg cgagttacca ggccagggac cccaatttct ctccatggat 22740
ggcgctggat gagttccctc ctgggaccga ggacagcgcg cgcgcaggtc cgggatgccg 22800
ccgaccatat cgtccacagc ttcgagggct cagcccctcg gctcgcgttc gcccccaact 22860
ccgacgagga ggacaatgcc ggtggtgcag acgacagtga cgatgaggcc ggcgacccgg 22920
gcgtatcgga ttgatccccc aagcccccgc cattcttcag ttttttcttc ttttccttct 22980
tctaaggcct tcgggcctct tttttgtata gatcaactta atctgtaatc aaaaatgaag 23040
aaatttttgt gtcaatttca tcttgctgtg tgtatgagat gaggatgatc tgtgacgtgg 23100
tccttttgcg tcttagcttg attaagggct cgtgcccagg tcccagtcct caaaaggcgt 23160
gggtcggggc tagtgcctgg ggagatccac atgtcgagac tggccaggcc gggaacgtgg 23220
tgaccgaggg ttatgggtga cccgattgtg ggtttttgcc gattcccccc cggagttcac 23280
cacgccccgg ggcacggctc ggttctgggc cccgtttggc gattttagcc gacccgagcc 23340

cccgagggca ggattgagca cgagtgacct atttcaagtc aagattcttc aaaaggaaaa 23400
aaaaacacag atacagcctt taggaaattg aaactgcttt tattgaaata ctgaaataag 23460
agaaataaga atgtgcatgt gtggcagccc ccggccaacc ctgcacgccc gagggggtgc 23520
ggggttggcc cgagcccgaa acctgacacc cgaccccccc cctcaggggt agaagcgacg 23580
aaggtgttcg atgttccacg ggttaggcag ctcaatgccg tcgcccgtgg ccagccgtat 23640
ggagcccggc cgggggacgc cgaccactcg atacggaccc tcccacattg gtgagagctt 23700
gctcaatcca gcacgcgttt ggacgcggcg taggacgagg tcgtcgacgc agagtgatcg 23760
ggcccggacg tgacgctgat ggtagcgccg caggctctgc tggtagcgcg cggctctgag 23820
ggccgcgcgt cgccttcgct cttccaagta gtcgaggtca tctctgcgaa gctgatcttg 23880
atcagcctcg cagtacatgg tggcccgagg agacctcagg gtgagctcgg atgggagaac 23940
cgcttccgcg ccgtagacga ggaagaaagg cgtttccccg gttgctcggc ttggtgtagt 24000
tcggtttgcc cagagcaccg ctggcaactc ctcgatccat gaatcgccgt gcttcttgag 24060
tatgttgaag gtcttggttt taaggccttt gaggatttct gaattggcgc gctccacttg 24120
gccattgctt ctggggtggg caggtgaggc gaagcagagc ttgatgccca tgtcttcgca 24180
gtagtcgccg aagagttcac tagtgaattg ggtgccatta tccgtaataa tacggttagg 24240
cactccaaac cgggccgtga tgcccttaat gaatttaagt gcggagtgct tatcgatctt 24300
gacgaccgga taagcctcgg gccacttagt gaacttgtcg atcgcgacat acagatactc 24360
aaacccgccc ggggcccgcc taaacggtcc caggatatcg agcccccaga cagcaaatgg 24420
ccacgaaagt ggtatggtct gcagggcctg ggccggctga tggatttgct tggcgtggaa 24480
ttgacacgct ctacatcgcc ggaccaggtc gaccgcatca ttgagagctg tcggccaata 24540
gaaaccctgg cgaaaagctt taccaaccaa ggtgcgcgag gcggagtggg ctccgcattc 24600
gccttcatgg atatcggcaa gaagcacaac gccttgttcc cgaggaatgc acttcaggag 24660
gattccatta gccgcgcgcc gatagagggt cccttctacc agcacgtagc gtttggagat 24720
gcgatggacg cgttcactcc cttcgcggtc ctcgggtaaa gtcttatctg tgaggtatgc 24780
ttggatctcg gcaatccaag caatcaatct aagggagctg ggagcgctcc cctcgggtcc 24840
cgaggcctgg acttcaacgg gcctcggggg ccggtcaggc gcgtccgtct cccctaaggg 24900
gtcgggtcgc gccgacggct gggcaagcct ttcttcaaag gcgcccggtg gggtctgggc 24960
tcgcgtggac gcgagccgtg agagttcgtc ggcaatcatg ttatcccgtc tgggcacatg 25020
ccgaagctca atcccgtcaa aatggcgctc catacgccgt acttggcgca cgtaggcgtc 25080

catctgcggg tcagagcacc ggtactcctt acagacttgg ttaacgacca gctgggagtc 25140
gcctaacacc aggaggcggc ggatccccag tccagctgcc actctgagtc cggcaaggag 25200
tccctcgtac tctgccatat tgttggtcgc tcgaaagtcg aggcggacca agtatctgag 25260
gacgtctccg ctcggagagg tcaacgtgac ccccgcaccg gcgccctgaa gagacaggga 25320
gccgtcgaac tgcattaccc agtgggcggt gtgaggcagc tgcgaggggt ccgtgctggc 25380
ctcggggatt gagacgggct cgggagccgg ggtccactct gccacaaaat cggcgagagc 25440
ctggctcttg atagcgtggc gtggttcaaa gtgcaaatcg aactcagaaa gttcgattgc 25500
ccatttcacc acccgtcctg taccgtctcg attatgcaag atttgaccga gggggtaaga 25560
cgtaaccaca gtgacccgat gcgcctggaa ataatggcgc agtttcctcg aggccatcag 25620
aatagcgtaa agcatcttct gggcctgagg gtatcgggtt ttggcgtccc ggagggcctc 25680
actaacaaag tagacgggcc gctgcacctt tcggtggggc cgatcctctt cgctaggggc 25740
cgcatccctg gggcactctt cgtccaagca gcctcgcggg gcgcacttgt cttctgtgct 25800
gatgacctcg gggtcggagg ataacagggg cggccttccc acagtggctt tggggccgtc 25860
ctgggggtca ggggctcctg gcgtcgtcgg acaagcgggc aaagggccaa ctccggtcgt 25920
caggggcctt aggcctccgt tcggctcggg ggcctcttct ccctgctctt tcccgggtcg 25980
agtcagcaca gggttagcct cggggtcaaa gggcgatagg tgcggccttc ccacagtggc 26040
ctcagggcct tcctgggggt cgggggctcc tagcaccgtc tgacaagcgg gcagagggcc 26100
aactccggtc gtcgggggcc tcgggccacc gttcggctcg ggggcctctc ctccctgctc 26160
tctcccgggc caagtcggca cagggtgggg aagcgcgaaa tgagaattgt cctcatcgcg 26220
ctccacaacc aatgccgcac taactacttg cggggtcgcc gctaagtaga gtagcaaggg 26280
ctcgtctggc tccggggcga ccagaactgg gggagagctt agatacgcct tcaactgggt 26340
gagggcattt tcagcttcct tcgtccaggt aaacggtccg gagcgtttga gaagcttaaa 26400
taagggtaac gccttctctc ccagcctcga tatgaaccga cttagggcgg ccatgcaacc 26460
ggtgacgtat tgcacatccc taagtttgct gggggggcgc atccgctcta tagcccgtat 26520
cttctcgggg ttggcctcaa tgccccgggc agagaccaag aacccgagaa gcttgcccgc 26580
aggtacaccg aacacacact tatcggggtt taattttatg cgggcggagc ggagactctc 26640
aaaagtttcc gctagatcta tgagtaacgt ttcctggttg cgcgtcttta caaccaagtc 26700
atcgacataa gcttcaatat tacgtcctaa ttggctaccc aaagaaattc gagtagtacg 26760
ttgaaaagta ggacctgcat tctttaaccc gaaggcattt gtcgtataac aataggttcc 26820

tatgggggta atgaacgcag ttttttcctc atcctcccta gccatgcgaa tctgatggta 26880
accagagtat gcatctagaa aacacaaaag gtcgcacccc gcagtggagt cgacaatctg 26940
atctatgcga ggcagggggt aaggatcctt aggacatgcc ttgttaaggt cggtgtagtc 27000
gatgcacatc cgaagcttgc cgttcgcctt gggaacgacc accgggttcg ctagccactc 27060
ggcggggttg acgctgccat catatttttc ggcgatggtg ggccggaacc ttgggggcca 27120
acggacattc cgaagactcg ccacaaaggc tctacagccg acaccaccaa ccgggggcac 27180
ggagggctga ttcccgcgtc cgtgttgagg tgacactctg gacgaggaag cgccctccgt 27240
tgcgtgggca gcacttcggt cattacgccg gcgctcgatg ctggtgcggg cgtccggccc 27300
cccacgcaga tctttctggg tcgaaggagt cgacgaagga gtggcggccg aatggcgaac 27360
agcggctgcc gctcgtcgtg ccctccgtct tgacgacgcg gagccggtgg tagcagcacc 27420
agaggccttg gtggcggagg accgcccacc agcatctagg cgctgccgta ccgtcatgac 27480
taatttggcc acgtcgtcca gccatcgttg ggctggagac tccgggtcag ggacgacagg 27540
cgggtgacgt aagagcgcgc ccgcagcttg gagcgcgccc tggggcgtgc tgccgtcgcc 27600
gtagacgagg aggcgacgct ccccatctcg ccgttcttct ccatcgcccg cgatcggtga 27660
agtcgcggat ctttcgaccc tctcgagcgc ctccccccgc ttaggacttt ggcgtggagg 27720
gagcggtgga gtacgagctc gacggcgtgg gttcggctcc ccgtcgtcgc cactcacact 27780
cggagagagg tcgtgcgcct ttgcttgctc ggccatcagg ctgaacagga aaagcttggc 27840
gcacacggaa gagtacgaga gctcagaaaa acacacactg agtcccctac ctggcgcgcc 27900
agatgacgga gcgtggggct cctcaccggg agaccgcgca ggcccccctt tgccggttcg 27960
gccggggact cagggtgaaa ttctaagctc tctgtatgtg gaaggttcgc gaccgtcgaa 28020
agagcataag acacgggcga tgtatacagg ttcgggccgc tgagaagcgt aataccctac 28080
tcctgtgttt tggggggatc tgtgtatgaa ggagctacaa agtatgagcc agcctctccc 28140
ttgttctggg ttccgaatct ggaaaagtcc agtccagtcc ccccctctaa gtgggcaagg 28200
tcctcctttt atatcttaag gggataccac atgcaccatc tccctccttt ctgtggggac 28260
ttaccctacc ttttcataaa tggacggaga tttgtatagt tgccgtccga atgaccttct 28320
gataggacgg cccataccta cctccacttc cgccgaaagc aggtgcgacg tgggattatg 28380
gctgtctgct gacgacatga ccagtgtcag actggtcaca aattgctcat tcctgtccac 28440
cacgcgtcag tttagcaatc tacatgttgg cccttcttca cacaacatct tgcctgtaat 28500
ggttaggatg aagcctggca tatatctaac caggactaac gtgccatctc taggaggtaa 28560

cacgctagct ccagctgggg acgagcgcct agaagccctc gtcctgacgg gatggggcga 28620
ggcgtgcgtc agatcgcctg tcgccaccta acccgcgatc tgaccggtct gtgactggtc 28680
acagaccgga taaacgagtg cactgcactt cgttacatgc ggcgtgacac gctcagccaa 28740
accgcaataa atgtggttag gtgagccccg ctgtgctcac ctaacccata cacgcggagc 28800
aaaaacccac gaggggtcgg ggcgcctcgg ccctcggggc cgaggcgggt gcggtccgac 28860
cccctcgggg ggactaagag gagggcgaac acatcaccct cgggcccgac gtcccccgag 28920
ggtgccaggc cacgtgggcg attgtgtctg cctcaaacct ctagtcatga tactcctgat 28980
cccatgtcat cgacaaggcc atccgaatgt attaaggagt aaaagttaca agaaaaaaca 29040
ccacaatgca ccaaggtgca tgaccacaca ccatacacta cccccaagca caaaccactg 29100
agggtgaagc ctagcaccaa acgaccgcca ctaagtgtga ccaaacgccg ctaggcctac 29160
ggcagcaaca catagatgag acttcgaaaa cgatgccacc aaggtggtca cgacatgtag 29220
gatgctgcca tcgtccatct aaaaagatgt ggttttcacc cagagaaact catcaagaag 29280
gggagagggt aacccttgac agcgccccaa ggaggttacg acgcccgaag gcgtagccgc 29340
tgccggtccg gtgaaccacc ggactaggct tccgcctagg accctatagc cttgatcgca 29400
gatcaccgtc caccactcag aaccaccaca cagacaaaag gtagcacgta gcttccaccg 29460
caccgcaccg acgccccttc gtcggccgac tccatcgaac caccatccct gagagctggc 29520
ccaggacccc tccgttccac cacccgccgg ccgccttgcc agttttggcc aaaggagaac 29580
ccgggactgg gtgacattgc ttcggcagcc tgagcttccc ccgctggcga gctgctgtct 29640
caatccaacc tagaaactcc ccgcaaaaga aggggatgag ctctaggaag ggcgagggtg 29700
ccgaccggca acgaggaaga caacccatcg actccagctc cctttgcact accatctggg 29760
cctgcgccaa tgccggatac gctgtcgctc cggctccggc gccacccacc tgcaccccct 29820
ttgcctggtc tccgcgcccc tcctggctgc gtcgcgccgc ccagctggcc gctaaggcca 29880
ccacgacggc cgcccggcta ccgaggcctg gccgcgccat gggacagctc gcgctggcac 29940
cagcgagcca cggccgtcgc gctgttgccg gcgccagcga gcacaaccgc cagctccaag 30000
ggccgagcat gccactgagc cgccgccgct gccgcccggg ccggctgcac gtcaccggcg 30060
cacacgaccg cacgccgcca cgctccgcct ccgcgcccga ggcagcccca tgccattgcc 30120
gcgcacctcg cccgcccgct gccgagccgc caccgcgcac cttgctgagc cgccaccgcc 30180
gtccctagcc gcctcgtgcc gccgccacgc cagatccagg cgcgggatgg ccggatccgg 30240
ccttgggggc gccggatccg ccgcctcccc acaccgccac ggcgtcacca cctccgaccg 30300

cagtgagggc ttcgtcgttt gccccatcct catcgcgtcg aggaggaaga cgccaagaaa 30360
aaagggcctc gccgctgcct tccttgctcg ctgccggctt cgccgccggc gagctccggc 30420
ggcggcgagg tgggggagaa gaagtgggga gtgggcagct agggtttttt cgccccccaa 30480
gccgcccgtg cgagagcgac ggtggggggg gggggacttt ccaacctctt ccagtgttct 30540
agttctccac gttatgtaac tcaatttgtt taaccataga aagtaagaaa cctaccagcg 30600
tgttaagctc tctttcattc cctttcttct tcctggtttt gcttccatca catgtcaagt 30660
gaagggttct taactaccat tactcctaca catctaattt ttttctcaga tctttcgcag 30720
gtatatattg atgctacatt ttatgatctt aagataatct ccttcacatt accctctgct 30780
gaaactttag cttgaaccgt catcttcacc acaatttgag cccaatttgc acagagcaca 30840
acgagcaata gcttgccctt acgttcatta tttagcatga actactacta actacccaag 30900
aatcaataca ccggtttaat aacgccattt tatcacgtta atatatgttt cattcaacac 30960
accggttttg gcacagttgc aaacttgcaa taaattcttt cctacttctc catcccataa 31020
tataacaaat tggtatgtct cgtctggtac taagttgcta tattatgaga tggagggagc 31080
acttcttttc ttccaaaata taagaatata gtattggatt agatattatc tagattcacg 31140
aattcgatta ggttgtctag atttatagtt gtatgtaatg tataattcgg taataggtta 31200
ttacctctcg ggatggaggg agtagttttg actttttttt ttcttataaa tcgctttgat 31260
ttttatatta gtcaaatttt atcgagttta actaagttta tagaaaaaaa ttagcaacat 31320
ttaagcacca cactagtttc attaaattta gcatggaata tattttgata atatatttgt 31380
tctgtgttaa aaatgctgct atatttttct ataaacgtag tcaaatttaa ataagttaga 31440
ctaaaaaaaa tcaaaacgac ttataatatg aaatggagga agtagtagac tataacaaat 31500
ttaaaccgtg ctttgatttt agagcatcac taatatgtta gcaataatct atccctaaaa 31560
tttatttttt ttcctaaact gaaaatagga agtggaaata ctcctccatc taagagagag 31620
cctaaattca ataaaaaact aaaaaactaa aggtggatcc ctctattaaa ctaccgcaaa 31680
aaatttatgt tttttttctc ttccacgcgc gcagaacaga tatctcgatc aagttagcat 31740
gtaaaatttt taaagagata ccttatacga ctccttccgt atttccaaaa gcaaacggat 31800
ttaaaatctg actcaaataa agatctatat atccaattta catgacacat gtttcgccga 31860
atttttatat taataataat taatattttt aaaattaaat tattagcaat ttgtttggag 31920
gatttatcaa aacaggatgg acgttgttta taacagcgtc tagacctaga cgcgcttgca 31980
aactgcggcc acccttttat cacacaaatt tttgacaatt tgacactttc caaaaattaa 32040

ttttataaat taaccgtgac caaaacttat ttaaaaataa tctttttgtt gagcgcaaaa 32100
tcgtatactt cagcgccaaa tagcacggcg ccgacctccc ccttcccctc ccctctatcc 32160
tccactgctg ccgcccacct ctccgtatca gctgcgtcgc gttggtttcc gccggcgctg 32220
ctgctgctgc accagtccgc tagggcgggc gggcatggcg cgccgcgccg cttcccgcgt 32280
ccgcgccggc gctgttggcg cccttcgctc ggaggctcgg acccaagggc gagggggccg 32340
cacggggggc agtggcgccg aggacgcacg ccacgtgttc gacgaattgc tccggcgtgg 32400
caggggcgcc tcgatctacg gcttgaactg cgccctcgcc gacgtcgcgc gtcacagccc 32460
cgcggccgcc gtgtcccgct acaaccgcat ggcccgagcc ggcgccgacg aggtaactcc 32520
caacttgtgc acctacggca ttctcatcgg ttcctgctgc tgcgcgggcc gcttggacct 32580
cggtttcgcg gccttgggca atgtcattaa gaaggatttt agagtggacg ccatcgcctt 32640
cactcctctg ctcaagggcc tctgtgctga caagaggacg agcgacgcaa tggacatagt 32700
gctccgcaga atgacccagc ttggctgcat accaaatgtc ttctcctaca atattcttct 32760
caaggggctg tgtgatgaga acagaagcca agaagctctc gagctgctcc aaatgatgcc 32820
tgatgatgga ggtgactgcc cacctgatgt ggtgtcgtat accactgtca tcaatggctt 32880
cttcaaggag ggggatctgg acaaagctta cggtacatac catgaaatgc tggaccgggg 32940
gattttacca aatgttgtta cctacagctc tattattgct gcgttatgca aggctcaagc 33000
tatggacaaa gccatggagg tacttaccag catggttaag aatggtgtca tgcctaattg 33060
caggacgtat aatagtatcg tgcatgggta ttgctcttca gggcagccga aagaggctat 33120
tggatttctc aaaaagatgc acagtgatgg tgtcgaacca gatgttgtta cttataactc 33180
gctcatggat tatctttgca agaacggaag atgcacggaa gctagaaaga tgttcgattc 33240
tatgaccaag aggggcctaa agcctgaaat tactacctat ggtaccctgc ttcaggggta 33300
tgctaccaaa ggagcccttg ttgagatgca tggtctcttg gatttgatgg tacgaaacgg 33360
tatccaccct aatcattatg ttttcagcat tctaatatgt gcatacgcta aacaagggaa 33420
agtagatcag gcaatgcttg tgttcagcaa aatgaggcag caaggattga atccggatac 33480
agtgacctat ggaacagtta taggcatact ttgcaagtca ggcagagtag aagatgctat 33540
gcgttatttt gagcagatga tcgatgaaag actaagccct ggcaacattg tttataactc 33600
cctaattcat agtctctgta tctttgacaa atgggacaag gctaaagagt taattcttga 33660
aatgttggat cgaggcatct gtctggacac tattttcttt aattcaataa ttgacagtca 33720
ttgcaaagaa gggagggtta tagaatctga aaaactcttt gacctgatgg tacgtattgg 33780

tgtgaagccc gatatcatta cgtacagtac tctcatcgat ggatattgct tggcaggtaa 33840
gatggatgaa gcaacgaagt tacttgccag catggtctca gttggaatga aacctgattg 33900
tgttacatat aatactttga ttaatggcta ctgtaaaatt agcaggatgg aagatgcgtt 33960
agttcttttt agggagatgg agagcagtgg tgttagtcct gatattatta cgtataatat 34020
aattctgcaa ggtttatttc aaaccagaag aactgctgct gcaaaagaac tctatgtcgg 34080
gattaccgaa agtggaacgc agcttgaact tagcacatac aacataatcc ttcatggcct 34140
ttgcaaaaac aatctcactg acgaggcact tcgaatgttt cagaacctat gtttgacgga 34200
tttacagctg gagactagga cttttaacat tatgattggt gcattgctta aagttggcag 34260
aaatgatgaa gccaaggatt tgtttgcagc tctctcggct aacggtttag tgccagatgt 34320
taggacctac agtttaatgg cagaaaatct tatagagcag gggttgctag aagaattgga 34380
tgatctattt ctttcaatgg aggagaatgg ctgtactgcc aactcccgca tgctaaattc 34440
cattgttagg aaactgttac agaggggtga tataaccagg gctggcactt acctgttcat 34500
gattgatgag aagcacttct ccctcgaagc atccactgct tccttgtttt tagatctttt 34560
gtctggggga aaatatcaag aatatcatag gtttctccct gaaaaatata agtcctttat 34620
agaatctttg agctgctgaa gccttttgca gctttgaaat tctgtgttgg agttcttttc 34680
tcctacagtt gtattagagg agggatcttc tctttatgtg taaatagcga ggtatgtatg 34740
tcacctctcc gaattatttt tactctggtt cctagacggt aaacaagcaa ttatgttctg 34800
cctttgatgc cagaaaaaac acaaaagttt gtcgttatct ctactaacgg atcataaagg 34860
aatttgtaac tggagtttca aacttaattt gtctaggcag tagttttggc attagatcca 34920
acattgtgta ggattcattt gtgtgtatca atctataggg tttcattaaa tttcgttaat 34980
gtgtactgtt taggtgttga atagtttgac ttgtttttta actgaacaaa agatactgaa 35040
atcgttccat tcaacaaaca catgttccgt taatgaaatt attgtacgtt acctttgttt 35100
ttcttactca caagtgtcct cttttcttat atcctataga ttggtacaac aaattattga 35160
ttcaattttg gttttgaaca ttgatgatcc tccctgcact attggtgcag ctgctcttct 35220
attcattttg tgaagtgatg tgagtacctc tcaatcccat ccttatgctt ctgtgcatgc 35280
ttcattccaa ttttttacgc atatcgattg ttttctttta tataacagtc cataaagata 35340
atcacatcat gacaaagtta tttatttcta cagtatagtt atataagtat tcaccagttt 35400
tccatgaata ttttggcatg tgattacaaa gaagattatt tgagaaaatc catgctttta 35460
tttcatcttt ttgtttgaag ttgaacttta atttatggtg taaatttcag ttattattgc 35520

tagcagctcg tactctttaa tggtataact tcacttgtgc ttattctcca atatctccct 35580
tcttgttgtt caggttcaag aaaatcattt gttggattca gaatctggtg tccattttct 35640
tcttaaatta ttaaatcctc cagtgaatct tgttgattcc aaagcaccat cgataggttc 35700
caaacttctt ggaatcagta aagttcaaat gcttaatgga tcaaataagg attctgactg 35760
catttcagag gaaatccttt caaaagttga agagattctc ttaagctgtc aagtgatcaa 35820
gtcgctcgac aaagatgaca agaaaacaac aaggccagaa ctgtgtccaa agtggcttgc 35880
tttgttgaca atggaaaatg catgcttgtc tgctgtttca gtagagggta agttttaatc 35940
aaatttcttg gtcatgattt ccctttatga ccattatatt tatttatatg agccaaataa 36000
gcagttgtca acttgtcata agttacatag cacctatttg caatattcat gggtggtttg 36060
cttagccctt ttcttcacct gcttttgatt gatgacttcc atctgtgttg cagaattgaa 36120
ttggagtagt ggactgcact agaagcacct atggccattg tcatactagg aaggttttcc 36180
cttatcaaat atttgattgt tacagagact tctgacacag tgtccagagt tggaggaaat 36240
tttaaagaga cattaaggga gatgggaggt cttgatagta tttttgacgt tatggtggat 36300
tttcattcaa cattggaggt gagatctcgc taacatcgca tattttacat ttcctttgtt 36360
caactctaat ggattgtgca ggcttgttcc ttttcgccat tttagcttta atgtgcttga 36420
agccacatga aagtaatgct tgtccagata catagccaaa ggttgttata ttttggggca 36480
tggaaaatgc ttgaggtagt aactattttc atcaggacat ggaaaattgg ctgcaacaca 36540
aattatgttg ttttatgttg caaaaatagt tttttaatac ttttttattc tgcatgtggt 36600
gttagtatct tacagttcct ctgatgatta tatcccccac gataataaca cttgaaacga 36660
taataacact tgacatatct acaccaagtg aacattattc atttggatgt tacttttcca 36720
gctatacttg ctgttcttgc atgtgtaagc aagtttggag taaattgcgc attaatttaa 36780
atgcttggtg ttcctatctg tgtacttttt attccccaac taataatgca atcatattac 36840
gctgataaac tgaataaata aattaacaat atacttctgg tggcaaacct tgtgtatcag 36900
aatctcataa aggatacatc cacttcagct ttggaccgaa atgaaggaac atctttgcaa 36960
agtgctgctc tcctcttgaa atgtttgaaa atattggaaa atgccatatt tctaagcgat 37020
gataacaagg taatgctcct tatatgttct gtttcagttt agtacccatt tccttcttct 37080
gtactatctt ctctcctgat ttgttctgtg caaaatgtgc aaacagtgcg actttgtatg 37140
tctgcttaac aatttctttt tcttcctgaa aaagcaatat gaactcttac attcattttg 37200
cttcttgcag acccatttgc ttaatatgag tagaaaattg aacccgaaac gctccttgct 37260

ttcttttgtt ggtgtcatta tcaatactat tgagttatta tcaggtattt ttcttaataa 37320
tacaatgtgt tcgctaacac aataaaatgt tttaaacatc cagtatgtta aagttgcagt 37380
ctgacgccta ttttgttttg ctgcagctct ttcaatactt cagaattctt ctgttgtttc 37440
cagctctaca tatccgaaat cgtctaaagt ctctcaacag agttactctg gtaataacaa 37500
acaccaattt tgtttgatca gttgatctcg ttggcttttc tatgcactgt ctcaatatag 37560
tttggtcgcc attcaagtct cactacagat gttgaacttg gcctgacacc aaatatttat 37620
aaaatgctac ctgatatttt taatatttca tgtttcctga cccagattat cttgttggtt 37680
cctcgtataa gtttaattag tgacattctt gaagctttgt tatgcagcag atgtcatggg 37740
gggaacttca tttaatgatg gaaagagcaa gaactcgaaa aaaaaaaact tttgtcgaac 37800
cagacacgtc attgttgctt atcttcaaaa tcagaagttt ctcatattac tatatcttct 37860
ggtagtgatg ctggtctgtc acagaaggca ttcaattgtt ctccatttat atcaagcaat 37920
ggggcatcaa gtggttcatt aggcgagagg cacagcaatg gtagtggttt gaagttgaat 37980
ataaaaaagg atcgtggcaa tgcaaatcca attagaggct caactggatg gatttcaata 38040
agagcgcaca gttctgatgg gaactccaga gaaatggcaa aaagactccg tctatcttaa 38100
aatgtaatca ccgacagtgg tggtggtgat gacccttttg catttgaccg ccgcgtcggc 38160
gtcgccacca cgtaatcgcc cacgtcgctg cccccgctgc cacgtcgtcg accgcgcacg 38220
gtaatcacac gcatctcgag gccgccgcta gctgatatct tctcatccgg ttgatttgtg 38280
attttggcgt tttgcagtgg gtgatggcgg ggggcgaccg tggccgaggc gtggagtgcc 38340
atccgcatca gggtgtatcg gccgcgctgc tccgccctgg tccgcaggct ttggcggcga 38400
gctggcggcg gagggagact gtggtgagat cggatttcgc cgctggtggt gtcgctacca 38460
tgggggattc gccgcaggcg ctctcaggtt tgcagcctcc tccactctct tccctttttt 38520
attttttttt ctcgcaaaat gtgttgtgat gttcgtctcg ctgggcaggc ctcatagcca 38580
ttaatgtagt ttgctggaac atttacattt ggaacgttgt tggcaattgc ttgacaaaat 38640
gtggaattgt ggaggggaga aaaatcattt gaacctgcag tgacaaaatt gccatctcta 38700
attttaaaac tgaaggtgtg gaaatcaaac ataatcattg ccagcgcatc attcttgtta 38760
accaccatga tatattgttg gttataacag ttagctccac accaaccttg aaggtgtcaa 38820
tagaatgttt agtataaatt gaggagaaca ggcagttgtt aagactttct aaagaacttg 38880
tagcagctaa tactagctat tgtgcatttg tgtttcatgg aatttgagca gcaatggata 38940
tttcttacta agatgtatga tgcaaaacaa aaaactatgt ctatacagtt tacatgtaat 39000

gtgcggatgc aaataaaatc atgtacatgg acaaactcat gggattcata ccgaattcca 39060
gaattgcatt tcttatgtgg ttacttttgt tgttgatttg gttaccagac atcgatgtga 39120
tttcaagggt cagaggggtt tgcttctacg cggtggctgc agttgcagca atctttttgt 39180
ttgtcgccat ggttgtggtt catccacttg tgctcctatt tgaccgatac cggaggagag 39240
ttcaggaaaa aaatttgaaa atacccattt tttgaaaaag atttacgttt atatacacta 39300
gtatgaagaa tttgcgaaaa tataactaat ccgcagatcg gttatgcggg agcgcaacaa 39360
aagtatggcg tggcggcgcg gagtggacgg ccgaggcgtt cgcgcggaat ggggctgcgg 39420
gaccgagcca gtctcgcttg ccggtaacgc ggaaccggta cgctcccgca gcgccagtgt 39480
gcggaaccgc ggcgccaaca tttttttact gcatggcact gtgtttaata ctgtttgaca 39540
ctgtttctgg tactgtttta cacagttccc gggtcagttc cgcacaatgg aggcgcggca 39600
ccgaccatga acaatgtgtg aacagtgctg cacaggttta aaacagtgta taaactgcgc 39660
tgcacagtgc tggagtcgct ggccactgcg gttccgcgtt ttggaaccgc gggaccgtcg 39720
cgattccgcg ttttggagct gccggaccat gacggttccg cgcaggatcg tcggtcccgt 39780
attttgaatc tgcggaaccg tcgctgtccc gcgtttccgt ttcgcgggat gcgtatattt 39840
ttataaaacc tctccatgca tgtatataaa cataaattat tgaaaaaata agtatatttg 39900
caaatttttt tcgagagctc agcactacat tgcaaagatt tgggcaactc tgacaatttc 39960
catgttctac aagcttgacg tcgagggaat ggagaacctg ccaccgaata gtagccctgc 40020
tatctatgtt gcgaaccatc agagtttttt ggatatctat acccttctaa ctctaggaag 40080
gtgtttcaag tttataagca agacaagtat atttatgttc cgaattattt gatgggcaat 40140
gtatctctta ggagtaattc ctttgcggcg tatggacagc aggagccagc tggtatggct 40200
gtagtctcat ccctgctttc ttaagtagac atatatgcaa ttacagaatt tggtaaacaa 40260
acaagatttt atgaatcata tatgatttgg gggaaaacac caaactctct ttggtggctg 40320
ccttgaacat agttctattc acacagttat agcaccttct ttaaaatgaa gaactttgtt 40380
gcatacacat atggccaaac cacataatga atttgtttta tttctatctt tgaatgttag 40440
caccttattt tcatgcatat catgctaatt tgcttgccca cgttgagtgg gaattttttt 40500
ccatgtttta taatttatat atgttctaga cttctagtcc acaatttatc tacttcatgt 40560
tcctgagcct ctagtatggc tggtagcaga ctaggtgctg agtgctgtcc atttttgcag 40620
actgaagaga ggagaaatac aggactgtcc gttgttagtc agatttgtaa aaatagactc 40680
tgatgtagtt tattttagcc cctattttat atttaacaat acaaatatat aacgtatcct 40740

aagaacttat cgtaatttag gagaagttgc tcgtttcatt aaattaaact gtgaagtaaa 40800
aatgtgtgct cgagtctgtc aatgcaatcc tgtgttcttg tttgaagata tggtgtaggg 40860
caggctagga tcgaacactg aatggtaaga ctgcttctgc cttcatttgt gcacttggtg 40920
ctgccacgcc gattaagcag tagaacaaag taattttgtc gtgcacaaat gagttatatt 40980
tcattgaaaa tcgaagtgaa aatgaaccaa aagatagaag aaaaggggaa acttggtaat 41040
tatatactcc acaaatttat tggtaagatt tgatattaga cgctcgatta cttggcttaa 41100
gttaaggata tcaaatttgg ggaagcacca aaggaattat tgtgaaggag ttgtgggtgc 41160
ataacgttat ctactaggtt caaatcctag tgactatgaa tattaatgag taaggtaagg 41220
gatttattgt taattttagt ttctttaaga ttgtgtccgg gtacaccatt cggtaagtgt 41280
aataatgttt tgtattggat tcacttgtgt tacgtgcatg tgatttacct tttcatttgt 41340
ttctgcgttc tgggtatgaa tttgacgaga ttccatggtc agctcaacat atcagttact 41400
gcgtgtcaag cgatcttata tggtatgcgc acaagcgatt gtatacggat atgacagtat 41460
aacgtgtgat attgatacga tgttcctttc ctttataaag gaacaaagac tttttaaaaa 41520
aaaagaaggg gtattactaa aaaccaaaat gtcaaaaaca aaatatcagt gcacatggca 41580
agtgtgcacg agcaatagct tgcccttacg ttcattattt agcatgtact actactaact 41640
acgcaaaaat caattcaccg attattaaac tgttaacatc attttagcac gttaacatat 41700
gtttcattca acacaccggt tttggcacat ttacaaactt gcaaagttgc aatactccct 41760
tcgttacata gcataagaga ttttaggtga atgtgacaca tctatccaaa ttcattatac 41820
tagaatgtat caccgcctcc acgccgggag ggagagcgcc gccggtggag aaagggggag 41880
ggagtggtcg aggggaacca gtagggtgcc ctccccgtcg ccgcctcccc gtggccgcgc 41940
cggcgagaca ggaggaagag ggggatatgg agcggcgccg ccggtgaggg cgcgcgcgcg 42000
ggggggagcg gcgacgccgg tgaggaaggg aaggggagtg gtggctttga gagagatagg 42060
ggggaggaaa aatgatttta gagttagggt ttgggctgct gagtttttat atagatcggg 42120
atcaatcagg accgtccatc agatcggaca actacggctt ctcccgcgtt gggccgggtg 42180
ccactcctag gttgcccaca ctattgggcc acatgtacgc tccgcgtgaa ataagttcac 42240
tttaggtcct ttaagttgcc tctgaattgt tcccaggccg gccgcactat tgggccaccc 42300
cataggccat gtgtacgctc cgcacagaat aatttcgctt tagctccctt aatttgtccc 42360
ctcaaactcc taaaaccagt gcaaatcttt aatttttagt tcacccattg caactcacgg 42420
gcatatttgc tagtgacata taatatgaaa cgaaggatgt agcagactat agaatttaaa 42480

ctgtgctttc attttagagc atcactaact gttatttaga tttttattta aataaatgct 42540
gaaatgatgt ttttattatg aaaattagca ataaagctcc caaaatttca aaaaaaaatt 42600
aaaagagatt tattaatcat ggttaattta attaaaaatt aaatctaacc atatcatatt 42660
atttcacggt ccgtgatgag gaaatggcag ctgctatcac ttacggtggg agagaagggg 42720
cattgtttat ttttataact atctcttata actcccatga aactataaaa taaatataat 42780
cattatcata acattagttt tttttccatt gcaacgcaag ggtaattttt cagtacaata 42840
aaaaaaataa aagtgggcca ttctgaacgg aaatttctgg ttttttttcc caagagcgcc 42900
gcacacaact gcgcaagaga tcgatcgcga tcaccctgct cgtcgccgat ctcctacacc 42960
atccctgcca tctccttccc ctccactggc tgctgctgca cctgtcagct agggcggcca 43020
tggcgcgccg cgccgcttcc cgcgctgctg gcgcccttcg ctcggagggc tcgatccaag 43080
ggcgaggggg ccgcgcgggg ggcagtggcg gtggcgcgga ggacgcacgc cacgtgttcg 43140
acgaattgct ccgtcgtggc ataccagatg tcttctccta caatattctt ctcaacgggc 43200
tgtgtgatga gaacagaagc caagaagctc tcgagttact gcacataatg gctgatgatg 43260
gaggtgactg cccacctgat gtggtgtcgt acagcaccgt catcaatggc ttcttcaagg 43320
agggggatct ggacaaaatg cttgaccaga ggatttcgcc aaatgttgtg acctacaact 43380
ctattattgc tgcgctatgc aaggctcaaa ctgtggacaa ggccatggag gtacttacca 43440
ccatggttaa gagtggtgtc atgcctgatt gcatgacata taatagtatt gtgcatgggt 43500
tttgctcttc agggcagccg aaagaggcta ttgtatttct caaaaagatg cgcagtgatg 43560
gtgtcgaacc agatgttgtt acttataact cgctcatgga ttatctttgc aagaacggaa 43620
gatgcacgga agcaagaaag atttttgatt ctatgaccaa gaggggccta aagcctgata 43680
ttactaccta tggtaccctg cttcaggggt atgctaccaa aggagccctt gttgagatgc 43740
atggtctctt ggatttgatg gtacgaaacg gtatccaccc taatcattat gttttcagca 43800
ttctagtatg tgcatacgct aaacaagaga aagtagaaga ggcaatgctt gtattcagca 43860
aaatgaggca gcaaggattg aatccgaatg cagtgaccta tggaacagtt atagatgtac 43920
tttgcaagtc aggtagagta gaagatgcta tgctttattt tgagcagatg atcgatgaag 43980
gactaagacc tgacagcatt gtttataact ccctaattca tagtctctgt atctttgaca 44040
aatgggagaa ggctgaagag ttatttcttg aaatgttgga tcgaggcatc tgtcttagca 44100
ctattttctt taattcaata attgacagtc attgcaaaga agggagggtt atagaatctg 44160
gaaaactctt tgacttgatg gtacgaattg gtgtgaagcc cgatatcatt acccttggca 44220

ggtaagatgg atgaagcaat gaagttactt tctggcatgg tctcagttgg gttgaaacct 44280
aatactgtta cttatagcac tttgattaat ggctactgca aaattagtag gatggaagac 44340
gcgttagttc tttttaagga gatggagagc agtggtgtta gtcctgatat tattacgtat 44400
aacataattc tgcaaggttt atttcaaacc agaagaactg ctgctgcaaa agaactctat 44460
gtcaggatta ccgaaagtgg aatgcagatt gaactttgtt agatttaatt ggataattaa 44520
tccatttaaa tcaattaaat caaataaatt ccaaggctca ttatgctagg aattcatgtg 44580
aattcattct tctatgggat atcaatggga tgaagagttt tgagaattaa tccatttgat 44640
taaggaattg gtaacttata tcaattaatc ctaattgatg gatggttgat ggttgtgtag 44700
tggaggatgg ttcatggcta gttgatgaca attagttgct ctattcctct tcctattcca 44760
ttggtaactt acatcaatta ctcttaattg attgttggtt gatggttgtg tagtggagga 44820
tggttcatgg ctagttgatg acaattagtt gctccattcc tcttcctatt ccatgactct 44880
tactcttcat cttccattcc tcttataaaa tgagaatgga tttgatctcc cgcgagaaga 44940
agaagacaca ctttcatcca ttttcaaaag ctgttgctgc tacggtaatc ccatcccgac 45000
gagtgtgtgc acacgcgttg ggagagtagg cctccgaaac cacgcgttgc tgcgacgttt 45060
gcacagacgg gcgggcgatc aggtttttgg ggagcgcaag gcgcgactac tcactgttcg 45120
tcaacatcta cttcatcttc accaacatgt cgaacactgg agacaaggag aaggagactc 45180
ccgtcaacac caacggaggc aatactgcct caaactccag cggaggacca ttcttggggt 45240
ataaccttat tacattattt caattagaag ttttactgtt aatgttcatc gcaatgtcaa 45300
cattgtgtca ttatgtgatt gttgatgctt attcaacgtt aagcatgctc atgttgatta 45360
cattcaccac tatcactgga tcaaatccta ttgtaaatat catgtttatt atcttgttat 45420
tttggattaa aatatgccga attatgacca aatttccaac aaacttagca catacaacat 45480
aatccttcat ggactttgca aaaacaaact cactgatgat gcacttcgaa tgtttcagaa 45540
cctatgtttg atggatttga agcttgaggc taggactttc aacattatga ttgatgcatt 45600
gcttaaagtt ggcagaaatg atgaagccaa ggatttgttt gttgctttct cgtctaacgg 45660
tttagtgccg aattattgga cgtacagatt gatggctgaa aatattatag gacaggggtt 45720
gctagaagaa ttggatcaac tctttctttc aatggaggac aatggctgta ctgttgactc 45780
tggcatgcta aatttcattg ttagggaact gttgcagaga ggtgagataa ccagggctgg 45840
cacttacctt tccatgattg atgagaagca cttttccctc gaagcatcca ctgcttcctt 45900
gtttatagat cttttgtctg ggggaaaata tcaagaatat catatatttc tccctgaaaa 45960

atacaagtcc tttatagaat ctttgagctg ctgaagcatt ttgcagcttt gaaattctgt 46020
gttggaattc ttttctccta cagtccgatt agaggaggga tcttctctgt atgtgtaaat 46080
agcgaggtat gtatgtcacc tctccgaatt attttgactg tggttcctgg actgtaaaca 46140
agctattatc ttctggtgtt gatgccagaa aaaacacaaa agtttgtcgt tatctctact 46200
aacggatcat aaaggggttt gtaactggag tttcaaactt aaggtatcta ggcagtaggt 46260
atatattgat cctacatctt atgatcttaa gatgatatcc ttctcattat cctctgctga 46320
aactttagct tgaaccgtca tctacaccac aatttgagcc ccttagcaca gagcacaacg 46380
agcaatagct tgcccttacg ttcattattt agcatgcact actactaact acccaataat 46440
caatacatcg gttattaaac tgtttgtaca gtttaataat gtcattttat cacgttaaca 46500
tatgtttcat tcaacaccac accggttttg gcacagttgc aaacttgcaa taacattttt 46560
actacttctc cgccccataa tataacaatc tcgttccata ctatattgct atattacggg 46620
acggatgaag tacttctttc cttccaaaat ataagaatct agtcctagat tagatattat 46680
ttggattcac gaatttgatt aggctatcta gatttgtagt cgtatgtaat gtctaattcg 46740
gtaataggtt attacctctt tggatggagg gagtagtttt tatttcgtac tccctctgtt 46800
tcatattata agttgttttg actttttctt tagtcaaatt ttattgagtt tgactaaatt 46860
tatagaaaaa aaattagcaa catttaagca ccacattagt ttcattaaat gtagcatgga 46920
atatattttt ataatatgtt tgttttttta ttaaaatgct actatatttt tctataaatg 46980
tagccaaatt taaagaagtt tgattacgaa aaaaaatcaa aatgacatat aatatgaaac 47040
tgaggatgta gcagactata gcaaatttaa actatgcttt tattttagag catcaccaaa 47100
agagatagcc taaatcttat cttaactaat taaaatattc ataattttcc tttcgtcaca 47160
ttaaattttc gtccgtaaat ccgattgaaa tccaactaga caatccaaaa aatagagaaa 47220
aagaacagaa aaaataataa aaagcacaca aatcttatct caatcccgcg ggaagctgcc 47280
gatgccgccg aatccgctcg agcgccgccg ccgccgctca cggggaacga tgtcgctgct 47340
atcgcacgtg gtatgggagg gcgccgccgc cgctgcttgg gagataggat atggagagag 47400
aaggaaatgt gagggagggt taggtttttc cccattcgta tcttcagcga cacggaggcg 47460
atccaagctg tccatcagat cagacggctc agaacgcctc catcttcagg ccgcgcatgc 47520
ttgatgggcc gagggaaggc cggagggtcg aacaaacgta gtcagaggag gagttggagg 47580
aggtaaagta gaatttattt gcgggctgag atagtaaatg gactgaaaat ggcccataga 47640
gaaattggga attttattta aataaatgtt gaaaaggtgt ttatattatc aaaattagaa 47700

attaagctcc gaaaatttta aaaaatattc aaagagcatt attaatcatg attaatttaa 47760
taaaaattaa atccaaccat atcatattat ttcacggcgc gcagtaggaa aatgcgcagc 47820
tgttgtcgct tacggtggga gagaagggac attgtttatt ttcagaacta tcttttataa 47880
ctcccatgga actttaaaat aaatataatc attattatag cattagtttt tttctgtctt 47940
ttttttcccc aagagcgccg cgcagaagag atcgatcgcg atctccctgc cccgacgtcg 48000
ccggccgatc tctcattctc tccacgccct gctcgtcgcc gatctcctac accatccctg 48060
ccatctcctc cttcccctcc cctctatcct ccactggtgc cgcccacctc tccgtataag 48120
acaaactgcg ttgcggcgtt ggtttccgcc ggcgctgctg ctgcacctgt cagctagggc 48180
gggcatggcg cgccgcgccg cttcccgcgc tgttggcgcc cttcgctcgg acggctcgat 48240
ccaagggcga ggaggccgcg cggggggcag tggcgccgag gacgcacgcc acgtgttcga 48300
cgaattgctc cggcgtggca ggggcgcctc gatctacggc ttgaaccgcg ccctcgccga 48360
cgtcgcgcgt cacagccccg cggccgccgt gtcccgctac aaccgcatgg cccgagctgg 48420
cgccgacgag gtaactcccg acttgtgcac ctacggcatt ctcatcggtt gctgctgccg 48480
cgcgggccgc ttggacctcg gtttcgcggc cttgggcaat gtcattaaga agggatttag 48540
agtggaagcc atcaccttca ctcctctgct caagggcctc tgtgccgaca agaggacgag 48600
cgacgcaatg gacatagtgc tccgcagaat gaccgagctc ggttgcatac caaatgtctt 48660
ctcctacaat aatcttctca acgggctgtg tgatgagaac agaagccaag aagctctcga 48720
gttgctgcac atgatggctg atgatcgagg aggaggtagc ccacctgatg tggtgtcgta 48780
taccactgtc atcaatggct tcttcaaaga gggggattca gacaaagctt acagtacata 48840
ccatgaaatg ctggaccggg ggattttacc tgatgttgtg acctacagct ctattattgc 48900
tgcgttatgc aagggtcaag ctatggacaa gccatggagg tacttaccac gatggttaag 48960
aatggtgtca tgcctgattg catgacatat aatagttatt tcttgaaatg ttggatcgag 49020
gcatttgtct ggacactatt ttctttaatt caataattga cagtcattgc aaagaaggga 49080
gggttataga atctgaaaaa ctctttgacc tgatggtacg tattggtgtg aagcctgata 49140
tcattacata cagtacactc atcgatggat attgcttggc aggtaagatg gatgaagcaa 49200
tgaagttact ttctggcatg gtctcagttg ggttgaaacc taatactgtt acttatagca 49260
ctttgattaa tggctactgc aaaattagta ggatggaaga cgcgttagtt ctttttaagg 49320
agatggagag cagtggtgtt agtcctgata ttattacgta taacataatt ctgcaaggtt 49380
tatttcaaac cagaagaact gctgctgcaa aagaactcta tgtcaggatt accgaaagtg 49440

gaacgcagat tgaacttagc acatacaaca taatccttca tggactttgc aaaaacaaac 49500
tcactgatga tgcacttcag atgtttcaga acctatgttt gatggatttg aagcttgagg 49560
ctaggacttt caacattatg attgatgcat tgcttaaagt tggcagaaat gatgaagcca 49620
aggatttgtt tgttgctttc tcgtctaacg gtttagtgcc gaattattgg acgtacaggt 49680
tgatggctga aaatattata ggacaggggt tgctagaaga attggatcaa ctctttcttt 49740
caatggagga caatggctgt actgttgact ctggcatgct aaatttcatt gttagggaac 49800
tgttgcagag aggtgagata accagggctg gcacttacct ttccatgatt gatgagaagc 49860
acttttccct cgaagcatcc actgcttcct tgtttataga tcttttgtct gggggaaaat 49920
atcaagaata ttataggttt ctccctgaaa aatacaagtc ctttatagaa tctttgagct 49980
gctgaagcat tttgcagctt tgaaattctg tgttggaatt cttttctcct acagtcctat 50040
tagaggaggg atcttctctg tatgtgtaaa tagcgaggta tgtatgccac ctctccgaat 50100
tatttttact gtggttccta gactgtaaac aagcaattat gttatgctgt tgatgccaga 50160
aaaaacataa aagtttgtcg ttatctctac taacggatca taaaggattt tgtgactgga 50220
gtttcaaact taatgtgtct aggcagtaat tttgacatta gatccaaaac aatttatagg 50280
gtttcattaa atttcatcta tgtgtactgt ttaggtgttg aatagtttga cttgtttttt 50340
aactgaacaa aagatatgtc tgaagctttg ttctttacca aatgcagtac tgatcatcac 50400
aatatatttt ttatggaaca agattggatt gtatagaatg gtttccgatc tgattatctt 50460
atctcaacgt attattatgc acatgtacta atcatgaaat atctgatgga atgatgtttc 50520
tatttacctg tgtgaggcag caaggagtga gatggataac accacatact ccctctatcc 50580
cagaatataa gaagttttag agttggacac gattattaag aaagtaggta gaagtgagta 50640
gtggagggtt gtgattgcat gagtagtgga ggtaggtggg aaaagtgaat ggtggagggt 50700
tgtgattggt tgggaagaga atgttggtag agaagttgtt atattttggg gagtacatta 50760
ttattctaga acaatactgt tgtgctcaag aagcgttcca aagatgtttc acaacctgtg 50820
ctcgatgggt tttgagctta atcctgggac attcagtatc atgatctgtc tcattcttaa 50880
acatggaata aaggatgaca gcatgatttc tttgtctcta taatcttttg gctacccaca 50940
gataatagct gtaaatctat actactttaa aaggagtagt ggtggtggtg agtggtgaat 51000
ctgccaccac cccaccacca actctcaaaa ttctgacatg tgggatcact gtcaatccct 51060
tctccaagac atgtgggatc actgtcaatc ccttctccaa accaattgta tgatagaaca 51120
gtggaaatca cggacagacc atggagctct caaccataat catccttgcg agttaataac 51180

aaatggagcg taaacttggc aagcaaaaaa ctcaaattaa ttctaaaatt aagctctagg 51240
attcaaaata gatttcctct ctgcattgtg ctgttatgat ttttaattcc gtaacaacgc 51300
aaatgcattt tgctagtctt ataaagaagg gttaatgcaa atattctgat taaatgattg 51360
tatctatgaa gtttgaatgc tagtggaagc tcctttgacc atgttttgtt gtgcgagcat 51420
ttaagagagt gaagagaatg cttctttggt gctgttctgg tatggaagga tccacagata 51480
aaattcaggt tctactgctt ctctgcttgt aattttcatg aagctgcagt gaataccttg 51540
ttgaccactt gatctgttgc tttgaaggag aatatagtag tggccaaggt tggtgacggt 51600
gatggtggca tgtgatcccc cagatcttca gtgacccaga gaggagggga cggcgcgtgg 51660
tgagctacaa ggcatactca gtggagggca agatcaaggc ctcccgtccg taggggactc 51720
cgctgcatca aggccaactg ctccgaactg atcaatttct ggtacggatc acttctcctt 51780
tccttttttt tttcacctta agcactctct tgattcttcg ctgctacctc ccttaatttc 51840
tttcaatata ttgtggcact tgatcatggc ggagacccac cttccagtgt gaatggattt 51900
tgtcaaagaa ctaaatttat tccattagct tattttctga ttacatggaa gacattcttt 51960
tctggaataa atacagaact aaatcctgtt tcctgaataa aagttgttag tgtgtggcat 52020
ggtgcatttc cgcgcttcta aattttataa aacctgttca ttcaatttga acctgcatcc 52080
aatccaatat tttaggtgca gacaggtgct tgcggtcagg ttaaagaagt tggcaaaaat 52140
gcttctgaag aaaggttaat tgttgtttca tctcaggagg taatatgcag atgattattc 52200
caattggcat tgccttgcca tttttatcac gagtctttac aattttatat cctcctacat 52260
attctttcca gattccagat gatccagtgt ctccaacaat tgaggcgctt attttgctcc 52320
atagtaaagc aagtacactt gctgagaacc accagttgac aacacggctt gttgtaccat 52380
caaacaaagt tggttgtatt cttggggaag gtggaaaggt aattactgaa atgagaagac 52440
ggactggggc tgaaatccga gtctactcaa aagcagataa acctaagtac ctgtcttttg 52500
atgaggagct tgtgcaggta atttatttgg ccatacctac accagagatc catatattac 52560
ttttataact gcagttttta cttgttaaca tttcattgtg cttttacatt tgttccaagc 52620
tttcaggttg ctgggcttcc agctattgaa agaggagccc tgacagagat tgcttcgagg 52680
ctttgaacta ggacactcag agatggaagt tcttccaata atccgacacc ttttgcccct 52740
gttgatggtc ctcctgttga tatcttgcct aacaaggaat tcatgctata tggacgatct 52800
gctaatagtc ccccatatgg agggcctgct aatgatccac catatggaag acctgccatt 52860
gatccaccat atggaagacc aatatccaca atatggaaga cctgccaatg atccaccata 52920

tagaagacct gtcaatgata catcatattg agggttggac aatgatgggc ctcgtgatca 52980
ggcccggtcc tgaggggggt cgaatggggc gatcgctccg ggcccccgat tcccagggcc 53040
cccacctatc tgtgcaacga gtagtagcga tcttccagcg cgcaacgtga ggcgatgttt 53100
ctccgtgatt tcgccggcct gcaactgcga gatcgcgagt ataacgatca gccgatcgat 53160
ctcatctgcc gactgccatg ctgatgccac acgcaagcgc agcatatcag ccttatcttg 53220
gttgatcggc atgctggacg agcacatctg ttgtcgcatc aactgctgac tgctatatat 53280
gtgctggtgc tgaatcgatc gattgtcgtc gcggaagtga agaacaacca cggcactgct 53340
gcctgctggg ctctagccgc catcagtaag tacgctatac tgcctatcta gatctagatc 53400
gagattacat agtggaatta tctgtttata acaaaattac aaggtatcaa ttgataattt 53460
aaggttataa ccgtacaaac ttcagtgatt tgctggtttc acattggtta gatttgtttc 53520
aactaatttg gtacttctgt agccttgtaa tttacgaatc tagtattaat attttcttaa 53580
gtattagcct gttccttgat attatgctgt tgagaaagta tgcaatagat aacaaaaaca 53640
agtaggtgtg ttgaggatgc tcaagagtaa tacagccact tcaataattc tgatattatc 53700
aggacatcat caataattct gcgcctacaa atcttcaaag aaaattttaa tataatgcgt 53760
atgatttttt aaatacgaat attgattgct atttaaagat atttatatta tatggtaatt 53820
attatttgaa ggtttataat aaaggcctcc gtttttagtt tcacgctggg ccttcagaat 53880
ctcaggaccg gccctgctca tgatc 53905

<210> 29
<211> 24
<212> DNA
<213> artificial sequence
<220>
<223> Oligonucleotide primer for amplification
<400> 29
atcaggagcc ttcaaattgg gaac 24

<210> 30
<211> 24

<212> DNA
<213> artificial sequence
<220>
<223> Oligonucleotide primer for amplification
<400> 30
ctcgcaaatt gcttaatttt gacc 24

<210> 31
<211> 24
<212> DNA
<213> artificial sequence
<220>
<223> Oligonucleotide primer for amplification
<400> 31
tgaaggagtt atgggtgcgt gacg 24

<210> 32
<211> 24
<212> DNA
<213> artificial sequence
<220>
<223> Oligonucleotide primer for amplification
<400> 32
ttgccgagca cacttgccat gtgc 24

<210> 33
<211> 24
<212> DNA
<213> artificial sequence

<220>
<223> Oligonucleotide primer for amplification
<400> 33
gcgacgcaat ggacatagtg ctcc 24

<210> 34
<211> 24
<212> DNA
<213> artificial sequence
<220>
<223> Oligonucleotide primer for amplification
<400> 34
ttacctgcca agcaatatcc atcg 24

<210> 35
<211> 24
<212> DNA
<213> artificial sequence
<220>
<223> Oligonucleotide primer for amplification
<400> 35
aaggcatact cagtggaggg caag 24

<210> 36
<211> 24
<212> DNA
<213> artificial sequence
<220>
<223> Oligonucleotide primer for amplification

<400> 36
ttaacctgac cgcaagcacc tgtc 24

<210> 37
<211> 24
<212> DNA
<213> artificial sequence
<220>
<223> Oligonucleotide primer for amplification
<400> 37
tggatggact atgtggggtc agtc 24

<210> 38
<211> 24
<212> DNA
<213> artificial sequence
<220>
<223> Oligonucleotide primer for amplification
<400> 38
agtggaagtg gagagagtag ggag 24

<210> 39
<211> 24
<212> DNA
<213> artificial sequence
<220>
<223> Oligonucleotide primer for amplification
<400> 39
ccctccaaca cataaatggt tgag 24

<210> 40
<211> 24
<212> DNA
<213> artificial sequence
<220>
<223> Oligonucleotide primer for amplification
<400> 40
tttctgccag gaaactgtta gatg 24

<210> 41
<211> 24
<212> DNA
<213> artificial sequence
<220>
<223> Oligonucleotide primer for amplification
<400> 41
gcgatcttat acgcatacta tgcg 24

<210> 42
<211> 24
<212> DNA
<213> artificial sequence
<220>
<223> Oligonucleotide primer for amplification
<400> 42
aaagtctttg ttccttcacc aagg 24

<210> 43

<211>  26
<212>  DNA
<213>  artificial sequence
<220>
<223>  Oligonucleotide primer for amplification
<400>  43
gaggatttat caaaacagga tggacg     26

<210>  44
<211>  24
<212>  DNA
<213>  artificial sequence
<220>
<223>  Oligonucleotide primer for amplification
<400>  44
tgggcggcag cagtggagga taga     24

<210>  45
<211>  24
<212>  DNA
<213>  artificial sequence
<220>
<223>  Oligonucleotide primer for amplification
<400>  45
aagaagggag ggttatagaa tctg     24

<210>  46
<211>  24
<212>  DNA

<213>  artificial sequence
<220>
<223>  Oligonucleotide primer for amplification
<400>  46
atatcaggac taacaccact gctc     24

<210>  47
<211>  24
<212>  DNA
<213>  artificial sequence
<220>
<223>  Oligonucleotide primer for amplification
<400>  47
acgagtagta gcgatcttcc agcg     24

<210>  48
<211>  24
<212>  DNA
<213>  artificial sequence
<220>
<223>  Oligonucleotide primer for amplification
<400>  48
cagcgtgaaa ctaaaaacgg aggc     24

<210>  49
<211>  24
<212>  DNA
<213>  artificial sequence
<220>

<223>  Oligonucleotide primer for amplification
<400>  49
atcccacatc atcataatcc gacc     24


<210>  50
<211>  25
<212>  DNA
<213>  artificial sequence
<220>
<223>  Oligonucleotide primer for amplification
<400>  50
agcttctccc ttggatacgg tggcg     25


<210>  51
<211>  24
<212>  DNA
<213>  artificial sequence
<220>
<223>  Oligonucleotide primer for amplification
<400>  51
atttgttggt tagttgcggc tgag     24


<210>  52
<211>  24
<212>  DNA
<213>  artificial sequence
<220>
<223>  Oligonucleotide primer for amplification
<400>  52

gcccaaactc aaaaggagag aacc     24

<210>  53
<211>  24
<212>  DNA
<213>  artificial sequence
<220>
<223>  Oligonucleotide primer for amplification
<400>  53
cctcaagtct cccctaaagc cact     24

<210>  54
<211>  24
<212>  DNA
<213>  artificial sequence
<220>
<223>  Oligonucleotide primer for amplification
<400>  54
gctctactgc tgataaaccg tgag     24

<210>  55
<211>  24
<212>  DNA
<213>  artificial sequence
<220>
<223>  Oligonucleotide primer for amplification
<400>  55
tggatggact atgtggggtc agtc     24

<210> 56
<211> 24
<212> DNA
<213> artificial sequence
<220>
<223> Oligonucleotide primer for amplification
<400> 56
agtggaagtg gagagagtag ggag 24

<210> 57
<211> 24
<212> DNA
<213> artificial sequence
<220>
<223> Oligonucleotide primer for amplification
<400> 57
tacgacgcca tttcactcca ttgc 24

<210> 58
<211> 24
<212> DNA
<213> artificial sequence
<220>
<223> Oligonucleotide primer for amplification
<400> 58
catttctcta tgggcgttgc tctg 24

<210> 59
<211> 26

<212> DNA
<213> artificial sequence
<220>
<223> Oligonucleotide primer for amplification
<400> 59
acctgtaggt atggcacctt caacac 26

<210> 60
<211> 26
<212> DNA
<213> artificial sequence
<220>
<223> Oligonucleotide primer for amplification
<400> 60
ccaaggaacg aagttcaaat gtatgg 26

<210> 61
<211> 24
<212> DNA
<213> artificial sequence
<220>
<223> Oligonucleotide primer for amplification
<400> 61
tgatgtgttt gggcatccct ttcg 24

<210> 62
<211> 24
<212> DNA
<213> artificial sequence

<220>
<223> Oligonucleotide primer for amplification
<400> 62
gagatagggg acgacagaca cgac 24

<210> 63
<211> 26
<212> DNA
<213> artificial sequence
<220>
<223> Oligonucleotide primer for amplification
<400> 63
tcctatggct gtttagaaac tgcaca 26

<210> 64
<211> 24
<212> DNA
<213> artificial sequence
<220>
<223> Oligonucleotide primer for amplification
<400> 64
caagttcaaa cataactggc gttg 24

<210> 65
<211> 24
<212> DNA
<213> artificial sequence
<220>
<223> Oligonucleotide primer for amplification

<400>  65

cactgtcctg taagtgtgct gtgc     24

<210>  66

<211>  24

<212>  DNA

<213>  artificial sequence

<220>

<223>  Oligonucleotide primer for amplification

<400>  66

caagcgtgtg ataaaatgtg acgc     24

<210>  67

<211>  24

<212>  DNA

<213>  artificial sequence

<220>

<223>  Oligonucleotide primer for amplification

<400>  67

tgcctactgc cattactatg tgac     24

<210>  68

<211>  24

<212>  DNA

<213>  artificial sequence

<220>

<223>  Oligonucleotide primer for amplification

<400>  68

acatactacc gtaaatggtc tctg     24

## Description

### FIELD OF THE INVENTION

The present invention relates to a method for providing and inhibiting the rice fertility, and discerning the presence of the rice restorer gene by using the rice restorer gene to the rice BT type cytoplasmic male sterility.

The present application claims priority based on Japanese Patent Application No. 2001-285247 filed on September 19, 2001, Japanese Patent Application No. 2001-309135 filed on October 4, 2001 and Japanese Patent Application No. 2002-185709 filed on June 26, 2002. The entire disclosures of the three patent applications are incorporated herein.

### PRIOR ART

Rice is a self-fertilizing plant, so in order to perform crossing between varieties, self-fertilization must first be avoided by removing all stamens in a glumaceous flower just before flowering and, then fertilization is effected with pollens from the parent variety with which it is to be crossed. However, this manual crossing method is entirely unsuitable for producing a large quantity of hybrid seeds on a commercial scale.

Accordingly, hybrid rice is produced by the triple-crossing system which makes use of cytoplasmic male sterility. In the triple-crossing system, the following three lines are employed, i.e., a sterile line having male sterile cytoplasm, a restorer line having Rf-1 gene and a maintainer line having the same nuclear gene as that of the sterile line but not having any sterile cytoplasm. By using these three lines, (i) hybrid seeds can be obtained through fertilization of the sterile line with the pollen of the restorer line whereas (ii) the sterile line can be maintained through its fertilization with the pollen of the maintainer line.

When employing the BT type male sterile cytoplasm in the triple-crossing system, it is important to breed rice of the restorer line and to this end, it is necessary to ensure that the rice at every stage of breeding maintains Rf-1 gene and that the Rf-1 gene is homozygous at the final stage. It also becomes necessary in the triple-crossing system to check to ensure that the variety used as the restorer line possesses Rf-1 gene, or to check for the presence of Rf-1 gene in order to ensure that the resulting hybrid seeds have restored fertility.

In order to genotype the locus of Rf-1 gene in a plant, it has been necessary that F1 plants be first formed from hybrid seeds obtained by crossing the plant to be genotyped to a standard line and then self-fertilized, followed by investigating the incidence of individuals that can produce seeds at a frequency higher than a certain level (e.g. 70∼80% or more). The standard line refers to the maintainer line, the sterile line or a set of the two lines, and it is appropriately chosen depending upon whether the cytoplasm of the individual under test is of BT type or normal type or unknown. If the standard line is a sterile line, it is crossed to the individual under test as the female parent and if the standard line is a maintainer line, it is crossed as the male parent.

However, these techniques require a huge amount of labor and time to carry out. As a further problem, fertilization for seed production is sensitive to environmental factors and if an investigation is made in an unfavorable environment such as cold climate or insufficient daylight, sterility may be caused irrespective of the genotype constitution, with the result that genotyping of the locus of Rf-1 gene cannot be performed accurately.

With a view to solving these problems, it has recently been proposed that Rf-1 gene be checked for its presence by a technique of molecular biology. The technical idea of this technique lies in checking for the presence or absence of Rf-1 gene by detecting base sequences linked to Rf-1 gene (such sequences are hereunder referred to as DNA markers). Note that it is not possible to directly detect Rf-1 gene since the DNA sequence of Rf-1 gene has not been clarified so far.

For example, it has been reported that the locus of Rf-1 gene in rice is present on chromosome 10 and located between DNA marker (RFLP marker) loci G291 and G127 which can be used in restriction fragment length polymorphism analysis (RFLP) (Fukuta et al., 1992, Jpn J. Breed. 42 (supl. 1) 164-165). This is a known method of genotyping the locus of Rf-1 gene by investigating the genotypes of DNA marker loci G291 and G127 which are linked to Rf-1 gene.

However, the conventional molecular biology techniques have several problems. First, they use RFLP markers which need to be detected by Southern blot analysis. In order to perform Southern blot analysis, DNA at the microgram level needs to be purified from the individual under test and, in addition, there is a need to carry out a sequence of steps comprising treatment with restriction enzymes, electrophoresis, blotting, hybridization with a probe and signal detection; this not only involves considerable labor but it also takes about one week to obtain the test results.

The second problem is that since the gene map distance between RFLP marker loci G291 and G127 is as long as about 30 cM (corresponding to about 9000 kbp in rice DNA), the probability for the occurrence of double recombination in the region would be a few percent and hence, it is not always guaranteed that the genotype of the locus of Rf-1 gene can be estimated correctly by the markers.

Thirdly, when the presence of Rf-1 gene is estimated by detecting RFLP marker loci G291 and G127, not only Rf-1 gene but also the gene region between those loci are introduced into the fertility restorer line selected as the result of breeding. As a consequence, the introduced DNA sequence will have a chromosomal region of 30 cM or longer from the Rf-1 gene donor parent, and this presents the risk of introducing a deleterious gene that may potentially be present within that region.

In order to solve these problems, there have been developed a dominant DNA marker (Japanese Patent Public Disclosure No. 222588/1995) and a co-dominant DNA marker (Japanese Patent Public Disclosure No. 313187/1997), both of which are linked to the locus of Rf-1 gene. These markers are linked to the locus of Rf-1 gene, their genetic distances from Rf-1 gene respectively being 1.6 ± 0.7 cM (corresponding to about 480 kbp in rice DNA) and 3.7 ± 1.1 cM (corresponding to about 1110 kbp in rice DNA), and their loci being on opposite sides of the locus of Rf-1 gene. Hence, the presence of Rf-1 gene can be estimated by detecting the presence of both the locus of the dominant PCR marker and that of the co-dominant PCR marker. The use of the co-dominant PCR marker also enables us to estimate as to whether the locus of Rf-1 gene is homozygous or heterozygous.

However, the use of these PCR markers still involve several problems. The co-dominant marker has a genetic distance of 3.7 ± 1.1 cM from the locus of Rf-1 gene, and the problem of potentially high frequency of recombination with the locus of Rf-1 gene has not been fully dissolved. As a result, speaking of the co-dominant marker itself, correct detection can be made as to whether it is homozygous or a heterozygous. However, if recombination occurs between the locus of the co-dominant marker and that of Rf-1 gene, the genotype of Rf-1 gene locus cannot be determined correctly, particularly as to whether it is homozygous or heterozygous. On the other hand, if the dominant marker is used to genotype the locus of Rf-1 gene, the marker will detect individuals indiscriminately irrespective of whether they are homozygous (Rf-1/Rf-1) or heterozygous (Rf-1/rf-1) with respect to Rf-1 gene. Therefore, even if the co-dominant marker is used in combination with the dominant marker in order to genotype the locus of Rf-1 gene, it is not possible to correctly distinguish individuals having Rf-1 gene homozygously from those having the gene heterozygously. Further, if no amplification product is obtained in PCR using the dominant marker, one cannot deny the possibility that this is due to some problems in the experimental procedure. As a further problem, since the genetic distance between the co-dominant marker and the dominant marker is as great as about 5.3 cM (around 1590 kbp), the size of the chromosomal region introduced from the Rf-1 gene donor parent cannot be limited to a sufficiently small value to prevent any concomitant introduction of a deleterious gene which may be contained in that region.

Japanese Patent Public Disclosure No. 139465/2000 describes co-dominant PCR markers that were developed on the basis of the base sequences of RFLP markers located in the neighborhood of Rf-1 gene on chromosome 10 of rice. However, most of those PCR markers are spaced from the Rf-1 gene by a genetic distance greater than about 1 cM.

### SUMMARY OF THE INVENTION

An object of the present invention is to provide methods for restoring rice fertility. A method of the present invention comprises introducing a nucleic acid into rice, wherein the nucleic acid has the base sequence of SEQ ID NO.27, or has a base sequence which is identical to at least 70% of the base sequence of SEQ ID NO.27, and which functions to restore fertility. Another method of the present invention comprises introducing a nucleic acid into rice, wherein the nucleic acid has the base sequence of bases 38538-54123 of SEQ ID NO.27, or has a base sequence which is identical to at least 70% of the base sequence of bases 38538-54123 of SEQ ID NO.27, and which functions to restore fertility. Still another method of the present invention comprises introducing a nucleic acid into rice, wherein the nucleic acid has the base sequence of bases 42357-53743, more preferably, bases 42132-48883 of SEQ ID NO.27, or has a base sequence which is identical to at least 70% of the base sequence of bases 42357-53743, more preferably, bases 42132-48883 of SEQ ID NO.27, and which functions to restore fertility. In an embodiment of the methods of the present invention, a base sequence which is identical to at least 70% of the base sequence of SEQ ID NO.27 or of the base sequence of bases 38538-54123 of SEQ ID NO.27 meets at least one of the following requirements 1) and 2):
1) a base corresponding to the base 45461 of SEQ ID NO.27 is A; and
2) a base corresponding to the base 49609 of SEQ ID NO.27 is A.

Another object of the present invention is to provide a method for discerning whether or not a subject rice individual or a seed thereof has the Rf-1 gene or not. The discerning method of the present invention utilizes a fact that a sequence determining the presence of the function of the rice restorer gene (the Rf-1 gene) positions between the polymorphism detection marker loci P4497 MboI and B56691 Xab I on rice chromosome 10.

In an embodiment of the methods of the present invention, the subject rice individual or the seed thereof is determined to have the Rf-1 gene, in the case that the nucleic acid having a base sequence which is identical to at least 70% of the base sequence of SEQ ID NO.27 or of the base sequence of bases 38538-54123 of SEQ ID NO.27, meets at least one of the following requirements 1) and 2):
1) a base corresponding to the base 45461 of SEQ ID No.27 is A; and
2) a base corresponding to the base 49609 of SEQ ID NO.27 is A.

Another object of the present invention is to provide a method for inhibiting the function of the Rf-1 gene to restore fertility. The inhibition method of the present invention comprises, in an embodiment, introducing an antisense having at least 100 continuous bases in length, and having a base sequence complementary to a nucleic acid having the base sequence of SEQ ID NO.27, or to a nucleic acid having a base sequence which is identical to at least 70% of the base sequence of SEQ ID NO.27, and which functions to restore fertility. In another embodiment, the inhibition methods of the present invention comprise introducing an antisense having at least 100 continuous bases in length, and having a base sequence complementary to a nucleic acid having the base sequence of bases 38538-54123 of SEQ ID NO.27, or to a nucleic acid having a base sequence which is identical to at least 70% of the base sequence of bases 38538-54123 of SEQ ID NO.27, and which functions to restore fertility.

Another object of the present invention is to provide a nucleic acid having the base sequence of SEQ ID NO.27, or a nucleic acid having a base sequence which is identical to at least 70% of the base sequence of SEQ ID NO.27, and which functions to restore fertility. The present invention also provides a nucleic acid having the base sequence of bases 38538-54123 of SEQ ID NO.27, or a nuclei acid having a base sequence which is identical to at least 70% of the base sequence of bases 38538-54123 of SEQ ID NO.27, and which functions to restore fertility. The present invention also provides a nucleic acid having the base sequence of bases 42357-53743, more preferably, bases 42132-48883 of SEQ ID NO.27, or a nucleic acid having a base sequence which is identical to at least 70% of the base sequence of bases 42357-53743, more preferably, bases 42132-48883 of SEQ ID NO.27, and which functions to restore fertility.

### BRIEF DESCRIPTION OF THE DRAWING

Fig. 1 shows the results of chromosomal walking started from the RFLP marker locus S12564.
Fig. 2 shows an alignment of lambda clone contigs in relation to the BAC clone AC068923.
Fig. 3 shows the chromosomal organization of recombinant pollens proximal to the Rf-1 locus (all fertile) as mapped in close proximity to the Rf-1 locus based on the genotypes at the marker loci of 10 individuals (RS1, RS2, RC1-8) generated from the pollens. White bars represent japonica regions and black bars represent indica regions.
Fig. 4 is a gene map in which the locus of Rf-1 gene on chromosome 10 of rice is positioned on a linkage map in relation to various markers; the values of map distance were calculated from the segregation data from 1042 F1 individuals.
Fig. 5 shows fragments from 10 genomic clones used for the identification of the Rf-1 region by complementation assays. Lambda clones obtained by chromosomal walking (thin lines) were used for complementation assays of the chromosomal regions shown by bold lines. XSF18 was found to contain a deletion shown by dotted line.
Fig. 6 shows the results of complementation assays using a 15.7 kb fragment from XSG16 (Example 10) and a 16.2 kb fragment from XSF18 (Example 8). The plant transformed with the 15.7 kb fragment from XSG16 has restored fertility as proved by ears bowing.

### BEST MODES FOR PERFORMING THE INVENTION

We began by restricting the Rf-1 locus to a very small region on chromosome 10. On this basis, we developed PCR markers proximal to the Rf-1 locus and found a method for detecting the Rf-1 gene by utilizing on the linkage of these PCR markers to the Rf-1 locus. Specifically, the presence of the Rf-1 gene is tested and individuals homozygous for the Rf-1 gene are selected by genotyping at the novel PCR marker loci proximal to the Rf-1 locus on the basis that the Rf-1 locus is mapped between the PCR marker loci S12564 Tsp509I and C1361 MwoI on chromosome 10 of rice. We previously filed a patent application for the method for detecting the Rf-1 gene under Japanese Patent Application No. 2000-247204 on August 17, 2000. The entire disclosure of the patent application is incorporated herein by reference.

### I. Methods for estimating the genotype at the Rf-1 locus described in Japanese Patent Application No. 2000-247204

Japanese Patent Application No. 2000-247204 describes methods for determining whether or not a rice individual or seed under test has the Rf-1 gene on the basis that the Rf-1 locus is mapped between the PCR marker loci S12564 Tsp509I and C1361 MwoI on chromosome 10 of rice.

### Markers

Primer pairs designed to be specific to particular regions near the locus of Rf-1 gene are used in PCR and the amplification products are treated with particular restriction enzymes; upon electrophoresis, rice of indica lines in some cases provide an observable band of a different size from that of rice of Japonica lines. This band which is characteristic of indica lines is herein referred to as the Rf-1 linked band. Now that it has been made clear by the present inventors that the locus of Rf-1 gene is located between PCR markers S12564 Tsp509I and C1361 MwoI on chromosome 10 of rice, the skilled artisan can appropriately develop and employ PCR markers that are present in the neighborhood of Rf-1 gene.

For instance, according to the invention, a rice individual under test is checked to see if its genome contains at least one of the PCR markers listed below, thereby determining whether the individual under test has Rf-1 gene linked to those PCR markers:
(1) marker 1: PCR marker R1877 EcoRI which, when rice genomic DNA is subjected to PCR with DNA primers having the sequences of SEQ ID NO:1 and SEQ ID NO:2, can detect polymorphisms between rice individuals of the japonica and indica lines depending on whether the amplification products have a recognition site for restriction enzyme EcoRI;
(2) marker 2: PCR marker G4003 HindIII (SEQ ID NO:19) which, when rice genomic DNA is subjected to PCR with DNA primers having the sequences of SEQ ID NO:3 and SEQ ID NO:4, can detect polymorphisms between rice individuals of the japonica and indica lines depending on whether the amplification products have a recognition site for restriction enzyme HindIII;
(3) marker 3: PCR marker C1361 MwoI (SEQ ID NO:20) which, when rice genomic DNA is subjected to PCR employing DNA primers having the sequences of SEQ ID NO:5 and SEQ ID NO:6, can detect polymorphisms between rice individuals of the japonica and indica lines depending on whether the amplification products have a recognition site for restriction enzyme MwoI;
(4) marker 4: PCR marker G2155 MwoI (SEQ ID NO:21) which, when rice genomic DNA is subjected to PCR with DNA primers having the sequences of SEQ ID NO:7 and SEQ ID NO:8, can detect polymorphisms between rice individuals of the japonica and indica lines depending on whether the amplification products have a recognition site for restriction enzyme MwoI;
(5) marker 5: PCR marker G291 MspI (SEQ ID NO:22) which, when rice genomic DNA is subjected to PCR with DNA primers having the sequences of SEQ ID NO:9 and SEQ ID NO:10, can detect polymorphisms between rice individuals of the japonica and indica lines depending on whether the amplification products have a recognition site for restriction enzyme MspI;
(6) marker 6: PCR marker R2303 BslI (SEQ ID NO:23) which, when rice genomic DNA is subjected to PCR with DNA primers having the sequences of SEQ ID NO:11 and SEQ ID NO:12, can detect polymorphisms between rice individuals of the japonica and indica lines depending on whether the amplification products have a recognition site for restriction enzyme Bs1I;
(7) marker 7: PCR marker S10019 BstUI (SEQ ID NO:24) which, when rice genomic DNA is subjected to PCR with DNA primers having the sequences of SEQ ID NO:13 and SEQ ID NO:14, can detect polymorphisms between rice individuals of the japonica and indica lines depending on whether the amplification products have a recognition site for restriction enzyme BstUI;
(8) marker 8: PCR marker S10602 KpnI (SEQ ID NO:25) which, when rice genomic DNA is subjected to PCR with DNA primers having the sequences of SEQ ID NO:15 and SEQ ID NO:16, can detect polymorphisms between rice individuals of the japonica and indica lines depending on whether the amplification products have a recognition site for restriction enzyme KpnI; and
(9) marker 9: PCR marker S12564 Tsp509I (SEQ ID NO:26) which, when rice genomic DNA is subjected to PCR with DNA primers having the sequences of SEQ ID NO:17 and SEQ ID NO:18, can detect polymorphisms between rice individuals of the japonica and indica lines depending on whether the amplification products have a recognition site for restriction enzyme Tsp509I.

Assuming that the locus of Rf-1 gene was highly likely to be located near the nine RFLP marker regions R1877, G291, R2303, S12564, C1361, S10019, G4003, S10602 and G2155 on chromosome 10 of rice (see the results of RFLP linkage analysis described in Fukuta et al., 1992, Jpn. J. Breed. 42 (supl. 1) 164-165 and the RFLP linkage map of rice described in Harushima et al., 1998, Genetics, 148, 479-494), the present inventors converted those RFLP markers to co-dominant PCR markers such as CAPS markers or dCAPS markers as described below in Reference example 1 (Michaels and Amasino, 1998, The Plant Journal, 14(3), 381-385; Neff et al., 1998, The Plant Journal, 14(3), 387-392). As a result of this conversion, the PCR markers above have been obtained.

Among these PCR markers, one group consisting of PCR markers R1877 EcoRI, G291 MspI (SEQ ID NO:22), R2303 BslI (SEQ ID NO:23) and S12564 Tsp509I (SEQ ID NO:26) and the other group consisting of PCR markers C1361 MwoI (SEQ ID NO:20), S10019 BstUI (SEQ ID NO:24), G4003 HindIII (SEQ ID NO:19), S10602 KpnI (SEQ ID NO:25) and G2155 MwoI (SEQ ID NO:21) are on opposite sides of the locus of Rf-1 gene on chromosome 10 of rice.

Therefore, in one embodiment, the presence of the Rf-1 gene is detected by detecting Rf-1 linked bands by (a) at least one PCR marker selected from the group consisting of PCR markers R1877 EcoRI, G291 MspI, R2303 BslI and S12564 Tsp509I, and (b) at least one PCR marker selected from the group consisting of PCR markers C1361 MwoI, S10019 BstUI, G4003 HindIII, S10602 KpnI and G2155 MwoI. In this case, at least S12564 Tsp509I from group (a) and at least C1361 MwoI from group (b) are preferably used as the closest PCR markers to the Rf-1 gene. If Rf-1 linked bands are detected with PCR markers of both (a) and (b) in the genome of the rice under test, it can be estimated with a high probability that the rice contains Rf-1 gene.

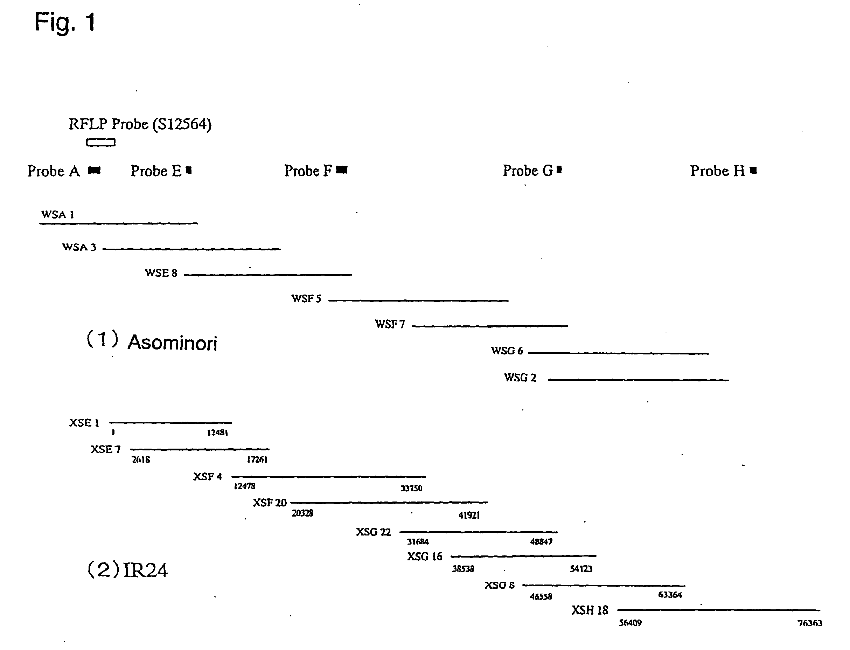

In another embodiment, Rf-1 linked bands are detected by at least two PCR markers of group (a) and at least two PCR markers of group (b) above. For example, a rice individual carrying the Rf-1 gene with a minimum of unwanted gene regions can be selected by picking up an individual in which Rf-1 linked bands are detected by markers of groups (a) and (b) more proximal to the Rf-1 gene but not detected by markers of groups (a) and (b) more distal from the Rf-1 gene on the gene map shown in Fig. 1. Again, it is preferred that at least one PCR marker of group (a) is S12564 Tsp509I and at least one PCR marker of group (b) is C1361 MwoI. Thus, the two PCR marker loci S12564 Tsp509I and C1361 MwoI are separated by a genetic distance of 0.3 cM. By utilizing this characteristic, the chromosomal region that is introduced from the Rf-1 gene donor parent can be narrowed down to a size of about 1 cM. This helps minimize the possibility of introducing into the restorer line a deleterious gene that may be present in the neighborhood of Rf-1 gene in the donor parent.

### Detection of the Rf-1 gene

In order to detect Rf-1 gene in the genome of a rice under test, any one of the above PCR markers is amplified from the genome of the rice by PCR using primers of SEQ ID NOS: 1-18 above and then detected by the polymerase chain reaction-restriction fragment length polymorphism method (PCR-RFLP). PCR-RFLP is a method that is applicable to the case where polymorphisms exist among variety lines at recognition sites of restriction enzymes in the sequences of PCR amplified DNA fragments and by which specific polymorphisms can conveniently be identified on the basis of cleavage patterns with those restriction enzymes (D.E. Harry et al., Theor. Appl. Genet. (1998), 97:327-336)

Restriction enzyme cleavage patterns show the bands as shown in Table 1 below on a visualized gel depending on the primer pair used.

**Table 1**

| | Approximate size (bp) of detected band |
|---|---|
| Detection of marker 1 (R1877 EcoRI) with primer pair 1 | |
| When the genome of test rice has Rf-1 gene homozygously | 1500 and 1700 |
| When the genome of test rice has Rf-1 gene heterozygously | 1500, 1700 and 3200 |
| When the genome of test rice has no Rf-1 gene | 3200 |

| Detection of marker 2 (G4003 HindIII) with primer pair 2 | |
|---|---|
| When the genome of test rice has Rf-1 gene homozygously | 362 |
| When the genome of test rice has Rf-1 gene heterozygously | 95, 267 and 362 |
| When the genome of test rice has no Rf-1 gene | 95 and 267 |

| Detection of marker 3 (C1361 MwoI) with primer pair 3 | |
|---|---|
| When the genome of test rice has Rf-1 gene homozygously | 50 and 107 |
| When the genome of test rice has Rf-1 gene heterozygously | 25, 50, 79 and 107 |
| When the genome of test rice has no Rf-1 gene | 25, 50 and 79 |

| Detection of marker 4 (G2155 MwoI) with primer pair 4 | |
|---|---|
| When the genome of test rice has Rf-1 gene homozygously | 25, 27 and 78 |
| When the genome of test rice has Rf-1 gene heterozygously | 25, 27, 78 and 105 |
| When the genome of test rice has no Rf-1 gene | 25 and 105 |

| Detection of marker 5 (G291 MspI) with primer pair 5 | |
|---|---|
| When the genome of test rice has Rf-1 gene homozygously | 25, 49 and 55 |
| When the genome of test rice has Rf-1 gene heterozygously | 25, 49, 55 and 104 |
| When the genome of test rice has no Rf-1 gene | 25 and 104 |

| Detection of marker 6 (R2303 BslI) with primer pair 6 | |
|---|---|
| When the genome of test rice has Rf-1 gene homozygously | 238, 655 and 679 |
| When the genome of test rice has Rf-1 gene heterozygously | 238, 655, 679 and 1334 |
| When the genome of test rice has no Rf-1 gene | 238 and 1334 |

| Detection of marker 7 (S10019 BstUI) with primer pair 7 | |
|---|---|
| When the genome of test rice has Rf-1 gene homozygously | 130, 218 and 244 |
| When the genome of test rice has Rf-1 gene heterozygously | 130, 218, 244 and 462 |
| When the genome of test rice has no Rf-1 gene | 130 and 462 |

| Detection of marker 8 (S10602 KpnI) with primer pair 8 | |
|---|---|
| When the genome of test rice has Rf-1 gene homozygously | 724 |
| When the genome of test rice has Rf-1 gene heterozygously | 117, 607 and 724 |
| When the genome of test rice has no Rf-1 gene | 117 and 607 |

| Detection of marker 9 (S12564 Tsp509I) with primer pair 9 | |
|---|---|
| When the genome of test rice has Rf-1 gene homozygously | 41 and 117 |
| When the genome of test rice has Rf-1 gene heterozygously | 26, 41, 91 and 117 |
| When the genome of test rice has no Rf-1 gene | 26. 41 and 91 |

### II. Identification of the Rf-1 locus

As described above, Japanese Patent Application No. 2000-247204 discloses RFLP-PCR markers based on our finding that the Rf-1 locus is mapped between DNA marker loci S12564 Tsp509I and C1361 MwoI. Fertility-restoring lines are established by backcrossing the Rf-1 gene into a normal japonica variety not containing the Rf-1 gene. If the method for identifying the Rf-1 locus described in Japanese Patent Application No. 2000-247204 is used during this process, not only the restoring lines can be established efficiently (within 2-3 years) but also the length of insert fragments can be controlled.

However, introduction by crossing inevitably introduce regions proximal to Rf-1 at the same time. Japanese Patent Application No. 2000-247204 showed that the Rf-1 locus is mapped between DNA marker loci S12564 Tsp509I and C1361 MwoI, but the distance between both loci is about 0.3 cM, i.e. about 90 kbp. If a deleterious gene existed proximal to Rf-1, it would be undeniable that the deleterious gene might be inserted together with the Rf-1 gene.

Thus, we searched for regions linked to the Rf-1 gene between DNA marker loci S12564 Tsp509I and C1361 MwoI by chromosomal walking and genetic analysis based on the close linkage between the Rf-1 locus and the DNA marker locus S12564 Tsp509I. As a result, we successfully identified the region of the Rf-1 locus including the Rf-1 gene upto about 76 kb and determined the entire base sequence of said region. According to the present invention, it is possible to introduce the function of a fertility restorer gene into BT male sterile cytoplasms by genetic engineering techniques.

Specifically, in Japanese Patent Application No. 2000-247204, linkage analyses on a population of 1042 individuals prepared by pollinating MS Koshihikari with MS-FR Koshihikari (heterozygous at the Rf-1 locus) revealed one recombinant between the Rf-1 and S12564 Tsp509I loci and two recombinants between the Rf-1 and C1361 MwoI loci (Reference examples 1-2 herein). In the present invention, 4103 individuals were added to the population to analyze a total of 5145 individuals. As a result, one recombinant between the Rf-1 and S12564 Tsp509I loci and six recombinants between the Rf-1 and C1361 MwoI loci were newly found with a total of 2 and 8 recombinants. These 10 individuals were tested by the high-precision segregation analysis of the present invention as recombinants proximal to the Rf-1 locus (Example 1).

The frequency of 8 recombinants between the Rf-1 and C1361 MwoI loci as compared with 2 recombinants between the Rf-1 and S12564 Tsp509I loci means that the S12564 Tsp509I locus is genetically closer to the Rf-1 locus than the C1361 MwoI locus. Genetic distance (expressed in recombination frequency: cM) and physical distance (expressed in the number of base pairs: bp) are not always proportional to each other, but it can be normally expected that physical distance decreases with genetic distance.

Thus, we tried to isolate the Rf-1 locus by chromosomal walking started from the S12564 Tsp509I locus (Example 2). Chromosomal walking was performed on a genomic library prepared from λ DASH II vector using the genomic DNA of an indica variety IR24 and a japonica variety Asominori. IR24 is a variety carrying Rf-1, while Asominori is a variety not carrying Rf-1. As a result of chromosomal walking, contigs covering a chromosomal region of about 76 kb (ordered sets of overlapping clones on a chromosome) were able to be prepared from genomic clones of IR24, and the entire base sequence (76363 bp) thereof was determined.

Then, 12 markers were newly developed on the basis of the base sequence data or the like obtained and a high-precision segregation analysis was performed on the 10 recombinants proximal to Rf-1 locus described above (Example 3). As a result, a 65 kb sequence included in the chromosomal region of about 76 kb above was shown to contain a sequence determining the presence of the function of the Rf-1 gene. This region is covered by a contig consisting of 8 genomic clones. Each clone has a length of about 12-22 kb and has overlapping domains of at least 4.7 kb. Genes for rice are known to have a wide range of lengths (from short ones to large ones), but most of them seem to have a length of several kbs or less. Thus, at least one of these 8 genomic clones is expected to contain the full-length Rf-1 gene.

We further restricted the Rf-1 gene region in the chromosomal region of about 76 kb above and performed complementation assays to directly demonstrate the presence of a fertility restoring ability.

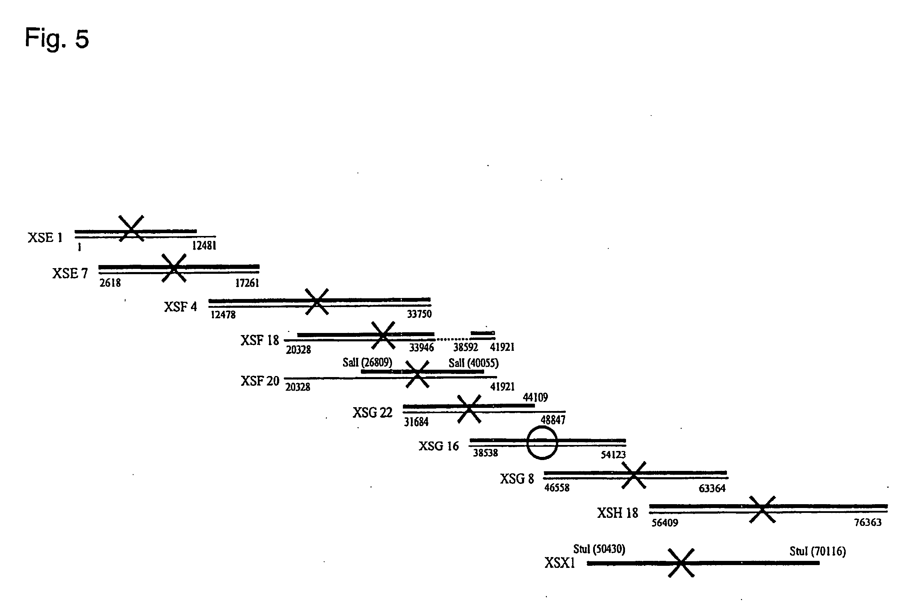

Specifically, 10 partial fragments (each 10-21 kb) in the above region of 76 kb were separately introduced into immature seeds of a male sterility line MS Koshihikari by genetic engineering techniques (Fig. 5). Of the 10 partial fragments used, 8 fragments are derived from 8 genomic clones previously obtained by chromosomal walking (XSE1, XSE7, XSF4, XSF20, XSG22, XSG16, XSG8 and XSH18 shown in Fig. 1 and described in Example 3). Additionally, fragments derived from 2 clones XSF18 and XSX1 were also analyzed by complementation assays. XSF18 is identical to XSF20 at the 5' and 3' ends (bases 20328 and 41921 of SEQ ID NO: 27, respectively), but lacks internal bases 33947-38591. This is because clone XSF18 was initially isolated but found to contain the above deletion during amplification after isolation, and therefore, the amplification step was freshly taken to isolate a complete clone designated XSF20 (Example 8). XSX1 is a clone freshly prepared from clones XSG8 and XSH18 by restriction enzyme treatment and ligation to contain sufficient overlapping domains because of the overlapping domains of both clones are relatively small (about 7 kb) (Example 13).

If the insert fragment completely contains the Rf-1 gene, transformed individuals at this generation restore fertility because Rf-1 is a dominant gene. In complementation assays plants transformed with each fragment were evaluated for seed fertility to find that those transformed with a 15.6 kb fragment (including bases 38538-54123 of SEQ ID NO: 27) derived from the λ phage clone XSG16 restored seed fertility (Example 10). Plants transformed with the other fragments were all sterile. These results showed that the above 15.6 kb fragment completely contains the Rf-1 gene. Moreover, a method for introducing the Rf-1 gene by genetic engineering techniques was provided by the present invention and demonstrated to be effective.

To further specify the region of the λ phage clone XSG16 in which the Rf-1 gene is contained, we evaluated seed fertility of shorter fragments than the 15.6 kb fragment (including bases 38538-54123 of SEQ ID NO: 27) by complementation assays. As a result, plants transformed with a 11.4 kb fragment derived from XSG16 (including bases 42357-53743 of SEQ ID NO: 27) were shown to restore seed fertility (Example 10(2)). Plants transformed with a further shorter 6.8 kb fragment (including bases 42132-48883 of SEQ ID NO: 27) also restored seed fertility (Example 10(3)). These results showed that the above 6.8 kb fragment contains the Rf-1 gene.

### III. Nucleic acids containing the Rf-1 locus

The present invention provides nucleic acids containing the locus of a fertility restorer gene (Rf-1). The nucleic acids containing the locus of a fertility restorer gene (Rf-1) of the present invention include a nucleic acid having the base sequence of SEQ ID NO.27, or a nucleic acid having a base sequence which is identical to at least 70% of the base sequence of SEQ ID NO.27, and which functions to restore fertility.

As described in Example 10, it was confirmed that the Rf-1 gene is completely contained in especially bases 38538-54123 of the base sequence of SEQ ID NO: 27. Accordingly, the present invention especially provides a nucleic acid having the base sequence of bases 38538-54123 of SEQ ID NO.27, or has a base sequence which is identical to at least 70% of the base sequence of bases 38538-54123 of SEQ ID NO.27, and which functions to restore fertility. As used herein, the term "the base sequence of SEQ ID NO: 27" refers to the entire sequence of SEQ ID NO: 27 or a part thereof participating in fertility restoring function, especially bases 38538-54123 according to the context. More preferably, it refers to bases 42357-53743, still more preferably bases 42132-48883.

In the examples below, a nucleic acid was isolated from a genomic library of indica rice IR24 containing the Rf-1 gene as a nucleic acid containing a fertility restorer gene (Rf-1) and determined to have the base sequence of SEQ ID NO: 27. However, the nucleic acid containing a fertility restorer gene (Rf-1) of the present invention can be derived from any indica variety carrying the RF-1 gene. The indica varieties carrying the Rf-1 gene include, but not specifically limited to, e.g. IR24, IR8, IR36, IR64, Chinsurah and BoroII. Known japonica varieties not carrying the Rf-1 gene include, but not limited to, Asominori, Koshihikari, Kirara 397, Akihikari, Akitakomachi, Sasanishiki, Kinuhikari, Nipponbare, Hatsuboshi, Koganebare, Hinohikari, Mineasahi, Aichinokaori, Hatsushimo, Akebono, Fujihikari, Minenoyukimochi, Kokonoemochi, Fukuhibiki, Dontokoi, Gohyakumangoku, Hanaechizen, Todorokiwase, Haenuki, Domannaka, Yamakikari, etc. The "indica" and "japonica" varieties are well known to those skilled in the art and the rice varieties encompassed by the present invention can be readily determined by those skilled in the art.

Nucleic acids of the present invention include DNA in both single-stranded and double-stranded forms, as well as the RNA complement thereof. DNA includes, for example, genomic DNA (including corresponding cDNA), chemically synthesized DNA, DNA amplified by PCR, and combinations thereof.

Nucleic acids containing the Rf-1 gene of the present invention preferably have the base sequence of SEQ ID NO: 27. More than one codon may encode the same amino acid, and this is called degeneracy of the genetic code. Thus, a DNA sequence not completely identical to SEQ ID NO: 27 may encode a protein having an amino acid sequence completely identical to SEQ ID NO: 27. Such a variant DNA sequence may result from silent mutation (e.g., occurring during PCR amplification), or can be a product of deliberate mutagenesis of a native sequence.

It is well known for those skilled in the art that even proteins having the same function may have different amino acid sequences depending on the varieties from which they are derived. The Rf-1 gene of the present invention includes such homologs and variants of the base sequence of SEQ ID NO: 27 so far as they functions to restore fertility. The expression "function to restore fertility" means that fertility is conferred on a rice individual or seed when such a DNA fragment is introduced. Fertility restoration may result from the expression of a protein by the Rf-1 gene or some function of the nucleic acid (DNA or RNA) per se of the Rf-1 gene in conferring fertility.

Whether or not a homolog or variant of the Rf-1 gene functions to restore fertility can be examined by, but not limited to, the following method, for example. A nucleic acid fragment under test is introduced into immature seeds obtained by pollinating MS Koshihikari (sterile line) with MS-FR Koshihikari according to the method of Hiei et al. (Plant Journal (1994), 6(2), p. 272-282). As the resulting transformants are cultured under normal conditions, the seeds mature only when the nucleic acid fragment under test functions to restore fertility.

The nucleic acid derived from a corresponding region of japonica Asominori not carrying the Rf-1 gene has the base sequence shown in SEQ ID NO: 28. Corresponding parts of SEQ ID NO: 28 and SEQ ID NO: 27 have an overall identity of about 98%. Thus, nucleic acids containing the locus of a fertility restorer gene (Rf-1) of the present invention are at least about 70%, preferably about 80% or more, more preferably 90% or more, still more preferably 95% or more, most preferably 98 or more% identical to SEQ ID NO: 27.

The percent identity may be determined by visual inspection and mathematical calculation. Alternatively, the percent identity of two nucleic acid sequences can be determined by comparing sequence information using the GAP computer program, version 6.0 described by Devereux et al., Nucl. Acids Res., 12:387 (1984) and available from the University of Wisconsin Genetics Computer Group (UWGCG). The preferred default parameters for the GAP program include: (1) a unary comparison matrix (containing a value of 1 for identities and 0 for non-identities) for bases, and the weighted comparison matrix of Gribskov and Burgess, Nucl. Acids Res., 14:6745 (1986), as described by Schwartz and Dayhoff, eds., Atlas of Protein Sequence and Structure, National Biomedical Research Foundation, pp. 353-358 (1979); (2) a penalty of 3.0 for each gap and an additional 0.10 penalty for each symbol in each gap; and (3) no penalty for end gaps. Other programs used by those skilled in the art of sequence comparison may also be used.

Nucleic acids of the present invention also include nucleic acids which are capable of hybridizing to the base sequence of SEQ ID NO: 27 under conditions of moderately stringent conditions and functions to restore fertility, and nucleic acids which are capable of hybridizing to the base sequence of SEQ ID NO: 27 under conditions of highly stringent conditions and functions to restore fertility.

As used herein, conditions of moderate stringency can be readily determined by those having ordinary skill in the art based on, for example, the length of the DNA. The basic conditions are set forth by Sambrook et al. Molecular Cloning: A Laboratory Manual, 2nd. Vol. 1, pp. 1.101-104, Cold Spring Harbor Laboratory Press, (1989), and include use of a prewashing solution for the nitrocellulose filters 5 x SSC, 0.5% SDS, 1.0 mM EDTA (pH 8.0), hybridization conditions of about 1 x SSC to 6 x SSC at about 40°C to 60°C (or other similar hybridization solution, such as Stark's solution, in about 50% formamide at about 42°C), and washing conditions of about 60°C, 0.5 x SSC, 0.1% SDS. The hybridization temperature is about 15-20°C lower when the hybridization solution contains about 50% formamide. Conditions of high stringency can also be readily determined by the skilled artisan based on, for example, the length of the DNA. Generally, conditions of high stringency include hybridization and/or washing conditions at higher temperatures and/or lower salt concentrations than in the conditions of moderate stringency described above. For example, such conditions include hybridization conditions of 0.1 x SSC to 0.2 x SSC at about 60-65°C and/or washing conditions of 0.2 x SSC, 0.1% SDS at about 65-68°C. The skilled artisan will recognize that the temperature and wash solution salt concentration can be adjusted as necessary according to factors such as the length of the probe.

DNAs of the present invention also include nucleic acids that differ from the base sequence of SEQ ID NO: 27 due to deletions, insertions or substitutions of one or more bases while retaining a fertility restoring function. So far as a fertility restoring function is retained, the number of bases to be deleted, inserted or substituted is not specifically limited, but preferably 1 to several thousands, more preferably 1-1000, still more preferably 1-500, even more preferably 1-200, most preferably 1-100.

Once the Rf-1 gene is further specified on the basis of the descriptions herein, it can be used by those skilled in the art after nucleic acids such as other regions than the Rf-1 gene or intron regions in the Rf-1 gene are removed. A given amino acid may be replaced, for example, by a residue having similar physiochemical characteristics. Examples of such conservative substitutions include changes from one aliphatic residue to another, such as changes from one to another of Ile, Val, Leu, or Ala; changes from one polar residue to another, such as changes between Lys and Arg, Glu and Asp, or Gln and Asn; or changes from one aromatic residue to another, such as changes from one to another of Phe, Trp, or Tyr. Other well-known conservative substitutions include e.g. changes between entire regions having similar hydrophobic characteristics. Those skilled in the art can introduce desired deletions, insertions or substitutions by well-known gene engineering techniques using e.g. site-specific mutagenesis as described in Sambrook et al., Molecular Cloning: A Laboratory Manual, 2nd edition, Cold Spring Harbor Laboratory Press, (1989).

We compared an indica variety IR24 carrying the Rf-1 gene (SEQ ID NO: 27) with japonica varieties not carrying it such as Asominori (SEQ ID NO: 28) and a Nipponbare BAC clone deposited with GenBank (Accession No. AC068923). As a result, we found that the Rf-1 region of the indica variety containing the Rf-1 gene has at least the following single bases polymorphisms (SNP).
1) a base corresponding to the base 1239 of SEQ ID NO: 27 is A;
2) a base corresponding to the base 6227 of SEQ ID NO: 27 is A;
3) a base corresponding to the base 20680 of SEQ ID NO: 27 is G;
4) a base corresponding to the base 45461 of SEQ ID NO: 27 is A;
5) a base corresponding to the base 49609 of SEQ ID NO: 27 is A;
6) a base corresponding to the base 56368 of SEQ ID NO: 27 is T;
7) a base corresponding to the base 57629 of SEQ ID NO: 27 is C; and
8) a base corresponding to the base 66267 of SEQ ID NO: 27 is G.

Thus, nucleic acids containing the Rf-1 region of the present invention preferably meet one to all of the requirements 1)-8) above.

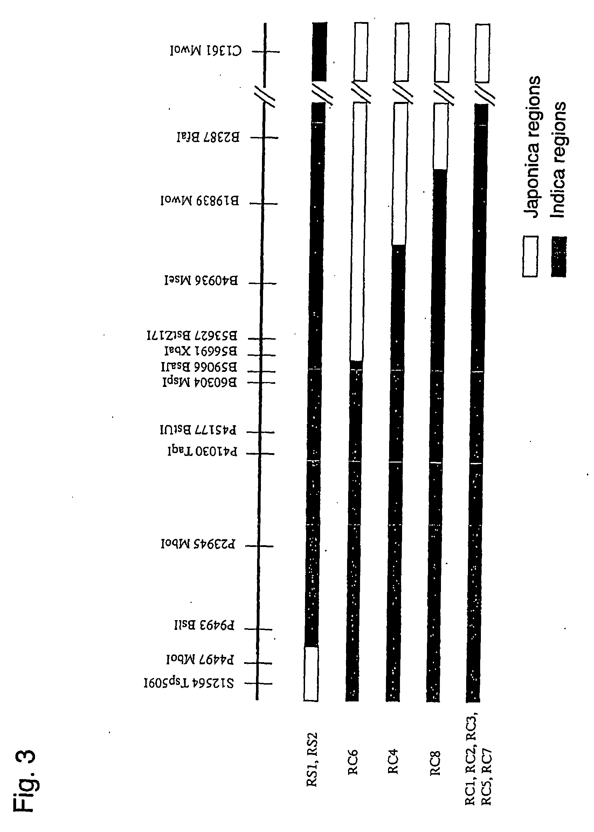

In Example 3 below, the chromosomal organizations of recombinants proximal to the Rf-1 gene (RS1-RS2, RC1-RC8) were tested in the Rf-1 region. The results showed that a sequence determining the presence of the function of the Rf-1 gene is contained in the base sequence of bases 1239-66267 of SEQ ID NO: 27, i.e. in a region from the P4497 MboI to B56691 XbaI loci (about 65 kb) as estimated at maximum (Fig. 3). However, there is a possibility that it is important for the expression of the genetic function of the Rf-1 gene that the Rf-1 gene is partially of the indica genotype, and that the genetic function may not be significantly changed whether the remaining regions are of the japonica or indica genotype. There may be an extreme case that the coding region is completely identical and only the promoter region is different between japonica and indica, and that the promoter region and the coding region are only partially included in the region from P4497 the MboI to B56691 XbaI loci (about 65 kb). Therefore, it cannot be concluded that the common indica region above (bases 1239-66267 of SEQ ID NO: 27) completely contains the entire Rf-1 gene. However, it is thought that at least SEQ ID NO: 27 completely contains the entire Rf-1 gene for the following reasons:
1) the size of a gene is normally several kilobases, and rarely exceeds 10 kb;
2) the genomic base sequence of IR24 determined by the present invention (SEQ ID NO: 27) completely contains the common indica region above;
3) the 5' end of SEQ ID NO: 27 is located 1238 bp upstream of the 5' end of the common indica region above and forms a part of another gene (S12564); and
4) the 3' end of SEQ ID NO: 27 is located 10096 bp downstream of the 3' end of the common indica region above.

In this way, we first succeeded in restricting the region of the Rf-1 gene to 76 kb. Thus, nucleic acids containing the region of the Rf-1 gene of the present invention are extremely less likely to contain other genes proximal to the Rf-1 gene as compared with those selected with the co-dominant marker locus at a genetic distance of about 1 cM (about 300 kb) from the Rf-1 gene described in a prior documents such as Japanese Patent Public Disclosure No. 2000-139465. Moreover, they are less likely to contain other genes than those selected with the DNA marker loci S12564 Tsp509I and C1361 MwoI (at a distance of about 0.3 cM between them) described in our prior Japanese Patent Application No. 2000-247204.

We further confirmed by complementation assays that the Rf-1 gene is completely contained in especially bases 38538-54123 of the base sequence of SEQ ID NO: 27. In an embodiment of the present invention, therefore, the base sequence at least 70% identical to the base sequence of SEQ ID NO: 27 or to the base sequence of bases 38538-54123 of SEQ ID NO: 27 meets at least one of the following requirements 1) and 2):
1) a base corresponding to the base 45461 of SEQ ID NO: 27 is A;
2) a base corresponding to the base 49609 of SEQ ID NO: 27 is A.

### IV. Method for restoring rice fertility

The present invention provides a method for restoring rice fertility comprising introducing a nucleic acid into rice, wherein the nucleic acid has the base sequence of SEQ ID NO.27, or has a base sequence which is identical to at least 70% of the base sequence of SEQ ID NO.27, and which functions to restore fertility. The methods of the present invention may comprise introducing a nucleic acid into rice, wherein the nuclei acid has a portion of SEQ ID NO: 27, especially bases 38538-54123, preferably bases 42357-53743, more preferably bases 42132-48883 of SEQ ID NO: 27 or has a base sequence which is identical to at least 70% identical of the base sequence of bases 38538-54123, preferably bases 42357-53743, more preferably bases 42132-48883 of SEQ ID NO: 27 and, which functions to restore fertility.

In the present invention, the nucleic acid containing the locus of a fertility restorer gene (Rf-1) that can be introduced into rice can be any one of the nucleic acids described above in "III. Nucleic acids containing the Rf-1 locus". The method for introducing the nucleic acid into rice is not specifically limited but can be any known method. Nucleic acids of the present invention can be introduced by known genetic engineering techniques or crossing. Genetic engineering techniques are preferably used because inclusion of other neighboring genes can be prevented and the period for establishing a line can be shortened.

Any suitable expression system for transduction by genetic engineering techniques can be employed. Recombinant expression vectors comprise a nucleic acid containing a fertility restorer gene (Rf-1) of the invention that can be introduced into rice, operably linked to suitable transcriptional or translational regulatory base sequences, such as those derived from a mammalian, microbial, viral, or insect gene.

Examples of regulatory sequences include transcriptional promoters, operators, or enhancers, an mRNA ribosomal binding site, and appropriate sequences which control transcription and translation initiation and termination. Base sequences are operably linked to a regulatory sequence when the regulatory sequence is functionally associated with the DNA sequences. Thus, a promoter base sequence is operably linked to a DNA sequence if the promoter base sequence controls the transcription of the DNA sequence. An origin of replication that confers the ability to replicate in rice, and a selection gene by which transformants are identified, are generally incorporated into expression vectors. As for selectable markers, those commonly used can be used by standard methods. Examples are genes resistant to antibiotics such as tetracycline, ampicillin, kanamycin, neomycin, hygromycin or spectinomycin.

In addition, a sequence encoding an appropriate signal peptide (native or heterologous) can be incorporated into expression vectors. A DNA sequence for a signal peptide (secretory leader) may be fused in frame to a nucleic acid sequence of the invention so that the DNA is initially transcribed, and the mRNA translated into a fusion protein containing the signal peptide.

The present invention also provides recombinant vectors containing a gene of the present invention. Methods for integrating a DNA fragment of a gene of the present invention into a vector such as a plasmid are described in e.g. Sambrook, J. et al, Molecular Cloning, A Laboratory Manual (2nd edition), Cold Spring Harbor Laboratory, 1.53 (1989). Commercially available ligation kits (e.g. available from TAKARA) can be conveniently used. Thus obtained recombinant vectors (e.g. recombinant plasmids) are transferred into host rice cells.

Vectors can be conveniently prepared by linking a desired gene to a recombinant vector available in the art (e.g. plasmid DNA) by standard methods. Plant transforming vectors are especially useful for conferring fertility on rice using a nucleic acid fragment of the present invention. Vectors for plants are not specifically limited so far as they can express the gene of interest in plant cells to produce the protein, but preferably include pBI221, pBI121 (Clontech), and vectors derived therefrom. Especially, examples of vectors for transforming rice belonging to monocotyledons include pIG121Hm and pTOK233 (Hiei et al., Plant J., 6, 271-282 (1994)), and pSB424 (Komari et al., Plant J., 10, 165-174 (1996)).

Transgenic plants can be prepared by replacing the β-glucuronidase (GUS) gene in the above vectors with a nucleic acid fragment of the present invention to construct a plant transforming vector and transfecting it into a plant. The plant transforming vector preferably comprises at least a promoter, a start codon, a desired gene (a nucleic acid sequence of the present invention or a part thereof), a stop codon and a terminator. It may also contain a DNA encoding a signal peptide, an enhancer sequence, non-translated 5' and 3' regions of the desired gene, a selectable marker region, etc., as appropriate. Promoters and terminators are not specifically limited so far as they are functional in plant cells, among which constitutive expression promoters include the 35S promoter initially contained in the above vectors as well as promoters for actin and ubiquitin genes.

Suitable methods for introducing a plasmid into a host cell include the use of calcium phosphate or calcium chloride/rubidium chloride, electroporation, electroinjection, chemical treatment with PEG or the like, the use of a gene gun described in Sambrook, J. et al., Molecular Cloning, A Laboratory Manual (2nd edition), Cold Spring Harbor Laboratory, 1.74(1989). Plant cells can be transformed by e.g. the leaf disc method [Science, 227, 129 (1985)] or electroporation [Nature, 319, 791 (1986)].

Methods for transferring a gene into a plant include the use of Agrobacterium (Horsch et al., Science, 227,129(1985); Hiei et al., Plant J., 6, 271-282(1994)), electroporation (Fromm et al., Nature, 319, 791(1986)), PEG (Paszkowski et al., EMBO J., 3, 2717(1984)), microinjection (Crossway et al., Mol. Gen. Genet., 202, 179 (1986)), particle bombardment (McCabe et al., Bio/Technology, 6, 923(1988)). Methods are not specifically limited so far as they are suitable for transfecting a nucleic acid into a desired plant.

Transduction by crossing can be performed as follows, for example. First, F₁ obtained by crossing an Rf-1 donor parent and a japonica variety is backcrossed with the japonica variety. The resulting individuals are screened for those homozygous for japonica at the S12564 Tsp509I locus and heterozygous at the P4497 MboI and B53627 BstZ17I loci and further backcrossed. The resulting individuals are screened for those heterozygous at the P4497 MboI and B56691 XbaI loci and homozygous for japonica at the B53627 BstZ17I locus and further backcrossed. Subsequently, about 10 cycles of screening each backcrossed generation for individuals heterozygous at the P4497 MboI and B56691 XbaI loci and subjecting them to the subsequent backcrossing are repeated. Finally, individuals heterozygous at the P4497 MboI and B56691 XbaI loci are self-fertilized and the resulting individuals are screened for those homozygous for indica at both loci, whereby a restorer line inheriting a limited chromosomal region from the P4497 MboI to B56691 XbaI loci from the Rf-1 donor parent can be obtained.

According to the present invention, nucleic acids containing a fertility restorer gene (Rf-1) were isolated, whereby the Rf-1 gene can be introduced into a rice variety using genetic engineering techniques to establish a restorer line. The present invention succeeded in restricting the Rf-1 region to 76 kb or less. Therefore, nucleic acids containing the Rf-1 locus of the present invention are extremely less likely to contain other genes neighboring the Rf-1 gene than those of the prior art. Moreover, the entire base sequence of the region containing the Rf-1 gene was determined by the present invention. Those skilled in the art can proceed with analysis of the Rf-1 gene itself on the basis of the description herein. Thus, only the Rf-1 gene can be introduced without including any neighboring gene. This is especially important when neighboring genes bring deleterious traits. Furthermore, restorer lines can be established in a shorter period such as 1-2 years than obtained by crossing.

In complementation assays described in Examples 4-13 herein, MS Koshihikari (having BT cytoplasm and a core gene substantially identical to Koshihikari) was actually transformed by an Agrobacterium-mediated method using fragments from 10 clones described in Fig. 5. The results demonstrated that fertility restorer lines are established from a nucleic acid containing the base sequence of bases 38538-54123, preferably bases 42357-53743, more preferably bases 42132-48883 of SEQ ID NO: 27.

Agrobacterium-mediated methods for establishing rice restorer lines are described in, but not limited to, Hiei et al., Plant J.,6, pp. 271-282(1994), Komari et al., Plant J.,10, p.165-174(1996), Ditta et al., Proc. Natl. Acad. Sci. USA 77: pp. 7347-7351(1980), etc.

First, a plasmid vector containing a nucleic acid fragment of interest to be inserted is prepared. Suitable plasmid vectors include e.g. pSB11, pSB22 and the like having a plasmid map described in Komari et al., Plant J., 10, pp. 165-174 (1996), supra. Alternatively, those skilled in the art can also construct an appropriate vector by themselves on the basis of plasmid vectors such as pSB11, pSB22 described above. In the examples herein below, an intermediate vector pSB200 having a hygromycin-resistant gene cassette was prepared on the basis of pSB11, and used. Specifically, a nopaline synthase terminator (Tnos) was first fused to a ubiquitin promoter and a ubiquitin intron (Pubi-ubiI). A hygromycin-resistant gene (HYG(R)) was inserted between ubiI and Tnos of the resulting Pubi-ubiI-Tnos complex to give a Pubi-ubiI-HYG(R)-Tnos assembly. This assembly was fused to a HindIII/EcoRI fragment of pSB11 (Komari et al., supra.) to give pKY205. Linker sequences for adding restriction enzyme sites NotI, NspV, EcoRV, KpnI, SacI, EcoRI were inserted into the Hind III site upstream of Pubi of this pKY205 to give pSB200 having a hygromycin-resistant gene cassette.

Then, E. coli cells (e.g. DH5a, JM109, MV1184, all commercially available from e.g. TAKARA) are transformed with the recombinant vector containing the nucleic acid inserted.

Thus transformed E. coli cells are used for triparental mating with an Agrobacterium strain preferably in combination with a helper E. coli strain according to e.g. the method of Ditta et al. (1980). Suitable Agrobacterium strains include Agrobacterium tumefaciens strains such as LBA4404/pSB1, LBA4404/pNB1, LBA4404/pSB3, etc. They all have a plasmid map described in Komari et al., Plant J., 10, pp. 165-174 (1996), supra. and can be used by those skilled in the art by constructing a vector by themselves. Suitable helper E. coli strains include, but not limited to, e.g. HB101/pRK2013 (available from Clontech). A report shows that E. coli cells carrying pRK2073 can also be used as helper E. coli though they are less common (Lemas et al., Plasmid 1992, 27, pp. 161-163).

Then, the Agrobacterium cells mated as intended are transformed into male sterility rice according to e.g. the method of Hiei et al (1994). Necessary immature rice seeds for transformation can be prepared by e.g. pollinating male sterility rice with a japonica variety.

Fertility restoration in transformed plants can be assessed by e.g. evaluating seed fertility in standing plants about one month after heading. Evaluation on standing plants means observation of plants grown in a field or the like. An alternative method is a laboratory study of grain ripening percentages in the ear.

### V. Methods for discerning the presence of the Rf-1 gene

According to the present invention, it was shown that a sequence determining the presence of the function of the Rf-1 gene is located between the polymorphism-detecting marker loci P4497 MboI and B56691 XbaI on rice chromosome 10. Moreover, complementation assays confirmed that the Rf-1 gene is completely contained in especially bases 38538-54123 of the base sequence of SEQ ID NO: 27.

Comparison of the base sequence of an indica variety carrying the Rf-1 gene (IR24) (SEQ ID NO: 27) with those of japonica varieties not carrying said gene (Asominori (SEQ ID NO: 28) and Nipponbare BAC clone AC068923) revealed the presence of polymorphisms between both varieties. As a result, it became possible to conveniently, rapidly and exactly discern whether or not a rice plant or seed under test carries the Rf-1 gene on the basis of polymorphisms in base sequence in regions neighboring the Rf-1 gene.

Therefore, the present invention also provides a method for discerning whether or not a subject rice individual or a seed thereof has the Rf-1 gene or not, wherein the method utilizing a fact that a sequence determining the presence of the function of the Rf-1 gene positions between the polymorphism detection marker loci P4497 MboI and B56691 Xba I on rice chromosome 10.

Polymorphisms can be detected by any known method. For example, known methods include assays for restriction fragment length polymorphisms (RFLPs); direct determination by sequencing; cutting a genomic DNA with a 8-base recognizing restriction enzyme, and then radioactivelly labeling the ends and further cutting the labeled digest with 6-base and 4-bases recognizing restriction enzyme and then developing the digest by two-dimensional electrophoresis (RLGS, Restriction Landmark Genome Scanning); etc. AFLP analysis (amplified fragment length polymorphism; P. Vos et al., Nucleic Acids Res. Vol. 23, pp. 4407-4414 (1995)) has also been developed wherein RFLP is amplified/detected by polymerase chain reaction (PCR).

For example, conventional methods involved detecting RFLPs via PCR amplification (conversion of RFLP markers into PCR markers) or detecting polymorphisms in microsatellites via PCR amplification (microsatellite markers) as illustrated below.

### Conversion of RFLP markers into PCR markers

A. PCR markers based on polymorphisms in genomic regions corresponding to RFLP probes (D.E. Harry, B.Temesgen, D.B. Neale; Codominant PCR-based markers for Pinus taeda developed from mapped cDNA clones, Theor. Appl. Genet. (1998) 97: pp. 327-336). After performing genomic PCR using primers designed for an RFLP marker probe sequence ("RFLP" is a polymorphism observed by Southern analysis using a DNA fragment as a probe. The base sequence of the DNA fragment used as a probe is called "RFLP marker probe sequence"), a PCR marker can be prepared by either of the following two procedures. A first procedure involves treating the products with a series of restriction enzymes to search for a restriction enzyme causing a fragment length polymorphism, and a second procedure involves searching for a polymorphism by varietal comparison of the base sequences of the products and preparing a PCR marker based on the polymorphism.
B. PCR markers based on identification of RFLP-causing sites. A PCR marker can be obtained by identifying an RFLP-causing site (a restriction enzyme recognition site carried by only one of two varieties compared) present in or near (normally within several kbs) an RFLP marker probe sequence.

### Microsatellite markers

Microsatellites are repeat sequences of about 2 to 4 bases such as (CA)ₙ that are present in great numbers in genomes. If a varietal polymorphism occurs in repetition number, a polymorphism can be observed in PCR product length by PCR using primers designed in adjacent regions, whereby the DNA polymorphism can be detected. Markers for detecting polymorphisms using microsatellites are called microsatellite markers (O. Parnaud, X. Chen, S.R. McCouch, Mol. Gen. Genet. (1996) 252: pp. 597-607).

Methods for detecting polymorphisms in the present invention are not specifically limited. From the viewpoint of efficiency and convenience, PCR-RFLP is preferred, which is a combination of PCR and RFLP to identify polymorphisms from their restriction enzyme cleavage patterns in cases where they exist among variety lines at restriction enzyme recognition sites in the sequences of DNA fragments amplified by PCR. PCR-RFLP is also called CAPS (cleaved amplified polymorphic sequence). If any suitable restriction enzyme recognition site is not present in a region showing polymorphisms, a modified CAPS called dCAPS (derived cleaved amplified polymorphic sequence) can also be used wherein restriction enzyme sites are introduced during PCR (Michaels, S.D. and Amasino, R.M. (1998), The Plant Journal 14(3) 381-385; A. Konieczny et al.,(1993), Plant J.4(2) pp. 403-410; Neff, M.M., Neff, J.D., Chory, J. and Pepper, A.E. (1998), The Plant Journal 14(3) 387-392). These methods are explained in more detail below.

### CAPS, dCAPS

The method for discerning of the present invention comprise, but not limited to:
i) preparing a pair of primers based on the base sequences of a site showing a polymorphism in the base sequences between indica and japonica varieties at the Rf-1 locus and its adjacent regions to amplify said base sequences;
ii) performing nucleic acid amplification reaction(s) using the genomic DNA of the subject rice individual or the seed thereof as a template; and
iii) discerning whether or not the subject rice individual or the seed thereof has the Rf-1 gene based on the polymorphism found in the nucleic acid amplification product.

The step of preparing a primer pair in i) preferably comprises any of the following means:
a) when a change containing a deleted region exists in the polymorphism in the nucleic acid amplification product, preparing a pair of primers for nucleic acid amplification to flank the deleted region to form a marker for detecting the polymorphism;
b) when a base change causing a difference in restriction enzyme recognition exists in the polymorphism in the nucleic acid amplification product, preparing a pair of primers for nucleic acid amplification to flank the base change site to form a marker for detecting the polymorphism; or
c) when a base change causing no difference in restriction enzyme recognition exists in the polymorphism in the nucleic acid amplification product, preparing a pair of primers for introducing a mismatch, wherein pair of primers contain the base change site and alters a region containing the base change site into a base sequence causing a difference in restriction enzyme recognition in the nucleic acid amplification product to form a marker for detecting the polymorphism.

Suitable polymorphic sites for discerning the presence of the Rf-1 gene in the present invention can be appropriately selected so that a polymorphism detecting marker can be prepared as described below on the basis of comparison of, but not limited to, the base sequence of an indica variety carrying the Rf-1 gene (IR24) (SEQ ID NO: 27) with those of japonica varieties not carrying said gene (Asominori (SEQ ID NO: 28) and Nipponbare BAC clone AC068923).

If the polymorphism found causes a difference in restriction enzyme recognition, for example, a pair of primers for nucleic acid amplification are prepared to flank the polymorphic site and used for detecting the polymorphism. Primers are preferably designed not to be specific for highly repeated sequences to avoid undesired products. If the polymorphism found does not cause a difference in restriction enzyme recognition, markers can be prepared by applying the dCAPS method described above. Primers for dCAPS markers are preferably designed not to be specific for repeat sequences and to provide a product length of preferably 50-300 bases, more preferably about 100 bases to ease identification of polymorphisms.

If the polymorphism found involves a microsatellite, nucleic acid amplification primers are prepared to flank the microsatellite and used to detect the polymorphism. Again, the primers are preferably designed not to be specific for repeat sequences.

### 1) Nucleic acid amplification

In the present invention, a pair of primers are preferably prepared for amplifying adjacent regions containing polymorphisms on the basis of the determined base sequence of the nucleic acid of a subject rice individual or seed at the Rf-1 locus. The primer pair is used to perform a nucleic acid amplification reaction with the genomic DNA of the subject rice individual or seed as a template. The nucleic acid amplification reaction is preferably polymerase chain reaction (PCR) (Saiki et al., 1985, Science 230, pp. 1350-1354).

The pair of primers for nucleic acid amplification can be prepared by any known method on the basis of the base sequence of a polymorphic site and adjacent regions thereto. Specifically, a primer pair can be prepared on the basis of the base sequence of a polymorphic site and adjacent regions thereto by a process comprising generating a single-stranded DNA having the same base sequence as the base sequence of the polymorphic site and adjacent regions thereto or a base sequence complementary to said regions or, if necessary, generating the single-stranded DNA containing a modification without affecting the binding specificity to the base sequence of the polymorphic site and adjacent regions thereto provided that the following conditions are satisfied:
1) the length of each primer should be 15-30 bases;
2) the proportion of G+C in the base sequence of each primer should be 30-70%;
3) the distribution of A, T, G and C in the base sequence of each primer should not be partially largely uneven;
4) the length of the nucleic acid amplification product amplified by the primer pair should be 50-3000 bases, preferably 50-300 bases; and
5) any complementary sequence segment should not occur with the base sequence of each primer or between the base sequences of the primers.

As used herein, the "adjacent regions" to a polymorphic site mean that an area containing both of a polymorphic site and adjacent regions thereto is within a distance suitable for nucleic acid amplification, preferably PCR. The adjacent regions amplified preferably have a length within the range of, but not limited to, about 50 bases to about 3000 bases, more preferably about 50 bases to about 2000 bases. To facilitate identification of polymorphisms, the product length is preferably 50-300 bases, more preferably about 100 bases. The adjacent regions preferably have a length within the range of, but not limited to, about 0 to about 3000 bases, more preferably about 0 to about 2000 bases, still more preferably about 0 to about 1000 bases on the 5' or 3' side of a polymorphic site.

Procedures and conditions for the nucleic acid amplification reaction are not specifically limited and are well known to those skilled in the art. Appropriate conditions can be applied by those skilled in the art depending on various factors such as the base sequence of the polymorphic site and adjacent regions thereto, the base sequence and length of the primer pair, etc. Generally, the nucleic acid amplification reaction can be performed under more stringent conditions (annealing reaction and nucleic acid elongation reaction at higher temperatures and less cycles) as the primer pair is longer or the proportion of G+C is higher or the distribution of A, T, G and C is evener. The use of more stringent conditions allows an amplification reaction with higher specificity.

The amplification reaction can be performed under conditions of, but not limited to, one cycle of 94°C for 2 min, 30 cycles of 94°C for 1 min, 58 °C for 1 min and 72°C for 2 min, and finally one cycle of 72°C for 2 min using 50 ng of a genomic DNA as a template, 200 µM of each dNTP and 5 U of ExTaq™ (TAKARA). The reaction can also be performed under conditions of one cycle of 94°C for 2 min, 30 cycles of 94°C for 1 min, 58°C for 1 min and 72°C for 1 min, and finally one cycle of 72°C for 2 min. In another embodiment, the reaction can also be performed under conditions of one cycle of 94°C for 2 min, 35 cycles of 94°C for 30 sec, 58°C for 30 sec and 72°C for 30 sec, and finally one cycle of 72°C for 2 min.

The subject rice (test rice) genomic DNA used as a template for PCR can be easily extracted from individuals or seeds by the method of Edwards et al. (Nucleic Acids Res. 8(6):1349, 1991). More preferably, DNA purified by standard techniques is used. An especially preferred extraction method is the CTAB method (Murray, M.G. et al., Nucleic Acids Res. 8(19):4321-5, 1980). The DNA is preferably used as a template for PCR at a final concentration of 0.5 ng/µL.

### 2) Preparation of markers for detecting polymorphisms

After examining whether or not a polymorphism is detected in the amplification product by the nucleic acid amplification reaction with a pair of primers, a marker for detecting the polymorphism is prepared on the basis of the polymorphism found. Non-limiting examples of polymorphisms that can be detected in the amplification product are as follows.

### a) A change containing a deleted region exists in the polymorphism in the nucleic acid amplification product.

In this case, a pair of primers for nucleic acid amplification are prepared to flank the deleted region to form a marker for detecting the polymorphism. If the deleted region has a sufficient size, the polymorphism can be detected from the difference in mobility by electrophoresing the amplification product on an agarose gel or an acrylamide gel, for example. The polymorphism can be detected when the difference in base pair numbers is about 5% or more in the case of agarose gel electrophoresis or when the difference in length is about 1 base or more in the case of sequencing acrylamide gel electrophoresis, for example. Alternatively, the polymorphism can be detected by hybridizing the nucleic acid amplification product using an oligobase or a DNA fragment having a complementary sequence to the base sequence excluding the deleted region as an analytical probe. Alternatively, the polymorphism can be confirmed by determining the base sequence of the amplification product, if desired. Known techniques for electrophoresis of nucleic acids, hybridization, sequencing and the like can be used as appropriate by those skilled in the art. In this case, the difference in the length of the amplification product directly reflects the polymorphism and markers for detecting polymorphisms on this basis are called ALP (amplicon length polymorphism) markers.

### b) A base change causing a difference in restriction enzyme recognition exists in the polymorphism in the nucleic acid amplification product.

In this case, a pair of primers for nucleic acid amplification are prepared to flank the base change site to form a marker for detecting the polymorphism. In this case, a base change causing a difference in restriction enzyme recognition occurs in the polymorphism of the nucleic acid amplification product, i.e. the nucleic acid amplification product may be cleaved or not with one or more specific restriction enzymes. Thus, the amplification product can be treated with the restriction enzymes and electrophoresed on e.g. an agarose gel to detect the polymorphism from the difference in mobility. The polymorphism can be confirmed by determining the base sequence of the amplification product, if desired.

In this case, the difference in the length of the restriction fragment of the amplification product by PCR or the like reflects the polymorphism and markers for detecting polymorphisms on this basis are called CAPS markers or PCR-RFLP markers (A. Konieczny et al., supra.)

This is exemplified by primer pairs P4497 MboI, P23945 MboI, P41030 TaqI, P45177 BstUI, B59066 BsaJI and B56691 XbaI in Example 1 below. Even if the polymorphism can be detected by the length of the nucleic acid amplification product as described in a) above, the polymorphism can be more easily detected by combination with restriction enzyme treatment.

### c) A base change causing no difference in restriction enzyme recognition exists in the polymorphism in the nucleic acid amplification product.

In this case, a pair of primers for introducing a mismatch are prepared that contains the base change site and alters a region containing the base change site into a base sequence causing a difference in restriction enzyme recognition in the nucleic acid amplification product to form a marker for detecting the polymorphism.

Specifically, a pair of primers based on the base sequences of regions naturally proximal to the Rf-1 gene cause a polymorphism in the nucleic acid amplification product but no difference in restriction enzyme recognition, and therefore, a mismatch is introduced into one or both of the primers to alter a region containing the base change site (polymorphism) into a base sequence causing a difference in restriction enzyme recognition in the nucleic acid amplification product. For example, the method described in Mikaelian et al., Nucl. Acids. Res. 20:376.1992 can be used as a standard technique for substituting, deleting or adding a specific base by PCR-mediated site-specific mutagenesis. The amplification product using the mismatch-introducing primers as a marker for detecting the polymorphism may be cleaved or not with one or more specific restriction enzymes because it has a difference in restriction enzyme recognition at the mismatch-introducing site. Therefore, the amplification product can be treated with the restriction enzymes and electrophoresed on e.g. an agarose gel to detect the polymorphism from the difference in mobility, as described in b) above.

The introduction of a mismatch must not affect not only the binding of the primers to a target plant genome but also the polymorphic base change. The polymorphic base change is used to introduce a mismatch near it so that a difference in restriction enzyme recognition occurs by a combination of both base change and mismatch. Methods for introducing such a mismatch are known to those skilled in the art and described in detail in Michaels, S.D. and Amasino, R.M. (1998), Neff, M.M., Neff, J.D., Chory, J. and Pepper, A.E. (1998), for example.

Markers in this case are improved CAPS markers described in b) above and called dCAPS (derived CAPS) markers. This is exemplified by P9493 BslI in Example 3 below.

If there are many extra restriction sites unrelated to varietal polymorphisms in the case of b) or c) above, it may be difficult to discern any difference in restriction site recognition based on polymorphisms. In this case, a mismatch may be introduced into a primer as appropriate to abolish unnecessary restriction sites. For example, a mismatch was introduced into the R-primer to abolish the MspI site unrelated to polymorphisms in B60304 MspI in Example 3.

Although the invention is not limited to any specific method, CAPS or dCAPS methods have several advantages over other RFLP methods. Specifically, analyses can be made with smaller amounts of samples than in RFLP, for example. Another advantage is that the time and labor required for analyses can be greatly reduced. Polymorphisms detected with PCR markers can be visualized by agarose gel electrophoresis that is easier than acrylamide gel electrophoresis used for microsatellite markers.

### Preferred embodiments of the discerning method of the present invention

Preferred embodiments of the method for discerning whether or not a subject rice has the Rf-1 gene are described below for illustrative purposes. In the examples herein, it was found that the base sequence of an indica variety IR24 carrying the Rf-1 gene (SEQ ID NO: 27) has at least the following polymorphisms 1)-8) as compared with corresponding regions of japonica varieties:
1) a base corresponding to the base 1239 of SEQ ID NO: 27 is A;
2) a base corresponding to the base 6227 of SEQ ID NO: 27 is A;
3) a base corresponding to the base 20680 of SEQ ID NO: 27 is G;
4) a base corresponding to the base 45461 of SEQ ID NO: 27 is A;
5) a base corresponding to the base 49609 of SEQ ID NO: 27 is A;
6) a base corresponding to the base 56368 of SEQ ID NO: 27 is T;
7) a base corresponding to the base 57629 of SEQ ID NO: 27 is C; and
8) a base corresponding to the base 66267 of SEQ ID NO: 27 is G.

In preferred embodiments of the present invention, therefore, the subject rice individual or seed is judged as carrying the Rf-1 gene when one to all of the requirements 1)-8) above are met.

We further verified that a region essential for the expression of the function of the Rf-1 gene is contained in especially bases 38538-54123, preferably bases 42357-53743, more preferably bases 42132-48883 in the base sequence of SEQ ID NO: 27. In an embodiment of the present invention, therefore, the subject rice individual or seed is determined to have the Rf-1 gene in the case that the nucleic acid having a base sequence which is identical to at least 70% of the base sequence of SEQ ID NO.27 or of the base sequence of bases 38538-54123 of SEQ ID NO.27, meets at least one of the following requirements 1) and 2):
1) a base corresponding to the base 45461 of SEQ ID NO.27 is A; and
2) a base corresponding to the base 49609 of SEQ ID NO.27 is A.

Known polymorphism detecting methods can be used to determine whether or not the above requirements are met. The base sequence of adjacent regions containing said sequence can also be directly determined. However, CAPS or dCAPS methods described above are preferably used because they are rapid and convenient. CAPS or dCAPS methods can be performed by a protocol comprising, for example:
i) preparing a pair of primers based on a base sequence of adjacent regions including any one of the following base;
   1) a base corresponding to the base 1239 of SEQ ID NO: 27;
   2) a base corresponding to the base 6227 of SEQ ID NO: 27;
   3) a base corresponding to the base 20680 of SEQ ID NO: 27;
   4) a base corresponding to the base 45461 of SEQ ID NO: 27;
   5) a base corresponding to the base 49609 of SEQ ID NO: 27;
   6) a base corresponding to the base 56368 of SEQ ID NO: 27;
   7) a base corresponding to the base 57629 of SEQ ID NO: 27; and
   8) a base corresponding to the base 66267 of SEQ ID NO: 27 is G.
   to amplify both the base of the above and adjacent regions thereto;
ii) performing nucleic acid amplification reaction(s) using the genome DNA of the subject rice individual or the seed thereof as a template; and
iii) discerning the presence of the Rf-1 in the subject rice individual or the seed thereof based on polymorphism found in said nucleic acid amplification product.

The detection of polymorphisms in the nucleic acid amplification product is performed by, but not limited to, discerning the subject rice individual or seed to have the Rf-1 gene when one to all of the requirements 1)-8) below are met:
1) a region including a base corresponding to the base 1239 of SEQ ID NO: 27 does not have any MboI recognition sequence;
2) a region including a base corresponding to the base 6227 of SEQ ID NO: 27 does not have any BslI recognition sequence;
3) a region including a base corresponding to the base 20680 of SEQ ID NO: 27 does not have any MboI recognition sequence;
4) a region including a base corresponding to the base 45461 of SEQ ID NO: 27 does not have any TaqI recognition sequence;
5) a region including a base corresponding to the base 49609 of SEQ ID NO: 27 does not have any BstUI recognition sequence;
6) a region including a base corresponding to the base 56368 of SEQ ID NO: 27 does not have any MspI recognition sequence;
7) a region including a base corresponding to the base 57629 of SEQ ID NO: 27 does not have any BsaJI recognition sequence; and
8) a region including a base corresponding to the base 66267 of SEQ ID NO: 27 does not have any XbaI recognition sequence.

However, the present invention is not limited to the restriction enzymes above so far as each polymorphism in the specific regions 1)-8) above can be detected.

Preferably, identification methods of the present invention comprise:
i) preparing a pair of primers based on a base sequence of adjacent regions including any one of the following base;
   1) a base corresponding to the base 45461; or
   2) a base corresponding to the base 49609; to amplify both the base of the above and adjacent regions thereto;
ii) performing nucleic acid amplification reaction(s) using the genome DNA of the subject rice individual or the seed thereof as a template; and
iii) discerning the presence of the Rf-1 in the subject rice individual or the seed thereof based on polymorphism found in said nucleic acid amplification product. The subject rice individual or seed thereof is determined to have the Rf-1 gene in step iii), although not limited to, when at least one of the following requirements 1) and 2) is met:
   1) a region including a base corresponding to the base 45461 of SEQ ID NO: 27 does not have any TaqI recognition sequence;
   2) a region including a base corresponding to the base 49609 of SEQ ID NO: 27 does not have any BstUI recognition sequence.

Primer pairs used for the amplification reaction can be appropriately selected by those skilled in the art to preferably satisfy the conditions above on the basis of the base sequence of SEQ ID NO: 27. Preferably, any primer pair having a base sequence selected from the group consisting of SEQ ID NOS: 39 and 40, SEQ ID NOS: 41 and 42, SEQ ID NOS: 43 and 44, SEQ ID NOS: 45 and 46, SEQ ID NOS: 47 and 48, SEQ ID NOS: 49 and 50, SEQ ID NOS: 51 and 52, and SEQ ID NOS: 53 and 54 is used. More preferably, the primer pair is selected from the group consisting of SEQ ID NOS: 45 and 46, and SEQ ID NOS: 47 and 48. If necessary, the sequences of the above primer pairs containing substitutions, deletions or additions while retaining the binding specificity for the base sequence of the polymorphic site and adjacent regions thereto can also be used as primers.

To examine the resulting PCR product for restriction fragment length polymorphisms, it is cleaved with restriction enzymes corresponding to the restriction sites present in PCR markers. Such cleavage is accomplished by incubation for several hours to a day at the recommended reaction temperature for the restriction enzymes used. The PCR amplified sample cleaved with the restriction enzymes can be analyzed by electrophoresis on an about 0.7% - 2% agarose gel or an about 3% MetaPhor™ agarose gel. The gel is visualized under UV light in ethidium bromide, for example.

In the most preferred embodiments of the present invention, restriction enzyme cleavage patterns show the bands as shown in Table 2 below on the visualized gel depending on the primer pair used.

**Table 2**

| | Approximate size (bp) of detected band |
|---|---|
| Amplified with P4497 MobI (SEQ ID NOS: 39 and 40) Restriction enzyme MboI | 730 |
| Test rice genome having the Rf-1 gene (homozygous) | |
| no | 385, 345 |
| Amplified with P9493 BslI (SEQ ID NOS: 41 and 42) Restriction enzyme BslI | 126 |

| Test rice genome having the Rf-1 gene (homozygous) | |
|---|---|
| no | 100, 26 |

| Amplified with P23945 MboI (SEQ ID NOS: 43 and 44) Restriction enzyme MboI | |
|---|---|
| Test rice genome having the Rf-1 gene (homozygous) | 160, 100 |
| no | 260 |

| Amplified with P41030 TaqI (SEQ ID NOS: 45 and 46) Restriction enzyme TaqI | |
|---|---|
| Test rice genome having the Rf-1 gene (homozygous) | 280 |
| no | 90, 190 |

| Amplified with P45177 BstUI (SEQ ID NOS: 47 and Restriction enzyme BstUI | 48) |
|---|---|
| Test rice genome having the Rf-1 gene (homozygous) | 20,65,730 |
| no | 20,65,175,555 |

| Amplified with B60304 MspI (SEQ ID NOS: 49 and 50) Restriction enzyme MspI | |
|---|---|
| Test rice genome having the Rf-1 gene (homozygous) | 330 |
| no | 220, 110 |

| Amplified with B59066 BsaJI (SEQ ID NOS: 51 and Restriction enzyme BsaJI 52) | |
|---|---|
| Test rice genome having the Rf-1 gene (homozygous) | 420 |
| no | 65, 355 |

| Amplified with B56691 XbaI (SEQ ID NOS: 53 and 54) Restriction enzyme XbaI | |
|---|---|
| Test rice genome having the Rf-1 gene (homozygous) | 670 |
| no | 140, 530 |

In Example 3 below, recombinants proximal to the Rf-1 gene having pollen fertility (RS1-RS2, RC1-RC8) were tested for the chromosomal organization of the Rf-1 region using 14 polymorphic markers including the 8 primer pairs described above. As a result, it was confirmed that all the plants carry the Rf-1 gene derived from the indica variety between P9493 BslI and 59066 BsaJI. This result showed that recombinant pollens having the chromosomal organization as shown in Fig. 3 have pollen fertility, i.e. the Rf-1 gene is functional in these pollens. This means that a sequence determining the presence of the function of the Rf-1 gene is included in the indica region common to these recombinant pollens, i.e. in a region from the P4497 MboI to B56691 XbaI loci (about 65 kb) as estimated at maximum.

In the present invention, chromosomal walking was started on the presumption that the S12564 Tsp509I locus should be vary proximal to the Rf-1 locus as judged from the frequency of appearance of individuals by crossing. In fact, the genetic distance between both loci has been calculated to be about 0.04 cM as the result of the high-precision segregation analysis of the present invention. Even one of markers known to be most closely linked to the Rf-1 locus as described in Japanese Patent Public Disclosure No. 2000-139465 is reported to have a genetic distance of 1 cM from the Rf-1 locus. Considering that 1 cM is estimated to be equivalent to 300 kb on average in rice, a considerable time should be required to restrict the Rf-1 gene region if chromosomal walking were started from the marker described in Japanese Patent Public Disclosure No. 2000-139465.

### VI. Method for inhibiting the function of Rf-1 gene to restore fertility

According to the present invention, the nucleic acid containing the locus of a fertility restorer gene (Rf-1) including the nucleic acids which function to restore fertility was isolated. The entire base sequence thereof was determined, whereby the fertility restoring function of the Rf-1 gene can be controlled by genetic engineering techniques. Thus, the present invention further provides a method for inhibiting the function of Rf-1 to restore fertility.

A method for inhibiting the function of the Rf-1 gene to restore fertility according to one embodiment of the present invention comprises introducing an antisense having at least 100 continuous bases in length, and having a base sequence complementary to a nucleic acid having the base sequence of SEQ ID NO.27, or to a nucleic acid having a base sequence which is identical to at least 70% of the base sequence of SEQ ID NO.27, and which functions to restore fertility.

In an embodiment, the method for inhibiting the function of the Rf-1 gene to restore fertility according to the present invention comprises introducing an antisense having at least 100 continuous bases in length, and being selected from base sequences complementary to a nucleic acid having the base sequence of bases 38538-54123, preferably bases 42357-53743, more preferably bases 42132-48883 of SEQ ID NO: 27, or to a nucleic acid having a base sequence which is identical to at least 70% of the base sequence of bases 38538-54123, preferably bases 42357-53743, more preferably bases 42132-48883 of SEQ ID NO: 27 and, which functions to restore fertility.

The antisense has a length of at least 100 bases or more, more preferably 500 bases or more, most preferably 1000 bases or more. From the viewpoint of technical convenience of introduction, it preferably has a length of 10000 bases or less, more preferably 5000 bases or less. The antisense can be synthesized by known methods. The antisense can be introduced into rice by known methods as described in e.g. Terada et al. (Plant Cell Physiol. 2000 Jul, 41(7), pp. 881-888).

It is also anticipated that Rf-1 disrupted lines can be established by screening variant lines containing a transposable element such as, but not limited to, Tos17 (Hirochika H. et al. 1996, Proc. Natl. Acad. Sci. USA 93, pp. 7783-7788) for a line containing the transposable element in the base sequence of SEQ ID NO: 27. In plants, gene disruption by homologous recombination has been studied. It may also be possible to inhibit fertility restoring function by establishing such a line in which the Rf-1 gene has been replaced by a variant Rf-1 gene using a nucleic acid having the base sequence of SEQ ID NO.27, or a nucleic acid having a base sequence which is identical to at least 70% of the base sequence of SEQ ID NO.27.

### References

1. Fukuta et al. 1992, Jpn J. Breed. 42 (supl.1) p.164-165.
2. Japanese Patent Public Disclosure No. HEI7(1995)-222588.
3. Japanese Patent Public Disclosure No. HEI9(1997)-313187.
4. Japanese Patent Public Disclosure No. 2000-139465.
5. Harushima et al. 1998, Genetics 148 p.479-494.
6. Michaels and Amasino 1998, The Plant Journal 14(3) p.381-385.
7. Neff et al. 1998, The plant Journal 14(3) p.387-392.
8. D.E. Harry, et al., Theor Appl Genet (1998) 97:p.327-336.
9. Hiei et al., Plant Journal (1994),6(2),p.272-282.
10. Komari et al., Plant Journal (1996) 10, p.165-174.
11. Ditta et al., Proc.Natl.Acad.Sci. USA (1980), 77: p.7347-7351,
12. P. Vos et al., Nucleic Acids Res. Vol.23, p. 4407-4414 (1995).
13. O.Parnaud,X. et al, Mol.Gen.Genet.(1996) 252:p.597-607.
14. A.Konieczny et al.,(1993),Plant J.4(2)p.403-410.
15. Edwards et al.,Nucleic Acids Res. 8(6): 1349, 1991.
16. Murray M.G. et al., Nucleic Acids Res. 8(19):4321-5, 1980.
17. Terada et al., Plant Cell Physiol. 2000 Jul, 41(7), p.881-888.
18. Hirochika H. et al. 1996, Proc.Natl.Acad.Sci.USA 93, p.7783-7788.

### Examples

The following examples further illustrate the present invention but are not intended to limit the technical scope of the invention. Those skilled in the art can readily add modifications/changes to the present invention on the basis of the description of the specification, and those modifications/changes are included in the technical scope of the present invention.

### Reference examples

The following reference examples are based on the examples described in our prior application (Japanese Patent Application No. 2000-247204 filed August 17, 2000).

### Reference example 1: Conversion of RFLP markers around Rf-1 gene to PCR markers

In this reference example, nine RFLP markers (i.e., R1877, G291, R2303, S12564, C1361, S10019, G4003, S10602 and G2155) around the locus of Rf-1 gene were converted to PCR markers.

### (1) Materials and methods

The following nine RFLP markers, R1877, G291, R2303, S12564, C1361, S10019, G4003, S10602 and G2155, were purchased from the National Institute of Agrobiological Sciences, the Ministry of Agriculture, Forestry and Fisheries of Japan. After determining the base sequences of the inserts in the vectors, experiments were conducted according to the following procedures. Among rice varieties herein, Asominori belongs to japonica, and IR24 belongs to indica.

### (2) Preparation of Asominori genomic library

Total DNA was extracted from green leaves of Asominori by the CTAB method. After partial digestion with MboI, the DNA was fractionated according to size by NaCl density gradient centrifugation (6-20% linear gradient, 20°C, 37,000 rpm, 4 hr, total volume = 12 mL). A portion of each fraction (about 0.5 mL) was subjected to electrophoresis and fractions containing 15-20 kb DNA were collected and purified. A library was constructed using Lambda DASH II (Stratagene) as a vector in accordance with the attached protocol. Giga Pack III Gold (Stratagene) was used for packaging. After packaging, 500 µL of SM Buffer and 20 µL of chloroform were added. After centrifugation, 20 µL of chloroform was added to the supernatant to make a library solution.

XL-1 Blue MRA (P2) was infected with 5 µL of a 50-fold dilution of the library solution, whereupon 83 plaques were formed. This corresponded to 4.15 x 10⁵ pfu per library, and hence, it was calculated that the plaques covered 8.3 x 10⁹ bp assuming that the average length of the inserted fragments was 20 kb. The library was therefore considered to have an adequate size for the rice genome (4 x 10⁸ bp).

### (3) Isolation of genomic clones containing R1877-, C1361- and G4003-marker regions.

As for C1361 and G4003, plasmids containing the RFLP marker probe were isolated and subjected to restriction enzyme treatment and electrophoresis to separate the RFLP marker probe portion; the desired DNA was recovered on a DNA recovery filter (Takara SUPREC-01). As for R1877, primers were designed that were specific to both ends of the marker probe and PCR was performed with the total DNA of Asominori used as a template; the amplification products were electrophoresed and recovered by the method described above. The recovered DNA was labelled with a Rediprime DNA Labelling System (Amersham Pharmacia) to prepare a probe for screening the library. PCR was performed in the usual manner (this also applies to the following description).

Screening of the library was performed in the usual manner after blotting the plaques onto Hybond-N+ (Amersham Pharmacia). After primary screening, areas of positive plaques were individually punched out, suspended in SM buffer and subjected to the second round of screening. After the second screening, the positive plaques were punched out and subjected to the third round of screening to isolate a single plaque.

The isolated plaque of interest was suspended in SM buffer and primary multiplication of the phage was performed by the plate lysate method. The resulting phage-enriched solution was subjected to secondary multiplication by shake culture and the phage DNA was purified with Lambda starter kit (QIAGEN).

For each marker, primary screening was conducted on eight plates. A 10 µL aliquot of the library solution was employed per plate. After the primary, second and third rounds of screening, four genomic clones in association with R1877 were isolated and three were isolated in association with each of C1361 and G4003.

### (4) Conversion of R1877 to PCR marker

The isolated genomic clones were analyzed to identify the causative site of RFLP, or the EcoRI site that exists in IR24 (indica rice) but not in Asominori (japonica rice), thereby converting R1877 to a PCR marker.

Specifically, the four isolated clones were subjected to the following analyses. First, T3 and T7 primers were used to determine the base sequences at both ends of the insert in each clone. Then, primers extending outwardly from both ends of the marker probe were designed. They were combined with T3 and T7 primers to give a combination of four primers in total, and employed in PCR with each clone used as the template.

In a separate step, each clone was digested with NotI and EcoRI, and electrophoresed to estimate the insert size and the length of each EcoRI fragment.

These analyses revealed the relative positions of the individual clones. In RFLP analysis, marker probe R1877 was reported to detect an EcoRI fragment of 20 kb in Nipponbare (japonica rice) and one of 6.4 kb in Kasalath (indica rice) (ftp://ftp.staff.or.jp/pub/geneticmap98/parentsouthern/chrl 0/R1877.JPG). This fact, taken together with the results of analysis described above, gave a putative position for the EcoRI site that existed in IR24 but not in Asominori. Hence, a primer combination (SEQ ID NO:1 x SEQ ID NO:2) that was designed to amplify the nearby region was employed to perform genomic PCR over 30 cycles, each cycle consisting of 94°C x 1 min, 58°C x 1 min and 72°C x 2 min. The PCR product was treated with EcoRI and subjected to electrophoresis on 0.7% agarose gel.

As a result, the expected polymorphisms were observed between Asominori and IR24. By treatment with EcoRI, the PCR product (∼3200 bp) was cleaved to yield 1500 bp and 1700 bp fragments in IR24 but not in Asominori. Mapping of the marker was made with an RIL (recombinant inbred line) of Asominori-IR24 with the results that the PCR marker was located in the same region as that of RFLP marker locus R1877, thereby confirming the conversion of RFLP marker R1877 to a PCR marker, which was named R1877 EcoRI in the present invention.

### (5) Conversion of G4003 to PCR marker

The isolated genomic clones were analyzed to identify the causative site of RFLP, or the HindIII site that existed in Asominori but not in IR24, thereby converting G4003 to a PCR marker.

By performing analyses similar to those employed for R1877, the relative positions of the three isolated clones were revealed. In RFLP analysis, marker probe G4003 was reported to detect a HindIII fragment of 3 kb in Nipponbare (japonica rice) and one of 10 kb in Kasalath (indica rice) (ftp://ftp.staff.or.jp/pub/geneticmap98/parentsouthern/chrl 0/R1877.JPG). This report, taken together with the analyses described above, led to a temporary conclusion that the HindIII site that existed in Asominori but not in IR24 would be at either one of two candidate sites. Hence, a primer combination (SEQ ID NOS: 3 and 4) that was designed to amplify the area in the neighborhood of each HindIII site was employed to perform genomic PCR over 35 cycles, each cycle consisting of 94°C x 30 sec, 58°C x 30 sec and 72°C x 30 sec. The PCR product was treated with HindIII and subjected to electrophoresis on 2% agarose gel. As a result, the HindIII site within the marker probe was found to have polymorphisms. By treatment with HindIII, the PCR product (362 bp) was cleaved to yield a 95 bp fragment and a 267 bp fragment in Asominori but not in IR24. Mapping of the site demonstrated the conversion of RFLP marker G4003 to a PCR marker, which was named G4003 HindIII (SEQ ID NO:19) in the present invention.

### (6) Conversion of C1361 to PCR marker

Primers were designed on the basis of the base sequence information of the isolated genomic clones. PCR was performed with the total DNAs of Asominori and IR24 being used as a template and the PCR product was recovered by known methods after electrophoresis. Using the recovered DNA as a template, the inventors analyzed the base sequence of each of the rice varieties with ABI Model 310 in search of mutations that would cause polymorphisms.

By performing analyses similar to those employed for R1877, approximate relative positions of the three isolated clones could be established. As it turned out, however, regions around the C1361 marker would be difficult to amplify by PCR or determine their base sequences, and hence, it would not be easy to identify the causative site of RFLP. Hence, the inventors took notice of the region capable of yielding a comparatively long PCR product (2.7 kb) and made an attempt to create a dCAPS marker.

Specifically, upon comparing the base sequences of the genomic PCR products of said region using Asominori and Koshihikari (both japonica rice) and Kasalath and IR24 (both indica rice), the inventors found six sites of polymorphism between japonica and indica. One of these six sites was used to create a dCAPS marker. To this end, with SEQ ID NO:5 and SEQ ID NO:6 used as primers, PCR was performed over 35 cycles, each cycle consisting of 94°C x 30 sec, 58°C x 30 sec and 72°C x 30 sec. The PCR product was treated with MwoI and analyzed by electrophoresis on 3% MetaPhor™ agarose gel. In Asominori, cleavage occurred at two sites to give three observable bands of about 25 bp, 50 bp and 79 bp, but in IR24 cleavage occurred at one site to give two observable bands of about 50 bp and 107 bp. Mapping demonstrated the conversion of RFLP marker C1361 to a PCR marker, which was named C1361 MwoI (SEQ ID NO:20) in the present invention.

### (7) Conversion of G2155 to PCR marker

Primers specific to both ends of the marker probe were designed and PCR was performed with the total DNA of Asominori, Koshihikari, IR24 or IL216 (a line produced by introducing Rf-1 gene into Koshihikari by back crossing; its genotype was Rf-1/Rf-1) being used as a template. Purification of the PCR product and searching for a mutation that would be useful for providing restriction fragment polymorphisms were performed by the methods already described above.

Specifically, as a result of comparing the base sequences of corresponding regions of the varieties under test, mutations were found at three sites between the variety/line (IR24 and IL216) having Rf-1 gene and the variety (Asominori and Koshihikari) not having Rf-1 gene. One of the three sites was utilized to create a dCAPS marker. To this end, SEQ ID NO:7 and SEQ ID NO:8 were used as primers to perform PCR over 35 cycles, each cycle consisting of 94°C x 30 sec, 58°C x 30 sec and 72°C x 30 sec. The PCR product was treated with MwoI and analyzed by electrophoresis on 3% MetaPhor™ agarose gel. In Asominori, cleavage occurred at one site to give two observable bands of about 25 bp and 105 bp, but in IR24, cleavage occurred at two sites to give three observable bands of about 25 bp, 27 bp and 78 bp. Mapping demonstrated the conversion of RFLP marker G2155 to a PCR marker, which was named G2155 MwoI (SEQ ID NO:21) in the present invention.

### (8) Conversion of G291 to PCR marker

Primers specific to internal sequences of the marker probe were designed and used in various combinations to perform PCR to find a primer combination that could yield an amplification product of the expected size. Using the selected primer combination, the inventors performed PCR with the total DNA of Asominori, Koshihikari, IR24 and IL216 used as a template. Purification of the PCR product and searching for a mutation that could be utilized in providing restriction fragment polymorphisms were performed by the methods already described above.

Specifically, using the primers designed to be specific for the marker probe sequence, the inventors performed genomic PCR of each variety under test and compared the base sequences of the products. As a result, mutations were found at four sites between the variety/line having Rf-1 gene (IR24 and IL216) and the variety (Asominori and Koshihikari) not having Rf-1 gene. One of the four sites was used to create a dCAPS marker. To this end, SEQ ID NO:9 and SEQ ID NO:10 were used as primers to perform PCR over 35 cycles, each cycle consisting of 94°C x 30 sec, 58°C x 30 sec and 72°C x 30 sec. The PCR product was treated with MspI and analyzed by electrophoresis on 3% MetaPhor™ agarose gel. In the varieties/lines having Rf-1 gene, cleavage occurred at two sites to give three observable bands of about 25 bp, 49 bp and 55 bp, but in the varieties not having Rf-1 gene, cleavage occurred at one site to give two observable bands of about 25 bp and 104 bp. Mapping demonstrated the conversion of RFLP marker G291 to a PCR marker, which was named G291 MspI (SEQ ID NO:22) in the present invention.

### (9) Conversion of R2303 to PCR marker

Primers specific to internal sequences of the marker probe were designed and PCR was performed with the total DNA of Asominori (japonica rice) and IR24 and Kasalath (indica rice) used as a template. Purification of the PCR product and searching for a mutation that could be used for providing restriction fragment polymorphisms were performed by the methods already described above.

As a result of comparing the base sequences of corresponding regions of the varieties under test, a mutation was found between japonica rice and indica rice. Since the mutation occurred at the BslI recognition site, the site was directly used to create a CAPS marker. To this end, SEQ ID NO:11 and SEQ ID NO:12 were used as primers and PCR was performed over 30 cycles, each cycle consisting of 94°C x 1 min, 58°C x 1 min and 72°C x 2 min. The PCR product was treated with BslI and analyzed by electrophoresis on 2% agarose gel. In japonica rice, cleavage occurred at one site to give two observable bands of about 238 bp and 1334 bp, but in indica rice, cleavage occurred at two sites to give three observable bands of about 238 bp, 655 bp and 679 bp. Mapping demonstrated the conversion of RFLP marker R2303 to a PCR marker, which was named R2303 BslI (SEQ ID NO:23) in the present invention.

### (10) Converting S10019 to PCR marker

S10019 was converted to a PCR marker in accordance with the method (9) of converting R2303 to a PCR marker.

Specifically, as a result of comparing the base sequences of corresponding regions of the varieties under test, a mutation was found between japonica rice and indica rice. Since the mutation occurred at the BstUI recognition site, the site was directly used to create a CAPS marker. To this end, SEQ ID NO:13 and SEQ ID NO:14 were used as primers and PCR was performed over 30 cycles, each cycle consisting of 94°C x 1 min, 58°C x 1 min and 72°C x 1 min. The PCR product was treated with BstUI and analyzed by electrophoresis on 2% agarose gel. In japonica rice, cleavage occurred at one site to give two observable bands of about 130 bp and 462 bp, but in indica rice, cleavage occurred at two sites to give three observable bands of about 130 bp, 218 bp and 244 bp. Mapping demonstrated the conversion of RFLP marker S10019 to a PCR marker, which was named S10019 BstUI (SEQ ID NO:24) in the present invention.

### (11) Conversion of S10602 to PCR marker

S10602 was converted to a PCR marker in accordance with the method (9) of converting R2303 to a PCR marker.

Specifically, as a result of comparing the base sequences of corresponding regions of the varieties under test, a mutation was found between japonica rice and indica rice. The mutation was used to create a CAPS marker. To this end, SEQ ID NO:15 and SEQ ID NO:16 were used as primers and PCR was performed over 33 cycles, each cycle consisting of 94°C x 1 min, 58°C x 1 min and 72°C x 1 min. The PCR product was treated with KpnI and analyzed by electrophoresis on 2% agarose gel. In japonica rice, cleavage occurred at one site to give two observable bands of about 117 bp and 607 bp, but in indica rice, no cleavage occurred, giving only an observable band of 724 bp.
Mapping demonstrated the conversion of RFLP marker S10602 to a PCR marker, which was named S10602 KpnI (SEQ ID NO:25) in the present invention.

### (12) Conversion of S12564 to PCR marker

S12564 was converted to a PCR marker in accordance with the method of converting R2303 to a PCR marker.

Specifically, as a result of comparing the base sequences of corresponding regions of the varieties under test, a mutation was found between japonica rice and indica rice. The mutation was used to create a dCAPS marker. To this end, SEQ ID NO:17 and SEQ ID NO:18 were used as primers and PCR was performed over 35 cycles, each cycle consisting of 94°C x 30 sec, 58°C x 30 sec and 72°C x 30 sec. The PCR product was treated with Tsp509I and analyzed by electrophoresis on 3% MetaPhor™ agarose gel. In japonica rice, cleavage occurred at two sites to give three observable bands of 26 bp, 41 bp and 91 bp, but in indica rice, cleavage occurred at one site to give two observable bands of 41bp and 117 bp. Mapping demonstrated the conversion of RFLP marker S12564 to a PCR marker, which was named S12564 Tsp509I (SEQ ID NO:26) in the present invention.

### Reference example 2: Mapping of Rf-1 Gene Locus

DNA was extracted from 1042 seedlings of the F1 population produced by pollinating MS Koshihikari with MS-FR Koshihikari, and the DNA extract was used in the analysis. MS Koshihikari (generation: BC10F1) was created by replacing the cytoplasm of Koshihikari with BT type male sterility cytoplasm. MS-FR Koshihikari was a line created by introducing Rf-1 gene from IR8 (supplied from National Institute of Agrobiological Sciences) into MS Koshihikari (the locus of Rf-1 gene being heterozygous).

First, each individual was investigated for the genotype at two marker loci R1877 EcoRI and G2155 MwoI described in Reference example 1 that would presumably be located on opposite sides of the locus of Rf-1 gene. Japonica type homozygotes with respect to either locus R1877 EcRI or G2155 MwoI were regarded as recombinants between these two marker loci. Then, each of such recombinants was investigated for the genotypes of G291 MspI, R2303 BslI, S12564 Tsp 509I, C1361 MwoI, S10019 BstUI, G4003 HindIII and S10602 KpnI loci, and the positions of recombination were identified.

The genotype investigation with respect to R1877 EcoRI and G2155 MwoI loci revealed that 46 individuals were recombinants around the locus of Rf-1 gene. Genotypes of the marker loci around the locus of Rf-1 gene were investigated and the results are shown in Table 3.

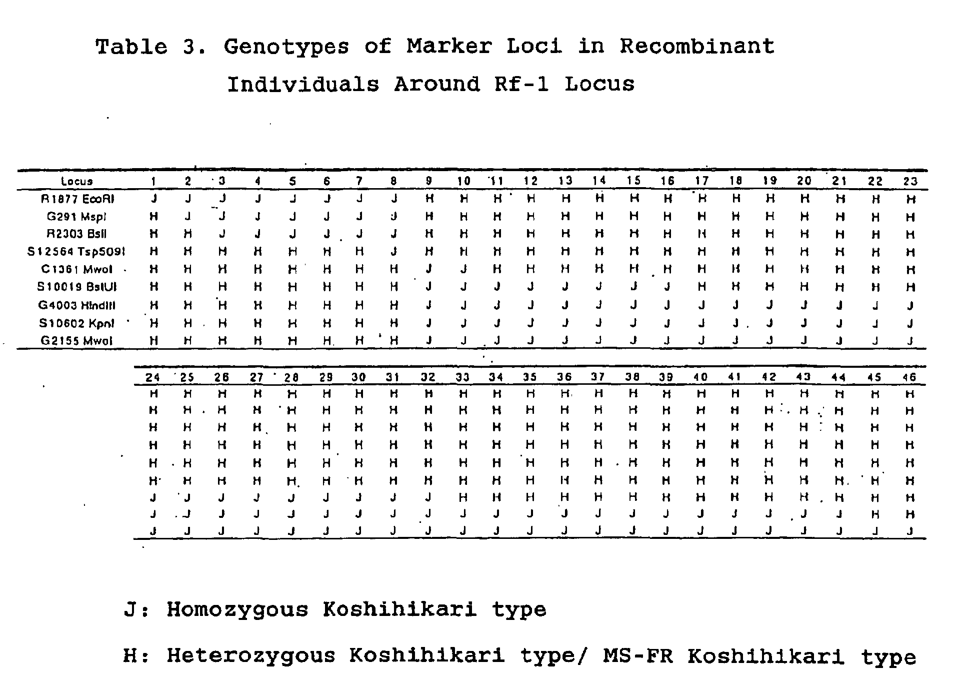

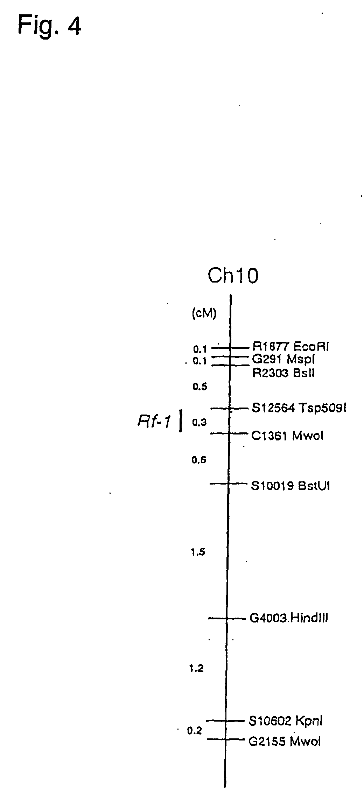

As shown in Table 3, recombinant 8 homozygous for japonica at the S12564 Tsp509I marker locus and recombinants 9 and 10 homozygous for japonica at the C1361 Mwo marker locus were obtained. As all of these recombinants restored fertility, the former was regarded as a recombinant between the Rf-1 and S12564 Tsp509I loci while the latter were regarded as recombinants between the Rf-1 and C1361 MwoI loci, showing that the Rf-1 gene is located between the S12564 Tsp509I and C1361 MwoI loci. Based on the report that only pollens carrying the Rf-1 gene have fertility in individuals having the BT type male sterile cytoplasm in the cross above (C.Shinjyo, JAPAN.J.GENETICS Vol.44, No.3:149-156(1969)), the Rf-1 gene locus could be located on a detailed linkage map (Fig. 4).

### Example 1: Acquisition of recombinant individuals proximal to the Rf-1 locus

### (Materials and Methods)

DNA was extracted from each of 4103 individuals of BC10F1 population produced by pollinating MS Koshihikari (generation: BC10F1) with MS-FR Koshihikari (generation: BC9F1, heterozygous at the Rf-1 locus), and genotyped at the S12564 Tsp509I and C1361 MwoI loci in the same manner as described in Reference example 2 above. Individuals having a genotype homozygous for Koshihikari at the S12564 Tsp509I locus were regarded as those generated by recombination between the Rf-1 and S12564 Tsp509I loci, while individuals having a genotype homozygous for Koshihikari at the C1361 MwoI locus were regarded as those generated by recombination between the Rf-1 and C1361 MwoI loci.

### (Results and Discussion)

A survey of 4103 individuals revealed one recombinant individual between the Rf-1 and S12564 Tsp509I loci and 6 recombinant individuals between the Rf-1 and C1361 MwoI loci. The previous survey of 1042 individuals obtained by crossing in Reference example 2 above had already revealed one recombinant individual between the Rf-1 and S12564 Tsp509I loci and 2 recombinant individuals between the Rf-1 and C1361 MwoI loci as shown in Table 3.

Thus, a total of 2 recombinant individuals between the Rf-1 and S12564 Tsp509I loci and 8 recombinant individuals between the Rf-1 and C1361 MwoI loci were able to be oabtained from 5145 individuals. These 10 individuals were tested by high-precision segregation analysis in the examples below.

### Example 2 chromosomal walking

### (1) First chromosomal walking

### (Materials and Methods)

A genomic library was constructed from the genomic DNA of Asominori japonica (not carrying Rf-1) using Lambda DASH II vector as described in Reference example 1 and tested by chromosomal walking.

PCR was routinely performed using total DNA of Asominori as a template in combination with the following primer pair: 5'-atcaggagccttcaaattgggaac-3' (SEQ ID NO: 29) and 5'-ctcgcaaattgcttaattttgacc-3' (SEQ ID NO: 30) designed for a partial base sequence (Accession No. D47284) of RFLP probe S12564. The resulting amplification products of about 1200 bp were electrophoresed on an agarose gel and then purified by QIAEXII (QIAGEN). The purified DNA was labeled with a rediprime DNA labelling system (Amersham Pharmacia) to give a library screening probe (probe A, Fig. 1).

The library was routinely screened after plaques were blotted onto Hybond-N⁺ (Amersham Pharmacia). Single plaques were separated, after which phage DNA was purified by the plate lysate method using Lambda Midi kit (QIAGEN).

### (Results and Discussion)

The results of terminal base sequence analysis and restriction enzyme fragment length analysis showed that two (WSA1 and WSA3) of 4 clones obtained by screening were in a relative position as shown in Fig. 1. The Asominori genomic base sequences corresponding to WSA1 and WSA3 were determined by primer walking (DNA Sequencer 377, ABI).

### (2) Second chromosomal walking

### (Materials and Methods)

In addition to the Asominori genomic library described above, an IR24 genomic library was similarly constructed from the genomic DNA of an indica variety IR24 (carrying Rf-1) and tested by chromosomal walking.

PCR was routinely performed using DNA of WSA3 as a template in combination with the following primer pair: 5'-tgaaggagttatgggtgcgtgacg-3' (SEQ ID NO: 31) and 5'-ttgccgagcacacttgccatgtgc-3' (SEQ ID NO: 32) designed for the Asominori genomic base sequence determined in (1). The resulting amplification products of 524 bp were purified and labeled by the method described above to give a library screening probe (probe E, Fig. 1).

Library screening and phage DNA purification were performed by the method described above.

### (Results and Discussion)

The results of terminal base sequence analysis and restriction enzyme fragment length analysis showed that one (WSE8) of 15 clones obtained by screening of the Asominori genomic library was in a relative position as shown in Fig. 1. The Asominori genomic base sequence corresponding to WSE8 was determined by primer walking.

The results of terminal base sequence analysis and restriction enzyme fragment length analysis showed that two (XSE1 and XSE7) of 7 clones obtained by screening of the IR24 genomic library were in a relative position as shown in Fig. 1. The IR24 genomic base sequences corresponding to XSE1 and XSE7 were determined by primer walking.

### (3) Third chromosomal walking

### (Materials and Methods)

The Asominori genomic library and IR24 genomic library described above were tested by chromosomal walking.

PCR was routinely performed using DNA of WSE8 as a template in combination with the following primer pair: 5'-gcgacgcaatggacatagtgctcc-3' (SEQ ID NO: 33) and 5'-ttacctgccaagcaatatccatcg-3' (SEQ ID NO: 34) designed for the Asominori genomic base sequence determined in (2). The resulting amplification products of 1159 bp were purified and labeled by the method described above to give a library screening probe (probe F, Fig. 1).

Library screening and phage DNA purification were performed by the method described above.

### (Results and Discussion)

The results of terminal base sequence analysis and restriction enzyme fragment length analysis showed that two (WSF5 and WSF7) of 8 clones obtained by screening of the Asominori genomic library were in a relative position as shown in Fig. 1. The Asominori genomic base sequences corresponding to WSF5 and WSF7 were determined by primer walking.

The results of terminal base sequence analysis and restriction enzyme fragment length analysis showed that two (XSF4 and XSF20) of 13 clones obtained by screening of the IR24 genomic library were in a relative position as shown in Fig. 1. The IR24 genomic base sequences corresponding to XSF4 and XSF20 were determined by primer walking.

### (4) Fourth chromosomal walking

### (Materials and Methods)

The Asominori genomic library and IR24 genomic library described above were tested by chromosomal walking.

PCR was routinely performed using DNA of WSF7 as a template in combination with the following primer pair: 5'-aaggcatactcagtggagggcaag-3' (SEQ ID NO: 35) and 5'-ttaacctgaccgcaagcacctgtc-3' (SEQ ID NO: 36) designed for the Asominori genomic base sequence determined in (3). The resulting amplification products of 456 bp were purified and labeled by the method described above to give a library screening probe (probe G, Fig. 1).

Library screening and phage DNA purification were performed by the method described above.

### (Results and Discussion)

The results of terminal base sequence analysis and restriction enzyme fragment length analysis showed that two (WSG2 and WSG6) of 6 clones obtained by screening of the Asominori genomic library were in a relative position as shown in Fig. 1. The Asominori genomic base sequences corresponding to WSG2 and WSG6 were determined by primer walking.

The results of terminal base sequence analysis and restriction enzyme fragment length analysis showed that three (XSG8, XSG16 and XSG22) of 14 clones obtained by screening of the IR24 genomic library were in a relative position as shown in Fig. 1. The IR24 genomic base sequences corresponding to XSG8, XSG16 and XSG22 were determined by primer walking.

### (5) Fifth chromosomal walking

### (Materials and Methods)

The IR24 genomic library described above was tested by chromosomal walking.

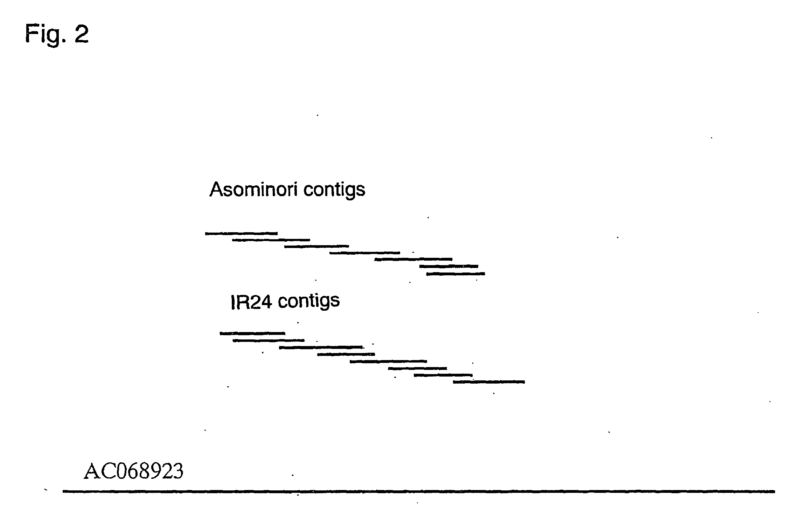

We perused the public website of TIGR (The Institute for Genomic Research) and found that a BAC (Bacterial Artificial Chromosome) clone (Accession No. AC068923) containing RFLP marker S12564 had been deposited with a public database (GenBank). This BAC clone contains the genomic DNA of Nipponbare japonica and it was shown from base sequence comparison to completely include the contig regions of Asominori and IR24 prepared in (1)-(4) (Fig. 2).

Thus, PCR was routinely performed using total DNA of IR24 as a template in combination with the following primer pair: 5'-tggatggactatgtggggtcagtc-3' (SEQ ID NO: 37) and 5'-agtggaagtggagagagtagggag-3' (SEQ ID NO: 38) designed to amplify a part of this BAC clone. The resulting amplification products of about 600 bp were purified and labeled by the method described above to give a library screening probe (probe H, Fig. 1).

Library screening and phage DNA purification were performed by the method described above.

### (Results and Discussion)

The results of terminal base sequence analysis and restriction enzyme fragment length analysis showed that one (XSH18) of 15 clones obtained by screening of the IR24 genomic library was in a relative position as shown in Fig. 1. The IR24 genomic base sequence corresponding to XSH18 was determined by primer walking.

### Example 3: High precision segregation analysis

### (1) Development of PCR marker P4497 MboI

Comparison between the genomic base sequence corresponding to the IR24 contig (SEQ ID NO: 27) and the genomic base sequence corresponding to the Asominori contig (SEQ ID NO: 28) determined in Example 2 revealed that the 1239th base,of SEQ ID NO: 27 is A while the 12631st base of SEQ ID NO: 28 corresponding to said position is G.

For detecting this change, fragments of about 730 bp are first amplified by PCR from a region surrounding said position using the following primer pair: P4497 MboI F: 5'-ccctccaacacataaatggttgag-3' (SEQ ID NO: 39) (corresponding to bases 853-876 of SEQ ID NO: 27)
(corresponding to bases 12247-12270 of SEQ ID NO: 28) and
P4497 MboI R: 5'-tttctgccaggaaactgttagatg-3' (SEQ ID NO: 40) (corresponding to bases 1583-1560 of SEQ ID NO: 27)
(corresponding to bases 12975-12952 of SEQ ID NO: 28).
The amplification products can be visualized by electrophoresis on an agarose gel after treatment with MboI. Thus, the change can be detected as a difference in mobility in the agarose gel due to the difference in the length of DNA after MboI treatment because the amplification products from Asominori DNA having an MboI recognition sequence (GATC) are cleaved with MboI while the amplification products from IR24 DNA are not cleaved with MboI for the lack of the MboI recognition sequence.

### (2) Development of PCR marker P9493 BslI

Comparison between the genomic base sequence corresponding to the IR24 contig (SEQ ID NO: 27) and the genomic base sequence corresponding to the Asominori contig (SEQ ID NO: 28) determined in Example 2 revealed that the 6227th base of SEQ ID NO: 27 is A while the 17627th base of SEQ ID NO: 28 corresponding to said position is C.

For detecting this change, fragments of 126 bp are first amplified by PCR from a region surrounding said position using the following primer pair:
P9493 BslI F: 5'-gcgatcttatacgcatactatgcg-3' (SEQ ID NO: 41) (corresponding to bases 6129-6152 of SEQ ID NO: 27)
(corresponding to bases 17529-17552 of SEQ ID NO: 28) and
P9493 BslI R: 5'-aaagtctttgttccttcaccaagg-3' (SEQ ID NO: 42) (corresponding to bases 6254-6231 of SEQ ID NO: 27)
(corresponding to bases 17654-17631 of SEQ ID NO: 28).
The amplification products can be visualized by electrophoresis on an agarose gel after treatment with BslI. Thus, the change can be detected as a difference in mobility in the agarose gel due to the difference in the length of DNA after BslI treatment because the amplification products from Asominori DNA having a BslI recognition sequence (CCNNNNNNNGG) are cleaved with BslI while the amplification products from IR24 DNA are not cleaved with BslI for the lack of the BslI recognition sequence.

This marker was developed by applying the dCAPS method (Michaels and Amasino 1998, Neff et al., 1998). Specifically, g is substituted for a at the base 6236 of SEQ ID NO: 27 and the base 17636 of SEQ ID NO: 28 by the use of P9493 BslI R primer described above. Thus, the fragments from Asominori DNA come to have a sequence of CCtttccttGG at 17626-17636 of SEQ ID NO: 28 so that they are cleaved with BslI.

### (3) Development of PCR marker P23945 MboI

Comparison between the genomic base sequence corresponding to the IR24 contig (SEQ ID NO: 27) and the genomic base sequence corresponding to the Asominori contig (SEQ ID NO: 28) determined in Example 2 revealed that the 20680th base of SEQ ID NO: 27 is G while the 32079th base of SEQ ID NO: 28 corresponding to said position is A.

For detecting this change, fragments of 260 bp are first amplified by PCR from a region surrounding said position using the following primer pair: P23945 MboI F: 5'-gaggatttatcaaaacaggatggacg-3' (SEQ ID NO: 43) (corresponding to bases 20519-20544 of SEQ ID NO: 27)
(corresponding to bases 31918-31943 of SEQ ID NO: 28) and
P23945 MboI R: 5'-tgggcggcagcagtggaggataga-3' (SEQ ID NO: 44) (corresponding to bases 20778-20755 of SEQ ID NO: 27)
(corresponding to bases 32177-32154 of SEQ ID NO: 28).
The amplification products can be visualized by electrophoresis on an agarose gel after treatment with MboI. Thus, the change can be detected as a difference in mobility in the agarose gel due to the difference in the length of DNA after MboI treatment because the amplification products from IR24 DNA having an MboI recognition sequence (GATC) are cleaved with MboI while the amplification products from Asominori DNA are not cleaved with MboI for the lack of the MboI recognition sequence.

### (4) Development of PCR marker P41030 TaqI

Comparison between the genomic base sequence corresponding to the IR24 contig (SEQ ID NO: 27) and the genomic base sequence corresponding to the Asominori contig (SEQ ID NO: 28) determined in Example 2 revealed that the 45461st base of SEQ ID NO: 27 is A while the 49164th base of SEQ ID NO: 28 corresponding to said position is G.

For detecting this change, fragments of 280 bp are first amplified by PCR from a region surrounding said position using the following primer pair:
P41030 TaqI F: 5'-aagaagggagggttatagaatctg-3' (SEQ ID NO: 45) (corresponding to bases 45369-45392 of SEQ ID NO: 27)
(corresponding to bases 49072-49095 of SEQ ID NO: 28) and
P41030 TaqI R: 5'-atatcaggactaacaccactgctc-3' (SEQ ID NO: 46) (corresponding to bases 45648-45625 of SEQ ID NO: 27)
(corresponding to bases 49351-49328 of SEQ ID NO: 28).
The amplification products can be visualized by electrophoresis on an agarose gel after treatment with TaqI. Thus, the change can be detected as a difference in mobility in the agarose gel due to the difference in the length of DNA after TaqI treatment because the amplification products from Asominori DNA having a TaqI recognition sequence (TCGA) are cleaved with TaqI while the amplification products from IR24 DNA are not cleaved with TaqI for the lack of the TaqI recognition sequence.

### (5) Development of PCR marker P45177 BstUI

Comparison between the genomic base sequence corresponding to the IR24 contig (SEQ ID NO: 27) and the genomic base sequence corresponding to the Asominori contig (SEQ ID NO: 28) determined in Example 2 revealed that the 49609th base of SEQ ID NO: 27 is A while the 53311st base of SEQ ID NO: 28 corresponding to said position is G.

For detecting this change, fragments of 812 bp are first amplified by PCR from a region surrounding said position using the following primer pair:
P45177 BstUI F: 5'-acgagtagtagcgatcttccagcg-3' (SEQ ID NO: 47) (corresponding to bases 49355-49378 of SEQ ID NO: 27)
(corresponding to bases 53057-53080 of SEQ ID NO: 28) and
P45177 BstUI R: 5'-cagcgtgaaactaaaaacggaggc-3' (SEQ ID NO: 48) (corresponding to bases 50166-50143 of SEQ ID NO: 27)
(corresponding to bases 53868-53845 of SEQ ID NO: 28).
The amplification products can be visualized by electrophoresis on an agarose gel after treatment with BstUI. Thus, the change can be detected as a difference in mobility in the agarose gel due to the difference in the length of DNA after BstUI treatment because the amplification products from IR24 DNA having a BstUI recognition sequence (CGCG) at two positions are cleaved into 3 fragments with BstUI while the amplification products from Asominori DNA having the BstUI recognition sequence at three positions are cleaved with BstUI into four fragments.

### (6) Development of PCR marker B60304 MspI

Comparison between the genomic base sequence corresponding to the IR24 contig (SEQ ID NO: 27) determined in Example 2 and the base sequence of the BAC clone described above (Accession No. AC068923) revealed that the 56368th base of SEQ ID NO: 27 is T while the base of AC068923 corresponding to said position is C.

For detecting this change, fragments of about 330 bp are first amplified by PCR from a region surrounding said position using the following primer pair:
B60304 MspI F: 5'-atcccacatcatcataatccgacc-3' (SEQ ID NO: 49) (corresponding to bases 56149-56172 of SEQ ID NO: 27) and
B60304 MspI R: 5'-agcttctcccttggatacggtggcg-3' (SEQ ID NO: 50) (corresponding to bases 56479-56455 of SEQ ID NO: 27).
The amplification products can be visualized by electrophoresis on an agarose gel after treatment with MspI. Thus, the change can be detected as a difference in mobility in the agarose gel due to the difference in the length of DNA after MspI treatment because the amplification products from Nipponbare DNA having an MspI recognition sequence (CCGG) are cleaved with MspI while the amplification products from IR24 DNA are not cleaved with MspI for the lack of the MspI recognition sequence.

This marker was developed by applying the dCAPS method. Specifically, t is substituted for g at base 56463 of SEQ ID NO: 27 by the use of B60304 MspI R primer. As a result, the MspI recognition sequence of bases 56460-56463 of SEQ ID NO: 27 changes from CCGG into ccgt so that the fragments from SEQ ID NO: 27 become unable to be cleaved with MspI. Thus, the fragments from IR24 have no MspI recognition sequence, while DNA from Nipponbare has the MspI recognition sequence at one position in a region corresponding to bases 56367-56370 of SEQ ID NO: 27.

### (7) Development of PCR marker B59066 BsaJI

Comparison between the genomic base sequence corresponding to the IR24 contig (SEQ ID NO: 27) determined in Example 2 and the base sequence of the BAG clone described above (Accession No. AC068923) revealed that the 57629th base of SEQ ID NO: 27 is C while the base of AC068923 corresponding to said position is CC.

For detecting this change, fragments of about 420 bp are first amplified by PCR from a region surrounding said position using the following primer pair:
B59066 BsaJI F: 5'-atttgttggttagttgcggctgag-3' (SEQ ID NO: 51) (corresponding to bases 57563-57586 of SEQ ID NO: 27) and
B59066 BsaJI R: 5'-gcccaaactcaaaaggagagaacc-3' (SEQ ID NO: 52) (corresponding to bases 57983-57960 of SEQ ID NO: 27).
The amplification products can be visualized by electrophoresis on an agarose gel after treatment with BsaJI. Thus, the change can be detected as a difference in mobility in the agarose gel due to the difference in the length of DNA after BsaJI treatment because the amplification products from Nipponbare DNA having a BsaJI recognition sequence (CCNNGG) are cleaved with BsaJI while the amplification products from IR24 DNA are not cleaved with BsaJI for the lack of the BsaJI recognition sequence.

### (8) Development of PCR marker B56691 XbaI

Comparison between the genomic base sequence corresponding to the IR24 contig (SEQ ID NO: 27) determined in Example 2 and the base sequence of the BAC clone described above (Accession No. AC068923) revealed that the 66267th base of SEQ ID NO: 27 is G while the base of AC068923 corresponding to said position is C.

For detecting this change, fragments of about 670 bp are first amplified by PCR from a region surrounding said position using the following primer pair:
B56691 XbaI F: 5'-cctcaagtctcccctaaagccact-3' (SEQ ID NO: 53) (corresponding to bases 66129-66152 of SEQ ID NO: 27) and
B56691 XbaI R: 5'-gctctactgctgataaaccgtgag-3' (SEQ ID NO: 54) (corresponding to bases 66799-66776 of SEQ ID NO: 27).
The amplification products can be visualized by electrophoresis on an agarose gel after treatment with XbaI. Thus, the change can be detected as a difference in mobility in the agarose gel due to the difference in the length of DNA after XbaI treatment because the amplification products from Nipponbare DNA having an XbaI recognition sequence (TCTAGA) are cleaved with XbaI while the amplification products from IR24 DNA are not cleaved with XbaI for the lack of the XbaI recognition sequence.

### (9) Development of PCR marker B53627 BstZ17I

Comparison between the genomic base sequence corresponding to the IR24 contig (SEQ ID NO: 27) determined in Example 2 and the base sequence of the BAC clone described above (Accession No. AC068923) revealed that the 69331st base of SEQ ID NO: 27 is T while the base of AC068923 corresponding to said position is C.

For detecting this change, fragments of about 620 bp are first amplified by PCR from a region surrounding said position using the following primer pair:
B53627 BstZ17I F: 5'-tggatggactatgtggggtcagtc-3' (SEQ ID NO: 55) (corresponding to bases 68965-68988 of SEQ ID NO: 27) and
B53627 BstZ17I R: 5'-agtggaagtggagagagtagggag-3' (SEQ ID NO: 56) (corresponding to bases 69582-69559 of SEQ ID NO: 27).
The amplification products can be visualized by electrophoresis on an agarose gel after treatment with BstZ17I. Thus, the change can be detected as a difference in mobility in the agarose gel due to the difference in the length of DNA after BstZ17I treatment because the amplification products from IR24 DNA having a BstZ17I recognition sequence (GTATAC) are cleaved with BstZ17I while the amplification products from Nipponbare DNA are not cleaved with BstZ17I for the lack of the BstZ17I recognition sequence.

### (10) Development of PCR marker B40936 MseI

Development of all the following PCR markers (10)-(12) relates to a study of the base sequences corresponding to further downstream regions (3') of base 76363 at the 3'end of SEQ ID NO: 27.

The following primer pair was designed for the base sequence of the BAC clone described above (Accession No. AC068923): 5'-tacgacgccatttcactccattgc-3' (SEQ ID NO: 57) and 5'-catttctctatgggcgttgctctg-3' (SEQ ID NO: 58). PCR was routinely performed using this primer pair in combination with total DNAs of MS-FR Koshihikari (genotype of the Rf-1 locus: Rf-1 Rf-1) and Koshihikari as templates. The resulting amplification products of about 1300 bp were electrophoresed on an agarose gel and then purified by QIAEXII (QIAGEN). Analysis of the base sequence of the purified DNA by a DNA sequencer 377 (ABI) showed several polymorphisms.

One of them can be detected by PCR amplification of a region surrounding said position using the following primer pair:
B40936 MseI F: 5'-acctgtaggtatggcaccttcaacac-3' (SEQ ID NO: 59) and
B40936 MseI R: 5'-ccaaggaacgaagttcaaatgtatgg-3' (SEQ ID NO: 60). The amplification products can be visualized by electrophoresis on an agarose gel after treatment with MseI. Thus, the change can be detected as a difference in mobility in the agarose gel due to the difference in the length of DNA after MseI treatment because the amplification products from MS-FR Koshihikari (Rf-1 Rf-1) DNA having an MseI recognition sequence (TTAA) are cleaved with MseI while the amplification products from Koshihikari DNA are not cleaved with MseI for the lack of the MseI recognition sequence.

This marker was developed by applying the dCAPS method.

### (11) Development of PCR marker B19839 MwoI

The following primer pair was designed for the base sequence of the BAC clone described above (Accession No. AC068923): 5'-tgatgtgtttgggcatccctttcg-3' (SEQ ID NO: 61) and 5'-gagataggggacgacagacacgac-3' (SEQ ID NO: 62). PCR was routinely performed using this primer pair in combination with total DNAs of MS-FR Koshihikari (genotype of the Rf-1 locus: Rf-1 Rf-1) and Koshihikari as templates. The resulting amplification products of about 1200 bp were electrophoresed on an agarose gel and then purified by QIAEXII (QIAGEN). Analysis of the base sequence of the purified DNA by a DNA sequencer 377 (ABI) showed several polymorphisms.

One of them can be detected by PCR amplification of a region surrounding said position using the following primer pair:
B19839 MwoI F: 5'-tcctatggctgtttagaaactgcaca-3' (SEQ ID NO: 63) and
B19839 MwoI R: 5'-caagttcaaacataactggcgttg-3' (SEQ ID NO: 64). The amplification products can be visualized by electrophoresis on an agarose gel after treatment with MwoI. Thus, the change can be detected as a difference in mobility in the agarose gel due to the difference in the length of DNA after MwoI treatment because the amplification products from Koshihikari DNA having an MwoI recognition sequence (GCNNNNNNNGC) are cleaved with MwoI while the amplification products from MS-FR Koshihikari (Rf-1 Rf-1) DNA are not cleaved with MwoI for the lack of the MwoI recognition sequence.

This marker was developed by applying the dCAPS method.

### (12) Development of PCR marker B2387 BfaI

The following primer pair was designed for the base sequence of the BAC clone described above (Accession No. AC068923): 5'-cactgtcctgtaagtgtgctgtgc-3' (SEQ ID NO: 65) and 5'-caagcgtgtgataaaatgtgacgc-3' (SEQ ID NO: 66). PCR was routinely performed using this primer pair in combination with total DNAs of MS-FR Koshihikari (genotype of the Rf-1 locus: Rf-1 Rf-1) and Koshihikari as templates. The resulting amplification products of about 1300 bp were electrophoresed on an agarose gel and then purified by QIAEXII (QIAGEN). Analysis of the base sequence of the purified DNA by a DNA sequencer 377 (ABI) showed several polymorphisms.

One of them can be detected by PCR amplification of a region surrounding said position using the following primer pair:
B2387 BfaI F: 5'-tgcctactgccattactatgtgac-3' (SEQ ID NO: 67) and
B2387 BfaI R: 5'-acatactaccgtaaatggtctctg-3' (SEQ ID NO: 68). The amplification products can be visualized by electrophoresis on an agarose gel after treatment with BfaI. Thus, the change can be detected as a difference in mobility in the agarose gel due to the difference in the length of DNA after BfaI treatment because the amplification products from Koshihikari DNA having an BfaI recognition sequence (CTAG) are cleaved with BfaI while the amplification products from MS-FR Koshihikari (Rf-1 Rf-1) DNA are not cleaved with BfaI for the lack of the BfaI recognition sequence.

### (13) Segregation analysis

Two recombinants between the Rf-1 and S12564 Tsp509I loci (RS1 and RS2) and 8 recombinants between the Rf-1 and C1361 MwoI loci (RC1 to RC8) obtained in Example 1 were genotyped at the 12 DNA marker loci developed in (1) to (12) above. The results are shown in Table 4 along with the genotypes of each recombinant at the S12564 Tsp509I and C1361 MwoI loci.

**Table 4.**

| Genotypes of recombinants proximal to the Rf-1 locus at various marker loci | | | | | | | | | | |
|---|---|---|---|---|---|---|---|---|---|---|
| Locus | RS1 | RS2 | RC1 | RC2 | RC3 | RC4 | RC5 | RC6 | RC7 | RC8 |
| S12564 Tsp509I | J | J | H | H | H | H | H | H | H | H |
| P4497 MboI | J | J | H | H | H | H | H | H | H | H |
| P9493 BsII | H | H | H | H | H | H | H | H | H | H |
| P23945 MboI | H | H | H | H | H | H | H | H | H | H |
| P41030 TaqI | H | H | H | H | H | H | H | H | H | H |
| P45177 BstUI | H | H | H | H | H | H | H | H | H | H |
| B60304 MspI | H | H | H | H | H | H | H | H | H | H |
| B59066 BsaJI | H | H | H | H | H | H | H | H | H | H |
| B56691 XbaI | H | H | H | H | H | H | H | J | H | H |
| B53627 BstZ17I | H | H | H | H | H | H | H | J | H | H |
| B40936 MseI | H | H | H | H | H | H | H | J | H | H |
| B19839 MwoI | H | H | H | H | H | J | H | J | H | H |
| B2387 BfaI | H | H | H | H | H | J | R | J | H | J |
| C1361 MwoI | H | H | J | J | J | J | J | J | J | J |
| J: Homozygous for Koshihikari | | | | | | | | | | |
| H: Heterozygous for Koshihikari /MS-FR Koshihikari | | | | | | | | | | |

Table 4 shows that all the recombinants have an indica-derived Rf-1 chromosomal region between P9493 BslI and 59066 BsaJI. This result showed that recombinant pollens having the chromosomal organization as shown in Fig. 3 have pollen fertility, i.e. the Rf-1 gene is functional in these pollens. This means that a sequence determining the presence of the function of the Rf-1 gene is included in the indica region common to these recombinant pollens, i.e. in a region from the P4497 MboI to B56691 XbaI loci (about 65 kb) as estimated at maximum.

However, there is a possibility that it is important for the expression of the genetic function of the Rf-1 gene that the Rf-1 gene is partially of the indica genotype, and that the genetic function may not be significantly changed whether the remaining regions are of the japonica or indica genotype. Therefore, it cannot be concluded that the common indica region above (bases 1239-66267 of SEQ ID NO: 27) completely contains the entire Rf-1 gene. However, it is thought that at least SEQ ID NO: 27 completely contains the entire Rf-1 gene for the following reasons:
1) the size of a gene is normally several kilobases, and rarely exceeds 10 kb;
2) the genomic base sequence of IR24 determined by the present invention (SEQ ID NO: 27) completely contains the common indica region above;
3) the 5' end of SEQ ID NO: 27 is located 1238 bp upstream of the 5' end of the common indica region above and forms a part of another gene (S12564); and
4) the 3' end of SEQ ID NO: 27 is located 10096 bp downstream of the 3' end of the common indica region above.

### Example 4: Complementation assay for a 9.7 kb fragment from XSE1

### (Materials and Methods)

The λ phage clone XSE1 (Figs. 1 and 5) was completely digested with NotI and electrophoresed on an agarose gel. The separated 9.7 kb fragment (including bases 1-9657 of SEQ ID NO: 27) was purified by QIAEXII (QIAGEN).

On the other hand, an intermediate vector pSB200 having a hygromycin-resistant gene cassette was prepared on the basis of pSB11 (Komari et al., supra.). Specifically, a nopaline synthase terminator (Tnos) was first fused to a ubiquitin promoter and a ubiquitin intron (Pubi-ubiI). A hygromycin-resistant gene (HYG(R)) was inserted between ubiI and Tnos of the resulting Pubi-ubiI-Tnos complex to give an assembly of Pubi-ubiI-HYG(R)-Tnos. This assembly was fused to a HindIII/EcoRI fragment of pSB11 to give pKY205. Linker sites for adding restriction enzyme sites NotI, NspV, EcoRV, KpnI, SacI, EcoRI were inserted into the Hind III site upstream of Pubi of this pKY205 to give pSB200 having a hygromycin-resistant gene cassette.

After the plasmid vector pSB200 was completely digested with NotI, DNA was recovered by ethanol precipitation. The recovered DNA was dissolved in TE solution and then dephosphorylated by CIAP (TAKARA). The reaction solution was electrophoresed on an agarose gel, and then a vector fragment was purified from the gel using QIAEXII (QIAGEN).

The two fragments prepared above, i.e. a 9.7 kb fragment from XSE1 and a vector fragment were subjected to a ligation reaction using DNA Ligation Kit Ver. 1 (TAKARA). After the reaction, DNA was recovered by ethanol precipitation. The recovered DNA was dissolved in pure water (prepared by a Millipore system) and then mixed with E. coli DH5a cells, and the mixture was electroporated. After electroporation, the solution was cultured with shaking in LB medium (37°C, 1 hr) and then plated on an LB plate containing spectinomycin and warmed (37°C, 16 hr). Plasmids were isolated from 24 of the resulting colonies. Their restriction enzyme fragment length patterns and boundary base sequences were analyzed to select desired E. coli cells transformed with recombinant plasmids.

The E. coli cells selected above were used for triparental mating with the Agrobacterium tumefaciens strain LBA4404/pSB1 (Komari et al., 1996) and the helper E. coli strain HB101/pRK2013 (Ditta et al., 1980) according to the method of Ditta et al. (1980). Plasmids were isolated from 6 of the colonies formed on an AB plate containing spectinomycin and their restriction enzyme fragment length patterns were analyzed to select desired Agrobacterium cells.

The Agrobacterium cells selected above were used to transform MS Koshihikari (having BT cytoplasm and a nucleus gene substantially identical to Koshihikari) according to the method of Hiei et al. (1994). Necessary immature seeds of MS Koshihikari for transformation can be prepared by pollinating MS Koshihikari with Koshihikari.

Transformed plants were transferred to a greenhouse under long-day conditions after acclimation. 48 individuals grown to a stage suitable for transplantation were transplanted into 1/5000a Wagner pots (4 individuals/pot), and transferred into a greenhouse under short-day conditions 3-4 weeks after transplantation. About one month after heading, seed fertility was tested on standing plants.

### (Results and Discussion)

All of the 48 transformed individuals were sterile. This indicates that the 9.7 kb insert fragment does not contain at least the full-length Rf-1 gene.

### Example 5: Complementation assay for a 14.7 kb fragment from XSE7

### (Materials and Methods)

The λ phage clone XSE7 (Figs. 1 and 5) was completely digested with EcoRI and then DNA was recovered by ethanol precipitation. The recovered DNA was dissolved in TE solution and then blunted by DNA Blunting Kit (TAKARA). The reaction solution was electrophoresed on an agarose gel to separate a 14.7 kb fragment (including bases 2618-17261 of SEQ ID NO: 27), which was purified by QIAEXII (QIAGEN).

On the other hand, the plasmid vector pSB200 was completely digested with SacI and then DNA was recovered by ethanol precipitation. The recovered DNA was dissolved in TE solution and then dephosphorylated by CIAP (TAKARA) and DNA was recovered by ethanol precipitation. The recovered DNA was dissolved in TE solution and then blunted by DNA Blunting Kit (TAKARA). The reaction solution was electrophoresed on an agarose gel, and then a vector fragment was purified from the gel using QIAEXII (QIAGEN).

The two fragments prepared above, i.e. the 14.7 kb fragment from XSE7 and the vector fragment were subjected to a ligation reaction using DNA Ligation Kit Ver. 1 (TAKARA). Subsequently, transformed plants were prepared and studied according to the method described in Example 4.

### (Results and Discussion)

All of the 48 transformed individuals were sterile. This indicates that the 14.7 kb insert fragment does not contain at least the full-length Rf-1 gene.

### Example 6: Complementation assay for a 21.3 kb fragment from XSF4

### (Materials and Methods)

The λ phage clone XSF4 (Figs. 1 and 5) was partially digested with NotI and electrophoresed on an agarose gel. The separated 21.3 kb fragment (including bases 12478-33750 of SEQ ID NO: 27) was purified by QIAEXII (QIAGEN).

On the other hand, the plasmid vector pSB200 was completely digested with NotI and then DNA was recovered by ethanol precipitation. The recovered DNA was dissolved in TE solution and then dephosphorylated by CIAP (TAKARA). The reaction solution was electrophoresed on an agarose gel, and then a vector fragment was purified from the gel using QIAEXII (QIAGEN).

The two fragments prepared above, i.e. the 21.3 kb fragment from XSF4 and the vector fragment were subjected to a ligation reaction using DNA Ligation Kit Ver. 1 (TAKARA). Subsequently, transformed plants were prepared and studied according to the method described in Example 4.

### (Results and Discussion)

All of the 48 transformed individuals were sterile. This indicates that the 21.3 kb insert fragment does not contain at least the full-length Rf-1 gene.

### Example 7: Complementation assay for a 13.2 kb fragment from XSF20

### (Materials and Methods)

The λ phage clone XSF20 (Figs. 1 and 5) was completely digested with SalI and then DNA was recovered by ethanol precipitation. The recovered DNA was dissolved in TE solution and then blunted by DNA Blunting Kit (TAKARA). The reaction solution was electrophoresed on an agarose gel to separate a 13.2 kb fragment (including bases 26809-40055 of SEQ ID NO: 27), which was purified by QIAEXII (QIAGEN).

On the other hand, the plasmid vector pSB200 was completely digested with EcoRV and then DNA was recovered by ethanol precipitation. The recovered DNA was dissolved in TE solution and then dephosphorylated by CIAP (TAKARA). The reaction solution was electrophoresed on an agarose gel, and then a vector fragment was purified from the gel using QIAEXII (QIAGEN).

The two fragments prepared above, i.e. the 13.2 kb fragment from XSF20 and the vector fragment were subjected to a ligation reaction using DNA Ligation Kit Ver. 1 (TAKARA). Subsequently, transformed plants were prepared and studied according to the method described in Example 4.

### (Results and Discussion)

All of the 44 transformed individuals were sterile. This indicates that the 13.2 kb insert fragment does not contain at least the full-length Rf-1 gene.

### Example 8: Complementation assay for a 16.2 kb fragment from XSF18

### (Materials and Methods)

The λ phage clone XSF18 is identical to XSF20 at the 5' and 3' ends (bases 20328 and 41921 of SEQ ID NO: 27, respectively), but lacks internal bases 33947-38591. Thus, it comprises bases 20328-33946 and 38592-41921 of SEQ ID NO: 27. This is because clone XSF18 was initially isolated but found to contain the above deletion during amplification after isolation, and therefore, the amplification step was freshly taken to isolate a complete clone designated XSF20.

The λ phage clone XSF18 (Fig. 5) was completely digested with NotI and electrophoresed on an agarose gel. The separated 16.2 kb fragment (including bases 21065-33946 and 38592-41921 of SEQ ID NO: 27) was purified by QIAEXII (QIAGEN).

On the other hand, the plasmid vector pSB200 was completely digested with NotI and then DNA was recovered by ethanol precipitation. The recovered DNA was dissolved in TE solution and then dephosphorylated by CIAP (TAKARA). The reaction solution was electrophoresed on an agarose gel, and then a vector fragment was purified from the gel using QIAEXII (QIAGEN).

The two fragments prepared above, i.e. the 16.2 kb fragment from XSF18 and the vector fragment were subjected to a ligation reaction using DNA Ligation Kit Ver. 1 (TAKARA). Subsequently, transformed plants were prepared and studied according to the method described in Example 4.

### (Results and Discussion)

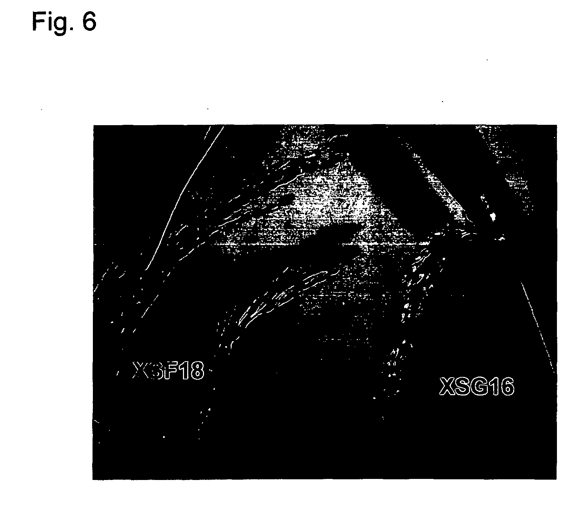

All of the 48 transformed individuals were sterile (Fig. 6). This indicates that the 16.2 kb insert fragment does not contain at least the full-length Rf-1 gene.

### Example 9: Complementation assay for a 12.6 kb fragment from XSG22

### (Materials and Methods)

The λ phage clone XSG22 (Figs. 1 and 5) was partially digested with NotI and electrophoresed on an agarose gel. The separated 12.6 kb fragment (including bases 31684-44109 of SEQ ID NO: 27) was purified by QIAEXII (QIAGEN).

On the other hand, the plasmid vector pSB200 was completely digested with NotI and then DNA was recovered by ethanol precipitation. The recovered DNA was dissolved in TE solution and then dephosphorylated by CIAP (TAKARA). The reaction solution was electrophoresed on an agarose gel, and then a vector fragment was purified from the gel using QIAEXII (QIAGEN).

The two fragments prepared above, i.e. the 12.6 kb fragment from XSG22 and the vector fragment were subjected to a ligation reaction using DNA Ligation Kit Ver. 1 (TAKARA). Subsequently, transformed plants were prepared and studied according to the method described in Example 4.

### (Results and Discussion)

All of the 48 transformed individuals were sterile. This indicates that the 12.6 kb insert fragment does not contain at least the full-length Rf-1 gene.

### Example 10: (1) Complementation assay for a 15.7 kb fragment from XSG16

### (Materials and Methods)

The λ phage clone XSG16 (Figs. 1 and 5) was partially digested with NotI and electrophoresed on an agarose gel. The separated 15.7 kb fragment (including bases 38538-54123 of SEQ ID NO: 27) was purified by QIAEXII (QIAGEN).

On the other hand, the plasmid vector pSB200 was completely digested with NotI and then DNA was recovered by ethanol precipitation. The recovered DNA was dissolved in TE solution and then dephosphorylated by CIAP (TAKARA). The reaction solution was electrophoresed on an agarose gel, and then a vector fragment was purified from the gel using QIAEXII (QIAGEN).

The two fragments prepared above, i.e. the 15.7 kb fragment from XSG16 and the vector fragment were subjected to a ligation reaction using DNA Ligation Kit Ver. 1 (TAKARA). Subsequently, transformed plants were prepared and studied according to the method described in Example 4.

### (Results and Discussion)

Of the 47 transformed individuals, at least 37 individuals clearly restored fertility (Fig. 6). This indicates that 15586 bases (bases 38538-54123 of SEQ ID NO: 27) derived from rice (IR24) in the 15.7 kb insert fragment include the full-length Rf-1 gene.

### (2) Complementation assay for an internal 11.4 kb fragment in XSG16

### (Materials and Methods)

The λ phage clone XSG16 was completely digested with AlwNI and BsiWI and then DNA was recovered by ethanol precipitation. The recovered DNA was dissolved in TE solution and then blunted by DNA Blunting Kit (TAKARA). The reaction solution was electrophoresed on an agarose gel to separate a 11.4 kb fragment, which was purified by QIAEXII (QIAGEN).

The plasmid vector pSB11 (Komari et al. Plant Journal, 1996) was completely digested with SmaI and then DNA was recovered by ethanol precipitation. The recovered DNA was dissolved in TE solution and then dephosphorylated by CIAP (TAKARA). The reaction solution was electrophoresed on an agarose gel, and then a vector fragment was purified from the gel using QIAEXII (QIAGEN).

The two fragments prepared above were subjected to a ligation reaction using DNA Ligation Kit Ver. 1 (TAKARA). After the reaction, DNA was recovered by ethanol precipitation. The recovered DNA was dissolved in pure water (prepared by a Millipore system) and then mixed with E. coli DH5a cells, and the mixture was electroporated. After electroporation, the solution was cultured with shaking in LB medium (37°C, 1 hr) and then plated on an LB plate containing spectinomycin and warmed (37°C, 16 hr). Plasmids were isolated from 14 of the resulting colonies, and their restriction enzyme fragment length patterns and boundary base sequences were analyzed to select desired E. coli cells.

The E. coli cells selected above were used for triparental mating with the Agrobacterium tumefaciens strain LBA4404/pSB4U (Takakura et al., Japanese Patent Application No. 2001-269982 (WO02/019803 A1)) and the helper E. coli strain HB101/pRK2013 (Ditta et al., 1980) according to the method of Ditta et al. (1980). Plasmids were isolated from 12 of the colonies formed on an AB plate containing spectinomycin and their restriction enzyme fragment length patterns were analyzed to select desired Agrobacterium cells.

The Agrobacterium cells selected above were used to transform MS Koshihikari (having BT cytoplasm and a nucleus gene substantially identical to Koshihikari) according to the method of Hiei et al. (1994). Necessary immature seeds of MS Koshihikari for transformation can be prepared by pollinating MS Koshihikari with Koshihikari.

Transformed plants were transferred to a greenhouse under long-day conditions after acclimation. 120 individuals grown to a stage suitable for transplantation were transplanted into 1/5000a Wagner pots (4 individuals/pot), and transferred into a greenhouse under short-day conditions about one month after transplantation. About one month after heading, one typical ear was sampled from each plant to evaluate seed fertility (the percentage of fertile paddies to total paddies).

### (Results and Discussion)

Of the 120 transformed individuals, 59 individuals showed seed fertility of 10% or more, among which 19 individuals showed seed fertility of 70% or more. This indicates that the 11.4 kb insert fragment (bases 42357-53743 of SEQ ID NO: 27) contains an essential Rf-1 gene region for expressing a fertility restoring function.

### (3) Complementation assay for an internal 6.8 kb fragment in XSG16

### (Materials and Methods)

The λ phage clone XSG16 was completely digested with HpaI and AlwNI and electrophoresed on an agarose gel. The separated 6.8 kb fragment was purified by QIAEXII (QIAGEN).

The subsequent procedures including the preparation of the plasmid vector pSB11 were performed according to the method in (2) above.

### (Results and Discussion)

Of the 120 transformed individuals, 67 individuals showed seed fertility of 10% or more, among which 26 individuals showed seed fertility of 70% or more. This indicates that the 6.8 kb insert fragment (bases 42132-48883 of SEQ ID NO: 27) contains an essential Rf-1 gene region for expressing a fertility restoring function.

### Example 11: Complementation assay for a 16.9 kb fragment from XSG8

### (Materials and Methods)

The λ phage clone XSG8 (Figs. 1 and 5) was completely digested with NotI and electrophoresed on an agarose gel. The separated 16.9 kb fragment (including bases 46558-63364 of SEQ ID NO: 27) was purified by QIAEXII (QIAGEN).

On the other hand, the plasmid vector pSB200 was completely digested with NotI and then DNA was recovered by ethanol precipitation. The recovered DNA was dissolved in TE solution and then dephosphorylated by CIAP (TAKARA). The reaction solution was electrophoresed on an agarose gel, and then a vector fragment was purified from the gel using QIAEXII (QIAGEN).

The two fragments prepared above, i.e. the 16.9 kb fragment from XSG8 and the vector fragment were subjected to a ligation reaction using DNA Ligation Kit Ver. 1 (TAKARA). Subsequently, transformed individuals were prepared and studied according to the method described in Example 4.

### (Results and Discussion)

All of the 48 transformed individuals were sterile. This indicates that the 16.9 kb insert fragment does not contain at least the full-length Rf-1 gene.

### Example 12: Complementation assay for a 20.0 kb fragment from XSH18

### (Materials and Methods)

The λ phage clone XSH18 (Figs. 1 and 5) was completely digested with NotI and electrophoresed on an agarose gel. The separated 20.0 kb fragment (including bases 56409-76363 of SEQ ID NO: 27) was purified by QIAEXII (QIAGEN).

On the other hand, the plasmid vector pSB200 was completely digested with NotI and then DNA was recovered by ethanol precipitation. The recovered DNA was dissolved in TE solution and then dephosphorylated by CIAP (TAKARA). The reaction solution was electrophoresed on an agarose gel, and then a vector fragment was purified from the gel using QIAEXII (QIAGEN).

The two fragments prepared above, i.e. the 20.0 kb fragment from XSH18 and the vector fragment were subjected to a ligation reaction using DNA Ligation Kit Ver. 1 (TAKARA). Subsequently, transformed individuals were prepared and studied according to the method described in Example 4.

### (Results and Discussion)

All of the 44 transformed individuals were sterile. This indicates that the 20.0 kb insert fragment does not contain at least the full-length Rf-1 gene.

### Example 13: Complementation assay for a 19.7 kb fragment from an overlapping region of XSG8 and XSH18

### (Materials and Methods)

A plasmid (XSG8SB200F) isolated from desired E. coli cells obtained by ligation in Example 11 was completely digested with SalI and StuI and electrophoresed on an agarose gel. The separated 12.8 kb fragment (including bases 50430-63197 of SEQ ID NO: 27) was purified by QIAEXII (QIAGEN).

On the other hand, a plasmid (XSH18SB200R) isolated from desired E. coli cells obtained by ligation in Example 12 was completely digested with SalI, StuI and XhoI and electrophoresed on an agarose gel to separate a 6.9 kb fragment (including bases 63194-70116 of SEQ ID NO: 27), which was purified by QIAEXII (QIAGEN).

Further, the plasmid vector pSB200 was completely digested with EcoRV and then DNA was recovered by ethanol precipitation. The recovered DNA was dissolved in TE solution and then dephosphorylated by CIAP (TAKARA). The reaction solution was electrophoresed on an agarose gel, and then a vector fragment was purified from the gel using QIAEXII (QIAGEN).

The three fragments prepared above, i.e. the 12.8 kb fragment from XSG8, the 6.9 kb fragment from XSH18 and the vector fragment were subjected to a ligation reaction using DNA Ligation Kit Ver. 1 (TAKARA). The ligation product contains a 19.7 kb fragment from an overlapping region of XSG8 and XSH18 (including 50430-70116 of SEQ ID NO: 27) (XSX1 in Fig. 5). Subsequently, transformed individuals were prepared and studied according to the method described in Example 4.

### (Results and Discussion)

All of the 40 transformed individuals were sterile. This indicates that the 19.7 kb insert fragment does not contain at least the full-length Rf-1 gene.

## Claims

1. A method for restoring rice fertility comprising introducing a nucleic acid into rice, wherein the nucleic acid has the base sequence of SEQ ID NO.27, or has a base sequence which is identical to at least 70% of the base sequence of SEQ ID NO.27, and which functions to restore fertility.

2. A method for restoring rice fertility comprising introducing a nucleic acid into rice, wherein the nucleic acid has the base sequence of bases 38538-54123 of SEQ ID NO.27, or has a base sequence which is identical to at least 70% of the base sequence of bases 38538-54123 of SEQ ID NO.27, and which functions to restore fertility.

3. A method for restoring rice fertility comprising introducing a nucleic acid into rice, wherein the nucleic acid has the base sequence of bases 42132-48883 of SEQ ID NO.27, or has a base sequence which is identical to at least 70% of the base sequence of bases 42132-48883 of SEQ ID NO.27, and which functions to restore fertility.

4. The method of any one of Claims 1-3, wherein the nucleic acid having a base sequence which is identical to at least 70% of the base sequence of SEQ ID NO.27 or of the base sequence of bases 38538-54123 of SEQ ID NO.27, and which meets at least one of the following requirements 1) and 2):
1) a base corresponding to the base 45461 of SEQ ID NO.27 is A; and
2) a base corresponding to the base 49609 of SEQ ID NO.27 is A.

5. The method of any one of Claims 1-4, wherein the nucleic acid comprising the rice restorer gene locus introduced into rice, does not comprise any constitutional gene other than the rice restorer gene.

6. A method for discerning whether or not a subject rice individual or a seed thereof has the rice restorer gene (the Rf-1 gene) or not, wherein the method utilizing a fact that a sequence determining the presence of the function of the Rf-1 gene positions between the polymorphism detection marker loci P4497 MboI and B56691 Xba I on rice chromosome 10.

7. The method of Claim 6 wherein the Rf-1 gene exists in a nucleic acid having the base sequence of SEQ ID NO.27, or a nucleic acid having a base sequence which is identical to at least 70% of the base sequence of SEQ ID NO.27.

8. The method of Claim 6 wherein the Rf-1 gene exists in a nucleic acid having the base sequence of bases 38538-54123 of SEQ ID NO.27, or a nucleic acid having a base sequence which is identical to at least 70% of the base sequence of bases 38538-54123 of SEQ ID NO.27.

9. The method of claim 6 wherein the Rf-1 gene exists in a nucleic acid having the base sequence of bases 42132-48883 of SEQ ID NO.27, or a nucleic acid having a base sequence which is identical to at least 70% of the base sequence of bases 42132-48883 of SEQ ID NO.27.

10. The method of any one of claims 7-9 wherein the subject rice individual or the seed thereof is determined to have the Rf-1 gene, in the case that the nucleic acid having a base sequence which is identical to at least 70% of the base sequence of SEQ ID NO.27 or of the base sequence of bases 38538-54123 of SEQ ID NO.27, meets at least one of the following requirements 1) and 2):
1) a base corresponding to the base 45461 of SEQ ID NO.27 is A; and
2) a base corresponding to the base 49609 of SEQ ID NO.27 is A.

11. The method of any one of Claims 7-10, wherein the method comprises:
i) preparing a pair of primers based on a base sequence of adjacent regions including any one of the following base;
1) a base corresponding to the base 45461; or
2) a base corresponding to the base 49609; to amplify both the base of the above and adjacent regions thereto;
ii) performing nucleic acid amplification reaction(s) using the genome DNA of the subject rice individual or the seed thereof as a template; and
iii) discerning the presence of the Rf-1 in the subject rice individual or the seed thereof based on polymorphism found in said nucleic acid amplification product.

12. The method of Claim 11 wherein the subject rice individual or the seed thereof is determined to have the Rf-1 gene, in the case that the step iii) meets least one of the following requirements 1) and 2):
1) a region including a base corresponding to the base 45461 of SEQ ID NO.27 does not have any TaqI recognition sequence; and
2) a region including a base corresponding to the base 49609 of SEQ ID NO.27 does not have any BstU recognition sequence.

13. The method of Claim 11 or 12 using a pair of primers having base sequences selected from the group consisting of SEQ ID NO:45 and 46, and SEQ ID NO:47 and 48.

14. A method for inhibiting the function of the Rf-1 gene to restore fertility by introducing an antisense having at least 100 continuous bases in length, and having a base sequence complementary to a nucleic acid having the base sequence of SEQ ID NO.27, or to a nucleic acid having a base sequence which is identical to at least 70% of the base sequence of SEQ ID NO.27, and which functions to restore fertility.

15. A method for inhibiting the function of the Rf-1 gene to restore fertility by introducing an antisense having at least 100 continuous bases in length, and being selected from base sequences complementary to a nucleic acid having the base sequence of bases 38538-54123 of SEQ ID NO.27, or to a nucleic acid having a base sequence which is identical to at least 70% of the base sequence of bases 38538-54123 of SEQ ID NO.27, and which functions to restore fertility.

16. A nucleic acid having the base sequence of SEQ ID NO.27, or a nucleic acid having a base sequence which is identical to at least 70% of the base sequence of SEQ ID NO.27, and which functions to restore fertility.

17. A nucleic acid having the base sequence of bases 38538-54123 of SEQ ID NO.27, or a nucleic acid having a base sequence which is identical to at least 70% of the base sequence of bases 38538-54123 of SEQ ID NO.27, and which functions to restore fertility.

18. A nucleic acid having the base sequence of bases 42132-48883 of SEQ ID NO.27, or a nucleic acid having a base sequence which is identical to at least 70% of the base sequence of bases 42132-48883 of SEQ ID NO.27, and which functions to restore fertility.
